# EUROPEAN PATENT APPLICATION

(11) **EP 1 437 344 A1**
(43) Date of publication of application: **14.07.2004**
(21) Application number: 02768103.0
(22) Date of filing: 27.09.2002
(51) Int. Cl.: C07C 275/38

(54) **BENZENE DERIVATIVES,PROCESS FOR PREPARING THE SAME AND USE THEREOF**

(30) Priority: 28.09.2001 JP 2001300564
(71) Applicant: Takeda Chemical Industries, Ltd., Osaka-shi, Osaka 541-0045 (JP)
(72) Inventor: MATSUMOTO, Takahiro, Kawabe-gun, Hyogo 666-0245 (JP); YAMAMOTO, Masataka, Osaka-shi, Osaka 546-0033 (JP); NAGABUKURO, Hiroshi, Osaka-shi, Osaka 533-0003 (JP); MOCHIZUKI, Manabu, Takedayakuhin-suitaryo, Suita-shi, Osaka 565-0823 (JP)
(74) Representative: Rickard, Timothy Mark Adrian
(86) International application number: PCT/JP2002/009995
(87) International publication number: WO 2003/029199

(57) **Abstract**

Novel benzene derivatives represented by the formula (I) : 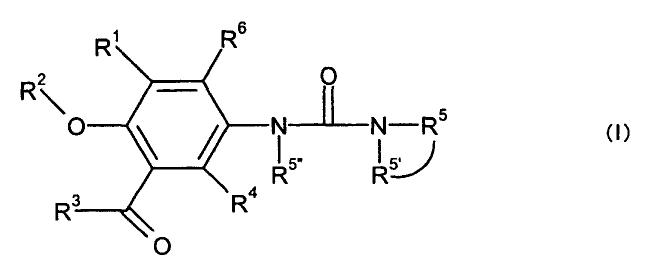 wherein R¹, R⁴ and R⁶ each independently represents a hydrogen atom, a halogen atom or a hydrocarbon group, R² represents a hydrocarbon group or a heterocyclic group, R³ represents a hydrocarbon group, NR^{7'}R⁷ or OR⁸ (wherein R^{7'} represents a hydrogen atom or a hydrocarbon group, R⁷ represents a non-aromatic group, or R^{7'} and R⁷ may form a ring with the adjacent nitrogen atom, and R⁸ represents a hydrocarbon group or a heterocyclic group), R⁵ represents a hydrocarbon group or a heterocyclic group (except for a quinolyl group), R^{5'} represents a hydrogen atom, or a hydrocarbon group, or R⁵ and R^{5'} may form a ring with the adjacent nitrogen atom, and R^{5"} represents a hydrogen atom or a hydrocarbon group, which have vanilloid receptor agonist activity and are useful as a drug such as an analgesic and an agent for preventing and/or treating urinary frequency and/or urinary incontinence.

## Description

### Technical Field

The present invention relates to a benzene derivative which is useful as medicines, and a preparation process and a use thereof.

### Background Art

There have so far been reported on various benzene derivatives which are useful as medicines. The benzene derivatives, which contain three substituents of a urea group, a carbonyl group and an ether group on benzene ring and are useful as medicines (anticancer agents, antiobestic agents), are disclosed in e.g. WO 01/25190, WO 00/47577, and JP 63-99056 A.

That is, WO 01/25190 discloses that a compound of the formula: 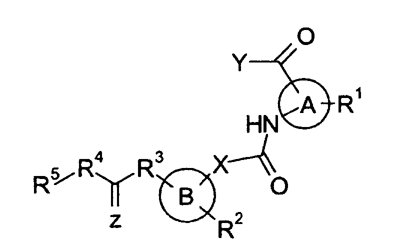 wherein A and B represent an aromatic ring such as a benzene ring; COX and NHCOX are present adjacent to each other and these substituents are bonded to a carbon atom in the aromatic ring A; X represents an alkylene group, an alkyleneoxy group or a single bond; Y represents an alkyl group, an alkoxy group, a hydroxy group or a substituted or unsubstituted amino group; R¹ represents a hydrogen atom, a halogen atom, a hydroxy group or an alkyl group, etc., provided that R¹ is not hydrogen atom when A is a benzene ring; R² represents a hydrogen atom, a halogen atom, a hydroxy group or an alkyl group, etc.; R³ and R⁴ represent a substituted or unsubstituted imino group, an oxygen atom or a single bond; R⁵ represents an alkyl group, a substituted or unsubstituted phenyl group, etc.; Z represents an oxygen atom or a sulfur atom, inhibits hyperplasia of cells.

WO 00/47577 discloses that a compound of the formula: 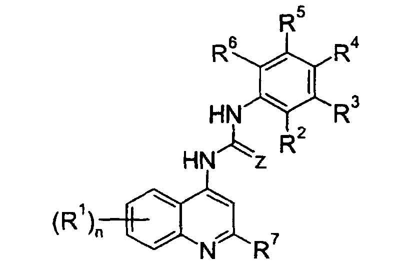 wherein Z represents an oxygen or sulfur atom; R₁ represents a (C₁₋₆)alkyl group, a (C₂₋₆)alkenyl group, a (C₁₋₆)alkoxy group, a halogen atom, a R⁸CO group or a NR⁹R¹⁰CO group; R², R³, R⁴, R⁵ and R⁶ each independently represents a (C₁₋₆)alkyl group, a (C₂₋₆)alkenyl group, a (C₁₋₆)alkoxy group, a (C₁₋₆)alkylthio group, a halogen atom, a hydrogen atom, a nitro group, a cyano group, an aryloxy group, an allyl(C₁₋₆)alkyloxy group, an aryl(C₁₋₆)alkyl group, a R⁸CO group, a R⁸SO₂NH group, a R⁸CON(R¹¹) group, a NR⁹R¹⁰ group, a NR⁹R¹⁰CO group, a COOR⁹ group, a heterocyclic ring or an alkyl group containing a heterocyclic ring; R⁷ represents a (C₁₋₆)alkyl group, a (C₂₋₆)alkenyl group, a (C₁₋₆)alkoxy group, a (C₁₋₆)alkythio group, a halogen atom, a hydroxy group, a nitro group, a cyano group, a NR⁹R¹⁰ group, a NR⁹R¹⁰CO group, a OCOR⁹ group, a N₃ group or a R⁸CON(R¹¹) group; R⁸ represents a (C₁₋₆)alkyl group or an aryl group; R⁹ and R¹⁰ each independently represents a hydrogen atom, a (C₁₋₆)alkyl group, an aryl group or an aryl(C₁₋₆)alkyl group; R¹¹ represents a hydrogen atom or a (C₁₋₆)alkyl group; n represents 0, 1, 2 or 3, is useful as an orexin receptor antagonist.

JP 63-99056 A discloses that a compound of the formula: 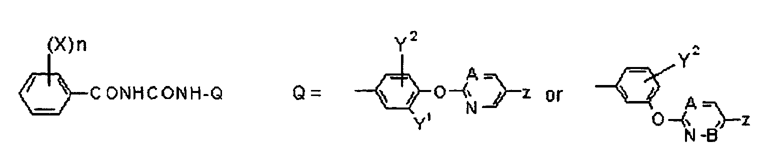 wherein, X represents a hydrogen atom, a halogen atom or a nitro group; n represents 1, 2 or 3; Y¹ represents an alkyl group, an alkoxy group or an alkoxycarbonyl group; Y² represents a hydrogen atom, a halogen atom, a nitro group, an alkyl group, an alkoxy group or an alkoxycarbonyl group; Z represents a hydrogen atom, a halogen atom, a trifluoromethyl group or a nitro group; either of A or B represents =CH- or N and the other represents =CH-, is useful as an anticancer agent.

In addition, capsaicin derivatives disclosed in U.S. Patent Nos. 5,099,030, 5,045,565, 5,403,868, 4,564,633, 4,544,669, 4,532,139, 4,544,668, 4,493,848, 4,460,602, 4,424,205, 4,443,473 and 4,401,663 are known as compounds having vanilloid receptor agonist activity.

U.S. Patent No. 4,313,958 discloses that capsaicin may be utilized as an analgesic agent. Further, JP 2001-513551 A discloses that resiniferatoxin may be utilized as a therapeutic agent for urinary incontinence. Still further, JP 2001-158738 A discloses capsaicinoid-like substances as an analgesic agent. WO 00/50387 also discloses benzene derivatives which are useful as medicines.

### Objects of the Invention

It has been desired earnestly to develop a compound that has vanilloid receptor agonist activity, and is useful as a medicine for treating acute/chronic, systemic and topical pain or inflammation, and for preventing or treating urinary frequency or urinary incontinence, etc. caused by overactivity of the bladder and cystitis. Therefore, an object of the present invention is to develop a compound which is useful as such medicine.

### Summary of the Invention

The present inventors have found that a novel compound represented by the following formula (I), and characterized by containing three substituents of an urea group, a carbonyl group and an ether group on a benzene ring in chemical structure, possesses an excellent analgesic activity, a preventing and/or therapeutic activity of urinary frequency and/or urinary incontinence, and also vanilloid receptor agonist activity, and have studied extensively thereon to have completed the present invention.

That is, the present invention provides:
(1) A compound represented by the formula (I): 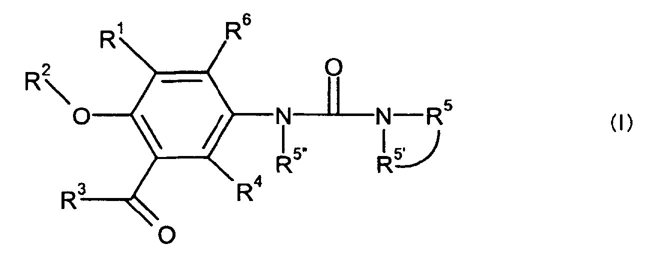 wherein R¹, R⁴ and R⁶ each independently represents a hydrogen atom, a halogen atom or an optionally substituted hydrocarbon group; R² represents an optionally substituted hydrocarbon group or an optionally substituted heterocyclic group; R³ represents an optionally substituted hydrocarbon group, NR^{7'}R⁷ or OR⁸ (wherein R^{7'} represents a hydrogen atom or an optionally substituted hydrocarbon group, and R⁷ represents an optionally substituted non-aromatic group, or R^{7'} and R⁷ may form an optionally substituted ring together with the adjacent nitrogen atom, and R⁸ represents an optionally substituted hydrocarbon group or an optionally substituted heterocyclic group); R⁵ represents an optionally substituted hydrocarbon group (except for an optionally substituted benzoyl group) or an optionally substituted heterocyclic group (except for a quinolyl group); R^{5'} represents a hydrogen atom, or an optionally substituted hydrocarbon group, or R⁵ and R^{5'} may form an optionally substituted ring together with the adjacent nitrogen atom; and R^{5"} represents a hydrogen atom or an optionally substituted hydrocarbon group; or a salt thereof,
(2) The compound as described in the above (1), wherein the optionally substituted hydrocarbon group represented by R¹, R², R³, R⁴, R⁵, R^{5'}, R^{5"}, R⁶, R^{7'} and R ⁸ each independently represents a C₁₋₆alkyl group, a C₂₋₆alkenyl group, a C₂₋₆alkynyl group, a C₃₋₁₀cycloalkyl group, a C₃₋₁₀cycloalkenyl group, a C₄₋₁₂cycloalkylalkyl group, a C₄₋₁₂cycloalkenylalkyl group, a C₆₋₁₄aryl group or a C₇₋₁₉aralkyl group, which may be independently substituted; the optionally substituted non-aromatic group represented by R⁷ represents a C₁₋₆alkyl group, a C₂₋₆alkenyl group, a C₂₋₆alkynyl group, a C₃₋₁₀ cycloalkyl group, a C₃₋₁₀cycloalkenyl group, a C₄₋₁₂cycloalkylalkyl group, a C₄₋₁₂cycloalkenylalkyl group, a C₇₋₁₉aralkyl group or a 5- to 12-membered non-aromatic heterocyclic group containing 1 to 4 hetero atoms consisting of 1 to 3 kinds of hetero atoms selected from an oxygen atom, a sulfur atom and a nitrogen atom as ring-constituting atoms, which may be independently substituted; the optionally substituted heterocyclic group represented by R², R⁵ and R⁸ each independently represents a 5- to 12-membered aromatic heterocyclic group, or a saturated or unsaturated non-aromatic heterocyclic group containing 1 to 4 hetero atoms consisting of 1 to 3 kinds of hetero atoms selected from an oxygen atom, a sulfur atom and a nitrogen atom as ring-constituting atoms, which may be substituted; and the ring formed by R⁷' and R⁷, and R⁵ and R⁵' together with the adjacent nitrogen atom, represents an optionally substituted 3- to 12-membered nitrogen-containing heterocyclic ring which may contain 1 to 3 hetero atoms selected from an oxygen atom, a sulfur atom and a nitrogen atom in addition to carbon atoms and one nitrogen atom,
(3) The compound as described in the above (1) or (2), wherein the substituents are 1 to 4 groups selected from a halogen atom; a nitro group; a cyano group; a hydroxy group; a mercapto group; a sulfo group; a sulfino group; a phosphono group; an optionally halogenated C₁₋₆alkyl group; an oxo group; an amidino group; an imino group; a C₁₋₄alkylenedioxy group; an optionally halogenated C₁₋₆alkoxy group; an optionally halogenated C₁₋₆alkylthio group; a carboxyl group; a formyl group; an optionally halogenated C₁₋₆alkyl-carbonyl group; a formyloxy group; an optionally halogenated C₁₋₆alkyl-carbonyloxy group; an optionally halogenated C₁₋₆alkoxy-carbonyl group; a C₇₋₁₁aralkyl group; a C₇₋₁₁aralkyloxy group; a C₇₋₁₁aralkyloxy-carbonyl group; a thiocarbamoyl group; an optionally halogenated C₁₋₆alkylsulfinyl group; an optionally halogenated C₁₋₆ₐₗₖylsulfonyl group; a sulfamoyl group; an optionally halogenated mono-C₁₋₆alkylsulfamoyl group; an optionally halogenated di-C₁₋₆alkylsulfamoyl group; a C₆₋₁₀arylsulfamoyl group; a C₆₋₁₀aryl group; a C₆₋₁₀aryloxy group; a C₆₋₁₀arylthio group; a C₆₋₁₀arylsulfinyl group; a C₆₋₁₀arylsulfonyl group; a C₆₋₁₀aryl-carbonyl group; a C₆₋₁₀aryl-carbonyloxy group; a group represented by the formula -CONR⁹R¹⁰ (wherein R⁹ and R¹⁰ represents independently (1) a hydrogen atom, (2) a C₁₋₆alkyl group which may have 1 to 4 substituents selected from a halogen atom and a hydroxy group, (3) a C₆₋₁₀aryl group which may have 1 to 4 substituents selected from a halogen atom, a hydroxy group, and an optionally halogenated C₁₋₆alkyl group, or (4) a 5- to 12-membered heterocyclic group containing 1 to 4 hetero atoms consisting of 1 to 3 kinds of hetero atoms selected from an oxygen atom, a sulfur atom and a nitrogen atom as ring-constituting atoms, which may have 1 to 4 substituents selected from a halogen atom and an optionally halogenated C₁₋₆alkyl group, or R⁹ and R¹⁰ may form a 3- to 8-membered nitrogen-containing heterocyclic ring together with the adjacent nitrogen atom, which may contain 1 to 3 hetero atoms selected from an oxygen atom, a sulfur atom and a nitrogen atom in addition to carbon atoms and one nitrogen atom); a group represented by the formula -NR⁹R¹⁰ (wherein R⁹ and R¹⁰ are as defined above); a group represented by the formula -NHCONR⁹R¹⁰ (wherein R⁹ and R¹⁰ are as defined above); a group represented by the formula -NR⁹COR¹⁰ (wherein R⁹ and R¹⁰ are as defined above); a group represented by the formula -NR⁹SO₂R¹⁰ (wherein R⁹ and R¹⁰ are as defined above); and a 5- to 12-membered heterocyclic group containing 1 to 4 hetero atoms consisting of 1 to 3 kinds of hetero atoms selected from an oxygen atom, a sulfur atom and a nitrogen atom as ring-constituting atoms,
(4) The compound as described in the above (1), wherein R¹ represents a hydrogen atom; R⁴ represents a hydrogen atom or an optionally halogenated C₁₋₄alkyl group; and R⁶ represents a hydrogen atom or an optionally halogenated C₁₋₄alkyl group,
(5) The compound as described in the above (1), wherein R² represents a C₇₋₁₉ aralkyl group which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxy-carbonyl group, an optionally halogenated C₁₋₆alkylthio-carbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C₆₋₁₀arylsulfamoyl group, and a di-C₆₋₁₀arylsulfamoyl group,
(6) The compound as described in the above (1), wherein R² represents a C₇₋₁₉aralkyl group which may have 1 to 4 substituents selected from a halogen atom, an optionally halogenated C₁₋₄alkyl group, a nitro group, a cyano group, and a C₁₋₄alkoxy-carbonyl group,
(7) The compound as described in the above (6), wherein the C₇₋₁₉aralkyl group represents a benzhydryl group,
(8) The compound as described in the above (1), wherein R³ represents a C₁₋₄alkyl group, a C₁₋₄alkylamino group or C₁₋₄alkoxy group,
(9) The compound as described in the above (1), wherein R⁵ represents a C₆₋₁₀aryl group, a pyridyl group or a C₇₋₁₁aralkyl group, each of which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxy-carbonyl group, an optionally halogenated C₁₋₆alkylthio-carbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C_{6-10ary}lsulfamoyl group, and a di-C₆₋₁₀arylsulfamoyl group,
(10) The compound as described in the above (1), wherein R⁵ represents a phenyl group which may have 1 or 2 C₁₋₄alkoxy groups,
(11) The compound as described in the above (1), wherein R¹ represents a hydrogen atom; R² represents a C₇₋₁₉ aralkyl group which may have 1 to 4 substituents selected from a halogen atom, an optionally halogenated C₁₋₄alkyl group, a nitro group, a cyano group, and a C₁₋₄alkoxycarbonyl group; R³ represents a C₁₋₄alkyl group, a C₁₋₄alkylamino group or C₁₋₄alkoxy group; R⁴ represents a hydrogen atom or an optionally halogenated C₁₋₄alkyl group; R⁵ represents a C₆₋₁₀aryl group, a pyridyl group or a C₇₋₁₁ aralkyl group, each of which may have 1 to 4 substituents selected from an optionally halogenated C₁₋₆alkyl group, a halogen atom, a nitro group, a cyano group, a carboxyl group, an amino group, an optionally halogenated C₁₋₆alkylcarbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxycarbonyl group, an optionally halogenated C₁₋₆alkylthiocarbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C₆₋₁₀arylsulfamoyl group, and a di C₆₋₁₀arylsulfamoyl group; and R⁶ represents a hydrogen atom or an optionally halogenated C₁₋₄alkyl group,
(12) The compound as described in the above (1), wherein R^{5'}, R^{5"}, and R^{7'} represent independently a hydrogen atom,
(13) The compound as described in the above (1), which is N-(4-benzhydryloxy-3-isobutyrylphenyl)-N'-(3,4-dimethoxyphenyl)urea,
   methyl 5-({[(3,4-dimethoxyphenyl)amino]carbonyl}amino)-2-[(4-fluorophenyl)(phenyl)methoxy]benzoate,
   methyl 5-({[(3,4-dimethoxyphenyl)amino]carbonyl}amino)-2-[(4-trifluoromethylphenyl)(phenyl)methoxy]benzoate,
   methyl 5-({[(3,4-dimethoxyphenyl)amino]carbonyl}amino)-2-[(2-chlorophenyl)(4'-chlorophenyl)methoxy]benzoate,
   N-(tert-butyl)-5-({[(3,4-dimethoxyphenyl)amino]carbonyl}amino)-2-{phenyl[4-(trifluoromethyl)phenyl]methoxy}benzamide,
   methyl 2-[(3,4-difluorophenyl)(phenyl)methoxy]-5-({[(3,4-dimethoxyphenyl)amino]carbonyl}amino)benzoate,
   methyl 2-{(2-chlorophenyl)[4-(trifluoromethyl)phenyl]methoxy}-5-({[(3,4-dimethoxyphenyl)amino]carbonyl}amino)benzoate,
   N-(tert-butyl)-2-{(2-chlorophenyl)[4-(trifluoromethyl)phenyl]methoxy)-5-({[(3,4-dimethoxyphenyl)amino]carbonyl}amino)benzamide,
   N-(tert-butyl)-2-[(4-chlorophenyl)(2-fluorophenyl)methoxy]-5-({[(3,4-dimethoxyphenyl)amino]carbonyl}amino) benzamide,
   N-(tert-butyl)-2-[(4-chlorophenyl)(3-fluorophenyl)methoxy]-5-({[(3,4-dimethoxyphenyl)amino]carbonyl}amino)benzamide, or
   N-(tert-butyl)-2-[(4-chlorophenyl)(4-fluorophenyl)methoxy]-5-({[(3,4-dimethoxyphenyl)amino]carbonyl}amino)benzamide.
(14) A prodrug of the compound as described in the above (1) or a salt thereof,
(15) A process for preparing a compound as described in the above (1) or a salt thereof, which comprises subjecting a compound represented by the formula: 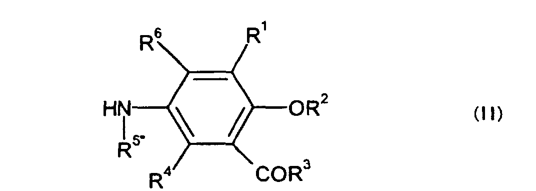 wherein each symbol is as defined in the above (1), or a salt thereof and a compound represented by the formula: 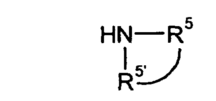 wherein each symbol is as defined in the above (1), or a salt thereof to urea synthesis reaction,
(16) A pharmaceutical composition comprising the compound of the above (1), a pharmaceutically acceptable salt or a prodrug thereof,
(17) A vanilloid receptor agonist comprising a compound represented by the formula: 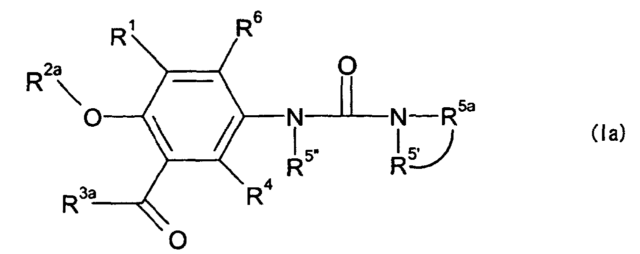 wherein R¹, R⁴ and R⁶ each independently represents a hydrogen atom, a halogen atom or an optionally substituted hydrocarbon group; R^{2a} represents an optionally substituted hydrocarbon group or an optionally substituted heterocyclic group; R^{3a} represents an optionally substituted hydrocarbon group, NR^{7'}R^{7a} or OR⁸ (wherein R^{7'} represents a hydrogen atom or an optionally substituted hydrocarbon group, R^{7a} and R⁸ represent independently an optionally substituted hydrocarbon group or an optionally substituted heterocyclic group, or R⁷' and R^{7a} may form an optionally substituted ring together with the adjacent nitrogen atom); R^{5a} represents an optionally substituted hydrocarbon group or an optionally substituted heterocyclic group, and R⁵' represents a hydrogen atom, or an optionally substituted hydrocarbon group, or R^{5a} and R⁵' may form an optionally substituted ring together with the adjacent nitrogen atom; and R⁵" represents a hydrogen atom or an optionally substituted hydrocarbon group], a pharmaceutically acceptable salt or a prodrug thereof,
(18) An agent for preventing and/or treating urinary frequency and/or urinary incontinence, which comprises the compound of the formula (Ia) as described in the above (17), a pharmaceutically acceptable salt or a prodrug thereof,
(19) An analgesic agent which comprises the compound of the formula (Ia) as described in the above (17), a pharmaceutically acceptable salt or a prodrug thereof,
(20) Use of the compound of the formula (Ia) as described in the above (17), a pharmaceutically acceptable salt or a prodrug thereof for manufacturing an agent for preventing and/or treating urinary frequency and/or urinary incontinence,
(21) Use of the compound of the formula (Ia) as described in the above (17), a pharmaceutically acceptable salt or a prodrug thereof for manufacturing an analgesic agent,
(22) Use of the compound of the formula (Ia) as described in the above (17), a pharmaceutically acceptable salt or a prodrug thereof for manufacturing a vanilloid receptor agonist,
(23) A method for preventing and/or treating urinary frequency and/or urinary incontinence, which comprises administrating to a mammal an effective amount of the compound of the formula (Ia) as described in the above (17), a pharmaceutically acceptable salt or a prodrug thereof, and
(24) An analgesic method which comprises administrating to a mammal an effective amount of the compound of the formula (Ia) as described in the above (17), a pharmaceutically acceptable salt or a prodrug thereof.

### Detailed Description of the Invention

In the above-mentioned formula (I), as the halogen atom represented by R¹, R⁴ and R⁶, for example, fluorine, chlorine, bromine, iodine, and the like may be used.

Examples of the "hydrocarbon group" in the "optionally substituted hydrocarbon group" represented by R¹, R⁴ and R⁶ include an aliphatic hydrocarbon group, an alicyclic hydrocarbon group, an alicyclic-aliphatic hydrocarbon group and an aromatic hydrocarbon group, and carbon number of these groups is preferably 1 to 16. Specifically, for example, an alkyl group, an alkenyl group, an alkynyl group, a cycloalkyl group, a cycloalkenyl group, a cycloalkylalkyl group, a cycloalkenylalkyl group, an aryl group, an aralkyl group, and the like are used.

Examples of the "alkyl group" include preferably a lower alkyl group, for example, a C₁₋₆alkyl group, etc. such as methyl, ethyl, propyl, isopropyl, butyl, isobutyl, sec-butyl, tert-butyl, pentyl, 1-ethylpropyl, hexyl, etc.

Examples of the "alkenyl group" include preferably a lower alkenyl group, for example, a C₂₋₇alkenyl group, etc. such as vinyl, 1-propenyl, allyl, isopropenyl, butenyl, isobutenyl, 2,2-dimethyl-pent-4-enyl, etc.

Examples of the "alkynyl group" include preferably a lower alkynyl group, for example, a C₂₋₆alkynyl group, etc. such as ethynyl, propargyl, 1-propynyl, etc.

Examples of the "cycloalkyl group" include preferably a lower cycloalkyl group, for example, a C₃₋₁₀cycloalkyl group, etc. such as cyclopropyl, cyclobutyl, cyclopentyl, cyclohexyl, cycloheptyl, cyclooctyl, bicyclo[2.2.1]heptanyl, adamanthyl, etc.

Examples of the "cycloalkenyl group" include preferably a lower cycloalkenyl group, for example, a C₃₋₆cycloalkenyl group, etc. such as cyclopropenyl, cyclobutenyl, cyclopentenyl, cyclohexenyl, etc.

Examples of the "cycloalkylalkyl group" include preferably a lower cycloalkylalkyl group, etc., for example, a C₄₋₁₂cycloalkylalkyl group, etc. such as cyclopropylmethyl, cyclopropylethyl, cyclobutylmethyl, cyclopentylmethyl, cyclohexylmethyl, cyclohexylethyl, etc.

Examples of the "cycloalkenylalkyl group" include preferably a lower cycloalkenylalkyl group, etc., for example, a C₄₋₁₂cycloalkenylalkyl group, etc. such as cyclopentenylmethyl, cyclohexenylmethyl, cyclohexenylethyl, cyclohexenylpropyl, cycloheptenylmethyl, cycloheptenylethyl, bicyclo[2.2.1]hept-5-en-2-ylmethyl, etc.

Examples of the "aryl group" include preferably a C₆₋₁₄aryl group, etc. such as phenyl, 1-naphthyl, 2-naphthyl, biphenyl, 2-anthryl, etc., for example, phenyl may be used.

Examples of the "aralkyl group" include preferably a C₇₋₁₉aralkyl group, etc. such as benzyl, benzhydryl, 1,1'-biphenyl-4-ylmethyl, 3,3-diphenylpropyl, 3-phenylpropa-2-enyl, phenylethyl, phenylpropyl, etc., for example, benzyl, benzhydryl, etc. may by used.

In addition, examples of the "hydrocarbon group" in the "optionally substituted hydrocarbon group" represented by R¹, R⁴ and R⁶ include a group formed by fusion of a cycloalkyl group in the above "cycloalkyl group" or "cycloalkylalkyl group" with a benzene ring (e.g., a polycyclic hydrocarbon group such as indanyl, etc.).

Examples of the substituents that the "hydrocarbon group" in the "optionally substituted hydrocarbon group" represented by R¹, R⁴ and R⁶ may have, include a halogen atom (e.g., fluorine, chlorine, bromine, iodine, etc.), a nitro group, a cyano group, a hydroxy group, a mercapto group, a sulfo group, a sulfino group, a phosphono group, an optionally halogenated C₁₋₆alkyl group (e.g., a C₁₋₆alkyl group such as methyl, ethyl, propyl, isopropyl, butyl, etc.; a mono-, di- or tri-halogeno-C₁₋₆alkyl group, etc. such as chloromethyl, dichloromethyl, trichloromethyl, fluoromethyl, difluoromethyl, trifluoromethyl, 2-bromoethyl, 2,2,2-trifluoroethyl, pentafluoroethyl, 3,3,3-trifluoropropyl, 4,4,4-trifluorobutyl, 5,5,5-trifluoropentyl, 6,6,6-trifluorohexyl, etc.), an oxo group, an amidino group, an imino group, an alkylenedioxy group (e.g., a C₁₋₄alkylenedioxy group, etc. such as methylenedioxy, ethylenedioxy, etc.), an optionally halogenated C₁₋₆alkoxy group (e.g., a C₁₋₆alkoxy group such as methoxy, ethoxy, propoxy, isopropoxy, butoxy, isobutoxy, pentyloxy, hexyloxy etc.; a mono-, di- or tri-halogeno-C₁₋₆alkoxy group, etc. such as trifluoromethoxy, etc.), an optionally halogenated C₁₋₆alkylthio group (e.g., a C₁₋₆alkylthio group such as methylthio, ethylthio, propylthio, isopropylthio, butylthio, isobutylthio, pentylthio, hexylthio, etc.; a mono-, di- or tri-halogeno-C₁₋₆alkylthio group, etc. such as of trifluoromethylthio, etc.), a carboxyl group, a formyl group, an optionally halogenated C₁₋₆alkyl-carbonyl group (e.g., a C₁₋₆alkyl-carbonyl group, etc. such as acetyl, propionyl, butyryl, isobutyryl, etc.), a formyloxy group, an optionally halogenated C₁₋₆alkyl-carbonyloxy group (e.g., a C₁₋₆alkyl-carbonyloxy group, etc. such as acetyloxy, propionyloxy, butyryloxy, isobutyryloxy, etc.), an optionally halogenated C₁₋₆alkoxycarbonyl group (e.g., a C₁₋₆alkoxy-carbonyl, etc. such as methoxycarbonyl, ethoxycarbonyl, propoxycarbonyl, butoxycarbonyl, etc.), a C₇₋₁₁aralkyl group (benzyl, etc.), a C₇₋₁₁aralkyloxy group (benzyloxy, etc.), an aralkyloxycarbonyl group (e.g., a C₇₋₁₁aralkyloxy-carbonyl, etc. such as benzyloxycarbonyl, etc.), a thiocarbamoyl group, an optionally halogenated C₁₋₆alkylsulfinyl group (e.g., a C₁₋₆alkylsulfinyl group such as methylsulfinyl, ethyl sulfinyl, etc.), an optionally halogenated C₁₋₆alkylsulfonyl group (e.g., a C₁₋₆alkylsulfonyl group such as methylsulfonyl, ethylsulfonyl, etc.), a sulfamoyl group, optionally halogenated mono-alkylsulfamoyl group (e.g., mono-C₁₋₆alkylsulfamoyl group, etc. such as methylsulfamoyl, ethylsulfamoyl, etc.), an optionally halogenated di-alkyl sulfamoyl group (e.g., a di-C₁₋₆alkylsulfamoyl group, etc. such as dimethylsulfamoyl, diethylsulfamoyl, etc.), an arylsulfamoyl group (e.g., a C₆₋₁₀arylsulfamoyl group, etc. such as phenylsulfamoyl, naphthylsulfamoyl, etc.), an aryl group (e.g., a C₆₋₁₀aryl group, etc. such as phenyl, naphthyl, etc.), an aryloxy group(e.g., a C₆₋₁₀aryloxy group, etc. such as phenyloxy, naphthyloxy, etc.), an arylthio group (e.g., a C₆₋₁₀arylthio group, etc. such as phenylthio, naphthylthio, etc.), an arylsulfinyl group (e.g., a C₆₋₁₀arylsulfinyl group such as phenylsulphinyl, naphthylsulfinyl, etc.), an arylsulfonyl group (e.g., a C₆₋₁₀arylsulfonyl group, etc. such as phenylsulfonyl, naphthylsulfonyl, etc.), an arylcarbonyl group (e.g., a C₆₋₁₀aryl-carbonyl group, etc. such as benzoyl, naphthoyl, etc.), an arylcarbonyloxy group (e.g., a C₆₋₁₀aryl-carbonyloxy group, etc. such as benzoyloxy, naphthoyloxy, etc.), an optionally substituted carbamoyl group [e.g., a group represented by the formula -CONR⁹R¹⁰ (wherein R⁹ and R¹⁰ each independently represents (1) a hydrogen atom, (2) a C₁₋₆alkyl group which may have 1 to 4 substituents selected from a halogen atom and a hydroxy group, (3) a C₆₋₁₀aryl group which may have 1 to 4 substituents selected from a halogen atom, a hydroxy group, and an optionally halogenated C₁₋₆alkyl group, or (4) a 5-to 12-membered heterocyclic group containing 1 to 4 hetero atoms consisting of 1 to 3 kinds of hetero atoms selected from an oxygen atom, a sulfur atom and a nitrogen atom as ring-constituting atoms, which may have 1 to 4 substituents selected from a halogen atom and an optionally halogenated C₁₋₆alkyl group, or R⁹ and R¹⁰ may form a 3- to 8-membered nitrogen-containing heterocyclic ring, which may contain 1 to 3 hetero atoms selected from an oxygen atom, a sulfur atom and a nitrogen atom in addition to carbon atoms and one nitrogen atom, together with the adjacent nitrogen atom (e.g., they may form a 3- to 8-membered (preferably, a 5-to 6-membered) nitrogen-containing heterocyclic ring such as aziridine, azetidine, pyrrolidine, pyrroline, pyrrole, imidazole, pyrazoline, imidazolidine, piperidine, morpholine, dihydropyridine, pyridine, piperazine, etc.)], an optionally substituted amino group (e.g., a group represented by the formula -NR⁹R¹⁰ (wherein R⁹ and R¹⁰ are as defined above), an optionally substituted ureido group (e.g., a group represented by the formula -NHCONR⁹R¹⁰ wherein R⁹ and R¹⁰ are as defined above), an optionally substituted carboxamide group (e.g., a group represented by the formula -NR⁹COR¹⁰ wherein R⁹ and R¹⁰ are as defined above), an optionally substituted sulfonamide group (e.g., a group represented by the formula -NR⁹SO₂R¹⁰ wherein R⁹ and R¹⁰ are as defined above), an optionally substituted heterocyclic group, and the like.

Examples of the "optionally substituted hydrocarbon group" in the substituents that the "hydrocarbon group" in the "optionally substituted hydrocarbon group" represented by R¹, R⁴ and R⁶ may have, include those as mentioned above, but when the substituents have a hydrocarbon group as a substituent, the hydrocarbon group is unsubstituted.

Examples of the "heterocyclic group" in the "optionally substituted heterocyclic group" represented by R¹, R⁴ and R⁶ include a 5- to 12-membered monocyclic or fused heterocyclic group, etc. containing 1 to 4 hetero atoms consisting of 1 to 3 kinds of hetero atoms selected from a nitrogen atom, a sulfur atom and an oxygen atom, such as pyridyl, pyrrolidinyl, piperazinyl, piperidinyl, 2-oxoazepinyl, furyl, decahydroisoquinolyl, quinolinyl, indolyl, isoquinolyl, thienyl, imidazolyl, morpholyl, etc. Examples of the "substituents" that the "optionally substituted heterocyclic group" may have, include the "substituents" in the "optionally substituted heterocyclic group" represented by R² which will be described below, but when the substituent is a heterocyclic group or a hydrocarbon group having a heterocyclic group, the heterocyclic group is unsubstituted.

Preferably, R¹ represents a hydrogen atom, R⁴ represents a hydrogen atom or an optionally halogenated C₁₋₄alkyl group (e.g., methyl, ethyl, isopropyl, tert-butyl, etc.), and R⁶ represents a hydrogen atom or an optionally halogenated C₁₋₄alkyl group (e.g., methyl, ethyl, isopropyl, tert-butyl, etc.).

As the "hydrocarbon group" in the "optionally substituted hydrocarbon group" represented by R², for example, those for the "hydrocarbon group" in the "optionally substituted hydrocarbon group" represented by the above-mentioned R¹, R⁴ and R⁶, preferably, an alkyl group, an alkenyl group and an alkynyl group may be used. As the "substituents", those for the "substituents" in the "optionally substituted hydrocarbon group" represented by the above-mentioned R¹, R⁴ and R⁶, may be used.

Examples of the "optionally substituted hydrocarbon group" represented by R², include preferably an optionally substituted C₇₋₁₉aralkyl group, particularly, an optionally substituted benzyl group, or an optionally substituted benzhydryl group. As R², an unsubstituted benzhydryl group, a 4-tert-butylbenzyl group, a 4-isopropylbenzyl group, etc is preferable.

Examples of the "optionally substituted heterocyclic group" represented by R², include a 5- to 12-membered aromatic heterocyclic group, or a saturated or unsaturated non-aromatic heterocyclic group, etc. which contains at least 1 hetero atom (preferably 1 to 4 hetero atoms, more preferably 1 to 2 hetero atoms) consisting of 1 to 3 kinds of hetero atoms (preferably 1 to 2 kinds of hetero atoms) selected from an oxygen atom, a sulfur atom, a nitrogen atom, etc. as ring-constituting atoms.

Examples of the "aromatic heterocyclic group" include an aromatic monocyclic heterocyclic group or an aromatic fused heterocyclic grop, etc.

Examples of the "aromatic monocyclic heterocyclic group" include a 5- to 6-membered aromatic monocyclic heterocyclic group, etc. such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl. etc.

Examples of the "aromatic fused heterocyclic group" include a 8- to 12-membered aromatic fused heterocyclic group (preferably, a heterocyclic group formed by fusion of the above-mentioned 5- or 6-membered aromatic monocyclic heterocyclic group with a benzene ring or a heterocyclic group formed by fusion of two above-mentioned 5- or 6-membered aromatic monocyclic heterocyclic groups which are the same or different) etc. such as benzofuranyl, isobenzofuranyl, benzothienyl, isobenzothienyl, indolyl, isoindolyl, 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.

Examples of the "saturated or unsaturated non-aromatic heterocyclic group" include a 3- to 8-membered (preferably 5- or 6-membered) saturated or unsaturated (preferably saturated) non-aromatic heterocyclic group (alicyclic heterocyclic group), etc. such as oxiranyl, azetidinyl, oxetanyl, thietanyl, pyrrolidinyl, tetrahydrofuryl, thiolanyl, piperidinyl, tetrahydropyranyl, thianyl, morpholyl, thiomorpholyl, piperazinyl, azepanyl, oxepanyl, thiepanyl, oxazepanyl, thiazepanyl, azocanyl, oxocanyl, thiocanyl, oxazocanyl, thiazocanyl, etc. These may be substituted with oxo, for example, may be 2-oxoazetidinyl, 2-oxopyrrolidinyl, 2-oxopiperidinyl, 2-oxoazepanyl, 2-oxoazocanyl, 2-oxotetrahydrofuryl, 2-oxotetrahydropyranyl, 2-oxothiolanyl, 2-oxothianyl, 2-oxopiperazinyl, 2-oxooxepanyl, 2-oxooxazepanyl, 2-oxothiepanyl, 2-oxothiazepanyl, 2-oxoazocanyl, 2-oxooxocanyl, 2-oxothiocanyl, 2-oxooxazocanyl, 2-oxothiazocanyl, and the like, preferably a 5-membered non-aromatic heterocyclic group such as 2-oxopyrrolidinyl, etc.

Examples of the "heterocyclic group" in the "optionally substituted heterocyclic group" represented by R², include preferably a 5- to 7-membered (preferably 5- or 6-membered, more preferably 5-membered) aromatic heterocyclic group which contains 1 to 3 (preferably 1 or 2) hetero atoms selected from an oxygen atom, a sulfur atom and a nitrogen atom, particularly a pyridyl group.

As the substituents that the "heterocyclic group" in the "optionally substituted heterocyclic group" represented by R² may have, for example, those for the "substituents" in the "optionally substituted hydrocarbon group" represented by R¹, R⁴ and R⁶ as described above may be used.

The "heterocyclic group" in the "optionally substituted heterocyclic group" represented by R² may have 1 to 5, preferably 1 to 3 substituents as described above at any possible position on the heterocyclic group, and when the number of the substituents is 2 or more, each substituent may be the same or different.

R² is preferably a C₇₋₁₉aralkyl group which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxy-carbonyl group, an optionally halogenated C₁₋₆alkylthio-carbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C₆₋₁₀arylsulfamoyl group, and a di-C₆₋₁₀arylsulfamoyl group.

R² is more preferably a C₇₋₁₉aralkyl group (a benzhydryl group, etc.) which may have 1 to 4 substituents selected from a halogen atom, an optionally halogenated C₁₋₄alkyl group, a nitro group, a cyano group and a C₁₋₄alkoxycarbonyl group.

R³ represents an optionally substituted hydrocarbon group, NR⁷'R⁷ or OR⁸, and as the "optionally substituted hydrocarbon group" represented by R³, those for the "optionally substituted hydrocarbon group" represented by R¹, R⁴ and R⁶ as described above may be used. Particularly, an optionally halogenated C₁₋₄alkyl group is preferred.

R⁷' in NR⁷'R⁷ represents the "hydrogen atom or optionally substituted hydrocarbon group", and as the "optionally substituted hydrocarbon group", those for the "optionally substituted hydrocarbon group" represented by R¹, R⁴ and R⁶ as described above may be used. As the substituents, those for the "substituents" in the "optionally substituted hydrocarbon group" represented by R¹, R⁴ and R⁶ as described above may be used. R⁷ represents a " optionally substituted non-aromatic group ", and as the "non-aromatic group", the aliphatic hydrocarbon group, the alicyclic hydrocarbon group, the alicyclic-aliphatic hydrocarbon group, non-aromatic heterocyclic group, and the like as described above (for example, a C₁₋₆alkyl group, a C₂₋₆alkenyl group, a C₂₋₆alkynyl group, a C₃₋₁₀cycloalkyl group, a C₃₋₁₀cycloalkenyl group, a C₄₋₁₂cycloalkylalkyl group, a C₄₋₁₂cycloalkenylalkyl group, a C₇₋₁₉aralkyl group, or a 5- to 12-membered non-aromatic heterocyclic group that contains 1 to 4 hetero atoms consisting of 1 to 3 kinds of hetero atoms selected from an oxygen atom, a sulfur atom and a nitrogen atom as ring-constituting atoms, which may have independently a substituent) may be used. As the substituents, those for the "substituents" in the "optionally substituted hydrocarbon group" represented by R¹, R⁴ and R⁶ as described above may be used. As R⁷, an optionally halogenated C₁₋₄alkyl group is preferred and as R⁷', a hydrogen atom is preferred.

Further, R⁷' and R⁷ may form an "optionally substituted ring" together with the adjacent nitrogen atom, and examples of the "optionally substituted ring" includes a "3- to 12-membered nitrogen-containing heterocyclic ring", etc. which may contain 1 to 3 hetero atoms selected from an oxygen atom, a sulfur atom and a nitrogen atom in addition to carbon atoms and one nitrogen atom. Specific examples of the "3- to 12-membered nitrogen-containing heterocyclic ring" include a non-aromatic heterocyclic ring such as aziridine, azetidine, pyrrolidine, piperidine, hexamethyleneimine, heptamethyleneimine, morpholine, thiomorpholine, pyrazolidine, piperazine, etc., an aromatic heterocyclic ring, etc. such as pyrazole, pyridine, quinoline, isoquinoline, pyrazine, pyrimidine, pyrrole, imidazole, pyridazine, isothiazoline, oxazole, isoxazole, indole. etc.

In addition, examples of the substituents in the "optionally substituted ring" include those for the "substituents" in the "optionally substituted hydrocarbon group" represented by R¹, R⁴ and R⁶ as described above.

R⁸ in OR⁸ represents the "optionally substituted hydrocarbon group" or the "optionally substituted heterocyclic group", and as the "optionally substituted hydrocarbon group" and the "optionally substituted heterocyclic group", those for the "optionally substituted hydrocarbon group" represented by R¹, R⁴ and R⁶ as described above, or those for the "optionally substituted heterocyclic group" represented by R² as described above may be used. As R⁸, particularly, an optionally halogenated C₁₋₄alkyl group is preferred.

R³ is preferably a C₁₋₄alkyl group, a C₁₋₄alkylamino group or a C₁₋₄alkoxy group.

R⁵ represents an "optionally substituted hydrocarbon group (except for an optionally substituted benzoyl group)" or a "optionally substituted heterocyclic group (except for a quinolyl group)". As these groups, those for the "optionally substituted hydrocarbon group" (except for an optionally substituted benzoyl group) represented by R¹, R⁴ and R⁶ as described above or those for the "optionally substituted heterocyclic group" (except for a quinolyl group) represented by R² as described above, may be used. As R⁵, particularly, an optionally substituted phenyl group, or an optionally substituted pyridyl group is preferred.

R⁵ is preferably a C₆₋₁₀aryl group, a pyridyl group or a C₇₋₁₁aralkyl group, each of which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxy-carbonyl group, an optionally halogenated C₁₋₆alkylthio-carbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C₆₋₁₀arylsulfamoyl group, and a di C₆₋₁₀arylsulfamoyl group, more preferably, a phenyl group that may have 1 or 2 C₁₋₄alkoxy groups.

R^{5'} represents the "hydrogen atom or optionally substituted hydrocarbon group", and examples of the "optionally substituted hydrocarbon group" includes those for the "optionally substituted hydrocarbon group" represented by R¹, R⁴ and R⁶ as described above. As the substituents, those for the "substituents" in the "optionally substituted hydrocarbon group" represented by R¹, R⁴ and R⁶ as described above may be used.

Further, R⁵ and R^{5'} may form an "optionally substituted ring" together with the adjacent nitrogen atom, and examples of the "optionally substituted ring", includes a "3- to 12-membered nitrogen-containing heterocyclic ring", etc. which may contain 1 to 3 hetero atoms selected from an oxygen atom, a sulfur atom and a nitrogen atom in addition to carbon atoms and one nitrogen atom. Examples of the "3-to 12-membered nitrogen-containing heterocyclic ring" include those for the "optionally substituted ring" formed by R^{7'} and R⁷ as described above together with the adjacent nitrogen atom. In addition, examples of the substituents in the "optionally substituted ring" include those for the "substituents" in the "optionally substituted hydrocarbon group" represented by R¹, R⁴ and R⁶ as described above.

R^{5"} represents the "hydrogen atom or optionally substituted hydrocarbon group", and examples of the "optionally substituted hydrocarbon group" include those for the "optionally substituted hydrocarbon group" represented by as described above R¹, R⁴ and R⁶. Examples of the substituents include those for the "substituents" in the "optionally substituted hydrocarbon group" represented by R¹, R⁴ and R⁶ as described above.

As R^{5'} and R^{5"}, a hydrogen atom is preferable.

The compound represented by the formula (I) of the present invention is preferably the compound wherein R¹ represents a hydrogen atom; R² represents a C₇₋₁₉aralkyl group which may have 1 to 4 substituents selected from a halogen atom, an optionally halogenated C₁₋₄alkyl group, a nitro group, a cyano group and a C₁₋₄alkoxy-carbonyl group; R³ represents a C₁₋₄alkyl group, a C₁₋₄alkylamino group or a C₁₋₄alkoxy group; R⁴ represents a hydrogen atom or an optionally halogenated C₁₋₄alkyl group; R⁵ represents a C₆₋₁₀aryl group, a pyridyl group or a C₇₋₁₁ₐᵣalkyl group, each of which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxy-carbonyl group, an optionally halogenated C₁₋₆alkylthio-carbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C₆₋₁₀arylsulfamoyl group, and a di-C₆₋₁₀arylsulfamoyl group; and R⁶ represents a hydrogen atom or an optionally halogenated C₁₋₄alkyl group.

Examples of the salt of the compound represented by the formula (I) include an acid addition salt such as an inorganic acid salt (e.g., hydrochloride, sulfate, hydrobromide, phosphate, etc.), an organic acid salt (e.g., acetate, trifluoroacetate, succinate, maleate, fumarate, propionate, citrate, tartarate, lactate, oxalate, methanesulfonate, benzensulfonate, p-toluenesulfonate, etc.), as well as a salt with a base, such as an alkali metal salt (e.g., potassium salt, sodium salt, lithium salt, etc.), an alkaline earth metal salt (e.g. calcium salt, magnesium salt, etc.), an ammonium salt, a salt with an organic base (e.g., trimethylamine salt, triethylamine salt, tert-butyldimethylamine salt, dibenzylmethylamine salt, benzyldimethylamine salt, N,N-dimethylaniline salt, pyridine salt, quinoline salt). Among others, a pharmaceutically acceptable salt is preferable.

In addition, the compound represented by the formula (I) or a salt thereof may be in the form of a hydrate or a solvate. Hereinafter, the compound including a salt thereof, a hydrate thereof and a solvate thereof will be also referred to as the compound (I).

The prodrug of the compound (I) means a compound which is converted to the compound (I) by a reaction with an enzyme, a gastric acid, etc. in a living body.

Examples of the prodrug of the compound (I) include a compound wherein if the compound (I) has an amino group, the amino group of the compound (I) is acylated, alkylated, or phosphorylated, (for example, a compound wherein the amino group of the compound (I) is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, or tert-butyl, etc.); a compound wherein if the compound (I) has a hydroxy group, the hydroxyl group of the compound (I) is acylated, alkylated, phosphorylated, or substituted with boric acid, (for example, a compound wherein the hydroxyl group of the compound (I) is substituted with acetyl, palmitoyl, propanoyl, pivaloyl, succinyl, fumaryl, alanyl, or dimethylaminomethylcarbonyl, etc.); a compound wherein if the compound (I) has a carboxyl group, the carboxyl group of the compound (I) is esterified or amidated, (for example, a compound wherein the carboxyl group is modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, or methylamide); and the like. These compounds can be produced from the compound (I) according to a per se known method. Also, the prodrug of compound (I) may be a compound which is converted into the compound (I) under the physiological conditions as described in "Development of Drugs", vol. 7, Molecular Design, pp. 163-198 published in 1990 by Hirokawa Publishing Company.

The prodrug of the compound (I) may be itself or a pharmaceutically acceptable salt thereof. In case that the prodrug of the compound (I) has an acidic group such as a carboxyl group, etc., the examples of these salts include salts with an inorganic base such as an alkali metal (e.g., sodium, potassium, and the like), an alkaline earth metal (calcium, magnesium, and the like), a transition metal, etc. (e.g., zinc, iron, copper, and the like), salts with an organic base such as organic amines (e.g., trimethylamine, triethylamine, pyridine, picoline, ethanolamine, diethanolamine, triethanolamine, dicyclohexylamine, N,N'-dibenzylethylenediamine, and the like), basic amino acids (e.g., arginine, lysine, ornithine, and the like), and so on.

In case that the prodrug of the compound (I) has a basic group such as an amino group, etc., examples of the salts include salts with an inorganic acid or organic acid (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like), an acidic amino acid (e.g., aspartic acid, glutamic acid, and the like), and so on.

The prodrug of the compound (I) may be in any form of hydrate or non-hydrate.

The compound (I) may contain one or more asymmetric carbon atoms in the molecule and the compounds having any one of R configuration and S configuration relating to the asymmetric carbon atoms are included in the scope of the present invention.

The compound (I) can be produced by, for example, the following preparation process 1: 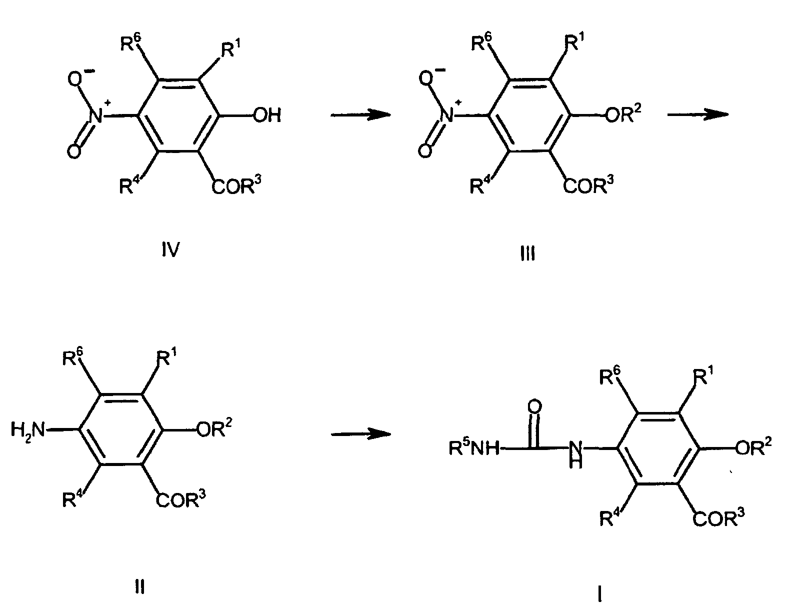 wherein each symbol is as defined above, or an analogous process thereto.

According to the process 1, first, a compound (IV) is subjected to alkylation to produce a compound (III).

The alkylation is carried out in the presence of a base and an alkyhalide in a solvent having no influence on the reaction, in accordance with a conventional method. Examples of the base include potassiun carbonate, sodium carbonate, sodium hydride, potassium hydride, and the like.

Examples of the alkylhalide include alkyl chloride, alkyl bromide, alkyl iodide, alkyl methanesulfonate, and the like.

The amounts of the base and the alkylhalide to be used are preferably about 1 to 5 molar equivalents to the compound (IV), respectively.

Examples of the solvent having no influence on the reaction include ethers such as tetrahydrofuran, etc.; halogenated hydrocarbons such as chloroform, etc.; aromatic hydrocarbons such as toluene, etc.; amides such as N,N-dimethylformamide, etc.; sulfoxides such as dimethylsulfoxide, etc.; or the like. These solvents may be used by mixing two or more solvents in an appropriate ratio. The amount of the solvent to be used is, for example, 1 to 100 times the volume of the compound (IV).

The reaction temperature is usually about -50 to about 250°C, preferably about 0°C to about 120°C.

The reaction time is usually about 0.5 to about 24 hours. The compound (III) thus produced may be isolated and purified through any known separation and purification means such as concentration, concentration under reduced pressure, solvent extraction, crystallization, recrystallization, phase transfer, chromatography, or the like. In addition, the compound (III) may be used without isolation in the subsequent reaction step.

Then, the compound (III) is subjected to reduction to produce the compound (II).

This reaction is carried out by using a reducing agent in a solvent having no influence on the reaction, in accordance with a conventional method.

Examples of the solvent having no influence on the reaction include alcohols such as methanol, etc.; ethers such as tetrahydrofuran, etc.; hydrocarbons such as ethyl acetate, etc.; water; or the like. These solvents may be used by mixing 2 or more solvents in an appropriate ratio. The amount of the solvent to be used is, for example, 1 to 100 times the volume of the compound (III).

Examples of the reducing agent include hydrogen in the presence of iridium carbon, sodium hydrosulfite, hydrogen iodide, or the like. The amount of the reducing agent to be used is preferably 1 to 100 molar equivalents.

The reaction temperature is usually about -50 to 200°C.

The reaction time is usually about 0.5 to 24 hours.

The compound (II) thus produced may be isolated and purified through any known separation and purification means such as concentration, concentration under reduced pressure, solvent extraction, crystallization, recrystallization, phase transfer, chromatography, or the like. In addition, the compound (II) may be used in the subsequent reaction step without isolation.

Then, the compound (II) is subjected to the urea synthesis reaction to produce the compound (I).

This reaction is carried out by using an urea formation agent in a solvent having no influence on the reaction, in accordance with a conventional method.

Examples of the urea formation agent include phenyl isocyanate, 3,4-dimethoxyphenyl isocyanate, 3-methoxyphenyl isocyanate, 4-methoxyphenyl isocyanate, or the like. In addition, the urea formation agent also includes a reagent formed in a reaction system by reacting a carbonylating agent in the presence of an appropriate amine, and then the reagent as such is reacted with the compound (II). Examples of the carbonylating agent include carbonyldiimidazole, phosgene, di(N-succinimidyl) carbonate, or the like. The amount of the urea formation agent to be used is preferably about 1 to 5 molar equivalents, relative to the compound (II).

Examples of the solvent having no influence on the reaction include ethers such as tetrahydrofuran, etc.; halogenated hydrocarbons such as chloroform, etc.; aromatic hydrocarbons such as toluene, etc.; amides such as N,N-dimethylformamide, etc.; sulfoxides such as dimethylsulfoxide, etc; or the like. These solvents may be used by mixing 2 or moresolvents in an appropriate ratio. The amount of the solvent to be used is 1 to 100 times the volume of the compound (II).

The reaction temperature is usually about -50 to about 250°C, preferably about 0°C to 120°C.

The reaction time is usually about 0.5 to 24 hours.

The compound (I) thus produced may be isolated and purified through any known separation and purification means such as concentration, concentration under reduced pressure, solvent extraction, crystallization, recrystallization, phase transfer, chromatography, or the like.

The compound (IV) to be used as a starting compound is commercially available, or can be prepared by per se known processes such as the processes described in "Bulletin of the Chemical Society of Japan", vol. 56, pp. 2762-2763 (1983), "Journal of Medicinal Chemistry", vol. 37, pp. 845-859 (1994), JP 5-294920 A, and the like or analogous processes thereof.

The compound (I) of the present invention and a prodrug thereof possess an excellent analgesic action, a preventing and/or therapeutic action of urinary frequency and/or urinary incontinence, and a pharmaceutical composition comprising them is useful as an analgesic agent, and an agent for preventing and/or treating urinary frequency and/or urinary incontinence, and the like. Therefore, the pharmaceutical composition is useful for treating acute/chronic, systemic and topical pain and/or inflammation, for example, treatment of gonarthritis, arthralgia such as lumbago, osteoarthritis, chronic articular rheumatism, fibromyalgia, Guillain-Barre syndrome, meralgia paraesthetica, pain by reflex sympathetic dystrophy syndrome, postoperative pain, diabetic neuralgia, herpes zoster pain, cancer pain, migraine, muscle pain, dental pain, myocardial infarction, reflex sympathetic nerve anomaly symptom, pain by trigeminal neuralgia, postmastectomy pain; analgesia for pain, etc. by burns; treatment of pain by inflammatory digestive system disease and enterokinesis; treatment of allergic rhinitis and vasomotor rhinitis; treatment of atopic dermatitis, psoriasis, lichen simplex chronicus, hemodialysis, itch by rash, etc.; treatment of urinary frequency and/or urinary incontinence by overactive bladder and cystitis; or the like. In addition, the pharmaceutical composition is also useful for treatment of clamacteric disorders, or flush or glow by administration of gonadotrophin agonist; treatment of emesis by anti-emetic or anticancer drug; prevention of obesity; inhibition of fat accumulation (fat metabolism enhancer); lowering cholesterol; enhancing secretion of adrenaline (increasing action of cardiac rate, etc.); lowering blood pressure; protection of gastric mucosa; enhancing secretion of saliva or stomach juice; lowering blood glucose; treatment of irritable bowel syndrome; treatment of toxic shock, septic shock, arterial sclerosis, cancer; prevention of progress of nerve tissue degenerative disease such as cerebral apoplexy (cerebral infarction, cerebral hemorrhage); prevention and/or treatment of motor neuron disease, Parkinson's disease, Alzheimer's disease, AIDS-associated dementia, Lewy body disease, cerebral neuropathy, peripheral neuropathy and prion disease.

In addition, the present inventors have found that a compound represented by the formula: 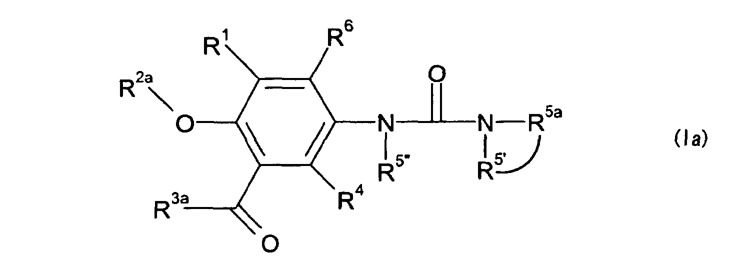 wherein R^{2a} represents an optionally substituted hydrocarbon group or an optionally substituted heterocyclic group; R^{3a} represents an optionally substituted hydrocarbon group, NR⁷'R^{7a} or OR⁸ (wherein R⁷' represents a hydrogen atom or an optionally substituted hydrocarbon group, R^{7a} and R⁸ each independently represents an optionally substituted hydrocarbon group or an optionally substituted heterocyclic group, or R^{7'} and R^{7a} may form an optionally substituted ring together with the adjacent nitrogen atom); R^{5a} represents an optionally substituted hydrocarbon group or an optionally substituted heterocyclic group; R^{5'} represents a hydrogen atom, or an optionally substituted hydrocarbon group, or R^{5a} and R^{5'} may form an optionally substituted ring together with the adjacent nitrogen atom; R^{5"} represents a hydrogen atom or an optionally substituted hydrocarbon group, R¹, R⁴ and R⁶ are as defined in the above-described formula (I), and the "optionally substituted hydrocarbon group", the "optionally substituted heterocyclic group" and the "ring formed together with the adjacent nitrogen atom" are as defined in the above-described formula (I), or a salt thereof (hereinafter, referred to as a compound (Ia)) including the compound (I) possess vanilloid receptor agonist activity.

Therefore, the present invention also provides a vanilloid receptor agonist comprising the compound (Ia) or a prodrug thereof.

The vanilloid receptor is a nociceptor which mediates pain, and an agonist thereof has the effect of desensitizing nerves. Therefore, the vanilloid receptor agonist of the present invention is an analgesic agent or an agent for preventing and/or treating urinary frequency and/or urinary incontinence, and useful for the prevention and/or treatment of diseases and disorders as described above.

The compound (I) or the compound (Ia) of the present invention may be administered orally or parenterally, and are particularly suitable for oral administration because they have no pungent taste. Further, these compounds can be used for the prevention and/or treatment of the above-mentioned diseases and disorders by formulating into forms suitable for the administration.

The compound (I) or the compound (Ia) of the present invention may be used in combination with other drugs for the prevention and/or treatment of diseases and disorders (e.g., other drugs for the prevention and/or treatment of central nervous system diseases).

The compound (I) or (Ia) has a low toxicity and can be safely administered orally or parenterally (e.g., topical, rectal, intravenous administration, etc.) as such or as a pharmaceutical composition, which is produced by compounding with a pharmaceutically acceptable carrier according to a per se known method, in the form of tablets (including sugar-coated tablets, film-coated tablets), powders, granules, capsules (including soft capsules), solutions, injectable preparations, nasal drops, suppositories, sustained-release formulations, plasters, chewing gums, or the like.

The content of the compound (I) or (Ia) in the formulation of the present invention is about 0.01 to about 100% by weight based on the total weight of the formulation. The dosage may vary depending on the administration subject, the administration route, the disease to be treated, and the like, but, for example, in case that it is administered orally to an adult (body weight 50 kg) as an analgesic agent, a daily dosage of the compound (I) or (Ia) of the present invention as the active ingredient is about 5 to 1000 mg/day, preferably, about 10 to 600 mg/day, more preferably about 10 to 300 mg/day, particularly preferably about 15 to 150 mg/day, and the dosage can be administered once a day, or 2 or 3 times daily.

When the compound (I) or (Ia) of the present invention may be used in combination with other drugs, these drugs, separately or simultaneously, may be formulated by mixing with pharmaceutically acceptable carriers, excipients, binders, diluents or the like, and can be administered orally or parenterally. When the drug is prepared separately, the drugs which are prepared separately may be mixed with a diluent or the like before using and then administered, or each of the preparations separately prepared may be administered, simultaneously or separately at an interval, to an identical subject. Kit products used for mixing the separately-prepared preparations with a diluent and the like before using and administering, (for example, an injectable kit including ampoules containing each powdery drug, and a diluent for mixing and solving with 2 or more drugs before using, and the like), kit products used for administering each of the separately-prepared preparations, simultaneously or separately at an interval, to an identical person, (for example, a tablet kit for administering 2 or more tablets, simultaneously or separately at an interval, wherein the tablet containing each drugs was put into the same or the separate bags and, if necessary, a column wherein the drug administration date is to be indicated was provided on the bag, and the like), or the like are also included in the pharmaceutical composition of the present invention.

The pharmaceutically acceptable carrier, which may be used for the preparations of the present invention includes a variety of organic or inorganic carrier substances, which have been conventionally employed as formulation materials, for example, an excipient, a lubricant, a binder, and a disintegrator in solid formulations; a solvent, a solubilizer, a suspending agent, an isotonizing agent, a buffering agent, and a soothing agent in liquid formulations; and the like. Also, if required, conventional additives such as a preservative, an antioxidant, a coloring agent, a sweetener, an adsorbent, a wetting agent, and the like can be used.

Examples of the excipient include lactose, refined sugar, D-mannitol, starch, corn starch, crystalline cellulose, light silicic anhydride, or the like.

Examples of the lubricant include magnesium stearate, calcium stearate, talc, colloidal silica, or the like.

Examples of the binder include crystalline cellulose, refined sugar, D-mannitol, dextrin, hydroxypropyl cellulose, hydroxypropylmethyl cellulose, polyvinyl pyrrolidone, starch, sucrose, gelatine, methyl cellulose, sodium carboxymethyl cellulose, or the like.

Examples of the disintegrator include starch, carboxymethyl cellulose, calcium carboxymethyl cellulose, croscarmellose sodium, sodium carboxymethyl starch, L-hydroxypropyl cellulose, or the like.

Examples of the solvent include water for injection, alcohol, propylene glycol, macrogol, sesame oil, corn oil, olive oil, or the like.

Examples of the solubilizer include polyethylene glycol, propylene glycol, D-mannitol, benzyl benzoate, ethanol, trisaminomethane, cholesterol, triethanolamine, sodium carbonate, sodium citrate, or the like.

Examples of the suspending agent include a surface active agent such as stearyltriethanolamine, sodium lauryl sulfate, laurlyaminopropionic acid, lecithin, benzalkonium chloride, benzethonium chloride, glycerin monostearate, or the like; hydrophilic high molecular weight substances such as polyvinyl alcohol, polyvinylpyrrolidone, sodium carboxymethyl cellulose, methyl cellulose, hydroxymethyl cellulose, hydroxyethyl cellulose, hydroxypropyl cellulose, or the like; etc.

Examples of the isotonizing agent include glucose, D-sorbitol, sodium chloride, glycerin, D-mannitol, or he like.

Examples of the buffering agent include a buffering solution of phosphate, acetate, carbonate, citrate or the like.

Examples of the soothing agent include benzyl alcohol or the like.

Examples of the preservative include paraoxybenzoic ester, chlorobutanol, benzyl alcohol, phenethyl alcohol, dehydroacetic acid, sorbic acid, or the like.

Examples of the antioxidant include sulfite, ascorbic acid, α-tocopherol, or the like.

### Examples

The present invention is hereinafter described in more detail by means of the following Examples and Test Examples, but these examples are merely illustrative, and are not intended to limit the present invention.

The genetic engineering procedures described below were based on the methods described in the textbook (Maniatis, et al., Molecular Cloning, Cold Spring Harbor Laboratory, 1989) or the methods described in the protocols attached to the reagents.

### Example 1

### N-[4-(Benzhydryloxy)-3-propionylphenyl]-N'-phenylurea

### (1) 1-[2-(Benzhydryloxy)-5-nitrophenyl]propan-1-one

To a solution (20 mL) of 1-(2-hydroxy-5-nitrophenyl)propan-1-one (1.00 g, 5.12 mmole) in DMF was added sodium hydride (60%, 184 mg, 4.61 mmole) under ice-cooling, the mixture was stirred at room temperature for 30 minutes, bromodiphenylmethane (1.52 g, 6.15 mmole) was added to the mixture, and the mixture was stirred at 70°C for 12 hours. Sodium hydride (60%, 184 mg, 4.61 mmole) was further added to the mixture under ice-cooling, and the mixture was stirred at room temperature for 30 minutes. Bromodiphenylmethane (1.52 g, 6.15 mmole) was added to the mixture, and the mixture was stirred at 70°C for 5 hours. The reaction solution was poured into a solution of ice-water containing ammonium chloride, and the mixture was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain 1.47 g (79.5%) of the titled compound as a solid.
¹H-NMR (CDCl₃) δ; 1.12 (3H, t, J = 7.4 Hz), 2.98 (2H, q, J = 7.4 Hz), 6.39 (1H, s), 6.97 (1 H, d, J = 9.2 Hz), 7.26 to 7.41 (10H, m), 8.15 (1H, dd, J = 9.2, 2.6 Hz), 8.53 (1H, d, J = 2.6 Hz)

### (2) 1-[5-Amino-2-(benzhydryloxy)phenyl]propan-1-one

To a mixed solution of 1-[2-(benzhydryloxy)-5-nitrophenyl]propan-1-one (1.40 g, 0.388 mmole) in ethyl acetate (50 mL) and methanol (50 mL) was added 5% iridium-carbon (300 mg), and the mixture was stirred for 3 hours under hydrogen atmosphere. The insolubles were filtered off, and the filtrate was concentrated, to obtain 450 mg (34.9%) of the titled compound as oil.
¹H-NMR (CDCl₃) δ; 1.05 (3H, t, J = 7.4 Hz), 2.95 (2H, q, J = 7.4 Hz), 6.14 (1H, s), 6.60 (1 H, dd, J = 2.6, 8.8 Hz), 6.68 (1H, d, J = 8.8 Hz), 6.95 (1H, d, J = 2.6 Hz), 7.15 to 7.42 (10H, m)

### (3) N-[4-(Benzhydryloxy)-3-propionylphenyl]-N'-phenylurea

To a solution (8 mL) of 1-[5-amino-2-(benzhydryloxy)phenyl]propan-1-one (430 mg, 1.30 mmole) in THF was added phenyl isocyanate (0.159 mL, 1.47 mmole) under ice-cooling, and the mixture was stirred at 0°C for 1 hour and at room temperature for 12 hours, was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate is 3:1) and recrystallized from hexane and ethyl acetate to obtain 372 mg (63.5%) of the titled compound as a solid.
¹H-NMR (CDCl₃) δ; 1.06 (3H, t, J = 7.4 Hz), 3.00 (2H, q, J = 7.4 Hz), 6.21 (1H, s), 6.82 (1 H, d, J = 9.2 Hz), 7.01 to 7.38 (18H, m), 7.62 (1H, dd, J = 2.6, 8.8 Hz)

### Example 2

### N-[(4-Benzhydryloxy)-3-propionylphenyl]-N'-(3,4-dimethoxyphenyl)urea

To a solution (4 mL) of 1-[5-amino-2-(benzhydryloxy)phenyl]propan-1-one (400 mg, 1.21 mmole) in tetrahydrofuran was added 3,4-dimethoxyphenyl isocyanate (0.216 mL, 1.45 mmole) at 0ºC and the mixture was stirred for 1 hour at 0°C and for 12 hours at room temperature. The solvent was distilled off under reduced pressure and the residue was recrystallized from ethyl acetate, to obtain 610 mg (98.7%) of the titled compound.
¹H-NMR (CDCl₃) δ; 1.07 (3H, t, J = 7.2 Hz), 3.01 (2H, q, J = 7.2 Hz), 3.85 (6H, s), 6.24 (1H, s), 6.75 to 7.65 (18H, m)

### Example 3

### N-[(4-Benzhydryloxy)-3-propionylphenyl]-N'-(3-methoxyphenyl)urea

To a solution (5 mL) of 1-[5-amino-2-(benzhydryloxy)phenyl]propan-1-one (200 mg, 0.603 mmole) and diisopropylethyl amine (0.121 mL, 0.723 mmole) in acetonitrile was added N,N-disuccinimidyl carbamate (185 mg, 0.723 mmole) at 0°C, and the mixture was stirred for 1 hour at 0°C. To this solution were added diisopropylethyl amine (0.121 mL, 0.723 mmole) and m-anicidine (0.146 mL, 1.30 mmole), and the mixture was stirred at room temperature for 12 hours. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), to obtain 80 mg (27.7%) of the titled compound as oil.
¹H-NMR (CDCl₃) δ; 1.07 (3H, t, J = 7.2 Hz), 3.00 (2H, q, J = 7.2 Hz), 3.77 (3H, s), 6.23 (1H, s), 6.27 to 7.64 (19H, m)

### Example 4

### N-[(4-Benzhydryloxy)-3-propionylphenyl]-N'-(4-methoxyphenyl)urea

To a solution (5 mL) of 1-[5-amino-2-(benzhydryloxy)phenyl]propan-1-one (200 mg, 0.603 mmole) and diisopropylethyl amine (0.121 mL, 0.723 mmole) in acetonitrile was added N,N-disuccinimidyl carbamate (185 mg, 0.723 mmole) at 0°C, and the mixture was stirred for 1 hour at 0°C. To this solution were added diisopropylethyl amine (0.121 mL, 0.723 mmole) and p-anicidine (160 mg, 1.30 mmole), and the mixture was stirred at room temperature for 12 hours. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 2:1), which was recrystallized from ethyl acetate to obtain 100 mg (34.5%) of the titled compound as a solid.
¹H-NMR (CDCl₃) δ; 1.06 (3H, t, J = 7.4 Hz), 2.97 (2H, q, J = 7.4 Hz), 3.78 (3H, s), 6.23 (1H, s), 6.68 (1H, s), 6.81 to 7.63 (18H, m)

### Example 5

### N-[4-(Benzhydryloxy)-3-butylphenyl]-N'-(4-methoxyphenyl)urea

### (1) 1-[2-(Benzhydryloxy)-5-nitrophenyl]butan-1-one

To a solution (30 mL) of 1-(2-hydroxy-5-nitrophenyl)butan-1-one (1.50 g, 7.18 mmole) in DMF was added sodium hydride (60%, 287 mg, 7.18 mmole) under ice-cooling, and the mixture was stirred at room temperature for 1 hour. Bromodiphenylmethane (2.13 g, 8.62 mmole) was added to the mixture, and the mixture was stirred for 3 hours at 70°C. The reaction solution was poured into ice-water, and the mixture was extracted with ethyl acetate. The extracted solution was washed with water, was dried with anhydrous magnesium sulfate, and the solvent was distilled off under reduced pressure. To the residue was added diisopropylether, to obtain 1.79 g (66.3%) of the titled compound as a solid.
¹H-NMR (CDCl₃) δ; 0.82 (3H, t, J = 7.4 Hz), 1.59 to 1.70 (2H, m), 2.92 (2H, t, J = 7.6 Hz), 6.39 (1H, s), 6.96 (1 H, d, J = 9.2 Hz), 7.35 to 7.41 (10H, m), 8.16 (1H, dd, J = 9.2, 3.0 Hz), 8.50 (1H, d, J = 3.0 Hz)

### (2) 1-[5-Amino-2-(benzhydryloxy)phenyl]butan-1-one

To a solution (100 mL) of 1-[2-(benzhydryloxy)-5-nitrophenyl]butan-1-one (1.74 g, 4.63 mmole) in ethyl acetate was added 5% iridium-carbon (200 mg), and the mixture was stirred for 12 hours under hydrogen atmosphere. The insolubles were filtered off, and the filtrate was concentrated to obtain 1.55 g (96.9%) of the titled compound as oil.
¹H-NMR (CDCl₃) δ; 0.77 (3H, t, J = 7.4 Hz), 1.50 to 1.64 (2H, m), 2.90 (2H, t, J = 7.4 Hz), 6.13 (1H, s), 6.60 (1 H, dd, J = 3.0, 8.4 Hz), 6.66 (1H, d, J = 8.4 Hz), 6.94 (1H, d, J = 3.0 Hz), 7.24 to 7.39 (10H, m)

### (3) N-[4-(Benzhydryloxy)-3-butylphenyl]-N'-(4-methoxyphenyl)urea

To a solution (3 mL) of 1-[5-amino-2-(benzhydryloxy)phenyl]butan-1-one (300 mg, 0.868 mmole) in THF was added 4-methoxyphenyl isocyanate (0.135 mL, 1.04 mmole) under ice-cooling, the mixture was stirred at 0°C for 1 hour and at room temperature for 2 hours, and the solvent was distilled off under reduced pressure. The residue was recrystallized from hexane and ethyl acetate, to obtain 331 mg (77.2%) of the titled compound.
¹H-NMR (CDCl₃) δ; 0.76 (3H, t, J = 7.2 Hz), 1.49 to 1.64 (2H, m), 2.92 (2H, t, J = 7.4 Hz), 3.78 (3H, s), 6.22 (1H, s), 6.72 to 7.66 (19H, m)

### Example 6

### N-[4-(Benzhydryloxy)-3-butylphenyl]-N'-(3-methoxyphenyl)urea

To a solution (3 mL) of 1-[5-amino-2-(benzhydryloxy)phenyl]butan-1-one (300 mg, 0.868 mmole) in THF was added 3-methoxyphenyl isocyanate (0.136 mL, 1.04 mmole) under ice-cooling, the mixture was stirred at 0°C for 1 hour and at room temperature for 12 hours, and the solvent was distilled off under reduced pressure. The residue was recrystallized from hexane and ethyl acetate, to obtain 420 mg (97.9%) of the titled compound.
¹H-NMR (CDCl₃) δ; 0.76 (3H, t, J = 7.0 Hz), 1.55 to 1.66 (2H, m), 2.95 (2H, t, J = 7.6 Hz), 3.75 (3H, s), 6.22 (1H, s), 6.58 to 7.67 (19H, m)

### Example 7

### N-[4-(Benzhydryloxy)-3-butylphenyl]-N'-(3,4-dimethoxyphenyl)urea

To a solution (6 mL) of 1-[5-amino-2-(benzhydryloxy)phenyl]butan-1-one (300 mg, 0.868 mmole) and diisopropylethyl amine (0.173 mL, 1.04 mmole) in acetonitrile was added N,N-disuccinimidyl carbamate (300 mg, 0.868 mmole) at 0°C, and the mixture was stirred for 1 hour at 0°C. To this solution were added diisopropylethyl amine (0.173 mL, 1.04 mmole) and 3.4-dimethoxyaniline (266 mg, 1.74 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), which was recrystallized from hexane and ethyl acetate, to obtain 337 mg (74.1%) of the titled compound as a solid.
¹H-NMR (CDCl₃) δ; 0.76 (3H, t, J = 7.2 Hz), 1.54 to 1.65 (2H, m), 2.94 (2H, t, J = 7.6 Hz), 3.85 (6H, s), 6.23 (1H, s), 6.71 to 7.67 (18H, m)

### Example 8

### N-[3-Acetyl-4-(benzhydryloxy)phenyl]-N'-(4-methoxyphenyl)urea

### (1) 1-[2-(benzhydryloxy)-5-nitrophenyl]ethanone

To a solution (30 mL) of 1-(2-hydroxy-5-nitrophenyl)ethanone (1.50 g, 8.28 mmole) in DMF was added sodium hydride (60%, 331 mg, 8.28 mmole) under ice-cooling, and the mixture was stirred at room temperature for 1 hour. Bromodiphenylmethane (2.46 g, 9.94 mmole) was added to the mixture, and the mixture was stirred for 3 hours at 70°C. The reaction solution was poured into ice-water, and the mixture was extracted with ethyl acetate. The extracted solution was washed with water, was dried with anhydrous magnesium sulfate, and the solvent was distilled off under reduced pressure. To the residue was added diisopropylether, to obtain 1.87 g (64.9%) of the titled compound as a solid.
¹H-NMR (CDCl₃) δ; 2.63 (3H, s), 6.42 (1H, s), 6.99 (1H, d, J = 9.2 Hz), 7.31 to 7.41 (10H, m), 8.18 (1H, dd, J = 9.2, 3.0 Hz), 8.60 (1H, d, J = 3.0 Hz)

### (2) 1-[5-Amino-2-(benzhydryloxy)phenyl]ethanone

To a solution (200 mL) of 1-(2-hydroxy-5-nitrophenyl)ethanone (1.80 g, 5.18 mmole) in ethyl acetate was added 5% iridium-carbon (180 mg), and the mixture was stirred for 12 hours under hydrogen atmosphere. The insolubles were filtered off, and the filtrate was concentrated, to obtain 1.45 g (88.4%) of the titled compound as a solid.
¹H-NMR (CDCl₃) δ; 2.57 (3H, s), 3.45 (2H, bs), 6.17 (1H, s), 6.64 (1 H, dd, J = 3.0, 8.7 Hz), 6.69 (1H, d, J = 8.7 Hz), 7.03 (1H, d, J = 3.0 Hz), 7.26 to 7.40 (10H, m)

### (3) N-[3-Acetyl-4-(benzhydryloxy)phenyl]-N'-(4-methoxyphenyl)urea

To a solution (3 mL) of 1-[5-amino-2-(benzhydryloxy)phenyl]ethanone (300 mg, 0.945 mmole) in THF was added 4-methoxyphenyl isocyanate (0.147 mL, 1.13 mmole) under ice-cooling, the mixture was stirred at 0°C for 1 hour and at room temperature for 2 hours, and the solvent was distilled off under reduced pressure. The residue was recrystallized from hexane and ethyl acetate, to obtain 421 mg (95.7%) of the titled compound.
¹H-NMR (CDCl₃) δ; 2.61 (3H, s), 3.78 (3H, s), 6.26 (1H, s), 6.77 to 7.75 (19H, m)

### Example 9

### N-[3-Acetyl-4-(benzhydryloxy)phenyl]-N'-(3-methoxyphenyl)urea

To a solution (3 mL) of 1-[5-amino-2-(benzhydryloxy)phenyl]ethanone (300 mg, 0.945 mmole) in THF was added 3-methoxyphenyl isocyanate (0.147 mL, 1.13 mmole) under ice-cooling, the mixture was stirred at 0°C for 1 hour and at room temperature for 2 hours, and the solvent was distilled off under reduced pressure. The residue was recrystallized from hexane and ethyl acetate, to obtain 350 mg (79.4%) of the titled compound.
¹H-NMR (CDCl₃) δ; 2.65 (3H, s), 3.75 (3H, s), 6.27 (1H, s), 6.57 to 7.86 (19H, m)

### Example 10

### N-[3-Acetyl-4-(benzhydryloxy)phenyl]-N'-(3,4-dimethoxyphenyl)urea

To a solution (3 mL) of 1-[5-amino-2-(benzhydryloxy)phenyl]ethanone (300 mg, 0.945 mmole) in THF was added 3,4-dimethoxyphenyl isocyanate (0.168 mL, 1.13 mmole) under ice-cooling, the mixture was stirred at 0°C for 1 hour and at room temperature for 2 hours, and the solvent was distilled off under reduced pressure. The residue was purified by silicagel column chromatography (hexane:ethyl acetate = 1:1), which was recrystallized from hexane and ethyl acetate, to obtain 400 mg (85.3%) of the titled compound as a solid.
H-NMR (CDCl₃) δ; 2.63 (3H, s), 3.86 (6H, s), 6.26 (1H, s), 6.68 to 7.81 (18H, m)

### Example 11

### N-[4-(4-tert-Butylbenzyloxy)-3-propionylphenyl]-N'-(3,4-dimethoxyphenyl)urea

### (1) 1-[2-(4-tert-Butylbenzyloxy)-5-nitrophenyl]propan-1-one

A solution of 1-(2-hydroxy-5-nitrophenyl)propan-1-one (500 mg, 2.56 mmole), potassium carbonate (424 mg, 3.07 mmole), 4-tert-butylbenzyl chloride (0.594 mL, 3.07 mmole) and DMF (5 mL) was stirred at 70°C for 12 hours, was poured into water, and was extracted with ethyl acetate. The solvent was distilled off under reduced pressure, to obtain 756 mg (86.5%) of the titled compound as a solid.
¹H-NMR (CDCl₃) δ; 1.15 (3H, t, J = 7.0 Hz), 1.34 (9H, s), 2.99 (2H, q, J = 7.0 Hz), 5.25 (2H, s), 7.11 (1 H, d, J = 9.2 Hz), 7.34 (2H, d, J = 8.4 Hz), 7.44 (2H, d, J = 8.4 Hz), 8.29 (1H, dd, J = 9.2, 2.8 Hz), 8.56 (1H, d, J = 2.8 Hz)

### (2) 1-[5-Amino-2-(4-tert-butylbenzyloxy)phenyl]propan-1-one

A mixed solution of 1-[2-(4-tert-butylbenzyloxy)-5-nitrophenyl]propan-1-one (700 mg, 2.05 mmole), 5% iridium-carbon (70 mg) and ethyl acetate (7 mL) was stirred for 8 hours at room temperature under hydrogen atmosphere. The insolubles were filtered off, and the solvent was distilled off under reduced pressure, to obtain 583 mg (95.0%) of the titled compound as a solid.
¹H-NMR (CDCl₃) δ; 1.11 (3H, t, J = 7.0 Hz), 1.33 (9H, s), 2.99 (2H, q, J = 7.0 Hz), 5.04 (2H, s), 6.76 (1 H, dd, J = 8.8, 3.0 Hz), 6.86 (1H, d, J = 8.8 Hz), 7.02 (1H, d, J = 3.0 Hz), 7.32 (2H, d, J = 8.4 Hz), 7.40 (2H, d, J = 8.4 Hz)

### (3) N-[4-(4-tert-Butylbenzyloxy)-3-propionylphenyl]-N'-(3,4-dimethoxyphenyl)urea

To a solution (2 mL) of 1-[5-amino-2-(4-tert-butylbenzyloxy)phenyl]propan-1-one (200 mg, 0.669 mmole) in THF was added 3,4-dimethoxyphenyl isocyanate (0.119 mL, 0.803 mmole) under ice-cooling, the mixture was stirred at 0°C for 1 hour and at room temperature for 12 hours, and the solvent was distilled off under reduced pressure. The residue was recrystallized from hexane and ethyl acetate, to obtain 293 mg (89.3%) of the titled compound.
¹H-NMR (CDCl₃) δ; 1.11 (3H, t, J = 7.2 Hz), 1.33 (9H, s), 3.02 (2H, q, J = 7.2 Hz), 3.85 (6H, s), 5.09 (2H, s), 6.75 to 7.83 (12 H, m)

### Example 12

### N-[4-(4-tert-Butylbenzyloxy)-3-propionylphenyl]-N'-(4-methoxyphenyl)urea

To a solution (3 mL) of 1-[5-amino-2-(4-tert-butylbenzyloxy)phenyl]propan-1-one (150 mg, 0.501 mmole) in THF was added 4-methoxyphenyl isocyanate (0.0776 mL, 0.599 mmole) under ice-cooling, the mixture was stirred at 0°C for 1 hour and at room temperature for 4 hours, and the solvent was distilled off under reduced pressure. The residue was recrystallized from hexane and ethyl acetate, to obtain 190 mg (82.3%) of the titled compound.
¹H-NMR (CDCl₃) δ; 1.10 (3H, t, J = 7.2 Hz), 1.33 (9H, s), 3.00 (2H, q, J = 7.2 Hz), 3.78 (3H, s), 5.07 (2H, s), 6.83 to 7.82 (13 H, m)

### Example 13

### N-[4-(4-tert-Butylbenzyloxy)-3-propionylphenyl]-N'-(3-methoxyphenyl)urea

To a solution (3 mL) of 1-[5-amino-2-(4-tert-butylbenzyloxy)phenyl]propan-1-one (150 mg, 0.501 mmole) in THF was added 3-methoxyphenyl isocyanate (0.0785 mL, 0.599 mmole) under ice-cooling, the mixture was stirred at 0°C for 1 hour and at room temperature for 12 hours, and the solvent was distilled off under reduced pressure. The residue was recrystallized from hexane and ethyl acetate, to obtain 193 mg (83.5%) of the titled compound.
¹H-NMR (CDCl₃) δ; 1.12 (3H, t, J = 7.4 Hz), 1.33 (9H, s), 3.02 (2H, q, J = 7.4 Hz), 3.76 (3H, s), 5.07 (2H, s), 6.60 to 7.82 (13 H, m)

### Example 14

### N-(3,4-Dimethoxyphenyl)-N'-[4-(4-isopropylbenzyloxy)-3-propionylphenyl]urea

### (1) 1-[2-(4-Isopropylbenzyloxy)-5-nitrophenyl]propan-1-one

A solution of 1-(2-hydroxy-5-nitrophenyl)propan-1-one (500 mg, 2.56 mmole), 4-isopropylbenzyl alcohol (587 mg, 3.84 mmole), 1,1-(azodicarbonyl)dipiperidine (967 mg, 3.84 mmole) and triphenylphosphine (1.01 g, 3.84 mmole) in toluene (5 mL), was stirred for 5 hours at 80°C, to the reaction solution were added 4-isopropylbenzyl alcohol (587 mg, 3.84 mmole), 1,1-(azodicarbonyl)dipiperidine (967 mg, 3.84 mmole) and triphenylphosphine (1.01 g, 3.84 mmole), and the mixture was stirred for 12 hours at 80°C. The reaction solution was poured into water, and the mixture was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 10:1), to obtain 480 mg (57.3%) of the titled compound as a solid.
¹H-NMR (CDCl₃) δ; 1.14 (3H, t, J = 7.4 Hz), 1.26 (6H, d, J = 7.0 Hz), 2.91 to 3.04 (3H, m), 5.24 (2H, s), 7.11 (1H, d, J = 9.2 Hz), 7.27 (2H, d, J = 8.4 Hz), 7.34 (2H, d, J = 8.4 Hz), 8.30 (1H, dd, J = 2.8, 9.2 Hz), 8.56 (1H, d, J = 2.8 Hz)

### (2) 1-[5-Amino-2-(4-isopropylbenzyloxy)phenyl]propan-1-one

A mixture of 1-[2-(4-isopropylbenzyloxy)-5-nitrophenyl]propan-1-one (470 mg, 1.44 mmole), 5% iridium-carbon (50 mg) and ethyl acetate (10 mL) was stirred for 12 hours under hydrogen atmosphere at room temperature, the insolubles were filtered off, and the filtrate was concentrated, to obtain 400 mg (97.8%) of the titled compound as oil.
¹H-NMR (CDCl₃) δ; 1.10 (3H, t, J = 7.0 Hz), 1.25 (6H, d, J = 7.0 Hz), 2.85 to 3.03 (3H, m), 5.03 (2H, s), 6.75 (1H, dd, J = 2.4, 8.8 Hz), 6.86 (1H, d, J = 8.8 Hz), 7.02 (1H, d, J = 2.4 Hz), 7.23 (2H, dd, J = 8.4 Hz), 7.32 (2H, d, J = 8.4 Hz)

### (3) N-(3,4-Dimethoxyphenyl)-N'-[4-(4-isopropylbenzyloxy)-3-propionylphenyl]urea

To a solution (4 mL) of 1-[5-amino-2-(4-isopropylbenzyloxy)phenyl]propan-1-one (200 mg, 0.701 mmole) in THF was added 3,4-dimethoxyphenyl isocyanate (0.125 mL, 0.842 mmole) under ice-cooling, the mixture was stirred at 0°C for 1 hour and at room temperature for 12 hours, and the solvent was distilled off under reduced pressure. The residue was recrystallized from hexane and ethyl acetate, to obtain 301 mg (89.8%) of the titled compound.
¹H-NMR (CDCl₃) δ; 1.10 (3H, t, J = 7.4 Hz), 1.25 (6H, d, J = 7.0 Hz), 2.89 to 3.06 (3H, m), 3.86 (6H, s), 5.06 (2H, s), 6.77 to 7.83 (12 H, m)

### Example 15

### N-(4-Methoxyphenyl)-N'-[4-(4-isopropylbenzyloxy)-3-propionylphenyl]urea

To a solution (1 mL) of 1-[5-amino-2-(4-isopropylbenzyloxy)phenyl]propan-1-one (100 mg, 0.350 mmole) in THF was added 4-methoxyphenyl isocyanate (0.0544 mL, 0.420 mmole) under ice-cooling, the mixture was stirred at 0°C for 1 hour and at room temperature for 2 hours, and the solvent was distilled off under reduced pressure. The residue was recrystallized from hexane and ethyl acetate, to obtain 123 mg (78.8%) of the titled compound.
¹H-NMR (CDCl₃) δ; 1.09 (3H, t, J = 7.5 Hz), 1.26 (6H, d, J = 6.9 Hz), 2.90 to 3.03 (3H, m), 3.78 (3H, s), 5.07 (2H, s), 6.79 to 7.81 (13 H, m)

### Example 16

### N-(3-Methoxyphenyl)-N'-[4-(4-isopropylbenzyloxy)-3-propionylphenyl]urea

To a solution (1 mL) of 1-[5-amino-2-(4-isopropylbenzyloxy)phenyl]propan-1-one (100 mg, 0.350 mmole) in THF was added 3-methoxyphenyl isocyanate (0.055 mL, 0.420 mmole) under ice-cooling, the mixture was stirred at 0°C for 1 hour and at room temperature for 2 hours, and the solvent was distilled off under reduced pressure. The residue was recrystallized from hexane and ethyl acetate, to obtain 150 mg (96.2%) of the titled compound.
¹H-NMR (CDCl₃) δ; 1.09 (3H, t, J = 7.2 Hz), 1.26 (6H, d, J = 6.9 Hz), 2.90 to 3.03 (3H, m), 3.79 (3H, s), 5.07 (2H, s), 6.79 to 7.81 (13 H, m)

### Example 17

### N-{4-[(5-Chloropyridin-3-yl)oxy]-3-propionylphenyl}-N'-(3,4-dimethoxyphenyl)urea

### (1) 1-{2-[(5-Chloropyridin-3-yl)oxy]-5-nitrophenyl}propan-1-one

A mixed solution of 1-(2-chloro-5-nitrophenyl)propan-1-one (500 mg, 2.34 mmole), 3-chloro-5-hydroxypyridine (302 mg, 2.34 mmole), potassium carbonate (323 mg, 2.34 mmole) in DMF (5 mL) was stirred for 30 minutes at 70°C, was poured into water, and was extracted with ethyl acetate. The extracted solution was washed with water, and was dried with anhydrous magnesium sulfate. The solvent was distilled off under reduced pressure, and the residue was purified by silicagel column chromatography (hexane:ethyl acetate = 10:1), to obtain 360 mg (50.1%) of the titled compound as a solid.
¹H-NMR (CDCl₃) δ; 1.22 (3H, t, J = 7.4 Hz), 3.04 (2H, q, J = 7.4 Hz), 6.98 (1H, d, J = 9.2 Hz), 7.44 to 7.46 (1H, m), 8.29 (1H, dd, J = 9.2, 3.0 Hz), 8.30 (1H, d, J = 3.0 Hz), 8.52 (1H, d, J = 2.0 Hz), 8.69 (1H, d, J = 2.6 Hz)

### (2) 1-{5-Amino-2-[(5-chloropyridin-3-yl)oxy]phenyl}propan-1-one

To a mixed solution of 1-{2-[(5-chloropyridin-3-yl)oxy]-5-nitrophenyl}propan-1-one (350 mg, 0.978 mmole) in methanol (14 mL) and water (3.5 mL) was added sodium hydrosulfite (579 mg, 3.33 mmole), and the mixture was stirred for 40 minutes at 80°C. Sodium hydrosulfite (1.16 g, 6.66 mmole) was added to the mixture, the mixture was stirred for 80 minutes at 80°C, and the solvent was distilled off under reduced pressure. The residue was extracted with ethyl acetate, was dried with anhydrous magnesium sulfate, and the solvent was distilled off under reduced pressure to obtain 108 mg (40.0%) of the titled compound as oil.
¹H-NMR (CDCl₃) δ; 1.11 (3H, t, J = 6.8 Hz), 2.86 (2H, q, J = 6.8 Hz), 6.83 to 6.84 (2H, m), 7.07 to 7.09 (1H, m), 7.15 to 7.17 (1H, m), 8.24 (1H, d, J = 2.6 Hz), 8.27 (1H, d, J = 2.6 Hz)

### (3) N-{4-[(5-Chloropyridin-3-yl)oxy]-3-propionylphenyl}-N'-(3,4-dimethoxyphenyl)urea

To a solution (1 mL) of 1-{5-amino-2-[(5-chloropyridin-3-yl)oxy]phenyl}propan-1-one (108 mg, 0.391 mmole) in THF was added 3,4-dimethoxyphenyl isocyanate (0.698 mL, 0.469 mmole) under ice-cooling, the mixture was stirred at 0°C for 1 hour and at room temperature for 2 hours, and the solvent was distilled off under reduced pressure. The residue was recrystallized from hexane and ethyl acetate, to obtain 130 mg (73.0%) of the titled compound.
¹H-NMR (CDCl₃) δ; 1.12 (3H, t, J = 7.0 Hz), 2.92 (2H, q, J = 7.0 Hz), 3.88 (6H, s), 6.76 to 7.27 (7H, m), 7.57 (1 H, d, J = 2.6 Hz), 7.83 (1H, dd, J = 2.6, 8.6 Hz), 8.26 (1H, d, J = 2.6 Hz), 8.33 (1H, d, J = 2.2 Hz)

### Example 18

### N-{4-[(5-Chloropyridin-3-yl)oxy]-3-propionylphenyl}-N'-(4-methoxyphenyl)urea

To a solution (1 mL) of 1-(5-amino-2-[(5-chloropyridin-3-yl)oxy]phenyl}propan-1-one (110 mg, 0.398 mmole) in THF was added 4-methoxyphenyl isocyanate (0.619 mL, 0.478 mmole) under ice-cooling, the mixture was stirred at 0°C for 1 hour and at room temperature for 4 hours, and the solvent was distilled off under reduced pressure. The residue was recrystallized from hexane and ethyl acetate, to obtain 114 mg (67.5%) of the titled compound.
¹H-NMR (CDCl₃) δ; 1.11 (3H, t, J = 7.4 Hz), 2.91 (2H, q, J = 7.4 Hz), 3.82 (3H, s), 6.85 to 7.28 (8H, m), 7.55 (1 H, d, J = 3.0 Hz), 7.82 (1H, dd, J = 3.0, 8.8 Hz), 8.26 (1H, d, J = 2.6 Hz), 8.33 (1H, d, J = 1.8 Hz)

### Example 19

### N-{4-[(5-Chloropyridin-3-yl)oxy]-3-propionylphenyl}-N'-(3-methoxyphenyl)urea

To a solution (1 mL) of 2-{5-amino-2-[(5-chloropyridin-3-yl)oxy]phenyl}propan-1-one (110 mg, 0.398 mmole) in THF was added 3-methoxyphenyl isocyanate (0.626 mL, 0.478 mmole) under ice-cooling, the mixture was stirred at 0°C for 1 hour and at room temperature for 4 hours, and the solvent was distilled off under reduced pressure. The residue was purified by silicagel column chromatography (hexane:ethyl acetate = 1:1), which was recrystallized from hexane and ethyl acetate, to obtain 88.0 mg (52.1%) of the titled compound as a solid.
¹H-NMR (CDCl₃) δ; 1.10 (3H, t, J = 7.2 Hz), 2.90 (2H, q, J = 7.2 Hz), 3.73 (3H, s), 6.58 to 7.77 (10H, m), 8.22 (1 H, d, J = 2.6 Hz), 8.33 (1H, d, J = 1.8 Hz)

### Example 20

### 2-Benzhydryloxy-N-(tert-butyl)-5-({[(4-methoxyphenyl)amino]carbonyl}amino)benzamide

### (1) 2-Benzhydryloxy-N-(tert-butyl)-5-nitrobenzamide

To a solution (10 mL) of N-(tert-butyl)-2-hydroxy-5-nitrobenzamide (500 mg, 2.10 mmole) in DMF was added sodium hydride (60%, 168 mg, 4.20 mmole) under ice-cooling, and the mixture was stirred for 30 minutes at room temperature. Bromodiphenylmethane (623 mg, 2.52 mmole) was added thereto, and the mixture was stirred for 3.5 hours at 70°C. Bromodiphenylmethane (623 mg, 2.52 mmole) was further added to the mixture, and the mixture was stirred at 70°C for 12 hours. The mixture was poured into ice-water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 9:1), to obtain 530 mg (62.4%) of the titled compound as oil.
¹H-NMR (CDCl₃) δ; 1.19 (9H, s), 6.45 (1H, s), 6.93 (1H, d, J = 9.2 Hz), 7.35 to 7.45 (10H, m), 7.61 (1H, s), 8.08 to 8.15 (1H, m), 9.06 to 9.08 (1H, m)

### (2) 5-Amino-2-benzhydryloxy-N-(tert-butyl)benzamide

A mixed solution of 2-benzhydryloxy-N-(tert-butyl)-5-nitrobenzamide (800 mg, 1.98 mmole), 5% iridium-carbon (100 mg) and ethyl acetate (20 mL) was stirred for 12 hours under hydrogen atmosphere. The insolubles were filtered off, and the filtrate was concentrated, to obtain 667 mg (90.0%) of the titled compound as oil.
¹H-NMR (CDCl₃) δ; 1.16 (9H, s), 6.18 (1H, s), 6.52 to 6.64 (2H, m), 7.29 to 7.37 (10H, m), 7.50 (1H, d, J = 3.0 Hz), 7.91 (1H, bs)

### (3) 2-Benzhydryloxy-N-(tert-butyl)-5-({[(4-methoxyphenyl)amino]carbonyl}amino)benzamide

To a solution (5 mL) of 5-amino-2-benzhydryloxy-N-(tert-butyl)benzamide (200 mg, 0.534 mmole) and diisopropylethyl amine (0.107 mL, 0.640 mmole) in acetonitrile was added N,N-disuccinimidyl carbamate (164 mg, 0.640 mmole) at 0°C, and the mixture was stirred for 1 hour at 0°C. This solution was added to a solution of diisopropylethyl amine (0.107 mL, 0.640 mmole) and p-anicidine (142 mg, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), which was recrystallized from hexane and ethyl acetate, to obtain 121 mg (43.4%) of the titled compound as a solid.
¹H-NMR (CDCl₃) δ; 1.16 (9H, s), 3.77 (3H, s), 6.26 (1H, s), 6.54 (1H, s), 6.76 to 7.73 (18H, m), 7.88 (1H, s)

### Example 21

### 2-Benzhydryloxy-N-(tert-butyl)-5-({[(3,4-dimethoxyphenyl)amino]carbonyl}amino)benzamide

To a solution (3 mL) of 5-amino-2-benzhydryloxy-N-(tert-butyl)benzamide (300 mg, 0.801 mmole) in THF was added 3,4-dimethoxyphenyl isocyanate (0.143 mL, 0.961 mmole) under ice-cooling, the mixture was stirred at 0°C for 1 hour and at room temperature for 12 hours, and the solvent was distilled off under reduced pressure. The residue was purified by silicagel column chromatography (hexane:ethyl acetate = 1:1), to obtain the titled compound as a solid. This compound was recrystallized from hexane and ethyl acetate.
¹H-NMR (CDCl₃) δ; 1.16 (9H, s), 3.82 (3H, s), 3.83 (3H, s), 6.27 (1H, s), 6.75 to 7.92 (19H, m)

### Example 22

### Methyl 5-(anilinocarbonylamino)-2-benzhydryloxybenzoate

### (1) Methyl 2-benzhydryloxy-5-nitrobenzoate

To a solution (200 mL) of methyl 2-hydroxy-5-nitrobenzoate (10.0 g, 50.7 mmole) in DMF was added sodium hydride (60%, 1.93 g, 48.2 mmole) under ice-cooling, the mixture was stirred for 30 minutes at room temperature, and bromodiphenylmethane (15.0 g, 60.9 mmole) was added thereto. The mixture was stirred at 70°C for 12 hours, was poured into ice-water containing ammonium chloride (2.71 g, 50.7 mmole), and was extracted with ethyl acetate. The extracted solution was washed with water, was dried with anhydrous magnesium sulfate, and the solvent was distilled off under reduced pressure. The residue was recrystallized from ethyl acetate and hexane, to obtain 11.3 g (61.4%) of the titled compound.
¹H-NMR (CDCl₃) δ; 3.99 (3H, s), 6.41 (1H, s), 7.02 (1H, d, J = 9.6 Hz), 7.24 to 7.55 (10H, m), 8.18 (1H, dd, J = 9.6, 3.0 Hz), 8.72 (1H, d, J = 3.0 Hz)

### (2) Methyl 5-amino-2-benzhydryloxybenzoate

A mixed solution of methyl 2-benzhydryloxy-5-nitrobenzoate (4.00 g, 11.0 mmole), 5% iridium-carbon (400 mg) and ethyl acetate (80 mL) was stirred for 4 hours under hydrogen atmosphere. The insolubles were removed, and the filtrate was concentrated, to obtain 3.60 g (98.1%) of the titled compound as oil.
¹H-NMR (CDCl₃) δ; 3.45 (2H, bs), 3.84 (3H, s), 6.13 (1H, s), 6.61 (1H, dd, J = 2.4, 8.8 Hz), 6.69 (1H, d, J = 8.8 Hz), 7.13 (1H, d, J = 2.4 Hz), 7.19 to 7.51 (10H, m)

### (3) Methyl 5-(anilinocarbonylamino)-2-benzhydryloxybenzoate

A mixed solution of methyl 5-amino-2-benzhydryloxybenzoate (3.60 g, 10.8 mmole), phenyl isocyanate (1.30 mL, 12.2 mmole) and THF (70 mL) was stirred at room temperature for 2 hours, was poured into water, and was extracted with ethyl acetate. The extracted solution was washed with water, was dried with anhydrous magnesium sulfate, and the solvent was distilled off under reduced pressure. The residue was recrystallized from ethyl acetate and diisopropylether, to obtain 2.77 g (56.6%) of the titled compound.
¹H-NMR (CDCl₃) δ; 3.84 (3H, s), 6.16 (1H, s), 6.76 (1H, d, J = 9.0 Hz), 6.99 to 7.49 (18H, m), 7.60 (1H, d, J = 2.6 Hz)

### Example 23

### Methyl 2-(benzhydryloxy)-5-({[(4-methoxyphenyl)amino]carbonyl}amino)benzoate

A mixed solution of methyl 5-amino-2-benzhydryloxybenzoate (200 mg, 0.600 mmole), 4-methoxyphenyl isocyanate (0.0876 mL, 0.678 mmole) and THF (2 mL) was stirred at room temperature for 2 hours, was poured into water, and was extracted with ethyl acetate. The extracted solution was washed with water, was dried with anhydrous magnesium sulfate, and the solvent was distilled off under reduced pressure. The residue was recrystallized from ethyl acetate and hexane, to obtain 200 mg (68.9%) of the titled compound.
¹H-NMR (CDCl₃) δ; 3.76 (3H, s), 3.86 (3H, s), 6.20 (1H, s), 6.55 (1H, s), 6.63 (1H, s), 6.80 to 7.60 (17H, m)

### Example 24

### Methyl 2-(benzhydryloxy)-5-({[(3-methoxyphenyl)amino]carbonyl}amino)benzoate

To a solution (13 mL) of methyl 5-amino-2-benzhydryloxybenzoate (500 mg, 1.50 mmole) and diisopropylethyl amine (0.308 mL, 1.80 mmole) in acetonitrile was added N,N-disuccinimidyl carbamate (461 mg, 1.80 mmole) at 0°C, and the mixture was stirred for 1 hour at 0°C. To this solution was added diisopropylethyl amine (0.308 mL, 1.80 mmole) and m-anicidine (0.202 mL, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 2:1), which was recrystallized from ether and ethyl acetate, to obtain 408 mg (56.4%) of the titled compound as a solid.
¹H-NMR (CDCl₃) δ; 3.71 (3H, s), 3.86 (3H, s), 6.18 (1H, s), 6.56 to 7.61 (19H, m)

### Example 25

### Methyl 2-(benzhydryloxy)-5-({[(4-ethoxyphenyl)amino]carbonyl}amino)benzoate

To a solution (10 mL) of methyl 5-amino-2-benzhydryloxybenzoate (333 mg, 1.00 mmole) and diisopropylethyl amine (0.205 mL, 1.20 mmole) in acetonitrile was added N,N-disuccinimidyl carbamate (307 mg, 1.20 mmole) at 0°C, and the mixture was stirred for 1 hour at 0°C. To this solution were added diisopropylethyl amine (0.205 mL, 1.20 mmole) and p-fenetidine (0.232 mL, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 2:1), which was recrystallized from hexane and ethyl acetate, to obtain 400 mg (80.5%) of the titled compound as a solid.
¹H-NMR (CDCl₃) δ; 1.40 (3H, t, J = 7.0 Hz), 3.87 (3H, s), 3.99 (2H, q, J = 7.0 Hz), 6.21 (1H, s), 6.41 (1H, s), 6.51 (1H, s), 6.81 to 7.60 (17H, m)

### Example 26

### Methyl 2-(benzhydryloxy)-5-({[(3,4-dimethoxyphenyl)amino]carbonyl}amino)benzoate

To a solution (13 mL) of methyl 5-amino-2-benzhydryloxybenzoate (500 mg, 1.50 mmole) and diisopropylethyl amine (0.308 mL, 1.80 mmole) in acetonitrile was added N,N-disuccinimidyl carbamate (461 mg, 1.80 mmole) at 0°C, and the mixture was stirred for 1 hour at 0°C. To this solution were added diisopropylethyl amine (0.308 mL, 1.80 mmole) and 3,4-dimethoxyaniline (275 mg, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), which was recrystallized from hexane and ethyl acetate, to obtain 352 mg (45.8%) of the titled compound as a solid.
¹H-NMR (CDCl₃) δ; 3.81 (6H, s), 3.86 (3H, s), 6.20 (1H, s), 6.65 to 7.61 (18H, m)

### Example 27

### Methyl 2-(benzhydryloxy)-5-({[(3-ethoxyphenyl)amino]carbonyl}amino)benzoate

To a solution (10 mL) of methyl 5-amino-2-benzhydryloxybenzoate (333 mg, 1.00 mmole) and diisopropylethyl amine (0.205 mL, 1.20 mmole) in acetonitrile was added N,N-disuccinimidyl carbamate (307 mg, 1.20 mmole) at 0°C, and the mixture was stirred for 1 hour at 0°C. To this solution were added diisopropylethyl amine (0.205 mL, 1.20 mmole) and m-fenetidine (0.239 mL, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), which was recrystallized from hexane and ethyl acetate, to obtain 265 mg (53.4%) of the titled compound as a solid.
¹H-NMR (CDCl₃) δ; 1.35 (3H, t, J = 7.0 Hz), 3.86 (3H, s), 3.95 (2H, q, J = 7.0 Hz), 6.20 (1H, s), 6.58 to 7.62 (19H, m)

### Example 28

### Methyl 2-(benzhydryloxy)-5-({[(3-nitrophenyl)amino]carbonyl}amino)benzoate

To a solution (10 mL) of methyl 5-amino-2-benzhydryloxybenzoate (333 mg, 1.00 mmole) and diisopropylethyl amine (0.205 mL, 1.20 mmole) in acetonitrile was added N,N-disuccinimidyl carbamate (307 mg, 1.20 mmole) at 0°C, and the mixture was stirred for 1 hour at 0°C. To this solution were added diisopropylethyl amine (0.205 mL, 1.20 mmole) and m-nitroaniline (0.239 mL, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), which was recrystallized from hexane and ethyl acetate, to obtain 310 mg (64.3%) of the titled compound as a solid.
¹H-NMR (CDCl₃) δ; 3.83 (3H, s), 6.12 (1H, s), 6.77 (1H, d, J = 9.2 Hz), 7.16 to 7.98 (18H, m)

### Example 29

### Methyl 2-(benzhydryloxy)-5-[(morpholin-4-ylcarbonyl)amino]benzoate

To a solution (10 mL) of methyl 5-amino-2-benzhydryloxybenzoate (333 mg, 1.00 mmole) and diisopropylethyl amine (0.205 mL, 1.20 mmole) in acetonitrile was added N,N-disuccinimidyl carbamate (307 mg, 1.20 mmole) at 0°C, and the mixture was stirred for 1 hour at 0°C. To this solution were added diisopropylethyl amine (0.205 mL, 1.20 mmole) and morpholine (0.105 mL, 1.20 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (ethyl acetate), which was recrystallized from hexane and ethyl acetate, to obtain 268 mg (60.0%) of the titled compound as a solid.
¹H-NMR (CDCl₃) δ; 3.42 (4H, t, J = 4.4 Hz), 3.71 (4H, t, J = 4.4 Hz), 3.88 (3H, s), 6.24 (1H, s), 6.27 (1H, s), 6.84 (1H, d, J = 9.2 Hz), 7.19 to 7.52 (11H, m), 7.65 (1H, d, J = 3.0 Hz)

### Example 30

### Methyl 2-(benzhydryloxy)-5-{[(1, 3-benzodioxol-5-ylamino)carbonyl]amino}benzoate

To a solution (10 mL) of methyl 5-amino-2-benzhydryloxybenzoate (333 mg, 1.00 mmole) and diisopropylethyl amine (0.205 mL, 1.20 mmole) in acetonitrile was added N,N-disuccinimidyl carbamate (307 mg, 1.20 mmole) at 0°C, and the mixture was stirred for 1 hour at 0°C. To this solution were added diisopropylethyl amine (0.205 mL, 1.20 mmole) and 3,4-methylenedioxyaniline (165 mg, 1.20 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), which was recrystallized from hexane and ethyl acetate, to obtain 354 mg (71.2%) of the titled compound as a solid.
¹H-NMR (CDCl₃) δ; 3.82 (3H, s), 5.79 (2H, s), 6.15 (1H, s), 6.48 to 7.56 (18H, m)

### Example 31

### Methyl 2-(benzhydryloxy)-5-({[(3-acetylphenyl)amino]carbonyl}amino)benzoate

To a solution (10 mL) of methyl 5-amino-2-benzhydryloxybenzoate (333 mg, 1.00 mmole) and diisopropylethyl amine (0.205 mL, 1.20 mmole) in acetonitrile was added N,N-disuccinimidyl carbamate (307 mg, 1.20 mmole) at 0°C, and the mixture was stirred for 1 hour at 0°C. To this solution were added diisopropylethyl amine (0.205 mL, 1.20 mmole) and 3'-aminoacetophenone (243 mg, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (ethyl acetate), which was recrystallized from ethyl acetate, to obtain 199 mg (40.2%) of the titled compound as a solid.
¹H-NMR (CDCl₃) δ; 2.59 (3H, s), 3.90 (3H, s), 6.26 (1H, s), 6.85 (1H, d, J = 8.8 Hz), 7.19 to 7.95 (16H, m), 8.07 (1H, s), 8.30 (1H, s)

### Example 32

### Methyl 2-(benzhydryloxy)-5-{[(pyridin-4-ylamino)carbonyl]amino}benzoate

To a solution (10 mL) of methyl 5-amino-2-benzhydryloxybenzoate (333 mg, 1.00 mmole) and diisopropylethyl amine (0.205 mL, 1.20 mmole) in acetonitrile was added N,N-disuccinimidyl carbamate (307 mg, 1.20 mmole) at 0°C, and the mixture was stirred for 1 hour at 0°C. To this solution were added diisopropylethyl amine (0.205 mL, 1.20 mmole) and 4-aminopyridine (169 mg, 1.80 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (ethyl acetate:THF = 4:1), which was recrystallized from ethyl acetate and hexane, to obtain 106 mg (23.4%) of the titled compound as a solid.
¹H-NMR (CDCl₃) δ; 3.88 (3H, s), 6.21 (1H, s), 6.84 (1H, d, J = 9.2 Hz), 7.22 to 7.50 (14H, m), 7.64 (1H, bs), 7.70 (1H, d, J = 2.8 Hz), 8.30 (2H, d, J = 6.0 Hz)

### Example 33

### 5-(Anilinocarbonylamino)-2-benzhydryloxy-N-(tert-butyl)benzamide

### (1) 5-(Anilinocarbonylamino)-2-benzhydryloxy benzoic acid

A mixed solution of methyl 5-(anilinocarbonylamino)-2-benzhydryloxybenzoate (1.60 g, 3.54 mmole), 1N aqueous solution of sodium hydroxide (7 mL) and methanol (70 mL) was heated to reflux for 12 hours, was poured into water, and was washed with ethyl acetate. The aqueous layer was neutralized with 1N hydrochloric acid, and was extracted with ethyl acetate. The extracted solution was washed with water, was dried with anhydrous magnesium sulfate, and the solvent was distilled off under reduced pressure. To the residue was added diisopropylether, and the mixture was filtered to obtain 939 mg (60.6%) of the titled compound.
¹H-NMR (CDCl₃) δ; 6.39 (1H, s), 6.88 to 7.02 (2H, m), 7.22 to 7.46 (14H, m), 7.72 (1H, d, J = 2.6 Hz), 7.93 (1H, dd, J = 2.6, 9.0 Hz), 8.01 (1H, s), 8.26 (1H, s)

### (2) 5-(Anilinocarbonylamino)-2-benzhydryloxy-N-(tert-butyl)benzamide

A solution (3 mL) of 5-(anilinocarbonylamino)-2-benzhydryloxy benzoic acid (300 mg, 0.684 mmole), tert-butyl amine (0.144 mL, 1.37 mmole), 1-hydroxy-1H-benzotriazole (157 mg, 1.03 mmole) and 1-ethyl-3-(3-dimethylaminopropyl)carbodiimide hydrochloride (164 mg, 0.855 mmole) in DMF was stirred at 0°C for 1 hour and at room temperature for 12 hours, and was poured into water. The mixture was extracted with ethyl acetate, the extracted solution was washed with water, and was dried with anhydrous magnesium sulfate. The solvent was distilled off under reduced pressure. The residue was purified by silicagel column chromatography (hexane:ethyl acetate = 1:1), which was recrystallized from hexane and ethyl acetate, to obtain 274 mg (81.1%) of the titled compound as a solid.
¹H-NMR (CDCl₃) δ; 1.18 (9H, s), 6.27 (1H, s), 6.77 (1 H, d, J = 8.8 Hz), 7.02 (1H, d, J = 7.0 Hz), 7.21 to 7.34 (14H, m), 7.46 (1H, s), 7.65 to 7.75 (3H, m), 7.89 (1H, s)

### Example 34

### 5-(Anilinocarbonylamino)-2-benzhydryloxy-N-(tert-pentyl)benzamide

A solution (2 mL) of 5-(anilinocarbonylamino)-2-benzhydryloxy benzoic acid (200 mg, 0.456 mmole), tert-pentyl amine (0.107 mL, 0.912 mmole), 1-hydroxy-1H-benzotriazole (105 mg, 0.687 mmole) and 1-ethyl-3-(3-dimethylaminopropyl)carbodiimide hydrochloride (109 mg, 0.570 mmole) in DMF was stirred at 0°C for 1 hour and at room temperature for 12 hours, and was poured into water. The mixture was extracted with ethyl acetate, the extracted solution was washed with water, and was dried with anhydrous magnesium sulfate. The solvent was distilled off under reduced pressure. The residue was purified by silicagel column chromatography (hexane:ethyl acetate = 2:1), which was recrystallized from hexane and ethyl acetate, to obtain 174 mg (75.3%) of the titled compound as a solid.
¹H-NMR (CDCl₃) δ; 0.65 (3H, t, J = 7.6 Hz), 1.10 (6H, s), 1.55 (2H, q, J = 7.6 Hz), 6.29 (1H, s), 6.79 (1H, d, J = 9.2 Hz), 7.02 (1 H, t, J = 7.4 Hz), 7.26 to 7.79 (19H, m)

### Example 35

### 5-(Anilinocarbonylamino)-2-benzhydryloxy-N-(1-methyl-1-phenylethyl)benzamide

A solution (2 mL) of 5-(anilinocarbonylamino)-2-benzhydryloxy benzoic acid (200 mg, 0.456 mmole), cumylamine (123 mg, 0.912 mmole), 1-hydroxy-1H-benzotriazole (105 mg, 0.687 mmole) and 1-ethyl-3-(3-dimethylaminopropyl)carbodiimide hydrochloride (109 mg, 0.570 mmole) in DMF was stirred at 0°C for 1 hour and at room temperature for 12 hours, and was poured into water. The mixture was extracted with ethyl acetate, the extracted solution was washed with water, and was dried with anhydrous magnesium sulfate. The solvent was distilled off under reduced pressure. The residue was purified by silicagel column chromatography (hexane:ethyl acetate = 2:1), which was recrystallized from hexane and ethyl acetate, to obtain 95.0 mg (37.5%) of the titled compound as a solid.
¹H-NMR (CDCl₃) δ; 1.38 (6H, s), 6.33 (1H, s), 6.81 (1H, d, J = 8.8 Hz), 6.96 (1H, t, J = 6.8 Hz), 7.09 to 7.63 (23H, m), 8.44 (1H, s)

### Example 36

### tert-Butyl 2-(benzhydryloxy)-5-({[(4-methoxyphenyl)amino]carbonyl}amino)benzoate

### (1) tert-Butyl 2-benzhydryloxy-5-nitrobenzoate

To a solution (20 mL) of tert-butyl 2-hydroxy-5-nitrobenzoate (1.17 g, 4.90 mmole) in DMF was added sodium hydride (60%, 235 mg, 5.87 mmole) under ice-cooling, and the mixture was stirred for 30 minutes at room temperature. Bromodiphenylmethane (1.45 g, 5.88 mmole) was added to the mixture, and the mixture was stirred at 70°C for 12 hours, and poured into ice-water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 10:1), to obtain 610 mg (30.6%) of the titled compound as oil.
¹H-NMR (CDCl₃) δ; 1.57 (9H, s), 6.38 (1H, s), 6.94 (1H, d, J = 9.2 Hz), 7.26 to 7.49 (10H, m), 8.13 (1H, dd, J = 9.2, 2.8 Hz), 8.51 (1H, d, J = 2.8 Hz)

### (2) tert-Butyl5-amino-2-benzhydryloxy benzoate

A mixed solution of tert-butyl 2-benzhydryloxy-5-nitrobenzoate (600 mg, 1.48 mmole), 5% iridium-carbon (60 mg) and ethyl acetate (10 mL) was stirred for 12 hours under hydrogen atmosphere. The insolubles were removed, and the filtrate was concentrated, to obtain 400 mg (72.1%) of the titled compound as oil.
¹H-NMR (CDCl₃) δ; 1.49 (9H, s), 6.13 (1H, s), 6.53 (1H, dd, J = 3.4, 8.4 Hz), 6.62 (1H, d, J = 8.4 Hz), 6.95 (1H, d, J = 3.4 Hz), 7.15 to 7.47 (10H, m)

### (3) tert-Butyl 2-(benzhydryloxy)-5-({[(4-methoxyphenyl)amino]carbonyl}amino)benzoate

A mixed solution of methyl 5-amino-2-benzhydryloxybenzoate (200 mg, 0.533 mmole), 4-methoxyphenyl isocyanate (0.0829 mL, 0.639 mmole) and THF (2 mL) was stirred under ice-cooling for 1 hour and at room temperature for 12 hours, was poured into water, and was extracted with ethyl acetate. The residue was recrystallized from ethyl acetate and hexane to obtain 139 mg (49.6%) of the titled compound.
¹H-NMR (CDCl₃) δ; 1.60 (9H, s), 3.79 (3H, s), 6.22 (1H, s), 6.33 (1H, s), 6.38 (1H, s), 6.77 to 7.46 (17H, m)

### Example 37

### Isopropyl 2-(benzhydryloxy)-5-({[(4-methoxyphenyl)amino]carbonyl}amino)benzoate

### (1) Isopropyl 2-benzhydryloxy-5-nitrobenzoate

To a solution (110 mL) of isopropyl 2-hydroxy-5-nitrobenzoate (5.64 g, 25.0 mmole) in DMF was added sodium hydride (60%, 1.10 g, 27.5 mmole) under ice-cooling, the mixture was stirred for 30 minutes at room temperature. Bromodiphenylmethane (7.41 g, 30.0 mmole) was added to the mixture, the mixture was stirred at 70°C for 12 hours, was poured into ice-water, and was extracted with ethyl acetate. The extracted solution was washed with water, was dried with anhydrous magnesium sulfate, and the solvent was distilled off under reduced pressure. The residue was recrystallized from diisopropylether, to obtain 3.28 g (33.5%) of the titled compound.
¹H-NMR (CDCl₃) δ; 1.35 (6H, d, J = 6.2 Hz), 5.25 to 5.37 (1H, m), 6.41 (1H, s), 6.99 (1H, d, J = 9.6 Hz), 7.24 to 7.52 (10H, m), 8.15 (1H, dd, J = 9.6, 3.0 Hz), 8.64 (1H, d, J = 3.0 Hz)

### (2) Isopropyl 5-amino-2-benzhydryloxy benzoate

A mixed solution of isopropyl 2-benzhydryloxy-5-nitrobenzoate (3.23 g, 8.25 mmole), 5% iridium-carbon (300 mg) and ethyl acetate (100 mL) was stirred for 12 hours under hydrogen atmosphere. The insolubles were filtered off, and the filtrate was concentrated, to obtain 2.80 g (94.0%) of the titled compound as oil.
¹H-NMR (CDCl₃) δ; 1.27 (6H, d, J = 6.2 Hz), 5.17 to 5.29 (1H, m), 6.16 (1H, s), 6.57 (1H, dd, J = 2.6, 8.8 Hz), 6.66 (1H, d, J = 8.8 Hz), 7.08 (1H, d, J = 2.6 Hz), 7.19 to 7.49 (10H, m)

### (3) Isopropyl 2-(benzhydryloxy)-5-({[(4-methoxyphenyl)amino]carbonyl}amino)benzoate

A mixed solution of isopropyl 5-amino-2-benzhydryloxy benzoate (300 mg, 0.830 mmole), 4-methoxyphenyl isocyanate (0.129 mL, 0.996 mmole) and THF (2 mL) was stirred under ice-cooling for 1 hour and at room temperature for 12 hours, was poured into water, and was extracted with ethyl acetate. The residue was recrystallized from ethyl acetate and hexane to obtain 376 mg (88.9%) of the titled compound.
¹H-NMR (CDCl₃) δ; 1.28 (6H, d, J = 6.2 Hz), 3.75 (3H, s), 5.17 - 5.30 (1H, m), 6.22 (1H, s), 6.58 (1H, s), 6.68 (1H, s), 6.80 to 7.52 (17H, m)

### Example 38

### Methyl 2-(benzhydryloxy)-5-({[(4-hydroxy-3-methoxybenzyl)amino]carbonyl}amino)benzoate

To a solution (5 mL) of methyl 5-amino-2-benzhydryloxybenzoate (200 mg, 0.600 mmole) and diisopropylethyl amine (0.123 mL, 0.720 mmole) in acetonitrile was added N,N-disuccinimidyl carbamate (184 mg, 0.720 mmole) at 0°C, and the mixture was stirred for 1 hour at 0°C. To this solution were added diisopropylethyl amine (0.247 mL, 1.44 mmole) and 4-hydroxy-3-methoxybenzyl amine hydrochloride (136 mg, 0.720 mmole), and the mixture was stirred for 12 hours at room temperature. The solvent was distilled off under reduced pressure, and the residue was poured into water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:2), which was recrystallized from hexane and ethyl acetate, to obtain 187 mg (68.2%) of the titled compound as a solid.
¹H-NMR (CDCl₃) δ; 3.82 (3H, s), 3.86 (3H, s), 4.28 (2H, d, J = 5.4 Hz), 4.96 (1H, t, J = 5.4 Hz), 5.59 (1H, s), 6.22 (1H, s), 6.32 (1H, s), 6.70 to 7.51 (15H, m), 7.61 (1H, d, J = 3.0 Hz)

### Example 39

### N-[4-(2-Chlorobenzyloxy)-3-propionylphenyl]-N'-(3,4-dimethoxyphenyl)urea

### (1) 1-[2-(2-Chlorobenzyloxy)-5-nitrophenyl]propan-1-one

A solution of 1-(2-hydroxy-5-nitrophenyl)propan-1-one (500 mg, 2.56 mmol), potassium carbonate (424 mg, 3.07 mmol), 2-chlorobenzylchloride (0.388 mL, 3.07 mmol) and DMF (5 mL) was stirred at 70°C for 12 hours, was poured into water, and was extracted with ethyl acetate. The solvent was distilled off under reduced pressure, to obtain the titled compound as a solid. 630 mg (76.9%)
¹H-NMR (CDCl₃) δ; 1.14 (3H, t, J = 7.4 Hz), 2.97 (2H, q, J = 7.4 Hz), 5.37 (2H, s), 7.11 (1 H, d, J = 9.2 Hz), 7.26 to 7.49 (4H, m), 8.33 (1H, dd, J = 9.2, 3.0 Hz), 8.57 (1H, d, J = 3.0 Hz)

### (2) 1-[5-Amino-2-(2-chlorobenzyloxy)phenyl]propan-1-one

To a mixed solution (30 mL) of 1-[2-(2-chlorobenzyloxy)-5-nitrophenyl]propan-1-one (610 mg, 1.91 mmol) in methanol and water (8 mL) was added sodium hydrosulfite (4.53 g, 26.0 mmol) for 4 hours at 80°C. The mixture was stirred for 1 hour at 80°C and the solvent was distilled off under reduced pressure. The residue was extracted with ethyl acetate, was dried with anhydrous magnesium sulfate, and the solvent was distilled off under reduced pressure, to obtain the titled compound as a solid. 220 mg (39.8%)
¹H-NMR (CDCl₃) δ; 1.12 (3H, t, J = 7.2 Hz), 2.99 (2H, q, J = 7.2 Hz), 3.53 (2H, bs), 5.17 (2H, s), 6.77 (1H, dd, J = 3.0, 8.6 Hz), 6.84 (1H, d, J = 8.6 Hz), 7.01 (1H, d, J = 3.0 Hz), 7.26 to 7.53 (4 H, m)

### (3) N-[4-(2-Chlorobenzyloxy)-3-propionylphenyl]-N'-(3,4-dimethoxyphenyl)urea

To a solution (1 mL) of 1-[5-amino-2-(2-chlorobenzyloxy)phenyl]propan-1-one (100 mg, 0.345 mmol) in THF was added 3,4-dimethoxyphenyl isocyanate (0.0616 mL, 0.414 mmol) under ice-cooling, the mixture was stirred at 0°C for 1 hour and at room temperature for 12 hours, and the solvent was distilled off under reduced pressure. The residue was recrystallized from hexane and ethyl acetate. 138 mg (85.2%)
¹H-NMR (CDCl₃) δ; 1.11 (3H, t, J = 6.8 Hz), 3.00 (2H, q, J = 6.8 Hz), 3.85 (6H, s), 5.21 (2H, s), 6.77 to 7.82 (12 H, m)

The following compounds were obtained in the same manner as Example 39.

### Example 40

### N-[4-(2-Chlorobenzyloxy)-3-propionylphenyl]-N'-(3-methoxyphenyl)urea

¹H-NMR (CDCl₃) δ; 1.12 (3H, t, J = 7.4 Hz), 3.01 (2H, q, J = 7.4Hz), 3.76 (3H, s), 5.20 (2H, s), 6.59 to 7.83 (13 H, m)

### Example 41

### N-[4-(1,1'-Biphenyl-4-ylmethoxy)-3-propionylphenyl]-N'-(3,4-dimethoxyphenyl)urea

### (1) 1-(2-(1,1'-Biphenyl-4-ylmethoxy)-5-nitrophenyl]propan-1-one

¹H-NMR (CDCl₃) δ; 1.16 (3H, t, J = 7.0 Hz), 3.01 (2H, q, J = 7.0 Hz), 5.32 (2H, s), 7.14 (1 H, d, J = 9.2 Hz), 7.37 to 7.68 (9H, m), 8.32 (1H, dd, J = 9.2, 2.8 Hz), 8.58 (1H, d, J = 2.8Hz)
IR (KBr) cm⁻¹; 1676, 1607, 1584, 1510, 1480, 1420, 1345, 1277, 982, 766, 750

### (2) 1-[5-Amino-2-(1,1'-biphenyl-4-ylmethoxy)phenyl]propan-1-one

¹H-NMR (CDCl₃) δ; 1.12 (3H, t, J = 7.4 Hz), 3.00 (2H, q, J = 7.4Hz), 5.11 (2H, s), 6.77 (1H, dd, J = 3.0, 8.4 Hz), 6.88 (1H, d, J = 8.4 Hz), 7.03 (1H, d, J = 3.0 Hz), 7.32 to 7.64 (9 H, m)

### (3) N-[4-(1,1'-Biphenyl-4-ylmethoxy)-3-propionylphenyl]-N'-(3,4-dimethoxyphenyl)urea

¹H-NMR (CDCl₃) δ; 1.12 (3H, t, J = 7.4 Hz), 3.03 (2H, q, J = 7.4 Hz), 3.85 (6H, s), 5.15 (2H, s), 6.78 to 7.85 (17 H, m)

### Example 42

### N-[4-(1,1'-Biphenyl-4-ylmethoxy)-3-propionylphenyl]-N'-(3-methoxyphenyl)urea

¹H-NMR (CDCl₃) δ; 1.13 (3H, t, J = 7.4 Hz), 3.04 (2H, q, J = 7.4 Hz), 3.78 (3H, s), 5.15 (2H, s), 6.61 to 7.85 (18 H, m)

### Example 43

### N-[4-(1,1'-Biphenyl-4-ylmethoxy)-3-propionylphenyl]-N'-(4-methoxyphenyl)urea

¹H-NMR (CDCl₃) δ; 1.11 (3H, t, J = 7.0 Hz), 3.01 (2H, q, J = 7.0 Hz), 3.77 (3H, s), 5.13 (2H, s), 6.83 to 7.83 (18 H, m)

### Example 44

### N-(3,4-Dimethoxyphenyl)-N'-[4-(4-ethylbenzyloxy)-3-propionylphenyl]urea

### (1) 1-[2-(4-Ethylbenzyloxy)-5-nitrophenyl]propan-1-one

A solution (5 mL) of 1-(2-hydroxy-5-nitrophenyl)propan-1-one (500 mg, 2.56 mmol), 4-ethylbenzyl alcohol (0.508 mL, 3.84 mmol), 1,1-(azodicarbonyl)dipiperidine (967 mg, 3.84 mmol) and triphenylphosphine (1.01 g, 3.84 mmol) in toluene was stirred at 80°C for 3 hours, to the reaction solution were added 4-ethylbenzyl alcohol (0.508 mL, 3.84 mmol), 1,1-(azodicarbonyl)dipiperidine (967 mg, 3.84 mmol), triphenylphosphine (1.01 g, 3.84 mmol) and toluene (5 mL), and the mixture was stirred at 80°C for 3 hours. To the reaction solution were further added 4-ethylbenzyl alcohol (0.508 mL, 3.84 mmol), 1,1-(azodicarbonyl)dipiperidine (967 mg, 3.84 mmol), triphenylphosphine (1.01 g, 3.84 mmol) and toluene (5 mL), and the mixture was stirred for 12 hours at 80°C. The reaction solution was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 10:1), to obtain the titled compound as oil. 770 mg (96.0%)
¹H-NMR (CDCl₃) δ; 1.13 (3H, t, J = 7.4 Hz), 1.24 (3H, t, J = 7.8 Hz), 2.65 (2H, q, J = 7.8 Hz), 2.97 (2H, q, J = 7.4 Hz), 5.24 (2H, s), 7.11 (1H, d, J = 9.2 Hz), 7.20 (2H, d, J = 8.0 Hz), 7.29 (2H, d, J = 8.0 Hz), 8.29 (1H, dd, J = 3.0, 9.2 Hz), 8.56 (1H, d, J = 3.0 Hz)

### (2) 1-[5-Amino-2-(4-ethylbenzyloxy)phenyl]propan-1-one

To a solution (80 mL) of 1-[2-(4-ethylbenzyloxy)-5-nitrophenyl]propan-1-one (750 mg, 2.39 mmol) in ethyl acetate was added 5% iridium carbon (75 mg), and the mixture was stirred for 12 hours under hydrogen atmosphere. The insolubles were filtered off, and the filtrate was concentrated, and the residue was purified by silicagel column chromatography (hexane:ethyl acetate = 1:1), to obtain titled compound as oil. 530 mg (78.3%)
¹H-NMR (CDCl₃) δ; 1.10 (3H, t, J = 7.5 Hz), 1.25 (3H, t, J = 7.8 Hz), 2.66 (2H, q, J = 7.8 Hz), 2.97 (2H, q, J = 7.5 Hz), 3.50 (2H, bs), 5.03 (2H, s), 6.76 (1H, dd, J = 3.0, 9.0 Hz), 6.86 (1H, d, J = 9.0 Hz), 7.02 (1H, d, J = 3.0 Hz), 7.21 (2H, d, J = 8.1 Hz), 7.31 (2H, d, J = 8.1 Hz)

### (3) N-(3,4-Dimethoxyphenyl)-N'-[4-(4-ethylbenzyloxy)-3-propionylphenyl]urea

To a solution (2 mL) of 1-[5-amino-2-(4-ethylbenzyloxy)phenyl]propan-1-one (200 mg, 0.706 mmol) in THF was added 3,4-dimethoxyphenyl isocyanate (0.126 mL, 0.847 mmol) under ice-cooling, the mixture was stirred at 0°C for 1 hour and at room temperature for 12 hours, and the solvent was distilled off under reduced pressure. The residue was recrystallized from hexane and ethyl acetate. 295 mg (90.2%)
¹H-NMR (CDCl₃) δ; 1.11 (3H, t, J = 7.4 Hz), 1.24 (3H, t, J = 7.6 Hz), 2.66 (2H, q, J = 7.6 Hz), 3.00 (2H, q, J = 7.4 Hz), 3.84 (6H, s), 5.06 (2H, s), 6.76 to 7.84 (12 H, m)

The following compounds were obtained in the same manner as Example 44.

### Example 45

### N-[4-(4-Ethylbenzyloxy)-3-propionylphenyl]-N'-(3-methoxyphenyl)urea

¹H-NMR (CDCl₃) δ; 1.11 (3H, t, J = 7.4 Hz), 1.24 (3H, t, J = 7.2 Hz), 2.66 (2H, q, J = 7.2 Hz), 3.01 (2H, q, J = 7.4 Hz), 3.76 (3H, s), 5.05 (2H, s), 6.58 to 7.84 (13 H, m)

### Example 46

### N-[4-(4-Ethylbenzyloxy)-3-propionylphenyl]-N'-(4-methoxyphenyl)urea

¹H-NMR (CDCl₃) δ; 1.09 (3H, t, J = 7.4 Hz), 1.25 (3H, t, J = 7.6 Hz), 2.66 (2H, q, J = 7.6 Hz), 2.98 (2H, q, J = 7.6 Hz), 3.78 (3H, s), 5.05 (2H, s), 6.828 to 7.82 (13 H, m)

### Example 47

### N-(3,4-Dimethoxyphenyl)-N'-[4-(2,4-dimethylbenzyloxy)-3-propionylphenyl]urea

### (1) 1-[2-(2,4-Dimethylbenzyloxy)-5-nitrophenyl]propan-1-one

¹H-NMR (CDCl₃) δ; 1.09 (3H, t, J = 7.4 Hz), 2.34 (6H, s), 2.90 (2H, q, J = 7.4 Hz), 5.21 (2H, s), 6.99 to 7.26 (4H, m), 8.32 (1H, dd, J = 2.6, 9.2 Hz), 8.55 (1H, d, J = 2.6 Hz)

### (2) 1-[5-Amino-2-(2,4-dimethylbenzyloxy)phenyl]propan-1-one

¹H-NMR (CDCl₃) δ; 1.06 (3H, t, J = 7.2 Hz), 2.33 (6H, s), 2.91 (2H, q, J = 7.2 Hz), 3.50 (2H, bs), 5.00 (2H, s), 6.78 (1H, dd, J = 2.8, 8.8 Hz), 6.87 (1H, d, J = 8.8 Hz), 7.00 to 7.26 (4H, m)

### (3) N-(3,4-Dimethoxyphenyl)-N'-[4-(2,4-dimethylbenzyloxy)-3-propionylphenyl]urea

¹H-NMR (CDCl₃) δ; 1.06 (3H, t, J = 7.4 Hz), 2.32 (6H, s), 2.93 (2H, q, J = 7.4 Hz), 3.85 (6H, s), 5.05 (2H, s), 6.77 to 7.86 (11 H, m)

### Example 48

### N-[4-(2,4-Dimethylbenzyloxy)-3-propionylphenyl]-N'-(3-methoxyphenyl)urea

¹H-NMR (CDCl₃) δ; 1.07 (3H, t, J = 7.4 Hz), 2.32 (6H, s), 2.95 (2H, q, J = 7.4 Hz), 3.77 (3H, s), 5.05 (2H, s), 6.59 to 7.87 (12 H, m)

### Example 49

### N-[4-(2,4-Dimethylbenzyloxy)-3-propionylphenyl]-N'-(4-methoxyphenyl)urea

¹H-NMR (CDCl₃) δ; 1.05 (3H, t, J = 7.4 Hz), 2.32 (6H, s), 2.94 (2H, q, J = 7.4 Hz), 3.79 (3H, s), 5.04 (2H, s), 6.76 to 7.84 (12 H, m)

### Example 50

### N-(3,4-Dimethoxyphenyl)-N'-[4-(4-methylbenzyloxy)-3-propionylphenyl]urea

### (1) 1-[2-(4-Methylbenzyloxy)-5-nitrophenyl]propan-1-one

¹H-NMR (CDCl₃) δ; 1.13 (3H, t, J = 7.0 Hz), 2.38 (3H, s), 2.96 (2H, q, J = 7.0 Hz), 5.23 (2H, s), 7.10 (1 H, d, J = 9.2 Hz), 7.22 (2H, d, J = 8.0 Hz), 7.30 (2H, d, J = 8.0 Hz), 8.29 (1H, dd, J = 9.2, 3.0 Hz), 8.56 (1H, d, J = 3.0 Hz)

### (2) 1-[5-Amino-2-(4-methylbenzyloxy)phenyl]propan-1-one

¹H-NMR (CDCl₃) δ; 1.09 (3H, t, J = 7.2 Hz), 2.36 (3H, s) , 2.97 (2H, q, J = 7.2 Hz), 3.50 (2H, bs), 5.02 (2H, s), 6.75 (1H, dd, J = 2.2, 8.4 Hz), 6.85 (1H, d, J = 8.4 Hz), 7.01 (1H, d, J = 2.2 Hz), 7.18 (2H, d, J = 8.0 Hz), 7.29 (2H, d, J = 8.0 Hz)

### (3) N-(3,4-Dimethoxyphenyl)-N'-[4-(4-methylbenzyloxy)-3-propionylphenyl]urea

¹H-NMR (CDCl₃) δ; 1.10 (3H, t, J = 7.2 Hz), 2.37 (3H, s), 3.00 (2H, q, J = 7.2 Hz), 3.85 (6H, s), 5.06 (2H, s), 6.76 to 7.85 (12 H, m)

### Example 51

### N-(3-Methoxyphenyl)-N'-[4-(4-methylbenzyloxy)-3-propionylphenyl]urea

¹H-NMR (CDCl₃) δ; 1.11 (3H, t, J = 7.2 Hz), 2.37 (3H, s), 3.00 (2H, q, J = 7.2 Hz), 3.76 (3H, s), 5.04 (2H, s), 6.58 to 7.86 (13 H, m)

### Example 52

### N-(4-Methoxyphenyl)-N'-[4-(4-methylbenzyloxy)-3-propionylphenyllurea

¹H-NMR (CDCl₃) δ; 1.08 (3H, t, J = 7.4 Hz), 2.36 (3H, s), 2.97 (2H, q, J = 7.4 Hz), 3.78 (3H, s), 5.05 (2H, s), 6.82 to 7.81 (13 H, m)

### Example 53

### N-(3,4-Dimethoxyphenyl)-N'-(4-neopentyl-3-propionylphenyl)urea

### (1) 1-[2-Neopentyloxy-5-nitrophenyl]propan-1-one

To a solution (10 mL) of 1-(2-hydroxy-5-nitrophenyl)propan-1-one (500 mg, 2.56 mmol) in DMF was added sodium hydride (60%, 123 mg, 3.07 mmol) under ice-cooling, and the mixture was stirred at room temperature for 1 hour. Neopentyl iodide (0.407 mL, 3.07 mmol) was added to the mixture, and the mixture was stirred for 2 hours at 120°C. Neopentyl iodide (0.407 mL, 3.07 mmol) was further added to the mixture, and the mixture was stirred at 120°C for 12 hours, and was poured into water. This solution was extracted with ethyl acetate, and the extracted solution was washed with water and was dried with anhydrous magnesium sulfate. The solvent was distilled off under reduced pressure, and the residue was purified by silicagel column chromatography (hexane:ethyl acetate = 9:1), to obtain the titled compound as a solid. 200 mg (29.5%)
¹H-NMR (CDCl₃) δ; 1.10 (9H, s), 1.21 (3H, t, J = 7.4 Hz), 3.04 (2H, q, J = 7.4 Hz), 3.81 (2H, s), 7.04 (1 H, d, J = 9.0 Hz), 8.31 (1H, dd, J = 9.0, 2.8 Hz), 8.54 (1H, d, J = 2.8Hz)

### (2) 1-[5-Amino-2-neopentyloxyphenyl]propan-1-one

1-[2-Neopentyloxy-5-nitrophenyl]propan-1-one (190 mg, 0.716 mmol) and 5% iridium carbon (20 mg) were added thereto, and the mixture was stirred for 12 hours under hydrogen atmosphere at room temperature. The insolubles were filtered off, and the filtrate was concentrated, to obtain the titled compound as'oil. 153 mg (90.5%)
¹H-NMR (CDCl₃) δ; 1.05 (9H, s), 1.17 (3H, t, J = 7.4 Hz), 3.04 (2H, q, J = 7.4 Hz), 3.48 (3H, bs), 3.61 (2H, s), 6.78 (2 H, d, J = 1.8 Hz), 7.02 (1H, t, J = 1.8 Hz)

### (3) N-(3,4-Dimethoxyphenyl)-N'-(4-neopentyl-3-propionylphenyl)urea

To a solution (3 mL) of 1-[5-amino-2-neopentyloxyphenyl]propan-1-one (143 mg, 0.608 mmol) in THF was added 3,4-dimethoxyphenyl isocyanate (0.108 mL, 0.729 mmol) under ice-cooling, the mixture was stirred at 0°C for 1 hour and at room temperature for 12 hours, and the solvent was distilled off under reduced pressure. The residue was recrystallized from hexane and ethyl acetate. 213 mg (84.5%)
¹H-NMR (CDCl₃) δ; 1.07 (9H, s), 1.18 (3H, t, J = 7.4 Hz), 3.08 (2H, q, J = 7.6 Hz), 3.67 (2H, s), 3.87 (6H, s), 6.76 to 6.94 (5H, m), 7.06 (1H, d, J = 1.8 Hz), 7.36 (1H, d, J = 3.0 Hz), 7.77 to 7.83 (1H, m)

The following compounds were obtained in the same manner as Example 53.

### Example 54

### N-(3,4-Dimethoxyphenyl)-N'-(4-isobutoxy-3-propionylphenyl)urea

### (1) 1-[2-Isobutoxy-5-nitrophenyl]propan-1-one

¹H-NMR (CDCl₃) δ; 1.09 (6H, d, J = 7.0 Hz), 1.20 (3H, t, J = 7.4 Hz), 2.14 to 2.28 (1H, m), 3.01 (2H, q, J = 7.4 Hz), 3.94 (2H, d, J = 6.6 Hz), 7.03 (1 H, d, J = 9.2 Hz), 8.31 (1H, dd, J = 9.2, 2.8 Hz), 8.56 (1H, d, J = 2.8Hz)

### (2) 1-[5-Amino-2-isobutoxyphenyl]propan-1-one

¹H-NMR (CDCl₃) δ; 1. 03 (6H, d, J = 7.0 Hz), 1.16 (3H, t, J = 7.4 Hz), 2.04 to 2.17 (1H, m), 3.01 (2H, q, J = 7.4 Hz), 3.50 (2H, bs), 3.73 (2H, d, J = 6.4 Hz), 6.77 (2 H, d, J = 1.8 Hz), 7.03 (1H, t, J = 1.8 Hz)

### (3) N-(3,4-Dimethoxyphenyl)-N'-(4-isobutoxy-3-propionylphenyl)urea

¹H-NMR (CDCl₃) δ; 1.05 (6H, d, J = 6.6 Hz), 1.17 (3H, t, J = 7.4 Hz), 2.05 to 2.20 (1H, m), 3.05 (2H, q, J = 7.4 Hz), 3.78 (2H, d, J = 6.2 Hz), 3.86 (6H, s), 6.73 to 7.09 (6H, m), 7.37 (1H, d, J = 2.8 Hz), 7.82 (1H, dd, J = 2.8, 9.2 Hz)

### Example 55

### N-(4-Cyclohexylmethoxy-3-propionylphenyl)-N'-(3,4-dimethoxyphenyl)urea

### (1) 1-(2-Cyclohexylmethoxy-5-nitrophenyl)propan-1-one

¹H-NMR (CDCl₃) δ; 1.08 to 1.90 (14H, m), 3.01 (2H, q, J = 7.2 Hz), 3.96 (3H, d, J = 5.8 Hz), 7.03 (1 H, d, J = 9.0 Hz), 8.30 (1H, dd, J = 9.0, 2.6 Hz), 8.55 (1H, d, J = 2.6Hz)

### (2) 1-(5-Amino-2-cyclohexylmethoxyphenyl)propan-1-one

¹H-NMR (CDCl₃) δ; 1.03 to 1.88 (14H, m), 3.00 (2H, q, J = 7.2 Hz), 3.47 (2H, bs), 3.75 (2H, d, J = 5.6 Hz), 6.77 (2 H, d, J = 2.0 Hz), 7.02 (1H, t, J = 2.0 Hz)

### (3) N-(4-Cyclohexylmethoxy-3-propionylphenyl)-N'-(3,4-dimethoxyphenyl)urea

¹H-NMR (CDCl₃) δ; 1.04 to 1.88 (14H, m), 3.04 (2H, q, J = 7.4 Hz), 3.81 (2H, d, J = 5.8 Hz), 3.86 (6H, s), 6.78 to 7.08 (6H, m), 7.35 (1H, d, J = 2.8 Hz), 7.80 (1H, dd, J = 2.8, 9.2 Hz)

### Example 56

### N-(4-Cyclohexylmethoxy-3-propionylphenyl)-N'-(3-methoxyphenyl)urea

¹H-NMR (CDCl₃) δ; 1.10 to 1.88 (14H, m), 3.05 (2H, q, J = 7.4 Hz), 3.78 (3H, s), 3.81 (2H, d, J = 6.0 Hz), 6.60 to 7.26 (7H, m), 7.38 (1H, d, J = 3.0 Hz), 7.80 (1H, dd, J = 3.0, 9.0 Hz)

### Example 57

### N-(4-Cyclohexylmethoxy-3-propionylphenyl)-N'-(4-methoxyphenyl)urea)

¹H-NMR (CDCl₃) δ; 1.12 to 1.87 (14H, m), 3.02 (2H, q, J = 7.4 Hz), 3.78 to 3.81 (5H, m), 6.82 to 7.26 (7H, m), 7.33 (1H, d, J = 3.0 Hz), 7.79 (1H, dd, J = 3.0, 8.8 Hz)

### Reference Example 1

### 1-(2-Hydroxy-5-nitrophenyl)-2-methylpropan-1-one

### (1) 4-Nitrophenyl 2-methylpropanate

To a solution (400 mL) of 4-nitrophenol (20.0 g, 144 mmol) and triethyl amine (24.0 mL, 173 mmol) in tetrahydrofuran was added dropwise isobutyl chloride (18.1 mL, 173 mmol) under ice-cooling, the mixture was stirred for 1 hour under ice-cooling, was poured into water, and was extracted with ethyl acetate. The extracted solution was washed with water, was dried with anhydrous magnesium sulfate, and the solvent was distilled off under reduced pressure, to obtain the titled compound as oil. 30.0 g (99.7%)
¹H-NMR (CDCl₃) δ; 1.34 (6H, d, J = 6.8 Hz), 2.78 - 2.92 (1H, m), 3.81 (3H, s), 7.27 (2 H, d, J = 9.2 Hz), 8.27 (2H, d, J = 9.2 H)

### (2) 1-(2-Hydroxy-5-nitrophenyl)-2-methylpropan-1-one

To a suspension (70 mL) of aluminium chloride (34.1 g, 256 mmol) in nitrobenzene was added dropwise a solution of 4-nitrophenyl 2-methylpropanate (15.0 g, 71.7 mmol) in nitrobenzene (70 mL). The mixture was stirred for 2 hours at room temperature, for 2 hours at 100°C and for 3 hours at 110 to 130°C, and the reaction solution was poured into ice-water. 1N hydrochloric acid (400 mL) was added to the mixture, and the mixture was extracted with ethyl acetate. The extracted solution was washed with water, and the solvent was distilled off under reduced pressure. The residue was purified by silicagel column chromatography (hexane:ethyl acetate = 20:1), to obtain the titled compound as oil.
3.40 g (22.7%)
¹H-NMR (CDCl₃) δ; 1.31 (6H, d, J = 6.6 Hz), 3.62 to 3.76 (1H, m), 7.11 (1 H, d, J = 9.2 Hz), 8.35 (1H, dd, J = 9.2, 2.6 Hz), 8.77 (1H, d, J = 2.6 Hz)

### Example 58

### N-(4-Benzhydryloxy-3-isobutyrylphenyl)-N'-(3,4-dimethoxyphenyl)urea

### (1) 1-[2-(Benzhydryloxy)-5-nitrophenyl]-2-methylpropan-1-one

A solution (10 mL) of 1-(2-hydroxy-5-nitrophenyl)-2-methylpropan-1-one (1.00 g, 4.78 mmol) in DMF was added to a solution of sodium hydride (60%, 191 mg, 4.78 mmol) under ice-cooling, and the mixture was stirred at room temperature for 0.5 hour. Bromodiphenylmethane (1.42 g, 5.74 mmol) was added to the mixture, and the mixture was stirred for 12 hours at 70°C. The reaction solution was poured into ice-water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 30:1), to obtain the titled compound as oil.
1.00 g (55.9%)
¹H-NMR (CDCl₃) δ; 1.10 (6H, d, J = 7.0 Hz), 3.37 to 3.44 (1H, m), 6.37 (1H, s), 6.95 (1 H, d, J = 9.2 Hz), 7.26 to 7.38 (10H, m), 8.15 (1H, dd, J = 9.2, 2.4 Hz), 8.35 (1H, d, J = 2.4 Hz)

### (2) 1-[5-Amino-2-(benzhydryloxy)phenyl]-2-methylpropan-1-one

1-[2-(Benzhydryloxy)-5-nitrophenyl]-2-methylpropan-1-one (950 mg, 2.53 mmol) and 5% iridium carbon (100 mg) were added thereto, and the mixture was stirred for 12 hours under hydrogen atmosphere at room temperature. The insolubles were filtered off, and the filtrate was concentrated, to obtain the titled compound as a solid.
630 mg (72.1%)
¹H-NMR (CDCl₃) δ; 1.03 (6H, d, J = 7.0 Hz), 3.43 to 3.57 (3H, m), 6.10 (1H, s), 6.58 (1H, dd, J = 3.4, 8.8 Hz), 6.65 (1 H, d, J = 8.8 Hz), 6.79 (1H, d, J = 3.4 Hz), 7.26 to 7.39 (10H, m)

### (3) N-(4-Benzhydryloxy-3-isobutylphenyl)-N'-(3,4-dimethoxyphenyl)urea

To a solution (4 mL) of 1-[5-amino-2-(benzhydryloxy)phenyl]-2-methylpropan-1-one (200 mg, 0.579 mmol) in THF was added 3,4-dimethoxyphenyl isocyanate (0.103 mL, 0.695 mmol) under ice-cooling, the mixture was stirred at 0°C for 1 hour, and the solvent was distilled off under reduced pressure. The residue was recrystallized from hexane and ethyl acetate. 278 mg (91.4%)
¹H-NMR (CDCl₃) δ; 1.06 (6H, d, J = 6.6 Hz), 3.51 to 3.58 (1H, m), 3.85 (6H, s), 6.21 (1H, s), 6.64 to 7.36 (17H, m), 7.55 (1H, dd, J = 3.0, 8.8 Hz)

### Example 59

### N-(4-Benzhydryloxy-3-isobutylphenyl)-N'-(3-methoxyphenyl)urea

To a solution (4 mL) of 1-[5-amino-2-(benzhydryloxy)phenyl]-2-methylpropan-1-one (200 mg, 0.579 mmol) in THF was added 3-methoxyphenyl isocyanate (0.0911 mL, 0.695 mmol) under ice-cooling, the mixture was stirred for 1 hour at 0°C, and the solvent was distilled off under reduced pressure. The residue was recrystallized from hexane and ethyl acetate. 272 mg (89.5%)
¹H-NMR (CDCl₃) δ; 1.05 (6H, d, J = 7.0 Hz), 3.51 to 3.62 (1H, m), 3.78 (3H, s), 3.75 (3H, s), 6.19 (1H, s), 6.58 to 7.35 (18H, m), 7.53 (1H, dd, J = 2.6, 9.0 Hz)

### Example 60

### N-(4-Benzhydryloxy-3-isobutylphenyl)-N'-(4-methoxyphenyl)urea

To a solution (4 mL) of 1-[5-amino-2-(benzhydryloxy)phenyl]-2-methylpropan-1-one (200 mg, 0.579 mmol) in THF was added 4-methoxyphenyl isocyanate (0.0900 mL, 0.695 mmol) under ice-cooling, the mixture was stirred at 0°C for 1 hour, and the solvent was distilled off under reduced pressure. The residue was recrystallized from hexane and ethyl acetate. 254 mg (88.8%)
¹H-NMR (CDCl₃) δ; 1.04 (6H, d, J = 6.6 Hz), 3.49 to 3.56 (1H, m), 3.79 (3H, s), 6.20 (1H, s), 6.51 to 7.36 (18H, m), 7.54 (1H, dd, J = 3.0, 8.8 Hz)

### Example 61

### N-(3,4-Dimethoxyphenyl)-N'-[4-(3,3-diphenylpropoxy)-3-propionylphenyl]urea

### (1) 1-[2-(3,3-Diphenylpropoxy)-5-nitrophenyl]propan-1-one

A solution (5 mL) of 1-(2-hydroxy-5-nitrophenyl)propan-1-one (500 mg, 2.56 mmol), 3,3-diphenylpropanol (765 mg, 3.84 mmol), 1,1-(azodicarbonyl)dipiperidine (969 mg, 3.84 mmol) and triphenylphosphine (1.01 g, 3.84 mmol) in toluene was stirred for 4 hours at 80°C, and to the reaction solution were added 3,3-diphenylpropanol (765 mg, 3.84 mmol), 1,1-(azodicarbonyl)dipiperidine (969 mg, 3.84 mmol), triphenylphosphine (1.01 g, 3.84 mmol) and toluene (5 mL). The mixture was stirred for 4 hours at 80°C. To the reaction solution were further added 3,3-diphenylpropanol (765 mg, 3.84 mmol), 1,1-(azodicarbonyl)dipiperidine (967 mg, 3.84 mmol), triphenylphosphine (1.01 g, 3.84 mmol) and toluene (5 mL), and the mixture was stirred for 12 hours at 80°C. The reaction solution was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 600 mg (60.2%)
¹H-NMR (CDCl₃) δ; 1.23 (3H, t, J = 7.2 Hz), 2.58 - 2.69 (2H, m), 3.04 (2H, q, J = 7.2 Hz), 4.07 to 4.25 (3H, m), 6.87 (1 H, d, J = 9.0 Hz), 7.15 to 7.36 (10H, m), 8.24 (1H, dd, J = 9.0, 2.8 Hz), 8.55 (1H, d, J = 2.8Hz)

### (2) 1-[5-Amino-2-(3,3-diphenylpropoxy)-phenyl]propan-1-one

1-[2-(3,3-Diphenylpropoxy)-5-nitrophenyl]propan-1-one (590 mg, 1.51 mmol) and 5% iridium carbon (100 mg) were added thereto, and the mixture was stirred for 12 hours under hydrogen atmosphere at room temperature. The insolubles were filtered off, and the filtrate was concentrated, to obtain the titled compound as a solid. 530 mg (97.4%)
¹H-NMR (CDCl₃) δ; 1.18 (3H, t, J = 7.4 Hz), 2.49 - 2.60 (2H, m), 3.04 (2H, q, J = 7.42 Hz), 3.47 (2H, bs), 3.90 (2 H, t, J = 6.2 Hz), 4.22 (1H, t, J = 6.2 Hz), 6.62 (1H, d, J = 8.4 Hz), 6.72 (1 H, dd, J = 8.4, 2.8 Hz), 7.02 (1H, d, J = 2.8 Hz), 7.16 to 7.34 (10H, m)

### (3) N-(3,4-Dimethoxyphenyl)-N'-[4-(3,3-diphenylpropoxy)-3-propionylphenyl]urea

To a solution (2 mL) of 1-[5-amino-2-(3,3-diphenylpropoxy)-phenyl]propan-1-one (200 mg, 0.556 mmol) in THF was added 3,4-dimethoxyphenyl isocyanate (0.0994 mL, 0.668 mmol) under ice-cooling, the mixture was stirred for 1 hour at 0°C, and the solvent was distilled off under reduced pressure. The residue was recrystallized from hexane and ethyl acetate. 285 mg (95.3%)
¹H-NMR (CDCl₃) δ; 1.23 (3H, t, J = 7.4 Hz), 2.52 - 2.62 (2H, m), 3.07 (2H, q, J = 7.4 Hz), 3.85 (6H, s), 3.97 (2H, t, J = 6.6 Hz), 4.21 (1H, t, J = 8.0 Hz), 6.73 to 7.37 (17H, m), 7.73 to 7.78 (1H, m)

### Example 62

### N-[4-(3,3-Diphenylpropoxy)-3-propionylphenyl]-N-(3-methoxyphenyl)urea

To a solution (2 mL) of 1-[5-amino-2-(3,3-diphenylpropoxy)-phenyl]propan-1-one (150 mg, 0.417 mmol) in THF was added 3-methoxyphenyl isocyanate (0.0648 mL, 0.500 mmol) under ice-cooling, the mixture was stirred for 1 hour at 0°C, and the solvent was distilled off under reduced pressure. The residue was recrystallized from hexane and ethyl acetate. 180 mg (84.9%)
¹H-NMR (CDCl₃) δ; 1.18 (3H, t, J = 7.2 Hz), 2.52 - 2.62 (2H, m), 3.05 (2H, q, J = 7.2 Hz), 3.79 (3H, s), 3.96 (2H, t, J = 6.2 Hz), 4.21 (1H, t, J = 8.0 Hz), 6.57 to 7.34 (18H, m), 7.71 to 7.77 (1H, m)

### Example 63

### N-[4-(3,3-diphenylpropoxy)-3-propionylphenyl]-N-(4-methoxyphenyl)urea

To a solution THF (2 mL) of 1-[5-amino-2-(3,3-diphenylpropoxy)-phenyl]propan-1-one (150 mg, 0.417 mmol) in was added 4-methoxyphenyl isocyanate (0.0655 mL, 0.500 mmol) under ice-cooling, the mixture was stirred at 0°C for 1 hour, and the solvent was distilled off under reduced pressure. The residue was recrystallized from hexane and ethyl acetate. 205 mg (96.7%)
¹H-NMR (CDCl₃) δ; 1.21 (3H, t, J = 7.4 Hz), 2.51 - 2.62 (2H, m), 3.08 (2H, q, J = 7.2 Hz), 3.76 (3H, s), 3.96 (2H, t, J = 6.6 Hz), 4.20 (1H, t, J = 7.6 Hz), 6.59 to 7.39 (18H, m), 7.73 to 7.79 (1H, m)

### Example 64

### Methyl [4-(3,4-dimethoxyphenylaminocarbonylamino)-2-propionylphenoxy]acetate

### (1) Methyl (4-nitro-2-propionylphenoxy)acetate

To a solution (20 mL) of 1-(2-hydroxy-5-nitrophenyl)propan-1-one (1.00 g, 5.12 mmol) in DMF was added sodium hydride (60%, 205 mg, 5.12 mmol) under ice-cooling, and the mixture was stirred at room temperature for 1 hour. Methyl bromoacetate (0.582 mL, 6.14 mmol) was added to the mixture under ice-cooling, and the mixture was stirred at room temperature for 6 hours, and was poured into water. The mixture was extracted with ethyl acetate, and was dried with anhydrous magnesium sulfate. The solvent was distilled off under reduced pressure, to obtain the titled compound as a solid. 1.29 g (94.2%)
¹H-NMR (CDCl₃) δ; 1.21 (3H, t, J = 7.4 Hz), 3.11 (2H, q, J = 7.4 Hz), 3.85 (3H, s), 4.86 (2H, s), 6.92 (1H, d, J = 9.2 Hz), 7.25 (2 H, d, J = 9.2 Hz), 8.27 (2H, d, J = 9.2, 3.0 H), 8.60 (1H, d, J = 3.0 Hz)

### (2) Methyl (4-amino-2-propionylphenoxy)acetate

Methyl (4-nitro-2-propionylphenoxy)acetate (1.26 g, 4.72 mmol), 5% iridium carbon (200 mg) and ethyl acetate (40 mL) stirred for 24 hours under hydrogen atmosphere. The solvent was distilled off under reduced pressure, to obtain the titled compound as a solid. 1.10 g (98.2%)
¹H-NMR (CDCl₃) δ; 1.17 (3H, t, J = 7.4 Hz), 3.08 (2H, q, J = 7.4 Hz), 3.80 (3H, s), 4.64 (2H, s), 6.69 (1H, d, J = 6.8 Hz), 6.76 (1H, dd, J = 3.0, 6.8 Hz), 7.02 (1 H, d, J = 3.0 Hz)

### (3) Methyl [4-(3,4-dimethoxyphenylaminocarbonylamino)-2-propionylphenoxy]acetate

To a solution (40 mL) of methyl (4-amino-2-propionylphenoxy) acetate (1.10 g, 4.64 mmol) in THF was added 3,4-dimethoxyphenyl isocyanate (0.827 mL, 5.56 mmol) under ice-cooling, the mixture was stirred for 2 hours under ice-cooling, and the solvent was distilled off under reduced pressure. The residue was recrystallized from hexane and ethyl acetate. 1.58 g (81.9%)
¹H-NMR (CDCl₃) δ; 1.18 (3H, t, J = 7.2 Hz), 3.15 (2H, q, J = 7.2 Hz), 3.82 (3H, s), 3.85 (6H, s), 4.68 (2H, s), 6.74 to 7.38 (7H, m), 7.84 to 7.88 (1H, m)

### Example 65

### [4-(3,4-Dimethoxyphenylaminocarbonylamino)-2-propionylphenoxy]acetic acid

To a mixed solution (50 mL) of methyl [4-(3,4-dimethoxyphenylaminocarbonylamino)-2-propionylphenoxy]acetate (1.40 g, 3.36 mmol) in THF and methanol (20 mL) was added 1N aqueous solution of sodium hydroxide (10 mL), and the mixture was stirred at room temperature for 4 hours. The reaction solution was poured into water, acidified with 1N hydrochloric acid, and was extracted with ethyl acetate. The solvent was distilled off under reduced pressure, and the residue was purified with ethyl acetate. 1.30 g (96.3%)
¹H-NMR (CDCl₃) δ; 1.16 (3H, t, J = 7.2 Hz), 3.14 (2H, q, J = 7.2 Hz), 3.4 (3H, s), 3.88 (3H, s), 4.67 (2H, s), 6.69 to 6.86 (3H, m), 7.35 (1H, d, J = 2.2 Hz), 7.49 (1H, d, J = 2.2 Hz), 7.83 (1H, dd, J = 3.0, 8.8 Hz), 7.97 (1H, s), 8.18 (1H, s)

### Example 66

### N-Benzhydryl-2-[4-(3,4-dimethoxyphenylaminocarbonylamino)-2-propionylphenoxy]acetamide

A solution (2 mL) of [4-(3,4-dimethoxyphenylaminocarbonylamino)-2-propionylphenoxy]acetic acid (200 mg, 0.497 mmol), 1-hydroxy-1H-benzotriazole (115 mg, 0.749 mmol) and 1-ethyl-3-(3-dimethylaminopropyl)carbodiimide hydrochloride (119 mg, 0.621 mmol) in DMF was stirred at 0°C for 1 hour. Aminodiphenylmethane (0.171 mL, 0.994 mmol) was added to the mixture, the mixture was stirred at room temperature for 12 hours, and was poured into water. The mixture was extracted with ethyl acetate, and the extracted solution was washed with water and was dried with anhydrous magnesium sulfate. The solvent was distilled off under reduced pressure. The residue was purified by silicagel column chromatography (tetrahydrofuran), to obtain the titled compound as a solid. This was recrystallized from ethyl acetate. 31.0 mg (11.0%)
¹H-NMR (CDCl₃) δ; 1.10 (3H, t, J = 7.4 Hz), 2.90 (2H, q, J = 7.4 Hz), 3.89 (6H, s), 4.61 (2H, s), 6.35 (1H, d, J = 9.2 Hz), 6.59 to 7.33 (17H, m), 7.89 (1H, d, J = 2.6 Hz), 8.74 (1H, d, J = 9.2 Hz)
IR (KBr) cm⁻¹; 3295, 1682, 1549, 1495, 1209, 1028, 700

### Example 67

### N-(4-Benzhydryloxy-3-isobutyrylphenyl)-N'-(3,4-dihydro-1, 4-benzodioxim-6-yl)urea

To a solution (6 mL) of 1-[5-amino-2-(benzhydryloxy)phenyl]-2-methylpropan-1-one (200 mg, 0.579 mmol) in acetonitrile were added diisopropylethyl amine (0.116 mL, 0.695 mmol) and N,N'-disuccinimidyl carbonate (178 mg, 0.695 mmol) under ice-cooling, and the mixture was stirred for 1 hour under ice-cooling. To the reaction solution were added diisopropylethyl amine (0.116 mL, 0.695 mmol) and 3,4-ethylenedioxyaniline (105 mg, 0.695 mmol) under ice-cooling, the mixture was stirred under ice-cooling for 1 hour and at room temperature for 12 hours. The reaction solution was poured into water and was extracted with ethyl acetate. The extracted solution was washed with water, and was dried with anhydrous magnesium sulfate. The solvent was distilled off under reduced pressure, and the residue was purified by silicagel column chromatography (hexane:ethyl acetate = 2:1), to obtain the titled compound as a solid.
113 mg (37.4%)
¹H-NMR (CDCl₃) δ; 1.04 (6H, d, J = 6.6 Hz), 3.46 to 3.60 (1H, m), 4.23 (4H, s), 6.20 (1H, s), 6.51 (1H, bs), 6.70 to 7.58 (17H, m)

### Example 68

### N-(4-Benzhydryloxy-3-isobutylphenyl)-N'-(3,4-diethoxyphenyl)urea

### (1) 1,2-Ethoxy-4-nitrobenzene

A mixture of 4-nitrocatechol (5.00 g, 32.2 mmol), potassium carbonate (10.7 g, 77.4 mmol), iodoethane (6.19 mL, 77.4 mmol) and DMF (100 mL) was stirred at 70°C for 12 hours, and the reaction solution was poured into water and was extracted with ethyl acetate. The extracted solution was washed with water, was dried with anhydrous magnesium sulfate, and the solvent was distilled off under reduced pressure, to obtain the titled compound as a solid. 6.50 g (95.6%)
¹H-NMR (CDCl₃) δ; 1.47 to 1.57 (6H, m), 4.12 to 4.25 (4H, m), 6.89 (1H, d, J = 9.2 Hz), 7.73 (1H, d, J = 2.6 Hz), 7.88 (1H, dd, J = 2.6, 9.2 Hz)

### (2) 3,4-Diethoxy aniline

A solution of 1, 2-ethoxy-4-nitrobenzene (6.30 g, 29.9 mmol), 10% palladium carbon (600 mg) and ethanol (300 mL) was stirred under hydrogen atmosphere for 6 hours. The insolubles were filtered off, and the filtrate was concentrated, to obtain the titled compound as oil. 5.02 g (92.9%)
¹H-NMR (CDCl₃) δ; 1.34 to 1.46 (6H, m), 3.94 to 4.08 (4H, m), 6.21 (1H, d, J = 2.8, 8.4Hz), 6.30 (1H, d, J = 2.6 Hz), 6.73 (1H, d, J = 8.4 Hz)

### (3) N-(4-Benzhydryloxy-3-isobutylphenyl)-N'-(3,4-diethoxyphenyl)urea

To a solution (6 mL) of 1-[5-amino-2-(benzhydryloxy)phenyl]-2-methylpropan-1-one (200 mg, 0.579 mmol) in acetonitrile were added diisopropylethyl amine (0.116 mL, 0.695 mmol) and N,N'-disuccinimidyl carbonate (178 mg, 0.695 mmol) under ice-cooling, and the mixture was stirred under ice-cooling for 1 hour. To the reaction solution were added diisopropylethyl amine (0.116 mL, 0.695 mmol) and 3,4-diethoxyaniline (126 mg, 0.695 mmol) under ice-cooling, and the mixture was stirred under ice-cooling for 1 hour and at room temperature for 12 hours. The reaction solution was poured into water and was extracted with ethyl acetate. The extracted solution was washed with water, and was dried with anhydrous magnesium sulfate. The solvent was distilled off under reduced pressure, and the residue was purified by silicagel column chromatography (hexane:ethyl acetate = 2:1), to obtain the titled compound as a solid.
236 mg (73.8%)
¹H-NMR (CDCl₃) δ; 1.04 (6H, d, J = 6.4 Hz), 1.39 to 1.46 (6H, m), 3.51 to 3.57 (1H, m), 3.99 to 4.11 (4H, m), 6.21 (1H, s), 6.64 to 7.58 (18H, m)

### Example 69

### N-[4-(Benzhydryloxy)-3-isobutylphenyl]-N'-(1H-benzoimidazol-6-yl)urea

### (1) 1H-benzoimidazol-5-amine

A mixture of 5-nitrobenzoimidazol (2.00 g, 12.3 mmol) and 10% palladium carbon (200 mg) in ethanol (100 mL) and THF (100 mL) was stirred under hydrogen atmosphere at room temperature for 4 hours. The insolubles were filtered off, the filtrate was evaporated under reduced pressure, to obtain the titled compound as a solid. 1.50 g (92.0%)
¹H-NMR (CDCl₃) δ; 6.67 (1H, dd, J = 2.2, 9.2 Hz), 6.86 (1H, d, J = 2.2 Hz), 7.43 (1H, d, J = 9.2 Hz), 7.84 (1H, s)

### (2) N-[4-(Benzhydryloxy)-3-isobutylphenyl]-N'-(1H-benzoimidazol-6-yl)urea

To a solution (6 mL) of 1-[5-amino-2-(benzhydryloxy)phenyl]-2-methylpropan-1-one (190 mg, 0.550 mmol) in acetonitrile were added diisopropylethyl amine (0.110 mL, 0.660 mmol) and N,N'-disuccinimidyl carbonate (169 mg, 0.660 mmol) under ice-cooling, and the mixture was stirred under ice-cooling for 1 hour. To the reaction solution were added diisopropylethyl amine (0.110 mL, 0.660 mmol) and 1H-benzoimidazol-5-amine (87.8 mg, 0.6605 mmol) under ice-cooling, and the mixture was stirred under ice-cooling for 1 hour and at room temperature for 12 hours. The reaction solution was poured into water and was extracted with ethyl acetate. The extracted solution was washed with water, and was dried with anhydrous magnesium sulfate. The solvent was distilled off under reduced pressure, and the residue was purified by silicagel column chromatography (ethyl acetate:methanol = 10:1). The fractions containing the titled compound were collected, which was washed with water, was dried with anhydrous magnesium sulfate, and the solvent was distilled off under reduced pressure, to obtain the titled compound as a solid. 102 mg (36.7%)
¹H-NMR (CDCl₃) δ; 0.96 (6H, d, J = 7.0 Hz), 3.39 to 3.46 (1H, m), 6.07 (1H, s), 6.63 to 6.77 (2H, m), 7.18 to 7.46 (15H, m), 8.16 (2H, bs)

### Example 70

### N-{4-[Bis(4-fluorophenyl)methoxy]-3-isobutylphenyl}-N'-(3,4-dimethoxyphenyl)urea

### (1) 1-{2-[Bis(4-fluorophenyl)methoxy]-5-nitrophenyl}-2-methylpropan-1-one

A mixture of 1-(2-hydroxy-5-nitrophenyl)-2-methylpropan-1-one (500 mg, 2.39 mmol), 4,4'-difluorobenzhydrol (1.05 g, 4.78 mmol), 40% solution of diethyl azodicarbonate in toluene (2.08 g, 4.78 mmol) and a solution of triphenylphosphine (1.25 g, 4.78 mmol) in DMF (5 mL) was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 9:1), to obtain the titled compound as oil. 600 mg (61.0%)
¹H-NMR (CDCl₃) δ; 1.10 (6H, d, J = 7.0 Hz), 3.29 to 3.36 (1H, m), 6.35 (1H, s), 6.91 (1 H, d, J = 9.0 Hz), 7.26 to 7.36 (8H, m), 8.15 (1H, dd, J = 9.0, 2.8 Hz), 8.33 (1H, d, J = 2.8 Hz)

### (2) 1-{5-Amino-2-[bis(4-fluorophenyl)methoxy]phenyl}-2-methylpropan-1-one

1-{2-[Bis(4-fluorophenyl)methoxy]-5-nitrophenyl}-2-methylpropan-1-one (600 mg, 1.46 mmol) and 5% iridium carbon (100 mg) were added thereto, and the mixture was stirred for 12 hours under hydrogen atmosphere at room temperature. The insolubles were filtered off, and the filtrate was concentrated. The residue was purified by silicagel column chromatography (1:1), to obtain the titled compound as a solid. 200 mg (35.9%)
¹H-NMR (CDCl₃) δ; 1.03 (6H, d, J = 7.0 Hz), 3.36 to 3.43 (1H, m), 3.48 (2H, bs), 6.06 (1H, s), 6.59 (2 H, d, J = 1.8 Hz), 6.77 (1H, t, J = 1.8 Hz), 6.98 to 7.08 (4H, m), 7.26 to 7.33 (4H, m)

### (3) N-{4-[Bis(4-fluorophenyl)methoxy]-3-isobutylphenyl}-N'-(3,4-dimethoxyphenyl)urea

To a solution (4 mL) of 1-{5-amino-2-[bis(4-fluorophenyl)methoxy]phenyl}-2-methylpropan-1-one (190 mg, 0.498 mmol) in THF was added 3,4-dimethoxyphenyl isocyanate (0.0889 mL, 0.598 mmol) under ice-cooling, the mixture was stirred at 0°C for 2 hours, and the solvent was distilled off under reduced pressure. The residue was recrystallized from hexane and ethyl acetate. 261 mg (93.5%)
¹H-NMR (CDCl₃) δ; 1.04 (6H, d, J = 7.0 Hz), 3.62 to 3.49 (1H, m), 3.86 (3H, s), 3.87 (3H, s), 6.17 (1H, s), 6.56 (1H, s), 6.73 to 7.59 (15H, m)

### Example 71

### N-[4-(Benzhydryloxy)-3-isobutylphenyl]-N'-(3,4-dihydro-2H-1, 5-benzodioxepin-7-yl)urea

To a solution (6 mL) of 1-[5-amino-2-(benzhydryloxy)phenyl]-2-methylpropan-1-one (200 mg, 0.579 mmol) in acetonitrile were added diisopropylethyl amine (0.116 mL, 0.695 mmol) and N,N'-disuccinimidyl carbonate (178 mg, 0.695 mmol) under ice-cooling and the mixture was stirred under ice-cooling for 1 hour. To the reaction solution was added diisopropylethyl amine (0.116 mL, 0.695 mmol) and 3,4-dihydro-2H-1, 5-benzooxepin-7-amine (115 mg, 0.695 mmol) under ice-cooling, and the mixture was stirred under ice-cooling for 1 hour and at room temperature for 2 hours. The reaction solution was poured into water and was extracted with ethyl acetate. The extracted solution was washed with water, and was dried with anhydrous magnesium sulfate. The solvent was distilled off under reduced pressure, and the residue was purified by silicagel column chromatography (hexane:ethyl acetate = 2:1), to obtain the titled compound as a solid. 147 mg (47.3%)
¹H-NMR (CDCl₃) δ; 1.04 (6H, d, J = 7.0 Hz), 2.14 to 2.19 (2H, m), 3.50 to 3.57 (1H, m), 4.13 to 4.21 (4H, m), 6.21 (1H, s), 6.65 (1H, bs), 6.79 to 7.57 (17H, m)

### Example 72

### N-[4-(Benzhydryloxy)-3-isobutylphenyl]-N'-(1,3-benzodioxol-5-yl)urea

To a solution (6 mL) of 1-[5-amino-2-(benzhydryloxy)phenyl]-2-methylpropan-1-one (200 mg, 0.579 mmol) in acetonitrile were added diisopropylethyl amine (0.116 mL, 0.695 mmol) and N,N'-disuccinimidyl carbonate (178 mg, 0.695 mmol) under ice-cooling, and the mixture was stirred under ice-cooling for 1 hour. To the reaction solution were added diisopropylethyl amine (0.116 mL, 0.695 mmol) and 3,4-(methylenedioxy)aniline (95.3 mg, 0.695 mmol) under ice-cooling, and the mixture was stirred under ice-cooling for 1 hour and at room temperature for 1 hour. The reaction solution was poured into water and was extracted with ethyl acetate. The extracted solution was washed with water, and was dried with anhydrous magnesium sulfate. The solvent was distilled off under reduced pressure, and the residue was purified by silicagel column chromatography (hexane:ethyl acetate = 1:1), to obtain the titled compound as a solid. 135 mg (45.9%)
¹H-NMR (CDCl₃) δ; 1.02 (6H, d, J = 7.0 Hz), 3.46 to 3.60 (1H, m), 5.86 (3H, s), 6.17 (1H, s), 6.57 to 7.53 (18H, m)

### Example 73

### N-{5-[({[4-(Benzhydryloxy)-3-isobutyrylphenyl]amino}carbonyl)amino]-2-methoxyphenyl}methane sulfonamide

### (1) N-(2-Methoxy-5-nitrophenyl)methane sulfonamide

To a solution (15 mL) of 2-amino-4-nitroanisol (5.00 g, 29.7 mmol) in pyridine was added dropwise methane sulfonylchloride (3.45 mL, 44.6 mmol), and the mixture was stirred at room temperature for 2 hours and was poured into water, to obtain the titled compound as a solid. 7.00 g (96.4%)
¹H-NMR (CDCl₃) δ; 3.09 (3H, s), 4.03 (3H, s), 6.98 to 7.02 (2H, m), 8.07 (1H, dd, J = 8.8, 2.6 Hz), 8.41 (1H, d, J = 2.6 Hz)

### (2) N-(5-Amino-2-methoxyphenyl)methane sulfonamide

To a mixed solution of N-(2-methoxy-5-nitrophenyl)methane sulfonamide (6.90 g, 28.0 mmol) in THF (150 mL) and ethanol (150 mL) was added 10% palladium carbon (690 mg), and the mixture was stirred under hydrogen atmosphere for 5 hours. The insolubles were filtered off, and the filtrate was concentrated, to obtain the titled compound as a solid. 6.00 g (99.0%)
¹H-NMR (CDCl₃) δ; 2.96 (3H, s), 3.52 (2H, bs), 3.80 (3H, s), 6.44 (1H, dd, J = 1.8, 5.6 Hz), 6.73 (1H, d, J = 5.6 Hz), 6.79 (1H, bs), 6.95 (1H, d, J = 1.8 Hz)

### (3) N-{5-[({[4-(Benzhydryloxy)-3-isobutyrylphenyl]amino}carbonyl)amino]-2-methoxyphenyl}methane sulfonamide

To a solution (6 mL) of 1-[5-amino-2-(benzhydryloxy)phenyl]-2-methylpropan-1-one (200 mg, 0.579 mmol) in acetonitrile were added diisopropylethyl amine (0.116 mL, 0.695 mmol) and N,N'-disuccinimidyl carbonate (178 mg, 0.695 mmol) under ice-cooling, and the mixture was stirred under ice-cooling for 1 hour. To the reaction solution were added diisopropylethyl amine (0.116 mL, 0.695 mmol) and N-(5-amino-2-methoxyphenyl)methane sulfonamide(150 mg, 0.695 mmol) under ice-cooling, and the mixture was stirred under ice-cooling for 1 hour and at room temperature for 12 hours. The reaction solution was poured into water and was extracted with ethyl acetate. The extracted solution was washed with water, and was dried with anhydrous magnesium sulfate. The solvent was distilled off under reduced pressure, and the residue was purified by silicagel column chromatography (hexane:ethyl acetate = 1:1), to obtain the titled compound as a solid. The product was recrystallized from hexane and ethyl acetate. 80.0 mg (23.5%)
¹H-NMR (CDCl₃) δ; 1.05 (6H, d, J = 7.0 Hz), 2.97 (3H, s), 3.46 to 3.56 (1H, m), 3.83 (3H, s), 6.21 (1H, s), 6.79 to 7.56 (19H, m)

### Example 74

### Methyl 2-[bis(3-fluorophenyl)methoxy]-5-({[(3,4-dimethoxyphenyl)amino]carbonyl}amino)benzoate

### (1) bis(3-fluorophenyl)methanol

To a solution (10 mL) of 3,3'-difluorobenzophenol (1.00 g, 4.58 mmol) in ethanol was added sodium borohydride (866 mg, 2.29 mmol), the mixture was stirred at room temperature for 2 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil.
982 mg (97.2%)
¹H-NMR (CDCl₃) δ; 2.31 (1H, d, J = 3.2 Hz), 5.81 (1H, d, J = 3.2 Hz), 6.92 to 7.37 (8H, m)

### (2) Methyl 2-[bis(3-fluorophenyl)methoxy]-5-nitrobenzoate

A mixture of methyl 2-hydroxy-5-nitrobenzoate (413 mg, 2.09 mmol), bis(3-fluorophenyl)methanol (922 mg, 4.19 mmol), 40% solution of diethyl azodicarbonate in toluene (1.82 g, 4.19 mmol) and solution (5 mL) of triphenylphosphine (1.10 g, 4.19 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 10:1), to obtain the titled compound as a solid. 410 mg (49.2%)
¹H-NMR (CDCl₃) δ; 4.02 (3H, s), 6.38 (1H, s), 6.96 to 7.41 (9H, m), 8.22 (1 H, dd, J = 2.8, 9.2 Hz), 8.76 (1H, d, J = 2.8 Hz)

### (3) Methyl 5-amino-2-[bis(3-fluorophenyl)methoxy]benzoate

Methyl 2-[bis(3-fluorophenyl)methoxy]-5-nitrobenzoate (400 mg, 1.00 mmol) and 5% iridium carbon (40 mg) were added thereto, and the mixture was stirred for 12 hours under hydrogen atmosphere at room temperature. The insolubles were filtered off, and the filtrate was concentrated. To the residue was added hexane, to obtain the titled compound as a solid. 188 mg (50.9%)
¹H-NMR (CDCl₃) δ; 3.87 (3H, s), 6.09 (1H, s), 6.64 (2 H, d, J = 1.6 Hz), 6.90 to 7.35 (9H, m)

### (4) Methyl 2-[bis(3-fluorophenyl)methoxy]-5-({[(3,4-dimethoxyphenyl)amino]carbonyl}amino)benzoate

To a solution of methyl 5-amino-2-[bis(3-fluorophenyl)methoxy]benzoate (184 mg, 0.498 mmol) in THF (3 mL) was added 3,4-dimethoxyphenyl isocyanate (0.0889 mL, 0.598 mmol) under ice-cooling, the mixture was stirred at 0°C for 2 hours, and the solvent was distilled off under reduced pressure. The residue was purified by silicagel column chromatography (hexane:ethyl acetate = 1:1), to obtain the titled compound as a solid. 106 mg (38.8%)
¹H-NMR (CDCl₃) δ; 3.81 (3H, s), 3.82 (3H, s), 3.88 (3H, s), 6.14 (1H, s), 6.66 to 7.60 (16H, m)
IR (KBr) cm⁻¹; 3326, 1726, 1651, 1611, 1593, 1557, 1514, 1497, 1451, 1414, 1221, 1165, 1136, 1082, 1028, 772, 754, 733

### Example 75

### Methyl 5-({[(3,4-dimethoxyphenyl)amino]carbonyl}amino)-2-[(4-fluorophenyl)(phenyl)methoxy]benzoate

### (1) (4-fluorophenyl)(phenyl)methanol

To a solution (100 mL) of p-fluorobenzophenol (10.0 g, 49.9 mmol) in ethanol was added sodium borohydride (945 mg, 25.0 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil.
9.63 g (96.3%)
¹H-NMR (CDCl₃) δ; 2.23 (1H, d, J = 3.8 Hz), 5.83 (1H, d, J = 3.8 Hz), 6.97 to 7.38 (9H, m)

### (2) Methyl 2-[(4-fluorophenyl)(phenyl)methoxy]-5-nitrobenzoate

A mixture of methyl 2-hydroxy-5-nitrobenzoate (1.62 g, 8.24 mmol), (4-fluorophenyl)(phenyl)methanol (2.00 g, 9.89 mmol), 40% solution of diethyl azodicarbonate in toluene (5.38 g, 12.4 mmol) and a solution (5 mL) of triphenylphosphine (2.59 g, 9.89 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 10:1), to obtain the titled compound as oil. 2.14 g (68.2%)
¹H-NMR (CDCl₃) δ; 3.98 (3H, s), 6.39 (1H, s), 6.98 to 7.52 (10H, m), 8.18 (1 H, dd, J = 3.0, 9.6 Hz), 8.72 (1H, d, J = 3.0 Hz)
IR (KBr) cm⁻¹; 1734, 1613, 1588, 1510, 1487, 1439, 1346, 1277, 1254, 1227, 1130, 1003, 824, 748, 698

### (3) Methyl 5-amino-2-[(4-fluorophenyl)(phenyl)methoxy]benzoate

Methyl 2-[(4-fluorophenyl)(phenyl)methoxy]-5-nitrobenzoate (2.10 g, 5.50 mmol) and 5% iridium carbon (210 mg) were added thereto, and the mixture was stirred under hydrogen atmosphere at room temperature for 2 days. The insolubles were filtered off, and the filtrate was concentrated. To the residue was added hexane, to obtain the titled compound as oil. 1.87 g (96.9%)
¹H-NMR (CDCl₃) δ; 3.49 (2H, bs), 3.83 (3H, s), 6.11 (1H, s), 6.58 to 7.48 (12H, m)

### (4) Methyl 5-({[(3,4-dimethoxyphenyl)amino]carbonyl}amino)-2-[(4-fluorophenyl)(phenyl)methoxy]benzoate

To a solution (10 mL) of methyl 5-amino-2-[(4-fluorophenyl)(phenyl)methoxy]benzoate (1.00 g, 2.85 mmol) in THF was added 3,4-dimethoxyphenyl isocyanate (0.509 mL, 3.42 mmol) under ice-cooling, the mixture was stirred at 0°C for 2 hours, and the solvent was distilled off under reduced pressure. The residue was purified by silicagel column chromatography (hexane:ethyl acetate = 1:2), to obtain the titled compound as a solid. 995 mg (65.9%)
¹H-NMR (CDCl₃) δ; 3.84 (3H, s), 3.85 (3H, s), 3.87 (3H, s), 6.20 (1H, s), 6.51 (1H, s), 6.61 (1H, s), 6.68 to 7.61 (15H, m)

### Example 76

### Methyl 2-[bis(2-fluorophenyl)methoxy]-5-({[(3,4-dimethoxyphenyl)amino]carbonyl}amino)benzoate

### (1) Bis(2-fluorophenyl)methanol

To a solution (200 mL) of 2-bromofluorobenzene (10.0 g, 57.1 mmol) in THF was added dropwise 1.6N solution of butyllithium in hexane (42.9 mL, 68.3 mmol) at -78°C, and the mixture was stirred at -78°C for 10 minutes. A solution (20 mL) of 2-fluorobenzaldehyde (7.09 g, 57.1 mmol) in THF was added dropwise to the mixture, and the mixture was stirred for 1 hour at -78 to -65°C. The reaction solution was poured into an aqueous solution of saturated ammonium chloride, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as a solid. 4.14 g (32.9%)
¹H-NMR (CDCl₃) δ; 2.41 (1H, d, J = 4.6 Hz), 6.42 (1H, d, J = 4.6 Hz), 6.98 to 7.49 (8H, m)

### (2) Methyl 2-[bis(2-fluorophenyl)methoxy]-5-nitrobenzoate

A solution (5 mL) of methyl 2-hydroxy-5-nitrobenzoate (1.49 g, 7.57 mmol), bis(2-fluorophenyl)methanol (2.00 g, 9.08 mmol), 40% solution of diethyl azodicarbonate in toluene (5.60 g, 12.9 mmol) and triphenylphosphine (2.38 g, 9.08 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 9:1), to obtain the titled compound as oil. 1.61 g (53.3%)
¹H-NMR (CDCl₃) δ; 3.98 (3H, s), 6.99 to 7.75 (10H, m), 8.26 (1 H, dd, J = 2.6, 9.4 Hz), 8.73 (1H, d, J = 2.6 Hz)

### (3) Methyl 5-amino-2-[bis(2-fluorophenyl)methoxy]benzoate

Methyl 2-[bis(2-fluorophenyl)methoxy]-5-nitrobenzoate (1.55 g, 3.88 mmol) and 5% iridium carbon (200 mg) were added thereto, and the mixture was stirred under hydrogen atmosphere at room temperature for 2 days. The insolubles were filtered off, and the filtrate was concentrated. The residue was purified by silicagel column chromatography (hexane:ethyl acetate = 1:1), to obtain the titled compound as oil. 800 mg (55.9%)
¹H-NMR (CDCl₃) δ; 3.48 (2H, bs), 3.83 (3H, s), 6.66 to 7.76 (12H, m)

### (4) Methyl 2-[bis(2-fluorophenyl)methoxy]-5-({[(3,4-dimethoxyphenyl)amino]carbonyl}amino)benzoate

To a solution (8 mL) of methyl 5-amino-2-[bis(2-fluorophenyl)methoxy]benzoate (780 mg, 2.11 mmol) in THF was added 3,4-dimethoxyphenyl isocyanate (0.377 mL, 2.53 mmol) under ice-cooling, the mixture was stirred at 0°C for 2 hours, and the solvent was distilled off under reduced pressure. The residue was recrystallized from hexane and ethyl acetate, to obtain the titled compound as a solid. 1.08 g (93.1%)
¹H-NMR (CDCl₃) δ; 3.84 (3H, s), 3.85 (3H, s), 3.87 (3H, s), 6.48 to 7.75 (17H, m)

### Example 77

### Methyl 5-({[(3,4-dimethoxyphenyl)amino]carbonyl}amino)-2-[(2-fluorophenyl)(4-fluorophenyl)methoxy]benzoate

### (1) (2-Fluorophenyl)(4-fluorophenyl)methanol

To a solution (100 mL) of 2,4'-difluorobenzophenol (10.0 g, 45.8 mmol) in ethanol was added sodium borohydride (867 mg, 22.9 mmol), the mixture was stirred at room temperature for 2 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 9.88 g (97.8%)
¹H-NMR (CDCl₃) δ; 2.32 (1H, d, J = 4.0 Hz), 5.83 (1H, d, J = 4.0 Hz), 6.95 to 7.53 (8H, m)

### (2) Methyl 2-[(2-fluorophenyl)(4-fluorophenyl)methoxy]-5-nitrobenzoate

A mixture of methyl 2-hydroxy-5-nitrobenzoate (2.23 g, 11.3 mmol), (2-fluorophenyl)(4-fluorophenyl)methanol (3.00 g, 13.6 mmol), 40% solution of diethyl azodicarbonate in toluene (7.87 g, 18.1 mmol) and a solution (5 mL) of triphenylphosphine (3.56 g, 13.6 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 9:1), to obtain the titled compound as a solid. 2.00 g (48.0%)
¹H-NMR (CDCl₃) δ; 3.99 (3H, s), 6.78 (1H, s), 7.01 to 7.70 (9H, m), 8.23 (1 H, dd, J = 2.6, 9.2 Hz), 8.74 (1H, d, J = 2.6 Hz)

### (3) Methyl 5-amino-2-[(2-fluorophenyl)(4-fluorophenyl)methoxy]benzoate

To a solution (100 mL) of methyl 2-[(2-fluorophenyl)(4-fluorophenyl)methoxy]-5-nitrobenzoate (2.00 g, 5.01 mmol) in ethyl acetate was added 5% iridium carbon (200 mg), and the mixture was stirred under hydrogen atmosphere at room temperature for 2 days. The insolubles were filtered off, and the filtrate was concentrated. The residue was purified by silicagel column chromatography (hexane:ethyl acetate = 1:1), to obtain the titled compound as oil. 1.79 g (96.8%)
¹H-NMR (CDCl₃) δ; 3.85 (3H, s), 6.49 (1H, s), 6.62 to 7.75 (11H, m)

### (4) Methyl 5-({[(3,4-dimethoxyphenyl)amino]carbonyl}amino)-2-[(2-fluorophenyl)(4-fluorophenyl)methoxy]benzoate

To a solution (60 mL) of methyl 5-amino-2-[(2-fluorophenyl)(4-fluorophenyl)methoxy]benzoate (1.74 g, 4.71 mmol) in THF was added 3,4-dimethoxyphenyl isocyanate (0.841 mL, 5.65 mmol) under ice-cooling, the mixture was stirred at 0°C for 2 hours, and the solvent was distilled off under reduced pressure. The residue was purified by silicagel column chromatography (hexane:ethyl acetate = 1:1), to obtain the titled compound as a solid. This was recrystallized from hexane and ethyl acetate. 743 mg (28.8%)
¹H-NMR (CDCl₃) δ; 3.83 (3H, s), 3.84 (3H, s), 3.88 (3H, s), 6.55 to 7.66 (17H, m)

### Example 78

### Methyl 2-[(2,6-difluorophenyl)(phenyl)methoxy]-5-({[(3,4-dimethoxyphenyl)amino]carbonyl}amino)benzoate

### (1) (2,6-Difluorophenyl)(phenyl)methanol

To a solution (100 mL) of 2,6-difluorobenzophenol (10.0 g, 45.8 mmol) in ethanol was added sodium borohydride (867 mg, 22.9 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 9.80 g (97.0%)
¹H-NMR (CDCl₃) δ; 2.75 - 2.82 (1H, m), 6.24 (1H, d, J = 8.8 Hz), 6.84 to 7.42 (8H, m)

### (2) Methyl 2-[(2, 6-difluorophenyl)(phenyl)methoxy]-5-nitrobenzoate (16d)

A mixture of methyl 2-hydroxy-5-nitrobenzoate (2.23 g, 11.3 mmol), (2, 6-difluorophenyl)(phenyl)methanol (3.00 g, 13.6 mmol), 40% solution of diethyl azodicarbonate in toluene (7.87 g, 18.1 mmol) and a solution (5 mL) of triphenylphosphine (3.56 g, 13.6 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as a solid. 410 mg (9.10%)
¹H-NMR (CDCl₃) δ; 3.99 (3H, s), 6.86 to 7.63 (10H, m), 8.24 (1 H, dd, J = 3.0, 9.2 Hz), 8.72 (1H, d, J = 3.0 Hz)

### (3) Methyl 5-amino-2-[(2, 6-difluorophenyl)(phenyl)methoxy]benzoate (17d)

To a solution (20 mL) of methyl 2-[(2, 6-difluorophenyl)(phenyl)methoxy]-5-nitrobenzoate (400 mg, 1.00 mmol) in ethyl acetate was added 5% iridium carbon (50 mg), and the mixture was stirred under hydrogen atmosphere at room temperature for 2 days. The insolubles were filtered off, and the filtrate was concentrated. To the residue was added hexane, to obtain the titled compound as oil. 360 mg (97.3%)
H-NMR (CDCl₃) δ; 3.52 (2H, bs), 3.82 (3H, s), 6.11 (1H, s), 6.598 to 7.61 (12H, m)

### (4) Methyl 2-[(2, 6-difluorophenyl)(phenyl)methoxy]-5-({[(3,4-dimethoxyphenyl)amino]carbonyl}amino)benzoate

To a solution (4 mL) of methyl 5-amino-2-[(2, 6-difluorophenyl)(phenyl)methoxy]benzoate (350 mg, 0.948 mmol) in THF was added 3,4-dimethoxyphenyl isocyanate (0.169 mL, 1.14 mmol) under ice-cooling, the mixture was stirred at 0°C for 2 hours, and the solvent was distilled off under reduced pressure. The residue was recrystallized from hexane and ethyl acetate. 467 mg (87.9%)
¹H-NMR (CDCl₃) δ; 3.84 (3H, s), 3.85 (3H, s), 3.87 (3H, s), 6.50 to 7.59 (17H, m)

### Example 79

### Methyl 2-[(4-chlorophenyl)(phenyl)methoxy]-5-({[(3,4-dimethoxyphenyl)amino]carbonyl}amino)benzoate

### (1) (4-Chlorophenyl)(phenyl)methanol

To a solution (100 mL) of 4-chlorobenzophenol (10.0 g, 46.2 mmol) in ethanol and THF (10 mL) was added sodium borohydride (876 mg, 23.1 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as a solid. 8.90 g (88.8%)
¹H-NMR (CDCl₃) δ; 2.20 (1H, d, J = 3.2 Hz), 5.82 (1H, d, J = 3.2 Hz), 7.26 to 7.36 (9H, m)

### (2) Methyl 2-[(4-chlorophenyl)(phenyl)methoxy]-5-nitrobenzoate

A mixture of methyl 2-hydroxy-5-nitrobenzoate (2.25 g, 11.4 mmol), (4-chlorophenyl)(phenyl)methanol (3.00 g, 13.7 mmol), 40% solution of diethyl azodicarbonate in toluene (7.95 g, 18.3 mmol) and a solution (8 mL) of triphenylphosphine (3.59 g, 13.7 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 4.43 g (97.6%)
¹H-NMR (CDCl₃) δ; 3.98 (3H, s), 6.38 (1H, s), 6.99 (1H, d, J = 9.4 Hz), 7.26 to 7.50 (9H, m), 8.19 (1 H, dd, J = 2.6, 9.4 Hz), 8.73 (1H, d, J = 2.6 Hz)

### (3) Methyl 5-amino-2-[(4-chlorophenyl)(phenyl)methoxy]benzoate

A mixture of methyl 2-[(4-chlorophenyl)(phenyl)methoxy]-5-nitrobenzoate (2.00 g, 5.03 mmol), iron (1.40 g, 25.1 mmol) and calcium chloride (279 mg, 2.52 mmol) in ethanol (32 mL) and water (8 mL) was heated to reflux for 2 hours, the insolubles were filtered off, and the filtrate was concentrated. The residue was purified by silicagel column chromatography (hexane:ethyl acetate = 1 :1 ), to obtain the titled compound as oil. 900 mg (48.6%)
¹H-NMR (CDCl₃) δ; 3.48 (2H, bs), 3.83 (3H, s), 6.09 (1H, s), 6.62 to 6.63 (2H, m), 7.12 to 7.46 (10H, m)

### (4) Methyl 2-[(4-chlorophenyl)(phenyl)methoxy]-5-({[(3,4-dimethoxyphenyl)amino]carbonyl}amino)benzoate

To a solution (20 mL) of methyl 5-amino-2-[(4-chlorophenyl)(phenyl)methoxy]benzoate (900 mg, 2.45 mmol) in THF was added 3,4-dimethoxyphenyl isocyanate (0.437 mL, 2.94 mmol) under ice-cooling, the mixture was stirred at 0°C for 2 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:2 ), to obtain the titled compound as a solid. 662 mg (49.4%)
¹H-NMR (CDCl₃) δ; 3.72 (3H, s), 3.74 (3H, s), 3.80 (3H, s), 6.09 (1H, s), 6.60 to 7.61 (17H, m)

### Example 80

### Methyl 5-({[(3,4-dimethoxyphenyl)amino]carbonyl}amino)-2-[(2-fluorophenyl)(phenyl)methoxy]benzoate

### (1) (2-Fluorophenyl)(phenyl)methanol

To a solution (100 mL) of p-fluorobenzophenol (10.0 g, 49.9 mmol) in ethanol was added sodium borohydride (945 mg, 25.0 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 9.80 g (97.0%)
¹H-NMR (CDCl₃) δ; 2.31 (1H, d, J = 4.8 Hz), 6.14 (1H, d, J = 4.8 Hz), 6.96 to 7.55 (9H, m)

### (2) Methyl 2-[(2-fluorophenyl)(phenyl)methoxy]-5-nitrobenzoate

A mixture of methyl 2-hydroxy-5-nitrobenzoate (2.42 g, 12.3 mmol), (2-fluorophenyl)(phenyl)methanol (3.00 g, 14.8 mmol), 40% solution of diethyl azodicarbonate (8.31 g, 19.7 mmol) and a solution (8 mL) of triphenylphosphine (3.88 g, 14.8 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as a solid. 2.88 g (61.4%)
¹H-NMR (CDCl₃) δ; 4.00 (3H, s), 6.79 (1H, s), 6.96 to 7.73 (10H, m), 8.21 (1 H, dd, J = 2.8, 9.2 Hz), 8.73 (1H, d, J = 2.8 Hz)

### (3) Methyl 5-amino-2-[(2-fluorophenyl)(phenyl)methoxy]benzoate

A mixture of methyl 2-[(2-fluorophenyl)(phenyl)methoxy]-5-nitrobenzoate (2.87 g, 7.53 mmol), iron (2.10 g, 37.7 mmol) and calcium chloride (418 mg, 3.77 mmol) in ethanol (54 mL) and water (13 mL) was stirred at 100°C for 5 hours. The insolubles were filtered off, and the filtrate was concentrated. The residue was purified by silicagel column chromatography (hexane:ethyl acetate = 1:1), to obtain the titled compound as oil. 1.89 g (71.3%)
¹H-NMR (CDCl₃) δ; 3.46 (2H, bs), 3.85 (3H, s), 6.52 (1H, s), 6.61 to 7.77 (12H, m)

### (4) Methyl 5-({[(3,4-dimethoxyphenyl)amino]carbonyl}amino)-2-[(2-fluorophenyl)(phenyl)methoxy]benzoate

To a solution (10 mL) of methyl 5-amino-2-[(2-fluorophenyl)(phenyl)methoxy]benzoate (1.87 g, 5.33 mmol) in THF was added 3,4-dimethoxyphenyl isocyanate (0.951 mL, 6.39 mmol) under ice-cooling, the mixture was stirred at 0°C for 2 hours, and the solvent was distilled off under reduced pressure. The residue was recrystallized from hexane and ethyl acetate. 1.77 g (62.5%)
¹H-NMR (CDCl₃) δ; 3.83 (3H, s), 3.84 (3H, s), 3.89 (3H, s), 6.55 to 7.70 (18H, m)

### Example 81

### Methyl 5-({[(3,4-dimethoxyphenyl)amino]carbonyl}amino)-2-[(4-trifluoromethylphenyl)(phenyl)methoxy]benzoate

### (1) (4-Trifluoromethylphenyl)(phenyl)methanol

To a solution (50 mL) of 4-trifluoromethylbenzophenol (5.00 g, 20.0 mmol) in ethanol was added sodium borohydride (378 mg, 10.0 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 4.85 g (96.2%)
¹H-NMR (CDCl₃) δ; 2.36 (1H, d, J = 3.2 Hz), 5.87 (1H, d, J = 3.2 Hz), 7.25 to 7.34 (5H, m), 7.50 (2H, d, J = 8.4 Hz), 7.58 (2H, d, J = 8.4 Hz)

### (2) Methyl 2-[(4-trifluoromethylphenyl)(phenyl)methoxy]-5-nitrobenzoate

A mixture of methyl 2-hydroxy-5-nitrobenzoate (1.93 g, 9.81 mmol), (4-trifluoromethylphenyl)(phenyl)methanol (3.00 g, 11.8 mmol), 40% solution of diethyl azodicarbonate in toluene (6.83 g, 15.7 mmol) and a solution (8 mL) of triphenylphosphine (3.09 g, 11.8 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 3.37 g (79.7%)
¹H-NMR (CDCl₃) δ; 4.00 (3H, s), 6.46 (1H, s), 7.00 (1H, d, J = 9.2 Hz), 7.26 to 7.72 (9H, m), 8.20 (1 H, dd, J = 3.0, 9.2 Hz), 8.74 (1H, d, J = 3.0 Hz)

### (3) Methyl 5-amino-2-[(4-trifluoromethylphenyl)(phenyl)methoxy]benzoate

A mixture of methyl 2-[(4-trifluoromethylphenyl)(phenyl)methoxy]-5-nitrobenzoate (3.30 g, 7.65 mmol), iron (2.14 g, 38.3 mmol) and calcium chloride (425 mg, 3.83 mmol) in ethanol (50 mL) and water (12 mL) was stirred at 100°C for 3 hours. The insolubles were filtered off, and the filtrate was concentrated. The residue was purified by silicagel column chromatography (hexane:ethyl acetate = 2:1), to obtain the titled compound as oil. 2.06 g (67.1%)
¹H-NMR (CDCl₃) δ; 3.46 (2H, bs), 3.84 (3H, s), 6.16 (1H, s), 6.62 to 6.64 (2H, m), 7.13 to 7.67 (10H, m)

### (4) Methyl 5-({[(3,4-dimethoxyphenyl)amino]carbonyl}amino)-2-[(4-trifluoromethylphenyl)(phenyl)methoxy]benzoate

To a solution (50 mL) of methyl 5-amino-2-[(4-trifluoromethylphenyl)(phenyl)methoxy]benzoate (2.00 g, 4.98 mmol) in THF was added 3,4-dimethoxyphenyl isocyanate (0.890 mL, 5.98 mmol) under ice-cooling, the mixture was stirred at 0°C for 2 hours, and the solvent was distilled off under reduced pressure. The residue was purified by silicagel column chromatography (hexane:ethyl acetate = 2:1), to obtain the titled compound as a solid. 1.87 g (64.7%)
¹H-NMR (CDCl₃) δ; 3.81 (3H, s), 3.83 (3H, s), 3.86 (3H, s), 6.23 (1H, s), 6.66 to 7.66 (17H, m)

### Example 82

### Methyl 5-({[(3,4-dimethoxyphenyl)amino]carbonyl}amino)-2-[(2-chlorophenyl)(phenyl)methoxy]benzoate

### (1) (2-Chlorophenyl)(phenyl)methanol

To a solution (100 mL) of 2-chlorobenzophenol (10.0 g, 46.2 mmol) in ethanol was added sodium borohydride (876 mg, 23.1 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 9.50 g (94.1%)
¹H-NMR (CDCl₃) δ; 2.35 (1H, d, J = 4.0 Hz), 6.22 (1H, d, J = 4.0 Hz), 7.18 to 7.63 (9H, m)

### (2) Methyl 2-[(2-chlorophenyl)(phenyl)methoxy]-5-nitrobenzoate

A mixture of methyl 2-hydroxy-5-nitrobenzoate (2.25 g, 11.4 mmol), (2-chlorophenyl)(phenyl)methanol (3.00 g, 13.7 mmol), 40% solution of diethyl azodicarbonate in toluene (7.95 g, 18.3 mmol) and a solution (8 mL) of triphenylphosphine (3.59 g, 13.7 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as a solid. 2.84 g (62.6%)
¹H-NMR (CDCl₃) δ; 3.98 (3H, s), 6.90 (1H, s), 7.02 (1H, d, J = 9.0 Hz), 7.25 to 7.76 (9H, m), 8.23 (1 H, dd, J = 2.8, 9.0 Hz), 8.72 (1H, d, J = 2.8 Hz)

### (3) Methyl 5-amino-2-[(2-chlorophenyl)(phenyl)methoxy]benzoate

A mixture of methyl 2-[(2-chlorophenyl)(phenyl)methoxy]-5-nitrobenzoate (2.80 g, 7.04 mmol), iron (1.97 g, 35.2 mmol) and calcium chloride (391 mg, 3.52 mmol) in ethanol (50 mL) and water (13 mL) was stirred at 100°C for 4 hours. The insolubles were filtered off, and the filtrate was concentrated. The residue was purified by silicagel column chromatography (hexane:ethyl acetate = 2:1), to obtain the titled compound as a solid. 1.23 g (47.5%)
¹H-NMR (CDCl₃) δ; 3.45 (2H, bs), 3.85 (3H, s), 6.63 to 6.73 (3H, m), 7.12 to 7.83 (10H, m)

### (4) Methyl 5-({[(3,4-dimethoxyphenyl)amino]carbonyl}amino)-2-[(2-chlorophenyl)(phenyl)methoxy]benzoate

To a solution (50 mL) of methyl 5-amino-2-[(2-chlorophenyl)(phenyl)methoxy]benzoate (1.20 g, 3.26 mmol) in THF was added 3,4-dimethoxyphenyl isocyanate (0.582 mL, 3.91 mmol) under ice-cooling, the mixture was stirred at 0°C for 2 hours, and the solvent was distilled off under reduced pressure. The residue was recrystallized from hexane and ethyl acetate. 1.70 g (95.5%)
¹H-NMR (CDCl₃) δ; 3.85 (3H, s), 3.86 (3H, s), 3.88 (3H, s), 6.42 (1H, s), 6.52 (1H, s), 6.73 to 7.78 (16H, m)

### Example 83

### Methyl 5-({[(3,4-dimethoxyphenyl)amino]carbonyl}amino)-2-[(2,4-dichlorophenyl)(phenyl)methoxy]benzoate

### (1) (2,4-Dichlorophenyl)(phenyl)methanol

To a solution of 2,4-dichlorobenzophenol (10.0 g, 39.8 mmol) in ethanol (100 mL) and THF (100 mL) was added sodium borohydride (753 mg, 19.9 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 9.40 g (93.0%)
¹H-NMR (CDCl₃) δ; 2.34 (1H, d, J = 3.6 Hz), 6.15 (1H, d, J = 3.6 Hz), 7.25 to 7.37 (7H, m), 7.57 (1H, d, J = 8.4 Hz)

### (2) Methyl 2-[(2,4-dichlorophenyl)(phenyl)methoxy]-5-nitrobenzoate

A mixture of methyl 2-hydroxy-5-nitrobenzoate (1.96 g, 9.92 mmol), (2,4-dichlorophenyl)(phenyl)methanol (3.00 g, 11.9 mmol), 40% solution of diethyl azodicarbonate in toluene (5.17 g, 11.9 mmol) and a solution (8 mL) triphenylphosphine (3.12 g, 11.9 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 4.00 g (93.2%)
¹H-NMR (CDCl₃) δ; 3.97 (3H, s), 6.83 (1H, s), 7.00 (1H, d, J = 9.2 Hz), 7.24 to 7.74 (8H, m), 8.24 (1 H, dd, J = 2.8, 9.2 Hz), 8.72 (1H, d, J = 2.8 Hz)

### (3) Methyl 5-amino-2-[(2,4-dichlorophenyl)(phenyl)methoxy]benzoate

A mixture of methyl 2-[(2,4-dichlorophenyl)(phenyl)methoxy]-5-nitrobenzoate (4.00 g, 9.25 mmol), iron (2.58 g, 46.3 mmol) and calcium chloride (513 mg, 4.63 mmol) in ethanol (64 mL) and water (16 mL) was stirred at 100°C for 5 hours. The insolubles were filtered off, and the filtrate was concentrated. The residue was purified by silicagel column chromatography (hexane:ethyl acetate = 5:1), to obtain the titled compound as a solid. 860 mg (24.1%)
¹H-NMR (CDCl₃) δ; 3.47 (2H, bs), 3.84 (3H, s), 6.56 (1H, s), 6.67 to 7.80 (11H, m)

### (4) Methyl 5-({[(3,4-dimethoxyphenyl)amino]carbonyl}amino)-2-[(2,4-dichlorophenyl)(phenyl)methoxy]benzoate

To a solution (10 mL) of methyl 5-amino-2-[(2,4-dichlorophenyl)(phenyl)methoxy]benzoate (840 mg, 2.17 mmol) in THF was added 3,4-dimethoxyphenyl isocyanate (0.388 mL, 2.61 mmol) under ice-cooling, the mixture was stirred at 0°C for 2 hours, and the solvent was distilled off under reduced pressure. The residue was recrystallized from hexane and ethyl acetate. 970 mg (77.0%)
¹H-NMR (CDCl₃) δ; 3.83 (3H, s), 3.84 (3H, s), 3.86 (3H, s), 6.64 to 7.74 (17H, m)

### Example 84

### Methyl 5-({[(3,4-dimethoxyphenyl)amino]carbonyl}amino)-2-[(2-chlorophenyl)(4'-chlorophenyl)methoxy]benzoate

### (1) (2-Chlorophenyl)(4'-chlorophenyl)methanol

To a solution of 2,4'-dichlorobenzophenone (10.0 g, 39.8 mmol) in ethanol (100 mL) and THF (100 mL) was added sodium borohydride (753 mg, 19.9 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 9.43 g (93.4%)
¹H-NMR (CDCl₃) δ; 2.39 (1H, d, J = 3.8 Hz), 6.20 (1H, d, J = 3.8 Hz), 7.18 to 7.58 (8H, m)

### (2) Methyl 2-[(2 -chlorophenyl)(4'-chlorophenyl)methoxy]-5-nitrobenzoate

A mixture of methyl 2-hydroxy-5-nitrobenzoate (1.96 g, 9.92 mmol), (2 -chlorophenyl)(4'-chlorophenyl)methanol (3.00 g, 11.9 mmol), 40% solution of diethyl azodicarbonate in toluene (5.17 g, 11.9 mmol) and a solution (8 mL) of triphenylphosphine (3.12 g, 11.9 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 2.83 g (65.9%)
¹H-NMR (CDCl₃) δ; 3.99 (3H, s), 6.87 (1H, s), 6.98 (1H, d, J = 9.0 Hz), 7.05 to 7.71 (8H, m), 8.23 (1 H, dd, J = 2.4, 9.0 Hz), 8.73 (1H, d, J = 2.4 Hz)

### (3) Methyl 5-amino-2-[(2-chlorophenyl)(4'-chlorophenyl)methoxy]benzoate

A mixture of methyl 2-[(2-dichlorophenyl)(4'-chlorophenyl)methoxy]-5-nitrobenzoate (2.80 g, 6.47 mmol), iron (2.02 g, 36.3 mmol) and calcium chloride (402 mg, 3.63 mmol) in ethanol (50 mL) and water (13 mL) was stirred at 100°C for 5 hours. The insolubles were filtered off, and the filtrate was concentrated. The residue was purified by silicagel column chromatography (hexane:ethyl acetate = 5:1), to obtain the titled compound as oil. 1.88 g (75.2%)
¹H-NMR (CDCl₃) δ; 3.47 (2H, bs), 3.84 (3H, s), 6.59 (1H, s), 6.66 to 6.67 (2H, m), 7.13 to 7.79 (9H, m)

### (4) Methyl 5-({[(3,4-dimethoxyphenyl)amino]carbonyl}amino)-2-[(2-chlorophenyl)(4'-chlorophenyl)methoxy]benzoate

To a solution (20 mL) of methyl 5-amino-2-[(2-dichlorophenyl)(4'-chlorophenyl)methoxy]benzoate (1.83 g, 4.74 mmol) in THF was added 3,4-dimethoxyphenyl isocyanate (0.846 mL, 5.69 mmol) under ice-cooling, the mixture was stirred at 0°C for 2 hours, was poured into water, and was extracted with ethyl acetate. The solvent was distilled off under reduced pressure, the residue was purified by silicagel column chromatography (hexane:ethyl acetate = 1:1), to obtain the titled compound as a solid. 1.58 g (57.5%)
¹H-NMR (CDCl₃) δ; 3.84 (3H, s), 3.85 (3H, s), 3.87 (3H, s), 6.48 (1H, s), 6.59 (1H, s), 6.68 to 7.74 (15H, m)

### Example 85

### Methyl 5-({[(3,4-dimethoxyphenyl)amino]carbonyl}amino)-2-[(3-chlorophenyl)(phenyl)methoxy]benzoate

### (1) (3-Chlorophenyl)(phenyl)methanol

To a solution of 3-chlorobenzophenone (10.0 g, 46.2 mmol) in ethanol (100 mL) and THF (100 mL) was added sodium borohydride (876 mg, 23.1 mmol), the mixture was stirred at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure, to obtain the titled compound as oil. 9.53 g (94.4%)
¹H-NMR (CDCl₃) δ; 2.55 (1H, d, J = 3.6 Hz), 5.80 (1H, d, J = 3.6 Hz), 7.25 to 7.40 (9H, m)

### (2) Methyl 2-[(3-chlorophenyl)(phenyl)methoxy]-5-nitrobenzoate

A mixture of methyl 2-hydroxy-5-nitrobenzoate (2.25 g, 11.4 mmol), (3-chlorophenyl)(phenyl)methanol (3.00 g, 13.7 mmol), 40% solution of diethyl azodicarbonate in toluene (7.95 g, 18.3 mmol) and a solution (8 mL) of triphenylphosphine (3.59 g, 13.7 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as a solid. 3.06 g (67.4%)
¹H-NMR (CDCl₃) δ; 4.01 (3H, s), 6.37 (1H, s), 6.99 (1H, d, J = 9.4 Hz), 7.23 to 7.57 (9H, m), 8.20 (1 H, dd, J = 3.0, 9.4 Hz), 8.74 (1H, d, J = 3.0 Hz)

### (3) Methyl 5-amino-2-[(3-chlorophenyl)(phenyl)methoxy]benzoate

A mixture of methyl 2-[(3-chlorophenyl)(phenyl)methoxy]-5-nitrobenzoate (2.90 g, 7.29 mmol), iron (2.04 g, 36.4 mmol) and calcium chloride (405 mg, 3.65 mmol) in ethanol (50 mL) and water (13 mL) was stirred at 100°C for 5 hours. The insolubles were filtered off, and the filtrate was concentrated. The residue was purified by silicagel column chromatography (hexane:ethyl acetate = 2:1), to obtain the titled compound as oil. 2.36 g (88.1%)
¹H-NMR (CDCl₃) δ; 3.48 (2H, bs), 3.86 (3H, s), 6.08 (1H, s), 6.62 to 7.55 (12H, m)

### (4) Methyl 5-({[(3,4-dimethoxyphenyl)amino]carbonyl}amino)-2-[(3-chlorophenyl)(phenyl)methoxy]benzoate

To a solution (50 mL) of methyl 5-amino-2-[(3-chlorophenyl)(phenyl)methoxy]benzoate (2.30 g, 6.25 mmol) in THF was added 3,4-dimethoxyphenyl isocyanate (1.12 mL, 7.50 mmol) under ice-cooling, the mixture was stirred at 0°C for 2 hours, and the solvent was distilled off under reduced pressure. The residue was recrystallized from hexane and ethyl acetate. 2.63 g (77.1%)
¹H-NMR (CDCl₃) δ; 3.83 (3H, s), 3.84 (3H, s), 3.90 (3H, s), 6.16 (1H, s), 6.57 to 7.61 (17H, m)

### Example 86

### N-(tert-Butyl)-5-({[(3,4-dimethoxyphenyl)amino]carbonyl}amino)-2-{phenyl[4-(trifluoromethyl)phenyl]methoxy}benzamide

A mixture of 5-({[(3,4-dimethoxyphenyl)amino]carbonyl}amino)-2-{phenyl[4-(trifluoromethyl)phenyl]methoxy}benzoic acid (500 mg, 0.883 mmol), 1-hydroxy-1H-benzotriazole (203 mg, 1.33 mmol), tert-butyl amine (0.186 mL, 1.77 mmol), 1-(3-dimethylaminopropyl)-3-ethylcarbodiimide hydrochloride (212 mg, 1.10 mmol), and a solution (5 mL) in DMF was stirred under ice-cooling for 1 hour and at room temperature for 12 hours, was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), to obtain the titled compound as a solid. 472 mg (86.0%)
¹H-NMR (CDCl₃) δ; 1.17 (9H, s), 3.81 (3H, s), 3.83 (3H, s), 6.29 (1H, s), 6.71 to 7.73 (18H, m)

### Example 87

### Methyl 5-({[(4-hydroxy-3-methoxyphenyl)amino]carbonyl}amino)-2-{phenyl[4-(trifluoromethyl)phenyl]methoxy}benzoate

### (1) 4-Amino-2-methoxyphenol

A mixture of 2-methoxy-4-nitrophenol (3.00 g, 17.7 mmol), 10% palladium carbon (300 mg), THF (100 mL) and methanol (100 mL) was stirred under hydrogen atmosphere at room temperature for 4 hours.

The insolubles were filtered off, and the solvent was distilled off under reduced pressure, to obtain the titled compound as a solid. 2.32 g (94.3%)
¹H-NMR (CDCl₃) δ; 3.40 (2H, bs), 5.50 (1H, bs), 6.21 (1H, dd, J = 8.2, 2.6Hz), 6.30 (1H, d, J = 2.6 Hz), 8.72 (1H, d, J = 8.2 Hz)

### (2) Methyl 5-({[(4-hydroxy-3-methoxyphenyl)amino]carbonyl}amino)-2-{phenyl[4-(trifluoromethyl)phenyl]methoxy}benzoate

A solution (9 mL) of methyl 5-amino-2-[(4-trifluoromethylphenyl)(phenyl)methoxy]benzoate (300 mg, 0.748 mmol), diisopropylethyl amine (0.149 mL, 0.898 mmol) and di(N-succinimidyl) carbonate (230 mg, 0.898 mmol) in acetonitrile was stirred for 1 hour under ice-cooling, diisopropylethyl amine (0.149 mL, 0.898 mmol) and 4-amino-2-methoxyphenol (125 mg, 0.898 mmol) were added to the solution under ice-cooling, and the mixture was stirred under ice-cooling for 1 hour and at room temperature for 12 hours.

The reaction mixture was poured into water, was extracted with ethyl acetate, was dried with anhydrous magnesium sulfate, and the solvent was distilled off under reduced pressure. The residue was purified by silicagel column chromatography (hexane:ethyl acetate = 1: 1), to obtain the titled compound as a solid. 67.2 mg (15.8%)
¹H-NMR (CDCl₃) δ; 3.84 (3H, s), 3.88 (3H, s), 5.56 (1H, s), 6.25 (1H, s), 6.50 to 7.67 (17H, m)

### Example 88

### Methyl 5-({[(3,4-dimethoxyphenyl)amino]carbonyl}amino)-2-[(3-trifluoromethylphenyl)(phenyl)methoxy]benzoate

### (1) Methyl 2-[(3-trifluoromethylphenyl)(phenyl)methoxy]-5-nitrobenzoate

A mixture of methyl 2-hydroxy-5-nitrobenzoate (651 mg, 3.30 mmol), (3-trifluoromethylphenyl)(phenyl)methanol (1.00 g, 3.96 mmol), 40% solution of diethyl azodicarbonate in toluene (2.30 g, 5.29 mmol) and a solution (1 mL) of triphenylphosphine (1.04 g, 3.96 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 1.39 g (97.9%)
¹H-NMR (CDCl₃) δ; 4.01 (3H, s), 6.46 (1H, s), 7.02 (1H, d, J = 9.2 Hz), 7.26 to 7.88 (9H, m), 8.20 (1 H, dd, J = 3.0, 9.2 Hz), 8.75 (1H, d, J = 3.0 Hz)

### (2) Methyl 5-amino-2-[(3-trifluoromethylphenyl)(phenyl)methoxy]benzoate

A mixture of methyl 2-[(3-trifluoromethylphenyl)(phenyl)methoxy]-5-nitrobenzoate (1.36 g, 3.16 mmol), iron (882 mg, 15.8 mmol) and calcium chloride (175 mg, 1.58 mmol) in ethanol (20 mL) and water (5 mL) was stirred at 100°C for 4 hours. The insolubles were filtered off, and the filtrate was concentrated. The residue was purified by silicagel column chromatography (hexane:ethyl acetate = 1:1), to obtain the titled compound as oil. 880 mg (69.3%)
¹H-NMR (CDCl₃) δ; 3.49 (2H, bs), 3.85 (3H, s), 6.17 (1H, s), 6.62 to 6.64 (2H, m), 7.14 to 7.84 (10H, m)

### (3) Methyl 5-({[(3,4-dimethoxyphenyl)amino]carbonyl}amino)-2-[(3-trifluoromethylphenyl)(phenyl)methoxy]benzoate

To a solution (10 mL) of methyl 5-amino-2-[(3-trifluoromethylphenyl)(phenyl)methoxy]benzoate (860 mg, 2.14 mmol) in THF was added 3,4-dimethoxyphenyl isocyanate (0.383 mL, 2.57 mmol) under ice-cooling, the mixture was stirred at 0°C for 2 hours, and the solvent was distilled off under reduced pressure. The residue was purified by silicagel column chromatography (hexane:ethyl acetate = 12:1), to obtain the titled compound as a solid. 815 mg (65.7%)
¹H-NMR (CDCl₃) δ; 3.84 (3H, s), 3.85 (3H, s), 3.90 (3H, s), 6.25 (1H, s), 6.59 (1H, s), 6.70 to 7.68 (15H, m), 7.87 (1H, s)

### Example 89

### Methyl 2-{bis[4-(trifluoromethyl)phenyl]methoxy}-5-({[(3,4-dimethoxyphenyl)amino]carbonyl}amino)benzoate

### (1) Bis[4-(trifluoromethyl)phenyl]methanol

To a solution (200 mL) of 2-bromo-4-trifluoromethylbenzene (10.0 g, 44.4 mmol) in THF was added dropwise 1.6N solution of butyllithium in hexane (33.2 mL, 53.1 mmol) at -78°C, and the mixture was stirred at -78°C for 10 minutes. A solution (20 mL) of 4-trifluoromethyl benzaldehyde (9.24 g, 53.1 mmol) in THF was added dropwise to the mixture, and the mixture was stirred at -78 to -65°C for 1 hour. The reaction solution was poured into an aqueous solution of saturated ammonium chloride, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 2:1), to obtain the titled compound as oil. 8.50 g (59.9%)
¹H-NMR (CDCl₃) δ; 2.51 (1H, d, J = 3.0 Hz), 5.94 (1H, d, J = 3.0 Hz), 7.49 (4H, d, J = 8.1 Hz), 7.60 (4H, d, J = 8.1 Hz)

### (2) Methyl 2-{bis[4-(trifluoromethyl)phenyl)methoxy}-5-nitrobenzoate

A mixture of methyl 2-hydroxy-5-nitrobenzoate (1.54 g, 7.82 mmol), bis[4-(trifluoromethyl)phenyl]methanol (3.00 g, 9.38 mmol), 40% solution of diethyl azodicarbonate in toluene (5.45 g, 12.5 mmol) and a solution (5 mL) of triphenylphosphine (2.46 g, 9.38 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 2.23 g (63.5%)
¹H-NMR (CDCl₃) δ; 4.01 (3H, s), 6.52 (1H, s), 6.97 (1H, d, J = 9.2 Hz), 7.59 to 7.72 (8H, m), 8.43 (1 H, dd, J = 3.0, 9.2 Hz), 8.77 (1H, d, J = 3.0 Hz)

### (3) Methyl 5-amino-2-{bis[4-(trifluoromethyl)phenyl]methoxy}benzoate

A mixture of methyl 2-{bis[4-(trifluoromethyl)phenyl]methoxy}-5-nitrobenzoate (2.22 g, 4.94 mmol), iron (1.38 g, 24.7 mmol) and calcium chloride (274 mg, 2.47 mmol) in ethanol (30 mL) and water (7.5 mL) was stirred at 100°C for 4 hours. The insolubles were filtered off, and the filtrate was concentrated. The residue was purified by silicagel column chromatography (hexane:ethyl acetate = 3:1), to obtain the titled compound as oil. 1.23 g (53.0%)
¹H-NMR (CDCl₃) δ; 3.52 (2H, bs), 3.84 (3H, s), 6.23 (1H, s), 6.62 to 6.63 (2H, m), 7.16 to 7.17 (1H, m), 7.56 to 7.61 (8H, m)

### (4) Methyl 2-{bis[4-(trifluoromethyl)phenyl]methoxy}-5-({[(3,4-dimethoxyphenyl)amino]carbonyl}amino)benzoate

To a solution (20 mL) of methyl 5-amino-2-{bis[4-(trifluoromethyl)phenyl]methoxy}benzoate (1.22 g, 2.60 mmol) in THF was added 3,4-dimethoxyphenyl isocyanate (0.464 mL, 3.12 mmol) under ice-cooling, the mixture was stirred at 0°C for 2 hours, and the solvent was distilled off under reduced pressure. The residue was purified by silicagel column chromatography (hexane:ethyl acetate = 1:1), to obtain the titled compound as a solid. 1.09 g (64.9%)
¹H-NMR (CDCl₃) δ; 3.83 (3H, s), 3.84 (3H, s), 3.87 (3H, s), 6.29 (1H, s), 6.59 to 7.64 (16H, m)

### Example 90

### Methyl 2-{(4-chlorophenyl)[4-(trifluoromethyl)phenyl]methoxy}-5-({[(3,4-dimethoxyphenyl)amino]carbonyl}amino)benzoate

### (1) (4-Chlorophenyl)[4-(trifluoromethyl)phenyl]methanol

To a solution (200 mL) of 4-bromochlorobenzene (10.0 g, 52.2 mmol) in THF was added dropwise 1.6N solution of butyllithium in hexane (39.2 mL, 62.6 mmol) at -78°C, and the mixture was stirred at -78°C for 10 minutes. A solution (30 mL) of 4-trifluoromethyl benzaldehyde (10.9 g, 62.6 mmol) in THF was added dropwise to the mixture, and the mixture was stirred at -78 to -65°C for 1 hour. The reaction solution was poured into an aqueous solution of saturated ammonium chloride, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 7: 1), to obtain the titled compound as oil. 6.70 g (44.7%)
¹H-NMR (CDCl₃) δ; 2.29 (1H, d, J = 3.0 Hz), 5.87 (1H, d, J = 3.0 Hz), 7.26 to 7.36 (4H, m), 7.48 (2H, d, J = 7.8 Hz), 7.60 (2H, d, J = 7.8 Hz)

### (2) Methyl 2-{(4-chlorophenyl)[4-(trifluoromethyl)phenyl]methoxy}-5-nitrobenzoate

A mixture of methyl 2-hydroxy-5-nitrobenzoate (1.72 g, 8.75 mmol), (4-chlorophenyl)[4-(trifluoromethyl)phenyl]methanol (3.00 g, 10.5 mmol), 40% solution of diethyl azodicarbonate in toluene (6.09 g, 14.0 mmol) and a solution (5 mL) of triphenylphosphine (2.75 g, 10.5 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 2.58 g (63.2%)
¹H-NMR (CDCl₃) δ; 4.00 (3H, s), 6.45 (1H, s), 6.97 (1H, d, J = 9.2 Hz), 7.26 to 7.70 (8H, m), 8.22 (1 H, dd, J = 3.0, 9.2 Hz), 8.76 (1H, d, J = 3.0 Hz)

### (3) Methyl 5-amino-2-{(4-chlorophenyl)[4-(trifluoromethyl)phenyl]methoxy}benzoate

A mixture of methyl 2-{(4-chlorophenyl)[4-(trifluoromethyl)phenyl]methoxy}-5-nitrobenzoate (2.50 g, 5.37 mmol), iron (1.50 g, 26.9 mmol) and calcium chloride (296 mg, 2.68 mmol) in ethanol (34 mL) and water (9 mL) was stirred at 100°C for 4 hours. The insolubles were filtered off, and the filtrate was concentrated. The residue was purified by silicagel column chromatography (hexane:ethyl acetate = 1: 1), to obtain the titled compound as oil. 850 mg (36.3%)
¹H-NMR (CDCl₃) δ; 3.52 (2H, bs), 3.84 (3H, s), 6.14 (1H, s), 6.61 to 6.63 (2H, m), 7.14 to 7.16 (1H, m), 7.29 (2H, d, J = 8.8 Hz), 7.40 (2H, d, J = 8.8 Hz), 7.60 (4H, s)

### (4) Methyl 2-{(4-chlorophenyl)[4-(trifluoromethyl)phenyl]methoxy}-5-({[(3,4-dimethoxyphenyl)amino]carbonyl}amino)benzoate

To a solution (10 mL) of methyl 5-amino-2-{(4-chlorophenyl)[4-(trifluoromethyl)phenyl]methoxy}benzoate (830 mg, 1.90 mmol) in THF was added 3,4-dimethoxyphenyl isocyanate (0.340 mL, 2.29 mmol) under ice-cooling, the mixture was stirred at 0°C for 2 hours, and the solvent was distilled off under reduced pressure. The residue was purified by silicagel column chromatography (hexane:ethyl acetate = 1:1), to obtain the titled compound as a solid. 810 mg (69.2%)
¹H-NMR (CDCl₃) δ; 3.84 (3H, s), 3.85 (3H, s), 3.87 (3H, s), 6.23 (1H, s), 6.52 (1H, s), 6.64 (1H, s), 6.69 to 7.64 (14H, m)

### Example 91

### Methyl 5-({[(3,4-dimethoxyphenyl)amino]carbonyl}amino)-2-{(4-fluorophenyl)[4-(trifluoromethyl)phenyl]methoxy}benzoate

### (1) (4-Fluorophenyl)[4-(trifluoromethyl)phenyl]methanol

To a solution (200 mL) of 4-bromofluorobenzene(10.0 g, 57.1 mmol) in THF was added dropwise 1.6N solution of butyllithium in hexane (42.8 mL, 68.5 mmol) at -78°C, and the mixture was stirred at -78°C for 10 minutes. A solution (30 mL) of 4-trifluoromethyl benzaldehyde (11.9 g, 68.5 mmol) in THF was added dropwise to the mixture, and the mixture was stirred at -78 to -65°C for 1 hour. The reaction solution was poured into an aqueous solution of saturated ammonium chloride, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 10:1), to obtain the titled compound as oil. 7.00 g (45.5%)
¹H-NMR (CDCl₃) δ; 2.37 (1H, d, J = 3.2 Hz), 5.87 (1H, d, J = 3.2 Hz), 6.99 to 7.08 (2H, m), 7.26 to 7.35 (2H, m), 7.48 (2H, d, J = 8.4 Hz), 7.60 (2H, d, J = 8.4 Hz)

### (2) Methyl 2-{(4-fluorophenyl)[4-(trifluoromethyl)phenyl]methoxy}-5-nitrobenzoate

A mixture of methyl 2-hydroxy-5-nitrobenzoate (1.82 g, 9.25 mmol), (4-fluorophenyl)[4-(trifluoromethyl)phenyl]methanol (3.00 g, 11.1 mmol), 40% solution of diethyl azodicarbonate in toluene (6.44 g, 14.8 mmol) and a solution (5 mL) of triphenylphosphine (2.91 g, 11.1 mmol) in DMF was stirred at room temperature for 12 hours, and the reaction solution was poured into ice-water and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 2.74 g (65.9%)
¹H-NMR (CDCl₃) δ; 3.99 (3H, s), 6.46 (1H, s), 6.96 to 7.65 (9H, m), 8.22 (1 H, dd, J = 2.8, 9.2 Hz), 8.76 (1H, d, J = 2.8 Hz)

### (3) Methyl 5-amino-2-{(4-fluorophenyl)[4-(trifluoromethyl)phenyl]methoxy}benzoate

A mixture of methyl 2-{(4-fluorophenyl)[4-(trifluoromethyl)phenyl]methoxy}-5-nitrobenzoate (2.70 g, 6.00 mmol), iron (1.68 g, 30.0 mmol) and calcium chloride (333 mg, 3.00 mmol) in ethanol (40 mL) and water (10 mL) was stirred at 100°C for 4 hours. The insolubles were filtered off, and the filtrate was concentrated. The residue was purified by silicagel column chromatography (hexane:ethyl acetate = 1:1), to obtain the titled compound as oil. 1.66 g (65.9%)
¹H-NMR (CDCl₃) δ; 3.49 (2H, bs), 3.84 (3H, s), 6.16 (1H, s), 6.62 to 7.60 (11H, m)

### (4) Methyl 5-({[(3,4-dimethoxyphenyl)amino]carbonyl}amino)-2-{(4-fluorophenyl)[4-(trifluoromethyl)phenyl]methoxy}benzoate

To a solution (10 mL) of methyl 5-amino-2-{(4-fluorophenyl)[4-(trifluoromethyl)phenyl]methoxy}benzoate (1.63 g, 3.89 mmol) in THF was added 3,4-dimethoxyphenyl isocyanate (0.694 mL, 4.66 mmol) under ice-cooling, the mixture was stirred at 0°C for 2 hours, and the solvent was distilled off under reduced pressure. The residue was purified by silicagel column chromatography (hexane:ethyl acetate = 1:1), to obtain the titled compound as a solid. 1.41 g (60.5%)
¹H-NMR (CDCl₃) δ; 3.83 (3H, s), 3.84 (3H, s), 3.86 (3H, s), 6.23 (1H, s), 6.57 to 7.63 (16H, m)

### Example 92

### 5-({[(3,4-Dimethoxyphenyl)amino]carbonyl}amino)-N-isopropyl-2-{phenyl[4-(trifluoromethyl)phenyl]methoxy}benzamide

A mixture of 5-({[(3,4-dimethoxyphenyl)amino]carbonyl}amino)-2-{phenyl[4-(trifluoromethyl)phenyl]methoxy}benzoic acid (300 mg, 0.530 mmol), 1-hydroxy-1H-benzotriazole (122 mg, 0.798 mmol),isopropyl amine (0.0903 mL, 1.06 mmol), 1-(3-dimethylaminopropyl)-3-ethylcarbodiimide hydrochloride (126 mg, 0.660 mmol) and DMF (3 mL) was stirred under ice-cooling for 1 hour and at room temperature for 12 hours, was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:1), to obtain the titled compound as a solid. This was recrystallized from hexane and ethyl acetate. 264 mg (82.0%)
¹H-NMR (CDCl₃) δ; 0.87 to 0.93 (6H, m), 3.85 (6H, s), 4.06 to 4.16 (1H, m), 6.33 (1H, s), 6.68 to 8.03 (18H, m)

### Example 93

### 5-({[(3,4-Dimethoxyphenyl)amino]carbonyl}amino)-N-(2,2-dimethyltetrahydro-2H-pyran-4-yl)-2-{phenyl[4-(trifluoromethyl)phenyl]methoxy}benzamide

A mixture of 5-({[(3,4-dimethoxyphenyl)amino]carbonyl}amino)-2-{phenyl[4-(trifluoromethyl)phenyl]methoxy}benzoic acid (300 mg, 0.530 mmol), 1-hydroxy-1H-benzotriazole (122 mg, 0.798 mmol), 2,2-dimethyltetrahydro-2H-pyran-4-yl amine (137 mg, 1.06 mmol), 1-(3-dimethylaminopropyl)-3-ethylcarbodiimide hydrochloride (126 mg, 0.660 mmol) and DMF (3 mL) was stirred under ice-cooling for 1 hour and at room temperature for 12 hours, was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 1:2), to obtain the titled compound as a solid. This was recrystallized from hexane and ethyl acetate. 315 mg (87.7%)
¹H-NMR (CDCl₃) δ; 0.66 to 1.07 (8H, m), 1.27 to 1.86 (2H, m), 3.32 to 3.60 (2H, m), 3.84 (6H, s), 4.02 to 4.19 (1H, m), 6.32 (1H, s), 6.66 to 7.98 (18H, m)

### Example 94

### Methyl 5-({[(6-methoxypyridin-3-yl)amino]carbonyl}amino)-2-{phenyl[4-(trifluoromethyl)phenyl]methoxy}benzoate

A solution of methyl 5-amino-2-[(4-trifluoromethylphenyl)(phenyl)methoxy]benzoate (300 mg, 0.748 mmol), diisopropylethyl amine (0.149 mL, 0.898 mmol), di(N-succinimidyl) carbonate (230 mg, 0.898 mmol) and acetonitrile (9 mL) was stirred for 1 hour under ice-cooling, diisopropylethyl amine (0.149 mL, 0.898 mmol) and 5-amino-2-methoxypyridine (111 mg, 0.898 mmol) were added to the mixture under ice-cooling, and the mixture was stirred under ice-cooling for 1 hour and at room temperature for 12 hours.

The reaction mixture was poured into water, was extracted with ethyl acetate, was dried with anhydrous magnesium sulfate, and the solvent was distilled off under reduced pressure. The residue was purified by silicagel column chromatography (hexane:ethyl acetate = 1:1), to obtain the titled compound as oil. 235 mg (56.9%)
¹H-NMR (CDCl₃) δ; 3.88 (6H, s), 6.26 (1H, s), 6.68 to 7.70 (16H, m), 7.98 (1H, d, J = 2.4 Hz)

### Example 95

### Methyl 5-[({[4-methoxy-3-(trifluoromethyl)phenyl]amino}carbonyl)amino]-2-{phenyl[4-(trifluoromethyl)phenyl]methoxy}benzoate

A solution of methyl 5-amino-2-[(4-trifluoromethylphenyl)(phenyl)methoxy]benzoate (300 mg, 0.748 mmol), diisopropylethyl amine (0.149 mL, 0.898 mmol), di(N-succinimidyl) carbonate (230 mg, 0.898 mmol) and acetonitrile (9 mL) was stirred for 1 hour under ice-cooling, diisopropylethyl amine (0.149 mL, 0.898 mmol) and 5-amino-2-methoxybenzotrifluoride (172 mg, 0.898 mmol) were added to the solution under ice-cooling, and the mixture was stirred under ice-cooling for 1 hour and at room temperature for 12 hours. The reaction solution was poured into water, extracted with ethyl acetate, was dried with anhydrous magnesium sulfate, and the solvent was distilled off under reduced pressure. The residue was purified by silicagel column chromatography (hexane:ethyl acetate = 1:1), to obtain the titled compound as a solid. 247 mg (53.5%)
¹H-NMR (CDCl₃) δ; 3.82 (3H, s), 3.88 (3H, s), 6.24 (1H, s), 6.73 to 7.66 (17H, m)

### Example 96

### Methyl 5-({[(3-fluoro-4-methoxyphenyl)amino]carbonyl}amino)-2-{phenyl[4-(trifluoromethyl)phenyl]methoxy}benzoate

A solution of methyl 5-amino-2-[(4-trifluoromethylphenyl)(phenyl)methoxy]benzoate (300 mg, 0.748 mmol), diisopropylethyl amine (0.149 mL, 0.898 mmol), di(N-succinimidyl) carbonate (230 mg, 0.898 mmol) and acetonitrile (9 mL) was stirred for 1 hour under ice-cooling, diisopropylethyl amine (0.149 mL, 0.898 mmol) and 3-fluoro-4 aniline (127 mg, 0.898 mmol) were added to the solution under ice-cooling, and the mixture was stirred under ice-cooling for 1 hour and at room temperature for 12 hours. The reaction solution was poured into water, extracted with ethyl acetate, was dried with anhydrous magnesium sulfate, and the solvent was distilled off under reduced pressure. The residue was purified by silicagel column chromatography (hexane:ethyl acetate = 2:1), to obtain the titled compound as a solid. 287 mg (67.5%)
¹H-NMR (CDCl₃) δ; 3.83 (3H, s), 3.88 (3H, s), 6.25 (1H, s), 6.63 to 7.67 (17H, m)

### Synthetic method of amide derivatives by combinatorial synthesis

The compounds of Examples 97 to 143 were synthesized according to the followings.

To a mixed solution of 5-(anilinocarbonylamino)-2-benzhydryloxy benzoic acid (0.0684 mmol), 1-hydroxy-7-azabenzotriazole (0.0821 mmol), 1-ethyl-3-(3-dimethylaminopropyl)carbodiimide (0.1026 mmol) in DMF (0.3 mL) and dichloromethane (0.7 mL) was added amine (0.0821 mmol), the mixture was stirred at room temperature for 2 hours, water and dichloromethane were added to the mixture, the layer of dichloromethane was separated by PTFE filter (1µm, made by Whatman Inc.), and was concentrated by Dry Thermo-unit PTU-1C. The residue was purified by preparative HPLC made by Gilson Inc. (PLRP-S column 5 µm 100 A, 50 x 25 mm, 40% to 100% aqueous solution of acetonitrile). The resulting compound was analyzed by LC MASS made by Gilson Inc. (Shiseido capsule pack C18 2 x 5 cm, λ = 220 nm, temperature 40°C, A luquid 0.05% trifluoroacetic acid solution: B liquid acetonitrile: 10 to 95% B liquid (for 4 minutes) 95% B liquid (for 1.5 minutes), electrospray ionization mass spectrum).

### Example 97

### 5-(Anilinocarbonylamino)-2-benzhydryloxy-N-butyl benzamide

20.6 mg
LC-MS: purity 99%, Rt = 3.84 mjn, m/z: 494 [M+H]⁺

### Example 98

### 5-(Anilinocarbonylamino)-2-benzhydryloxy-N-(cyclohexylmethyl)benzamide

23.6 mg
LC-MS: purity 96%, Rt = 4.15 mjn, m/z: 534 [M+H]⁺

### Example 99

### 5-(Anilinocarbonylamino)-2-benzhydryloxy-N-cyclopropyl benzamide

22.4 mg
¹H-NMR (CDCl₃) δ; 0.07 to 0.15 (2H, m), 0.56 to 0.65 (2H, m), 2.78 to 2.87 (2H, m), 6.22 (1H, s), 6.74 (1H, d, J = 9.2 Hz), 6.93 (1H, t, J = 7.4 Hz), 7.17 to 7.40 (14H, m), 7.70 (1H, d, J = 2.8 Hz), 7.93 (1H, dd, J = 2.8, 8.8 Hz), 8.00 (1H, s), 8.09 (1H, bs), 8.28 (1H, s)
LC-MS: purity 99%, Rt = 3.55 mjn, m/z: 478 [M+H]⁺

### Example 100

### 5-(Anilinocarbonylamino)-2-benzhydryloxy-N-(1,3-benzodioxol-5-ylmethylcyclohexylmethyl)benzamide

26.5 mg
LC-MS: purity 98%, Rt = 3.69 mjn, m/z: 572 [M+H]⁺

### Example 101

### 5-(Anilinocarbonylamino)-2-benzhydryloxy-N-(2-phenylethyl)benzamide

26.1 mg
LC-MS: purity 99%, Rt = 3.89 mjn, m/z: 542 [M+H]⁺

### Example 102

### 5-(Anilinocarbonylamino)-2-benzhydryloxy-N-(3-phenylpropyl)benzamide

27.0 mg
LC-MS: purity 99%, Rt = 4.00 mjn, m/z: 556 [M+H]⁺

### Example 103

### 5-(Anilinocarbonylamino)-2-benzhydryloxy-N-(benzhydryl)benzamide

30.7 mg
LC-MS: purity 99%, Rt = 4.13 mjn, m/z: 604 [M+H]⁺

### Example 104

### 5-(Anilinocarbonylamino)-2-benzhydryloxy-N-(2-methoxyethyl)benzamide

25.7 mg
LC-MS: purity 98%, Rt = 3.46 mjn, m/z: 496 [M+H]⁺

### Example 105

### 5-(Anilinocarbonylamino)-2-benzhydryloxy-N-(3-methylthiopropyl)benzamide

26.4 mg
LC-MS: purity 99%, Rt = 3.71 mjn, m/z: 526 [M+H]⁺

### Example 106

### 5-(Anilinocarbonylamino)-2-benzhydryloxy-N-(tetrahydrofuran-2-ylmethyl)benzamide

26.3 mg
LC-MS: purity 99%, Rt = 3.56 mjn, m/z: 522 [M+H]⁺

### Example 107

### 5-(Anilinocarbonylamino)-2-benzhydryloxy-N-[2-(1H-indol-3-yl)ethyl]benzamide

27.9 mg
LC-MS: purity 99%, Rt = 3.74 mjn, m/z: 581 [M+H]⁺

### Example 108

### 5-(Anilinocarbonylamino)-2-benzhydryloxy-N-(1-ethylpropyl)benzamide

26.7 mg
LC-MS: purity 97%, Rt = 3.93 mjn, m/z: 508 [M+H]⁺

### Example 109

### 5-(Anilinocarbonylamino)-2-benzhydryloxy-N-cyclohexyl benzamide (24)

24.9 mg
LC-MS: purity 99%, Rt = 4.00 mjn, m/z: 520 [M+H]⁺

### Example 110

### 5-(Anilinocarbonylamino)-2-benzhydryloxy-N-ethynyl benzamide (25)

24.1 mg
LC-MS: purity 100%, Rt = 3.50 mjn, m/z: 476 [M+H]⁺

### Example 111

### 5-(Anilinocarbonylamino)-2-benzhydryloxy-N-(4-trifluoromethylbenzyl)benzamide

24.3 mg
¹H-NMR (CDCl₃) δ; 4.51 (2H, d, J = 5.6 Hz), 6.29 (1H, s), 6.87 (1H, d, J = 9.2 Hz), 7.03 to 7.44 (19H, m), 7.55 (1H, s), 7.72 (1H, s), 7.83 (1H, d, J = 3.0 Hz), 7.92 (1H, dd, J = 3.0, 8.4 Hz), 8.63 (1H, t, J = 5.6 Hz)
LC-MS: purity 99%, Rt = 4.02 mjn, m/z: 596 [M+H]⁺

### Example 112

### 5-(Anilinocarbonylamino)-2-benzhydryloxy-N-[2-(3,4-dimethoxyphenyl)ethyl]benzamide

29.5 mg
LC-MS: purity 99%, Rt = 3.67 mjn, m/z: 602 [M+H]⁺

### Example 113

### 5-(Anilinocarbonylamino)-2-benzhydryloxy-N-(3,3-diphenylpropyl)benzamide

24.7 mg
LC-MS: purity 99%, Rt = 4.22 mjn, m/z: 632 [M+H]⁺

### Example 114

### 5-(Anilinocarbonylamino)-2-benzhydryloxy-N-(2,3-dihydro-1H-indene-2-yl)benzamide

27.4 mg
LC-MS: purity 99%, Rt = 3.96 mjn, m/z: 554 [M+H]⁺

### Example 115

### 5-(Anilinocarbonylamino)-2-benzhydryloxy-N-(3-isopropoxypropyl)benzamide

27.4 mg
LC-MS: purity 99%, Rt = 3.75 mjn, m/z: 538 [M+H]⁺

### Example 116

### 5-(Anilinocarbonylamino)-2-benzhydryloxy-N-(2-oxoazepan-3-yl)benzamide

29.0 mg
LC-MS: purity 99%, Rt = 3.30 mjn, m/z: 549 [M+H]⁺

### Example 117

### 5-(Anilinocarbonylamino)-2-benzhydryloxy-N-(2-furylmethyl)benzamide

24.6 mg
LC-MS: purity 99%, Rt = 3.65 mjn, m/z: 518 [M+H]⁺

### Example 118

### 5-(Anilinocarbonylamino)-2-benzhydryloxy-N-[3-(2-oxopyrrolidin-1-yl)propyl]benzamide

16.3 mg
LC-MS: purity 99%, Rt = 3.22 mjn, m/z: 563 [M+H]⁺

### Example 119

### N-[3-(Azocan-1-ylcarbonyl)-4-(benzhydryloxy)phenyl]-N'-phenylurea

20.1 mg
LC-MS: purity 99%, Rt = 3.89 mjn, m/z: 534 [M+H]⁺

### Example 120

### 5-(Anilinocarbonylamino)-2-benzhydryloxy-N-[2-(dimethylamino)ethyl]benzamide

22.5 mg
LC-MS: purity 99%, Rt = 2.62 mjn, m/z: 508 [M+H]⁺

### Example 121

### 5-(Anilinocarbonylamino)-2-benzhydryloxy-N-[3-(diethylamino)propyl]benzamide

21.1 mg
LC-MS: purity 98%, Rt = 2.72 mjn, m/z: 551 [M+H]⁺

### Example 122

### 5-(Anilinocarbonylamino)-2-benzhydryloxy-N-(2-piperidin-1-ylethyl)benzamide

24.4 mg
LC-MS: purity 99%, Rt = 2.73 mjn, m/z: 549 [M+H]⁺

### Example 123

### 5-(Anilinocarbonylamino)-2-benzhydryloxy-N-(2-morpholin-4-ylethyl)benzamide

24.4 mg
LC-MS: purity 99%, Rt = 2.63 mjn, m/z: 551 [M+H]⁺

### Example 124

### 5-(Anilinocarbonylamino)-2-benzhydryloxy-N-[3-(4-methylpiperidin-1-yl)propyl]benzamide

26.4 mg
LC-MS: purity 98%, Rt = 2.39 mjn, m/z: 578 [M+H]⁺

### Example 125

### 5-(Anilinocarbonylamino)-2-benzhydryloxy-N-{3-[methyl(phenyl)amino]propyl)benzamide

27.5 mg
LC-MS: purity 96%, Rt = 3.06 mjn, m/z: 585 [M+H]⁺

### Example 126

### 5-(Anilinocarbonylamino)-2-benzhydryloxy-N-(1-benzylpiperidin-4-yl)benzamide

25.6 mg
LC-MS: purity 98%, Rt = 2.85 mjn, m/z: 611 [M+H]⁺

### Example 127

### 5-(Anilinocarbonylamino)-2-benzhydryloxy-N-(2,2,6,6-tetramethylpiperidin-4-yl)benzamide

25.7 mg
LC-MS: purity 100%, Rt = 2.74 mjn, m/z: 577 [M+H]⁺

### Example 128

### 5-(Anilinocarbonylamino)-2-benzhydryloxy-N-(2-anilin oethyl)benzamide

20.4 mg
LC-MS: purity 98%, Rt = 3.49 mjn, m/z: 557 [M+H]⁺

### Example 129

### 5-(Anilinocarbonylamino)-2-benzhydryloxy-N-(pyridin-2-ylmethyl)benzamide

23.8 mg
LC-MS: purity 98%, Rt = 2.79 mjn, m/z: 529 [M+H]⁺

### Example 130

### 5-(Anilinocarbonylamino)-2-benzhydryloxy-N-(2-pyridin-4-ylethyl)benzamide

26.1 mg
LC-MS: purity 98%, Rt = 2.64 mjn, m/z: 543 [M+H]⁺

### Example 131

### 5-(Anilinocarbonylamino)-2-benzhydryloxy-N-[3-(1H-imidazol-1-yl)propyl]benzamide

27.2 mg
LC-MS: purity 99%, Rt = 2.64 mjn, m/z: 546 [M+H]⁺

### Example 132

### 5-(Anilinocarbonylamino)-2-benzhydryloxy-N-[2-(diisopropylamino)ethyl]benzamide

26.1 mg
LC-MS: purity 97%, Rt = 2.83 mjn, m/z: 565 [M+H]⁺

### Example 133

### 5-(Anilinocarbonylamino)-2-benzhydryloxy-N-[3-(dimethylamino)-2,2-dimethylpropyl]benzamide

19.7 mg
LC-MS: purity 98%, Rt = 2.78 mjn, m/z: 551 [M+H]⁺

### Example 134

### 5-(Anilinocarbonylamino)-2-benzhydryloxy-N-[3-(2-methylpiperidin-1-yl)propyl]benzamide

23.9 mg
LC-MS: purity 98%, Rt = 2.78 mjn, m/z: 577 [M+H]⁺

### Example 135

### 5-(Anilinocarbonylamino)-2-benzhydryloxy-N-(2-pyrrolidin-1-ylethyl)benzamide

21.7 mg
LC-MS: purity 95%, Rt = 2.67 mjn, m/z: 535 [M+H]⁺

### Example 136

### 5-(Anilinocarbonylamino)-2-benzhydryloxy-N-{2-[ethyl(3-methylphenyl)amino]ethyl}benzamide

26.9 mg
LC-MS: purity 98%, Rt = 3.27 mjn, m/z: 599 [M+H]⁺

### Example 137

### 5-(Anilinocarbonylamino)-2-benzhydryloxy-N-(1-benzylpyrrolidin-3-yl)benzamide

23.7 mg
LC-MS: purity 96%, Rt = 2.88 mjn, m/z: 597 [M+H]⁺

### Example 138

### 5-(Anilinocarbonylamino)-2-benzhydryloxy-N-{3-[bis(2-hydroxyethyl)amino]propyl}benzamide

25.0 mg
LC-MS: purity 97%, Rt = 2.57 mjn, m/z: 583 [M+H]⁺

### Example 139

### 5-(Anilinocarbonylamino)-2-benzhydryloxy-N-{2-[(5-nitropyridyl-2-yl)amino]ethyl}benzamide (54)

11.1 mg
LC-MS: purity 95%, Rt = 3.54 mjn, m/z: 603 [M+H]⁺

### Example 140

### 5-(Anilinocarbonylamino)-2-benzhydryloxy-N-(pyridin-4-ylmethyl)benzamide (55)

26.2 mg
LC-MS: purity 99%, Rt = 2.63 mjn, m/z: 529 [M+H]⁺

### Example 141

### 5-(Anilinocarbonylamino)-2-benzhydryloxy-N-(pyridin-3-ylmethyl)benzamide

27.1 mg
LC-MS: purity 99%, Rt = 2.66 mjn, m/z: 529 [M+H]⁺

### Example 142

### 5-(Anilinocarbonylamino)-2-benzhydryloxy-N-(pyridin-3-ylethyl)benzamide

25.3 mg
LC-MS: purity 99%, Rt = 2.66 mjn, m/z: 543 [M+H]⁺

### Example 143

### N-[4-(Benzhydryloxy)-3-(octahydroquinolin-1 (2H)-ylcarbonyl)phenyl]-N'-phenylurea

16.1 mg
LC-MS: purity 95%, Rt = 4.10 mjn, m/z: 560 [M+H]⁺

### Combinatorial synthesis

The compounds of Examples 144 to 227 were synthesized according to the followings.

To a solution (1 mL) of methyl 5-amino-2-benzhydryloxy benzoate (0.900 mmol), diisopropylethyl amine (0.108 mmol) in acetonitrile was added N,N-disuccinimidyl carbamate (0.108 mmol) at 0°C, and the mixture was stirred at 0°C for 40 minutes. This solution was added to diisopropylethyl amine (0.108 mmol), amine (0.09 mmol), and the mixture was stirred for 12 hours at room temperature. The solvent was evaporated by Dry Thermo-unit, to the residue were added water and dichloromethane, and the layer of dichloromethane was separated by PTFE filter (1 µm pore size, Whatman Inc.). Dichloromethane was evaporated by Dry Thermo-unit, and the residue was purified by preparative HPLC (PLRP-S column 5 µm 100 A, 50 x 25 mm, 40% to 100% aqueous solution of acetonitrile). The resulting compound was analyzed by LC MASS made by Gilson Inc. (Shiseido capsule pack C18 2 x 5 cm, λ = 220 nm, temperature 40°C, A solution 0.05% trifluoroacetic acid solution: B liquid acetonitrile: 10 to 95% B liquid (for 4 minutes) 95% B liquid (for 1.5 minutes), electrospray ionization mass spectrum).

### Example 144

### Methyl 2-(benzhydryloxy)-5-[({[2-(dimethylamino)ethyl]amino}carbonyl)amino]benzoate

18.3 mg, LC-MS: purity 98%, Rt = 2.57 mjn, m/z: 448 [M+H]⁺

### Example 145

### Methyl 2-(benzhydryloxy)-5-[({[3-(diethylamino)propyl]amino}carbonyl)amino]benzoate

19.8 mg, LC-MS: purity 96%, Rt = 2.66 mjn, m/z: 490 [M+H]⁺

### Example 146

### Methyl 2-(benzhydryloxy)-5-({[(2-piperazin-1-ylethyl)amino]carbonyl}amino)benzoate

19.5 mg, LC-MS: purity 96%, Rt = 2.69 mjn, m/z: 488 [M+H]⁺

### Example 147

### Methyl 2-(benzhydryloxy)-5-({[(2-morpholin-4-ylethyl)amino]carbonyl}amino)benzoate

18.3 mg
¹H-NMR (CDCl₃) δ; 2.41 - 2.51 (6H, m), 3.26 to 3.35 (2H, m), 3.61 to 3.65 (4H, m), 3.90 (3H, s), 5.17 (1H, bs), 6.25 (1H, s), 6.85 to 6.89 (2H, m), 7.20 to 7.53 (12H, m), 7.67 (1H, d, J = 2.6 Hz)
LC-MS: purity 100%, Rt = 2.59 mjn, m/z: 490 [M+H]⁺

### Example 148

### Methyl 2-(benzhydryloxy)-5-[({[3-(4-methylpiperazin-1-yl)propyl]amino}carbonyl)amino]benzoate

22.1 mg
LC-MS: purity 98%, Rt = 2.33 mjn, m/z: 517 [M+H]⁺

### Example 149

### Methyl 2-(benzhydryloxy)-5-{[({3-[methyl(phenyl)amino]propyl}amino)carbonyl]amino}benzoate

25.9 mg, LC-MS: purity 98%, Rt = 2.85 mjn, m/z: 524 [M+H]⁺

### Example 150

### Methyl 2-(benzhydryloxy)-5-({[(1-benzylpiperidin-4-yl)amino]carbonyl}amino)benzoate

24.7 mg, LC-MS: purity 99%, Rt = 2.80 mjn, m/z: 550 [M+H]⁺

### Example 151

### Methyl 2-(benzhydryloxy)-5-({[(2,2,6,6-tetramethylpiperidin-4-yl)amino]carbonyl}amino)benzoate

12.6 mg, LC-MS: purity 99%, Rt = 2.72 mjn, m/z: 516 [M+H]⁺

### Example 152

### Methyl 2-(benzhydryloxy)-5-({[(2-anilin oethyl)amino]carbonyl}amino)benzoate

25.5 mg, LC-MS: purity 98%, Rt = 3.01 mjn, m/z: 496 [M+H]⁺

### Example 153

### Methyl 2-(benzhydryloxy)-5-({[(piperidin-2-ylmethyl)amino]carbonyl}amino)benzoate

16.8 mg, LC-MS: purity 98%, Rt = 2.61 mjn, m/z: 468 [M+H]⁺

### Example 154

### Methyl 2-(benzhydryloxy)-5-({[(2-pyridin-4-ylethyl)amino]carbonyl}amino)benzoate

17.1 mg, LC-MS: purity 95%, Rt = 2.58 mjn, m/z: 482 [M+H]⁺

### Example 155

### Methyl 2-(benzhydryloxy)-5-[({[3-(1H-imidazol-1-yl)propyl]amino}carbonyl)amino]benzoate

13.5 mg
¹H-NMR (CDCl₃) δ; 1.94 to 2.04 (2H, m), 3.12 to 3.21 (2H, m), 3.88 (3H, s), 3.99 (2H, t, J = 7.0 Hz), 5.31 (1H, bs), 6.23 (1H, s), 6.84 (1H, d, J = 8.8 Hz), 6.92 to 7.52 (15H, m), 7.63 (1H, d, J = 2.6 Hz)
LC-MS: purity 100%, Rt = 2.59 mjn, m/z: 485 [M+H]⁺

### Example 156

### Methyl 2-(benzhydryloxy)-5-[({[2-(diisopropylamino)ethyl]amino}carbonyl)amino]benzoate

18.7 mg, LC-MS: purity 100%, Rt = 2.80 mjn, m/z: 504 [M+H]⁺

### Example 157

### Methyl 2-(benzhydryloxy)-5-[({[3-(dimethylamino)-2,2-dimethylpropyl]amino}carbonyl)amino]benzoate

22.4 mg, LC-MS: purity 98%, Rt = 2.68 mjn, m/z: 490 [M+H]⁺

### Example 158

### Methyl 2-(benzhydryloxy)-5-[({[3-(2-methylpiperidin-1-yl)propyl]amino}carbonyl)amino]benzoate

21.0 mg, LC-MS: purity 996%, Rt = 2.73 mjn, m/z: 516 [M+H]⁺

### Example 159

### Methyl 2-(benzhydryloxy)-5-({[(3-morpholin-4-ylpropyl)amino]carbonyl}amino)benzoate

23.7 mg, LC-MS: purity 96%, Rt = 2.60 mjn, m/z: 504 [M+H]⁺

### Example 160

### Methyl 2-(benzhydryloxy)-5-({[(2-pyrrolidin-1-ylethyl)amino]carbonyl}amino)benzoate

17.1 mg, LC-MS: purity 99%, Rt = 2.62 mjn, m/z: 474 [M+H]⁺

### Example 161

### Methyl 2-(benzhydryloxy)-5-{[({2-[ethyl(3-methylphenyl)amino]ethyl}amino)carbonyl]amino}benzoate

27.9 mg, LC-MS: purity 98%, Rt = 2.98 mjn, m/z: 538 [M+H]⁺

### Example 162

### Methyl 2-(benzhydryloxy)-5-({[(1-benzylpyrrolidin-3-yl)amino]carbonyl}amino)benzoate

26.5 mg, LC-MS: purity 97%, Rt = 2.85 mjn, m/z: 536 [M+H]⁺

### Example 163

### Methyl 2-(benzhydryloxy)-5-[({[2-(5-nitropyridin-2-yl)ethyl]amino}carbonyl)amino]benzoate

25.1 mg, LC-MS: purity 96%, Rt = 3.35 mjn, m/z: 542 [M+H]⁺

### Example 164

### Methyl 2-(benzhydryloxy)-5-({[(pyridin-4-ylmethyl)amino]carbonyl}amino)benzoate (21)

21.5 mg, LC-MS: purity 99%, Rt = 2.58 mjn, m/z: 468 [M+H]⁺

### Example 165

### Methyl 2-(benzhydryloxy)-5-({[(pyridin-3-ylmethyl)amino]carbonyl}amino)benzoate

22.5 mg, LC-MS: purity 100%, Rt = 2.58 mjn, m/z: 468 [M+H]⁺

### Example 166

### Methyl 2-(benzhydryloxy)-5-[({[2-(3-pyridin yl)ethyl]amino}carbonyl)amino]benzoate

20.4 mg, LC-MS: purity 99%, Rt = 2.59 mjn, m/z: 482 [M+H]⁺

### Example 167

### Methyl 2-(benzhydryloxy)-5-({[[2-(dimethylamino)ethyl](methyl)amino]carbonyl}amino)benzoate

18.0 mg, LC-MS: purity 99%, Rt = 2.58 mjn, m/z: 462 [M+H]⁺

### Example 168

### Methyl 2-(benzhydryloxy)-5-({[(1-benzylpyrrolidin-3-yl)amino]carbonyl}amino)benzoate

24.1 mg, LC-MS: purity 86%, Rt = 2.92 mjn, m/z: 550 [M+H]⁺

### Example 169

### Methyl 2-(benzhydryloxy)-5-({(ethyl(pyridin-4-ylmethyl)amino]carbonyl}amino)benzoate

25.2 mg
¹H-NMR (CDCl₃) δ; 1.21 (3H, t, J = 7.4 Hz), 3.35 (2H, q, J = 7.4 Hz), 3.90 (3H, s), 4.55 (2H, s), 6.25 (2H, bs), 6.84 (1H, d, J = 9.2 Hz), 7.19 to 7.53 (13H, m), 7.64 (1H, d, J = 3.2 Hz), 8.56 (2H, d, J = 5.8 Hz)
LC-MS: purity 98%, Rt = 2.66 mjn, m/z: 496 [M+H]⁺

### Example 170

### Methyl 2-(benzhydryloxy)-5-({[bis(pyridin-3-ylmethyl)amino]carbonyl}amino)benzoate

28.1 mg, LC-MS: purity 98%, Rt = 2.31 mjn, m/z: 559 [M+H]⁺

### Example 171

### Methyl 2-(benzhydryloxy)-5-{[(4-ethylpiperazin-1-yl)carbonyl]amino}benzoate

26.1 mg, LC-MS: purity 98%, Rt = 2.58 mjn, m/z: 474 [M+H]⁺

### Example 172

### Methyl 2-(benzhydryloxy)-5-({[4-(2-ethoxy-2-oxoethyl)piperazin-1-yl]carbonyl}amino)benzoate

22.8 mg, LC-MS: purity 99%, Rt = 2.67 mjn, m/z: 532 [M+H]⁺

### Example 173

### Methyl 2-(benzhydryloxy)-5-{[(4-benzylpiperazin-1-yl)carbonyl]amino}benzoate

25.3 mg, LC-MS: purity 99%, Rt = 2.77 mjn, m/z: 536 [M+H]⁺

### Example 174

### Methyl 2-(benzhydryloxy)-5-{[(4-pyridin-2-ylpiperazin-1-yl)carbonyl]amino}benzoate

28.7 mg, LC-MS: purity 100%, Rt = 2.66 mjn, m/z: 523 [M+H]⁺

### Example 175

### Methyl 2-(benzhydryloxy)-5-{[(4-benzhydrylpiperazin-1-yl)carbonyl]amino}benzoate

27.4 mg, LC-MS: purity 98%, Rt = 3.03 mjn, m/z: 612 [M+H]⁺

### Example 176

### Methyl 2-(benzhydryloxy)-5-{[(4-phenylpiperazin-1-yl)carbonyl]amino}benzoate

28.6 mg, LC-MS: purity 99%, Rt = 3.43 mjn, m/z: 522 [M+H]⁺

### Example 177

### Methyl 2-(benzhydryloxy)-5-({[4-(2-methoxyphenyl)piperazin-1-yl]carbonyl}amino)benzoate

28.8 mg, LC-MS: purity 99%, Rt = 3.12 mjn, m/z: 552 [M+H]⁺

### Example 178

### Methyl 2-(benzhydryloxy)-5-[(1,4'-bipiperidin-1-ylcarbonyl)amino]benzoate

16.8 mg, LC-MS: purity 100%, Rt = 2.66 mjn, m/z: 528 [M+H]⁺

### Example 179

### Methyl 2-(benzhydryloxy)-5-({[3-(dimethylamino)pyrrolidin-1-yl]carbonyl}amino)benzoate

18.9 mg, LC-MS: purity 99%, Rt = 2.61 mjn, m/z: 474 [M+H]⁺

### Example 180

### Methyl 2-(benzhydryloxy)-5-({[benzyl(1-benzylpyrrolidin-3-yl)amino]carbonyl}amino)benzoate

26.6 mg, LC-MS: purity 98%, Rt = 3.19 mjn, m/z: 626 [M+H]⁺

### Example 181

### Methyl 2-(benzhydryloxy)-5-({[bis(2-pyridin ylmethyl)amino]carbonyl}amino)benzoate

23.4 mg, LC-MS: purity 99%, Rt = 2.72 mjn, m/z: 559 [M+H]⁺

### Example 182

### Methyl 2-(benzhydryloxy)-5-{[(4-methyl-1,4-diazepin-1-yl)carbonyl]amino}benzoate

21.7 mg, LC-MS: purity 99%, Rt = 2.55 mjn, m/z: 474 [M+H]⁺

### Example 183

### Methyl 2-(benzhydryloxy)-5-({[4-(2-hydroxyethyl)piperazin-1-yl]carbonyl}amino)benzoate

24.6 mg, LC-MS: purity 99%, Rt = 2.52 mjn, m/z: 490 [M+H]⁺

### Example 184

### Methyl 2-(benzhydryloxy)-5-({[4-(1,3-benzodioxol-5-ylmethyl)piperazin-1-yl]carbonyl}amino)benzoate

25.4 mg, LC-MS: purity 98%, Rt = 2.78 mjn, m/z: 580 [M+H]⁺

### Example 185

### Methyl 2-(benzhydryloxy)-5-{[(4-pyrimidin-2-ylpiperazin-1-yl)carbonyl]amino}benzoate

27.1 mg, LC-MS: purity 99%, Rt = 2.90 mjn, m/z: 562 [M+H]⁺

### Example 186

### Methyl 2-(benzhydryloxy)-5-[({4-[(2E)-3-phenylpropen-2-yl]piperazin-1-yl}carbonyl)amino]benzoate

25.1 mg, LC-MS: purity 99%, Rt = 2.67 mjn, m/z: 532 [M+H]⁺

### Example 187

### Methyl 2-(benzhydryloxy)-5-[({benzyl[2-(dimethylamino)ethyl]amino}carbonyl)amino]benzoate

22.3 mg, LC-MS: purity 99%, Rt = 2.89 mjn, m/z: 538 [M+H]⁺

### Example 188

### Methyl 2-(benzhydryloxy)-5-({[methyl(1-methylpiperidin-4-yl)amino]carbonyl}amino)benzoate

19.2 mg, LC-MS: purity 98%, Rt = 2.60 mjn, m/z: 488 [M+H]⁺

### Example 189

### Methyl 2-(benzhydryloxy)-5-({[2-(pyrrolidin-1-ylmethyl)pyrrolidin-1-yl]carbonyl}amino)benzoate

8.1 mg, LC-MS: purity 93%, Rt = 2.77 mjn, m/z: 514 [M+H]⁺

### Example 190

### Methyl 2-(benzhydryloxy)-5-{[(4-pyrrolidin-1-ylpiperidin-1-yl)carbonyl]amino}benzoate

20.2 mg, LC-MS: purity 97%, Rt = 2.61 mjn, m/z: 514 [M+H]⁺

### Example 191

### Methyl 2-(benzhydryloxy)-5-({[(4-cyclohexylphenyl)amino]carbonyl}amino)benzoate

21.6 mg, LC-MS: purity 99%, Rt = 4.36 mjn, m/z: 535 [M+H]⁺

### Example 192

### Methyl 2-(benzhydryloxy)-5-{[(pyridin-2-ylamino)carbonyl]amino}benzoate

17.1 mg, LC-MS: purity 95%, Rt = 2.98 mjn, m/z: 454 [M+H]⁺

### Example 193

### Methyl 2-(benzhydryloxy)-5-({[(5-methylisoxazol-3-yl)amino]carbonyl}amino)benzoate

10.4 mg, LC-MS: purity 95%, Rt = 3.49 mjn, m/z: 461 [M+H]⁺

### Example 194

### Methyl 2-(benzhydryloxy)-5-({[(4-methylphenyl)amino]carbonyl}amino)benzoate

21.1 mg, LC-MS: purity 100%, Rt = 3.78 mjn, m/z: 489 [M+Na]⁺

### Example 195

### Methyl 2-(benzhydryloxy)-5-({[(4-hydroxyphenyl)amino]carbonyl}amino)benzoate

21.5 mg, LC-MS: purity 96%, Rt = 3.27 mjn, m/z: 491 [M+Na]⁺

### Example 196

### Methyl 2-(benzhydryloxy)-5-({[(4-fluorophenyl)amino]carbonyl}amino)benzoate

21.3 mg, LC-MS: purity 97%, Rt = 3.69 mjn, m/z: 471 [M+H]⁺

### Example 197

### Methyl 2-(benzhydryloxy)-5-({[(2-fluorophenyl)amino]carbonyl}amino)benzoate

16.5 mg, LC-MS: purity 98%, Rt = 3.75 mjn, m/z: 493 [M+Na]⁺

### Example 198

### Methyl 2-(benzhydryloxy)-5-({[(3-cyanophenyl)amino]carbonyl}amino)benzoate

18.2 mg, LC-MS: purity 84%, Rt = 3.75 mjn, m/z: 500 [M+Na]⁺

### Example 199

### Methyl 2-(benzhydryloxy)-5-({[(4-cyanophenyl)amino]carbonyl}amino)benzoate

11.2 mg, LC-MS: purity 85%, Rt = 3.64 mjn, m/z: 500 [M+Na]⁺

### Example 200

### Methyl 2-(benzhydryloxy)-5-({[(3,5-dimethylphenyl)amino]carbonyl}amino)benzoate

23.8 mg, LC-MS: purity 94%, Rt = 3.92 mjn, m/z: 481 [M+H]⁺

### Example 201

### Methyl 2-(benzhydryloxy)-5-({[(4-ethylphenyl)amino]carbonyl}amino)benzoate

23.8 mg, LC-MS: purity 96%, Rt = 3.92 mjn, m/z: 503 [M+H]⁺

### Example 202

### Methyl 2-(benzhydryloxy)-5-({[(3-fluoro-4-methylphenyl)amino]carbonyl}amino)benzoate

24.2 mg, LC-MS: purity 99%, Rt = 3.87 mjn, m/z: 507 [M+Na]⁺

### Example 203

### Methyl 2-(benzhydryloxy)-5-({[(2-fluoro-4-methylphenyl)amino]carbonyl}amino)benzoate

21.7 mg, LC-MS: purity 99%, Rt = 3.87 mjn, m/z: 507 [M+Na]⁺

### Example 204

### Methyl 2-(benzhydryloxy)-5-({[(4-chlorophenyl)amino]carbonyl}amino)benzoate

25.4 mg, LC-MS: purity 100%, Rt = 3.88 mjn, m/z: 509 [M+Na]⁺

### Example 205

### Methyl 2-(benzhydryloxy)-5-({[(3-chlorophenyl)amino]carbonyl}amino)benzoate

22.7 mg, LC-MS: purity 98%, Rt = 3.91 mjn, m/z: 509 [M+Na]⁺

### Example 206

### Methyl 2-(benzhydryloxy)-5-({[(2,4-difluorophenyl)amino]carbonyl}amino)benzoate

20.2 mg, LC-MS: purity 94%, Rt = 3.78 mjn, m/z: 511 [M+Na]⁺

### Example 207

### Methyl 2-(benzhydryloxy)-5-({[(3,4-difluorophenyl)amino]carbonyl}amino)benzoate

19.7 mg, LC-MS: purity 98%, Rt = 3.828 mjn, m/z: 511 [M+Na]⁺

### Example 208

### Methyl 2-(benzhydryloxy)-5-{[(1H-indol-5-ylamino)carbonyl]amino}benzoate

17.3 mg, LC-MS: purity 97%, Rt = 3.47 mjn, m/z: 492 [M+H]⁺

### Example 209

### Methyl 2-(benzhydryloxy)-5-{[(2,3-dihydro-1H-indene-5-ylamino)carbonyl]amino}benzoate

29.4 mg, LC-MS: purity 91%, Rt = 3.95 mjn, m/z: 493 [M+Na]⁺

### Example 210

### Methyl 2-(benzhydryloxy)-5-({[(4-acetylphenyl)amino]carbonyl}amino)benzoate

19.5 mg, LC-MS: purity 100%, Rt = 3.55 mjn, m/z: 517 [M+Na]⁺

### Example 211

### Methyl 2-(benzhydryloxy)-5-({[(4-isopropylphenyl)amino]carbonyl}amino)benzoate

22.6 mg, LC-MS: purity 94%, Rt = 3.95 mjn, m/z: 517 [M+Na]⁺

### Example 212

### Methyl 2-(benzhydryloxy)-5-({[(2-propylphenyl)amino]carbonyl}amino)benzoate

25.6 mg, LC-MS: purity 95%, Rt = 3.95 mjn, m/z: 495 [M+H]⁺

### Example 213

### Methyl 2-(benzhydryloxy)-5-[({[4-(dimethylamino)phenyl]amino}carbonyl)amino]benzoate

22.3 mg, LC-MS: purity 95%, Rt = 2.77 mjn, m/z: 496 [M+H]⁺

### Example 214

### Methyl 2-(benzhydryloxy)-5-({[(2-methoxy-5-methylphenyl)amino]carbonyl}amino)benzoate

25.6 mg, LC-MS: purity 98%, Rt = 3.91 mjn, m/z: 497 [M+H]⁺

### Example 215

### Methyl 2-(benzhydryloxy)-5-({[(4-ethoxyphenyl)amino]carbonyl}amino)benzoate

24.9 mg, LC-MS: purity 99%, Rt = 3.91 mjn, m/z: 497 [M+H]⁺

### Example 216

### Methyl 2-(benzhydryloxy)-5-({[(4-nitrophenyl)amino]carbonyl}amino)benzoate

7.6 mg, LC-MS: purity 86%, Rt = 3.77 mjn, m/z: 520 [M+Na]⁺

### Example 217

### Methyl 2-(benzhydryloxy)-5-({[(3-methylthiophenyl)amino]carbonyl}amino)benzoate

24.8 mg, LC-MS: purity 99%, Rt = 3.83 mjn, m/z: 499 [M+H]⁺

### Example 218

### Methyl 2-(benzhydryloxy)-5-({[(3-chloro-4-methylphenyl)amino]carbonyl}amino)benzoate

26.6 mg, LC-MS: purity 93%, Rt = 4.02 mjn, m/z: 501 [M+H]⁺

### Example 219

### Methyl 2-(benzhydryloxy)-5-{[(1-naphthylamino)carbonyl]amino}benzoate

¹H-NMR (CDCl₃) δ; 3.84 (3H, s), 6.20 (1H, s), 6.52 (1H, s), 6.70 (1H, s), 6.80 (1H, d, J = 9.2 Hz), 7.18 to 8.00 (19H, m)
22.6 mg, LC-MS: purity 98%, Rt = 3.58 mjn, m/z: 525 [M+Na]⁺

### Example 220

### Methyl 2-(benzhydryloxy)-5-{[(quinolin-6-ylamino)carbonyl]amino}benzoate

24.0 mg, LC-MS: purity 97%, Rt = 2.80 mjn, m/z: 504 [M+H]⁺

### Example 221

### Methyl 2-(benzhydryloxy)-5-{[(isoquinolin-5-ylamino)carbonyl]amino}benzoate

20.1 mg, LC-MS: purity 99%, Rt = 2.76 mjn, m/z: 504 [M+H]⁺

### Example 222

### Methyl 2-(benzhydryloxy)-5-({[(3-chloro-4-fluorophenyl)amino]carbonyl}amino)benzoate

23.0 mg, LC-MS: purity 98%, Rt = 3.92 mjn, m/z: 527 [M+Na]⁺

### Example 223

### Methyl 2-(benzhydryloxy)-5-({[(1-oxo-2,3-dihydro-1H-indane-5-yl)amino]carbonyl}amino)benzoate

11.4 mg
¹H-NMR (CDCl₃) δ; 2.66 (2H, t, J = 7.0 Hz), 3.06 (2H, t, J = 7.6Hz), 3.91 (3H, s), 6.23 (1H, s), 6.89 to 7.38 (18H, m)
LC-MS: purity 97%, Rt = 3.50 mjn, m/z: 529 [M+Na]⁺

### Example 224

### Methyl 2-(benzhydryloxy)-5-({[(4-tert-butylphenyl)amino]carbonyl}amino)benzoate

27.7 mg, LC-MS: purity 100%, Rt = 4.13 mjn, m/z: 509 [M+H]⁺

### Example 225

### Methyl 2-(benzhydryloxy)-5-({[(2-tert-butylphenyl)amino]carbonyl}amino)benzoate

19.6 mg
¹H-NMR (CDCl₃) δ; 1.36 (9H, s), 3.88 (3H, s), 6.11 (1H, s), 6.20 (1H, s), 6.24 (1H, s), 6.85 (1H, d, J = 8.4 Hz), 7.22 to 7.55 (16H, m)
LC-MS: purity 98%, Rt = 3.91 mjn, m/z: 509 [M+H]⁺

### Example 226

### Methyl 2-(benzhydryloxy)-5-{[({3-[(methylamino)carbonyl]phenyl}amino)carbonyl]amino}benzoate

2.6 mg, LC-MS: purity 92%, Rt = 3.25 mjn, m/z: 510 [M+H]⁺

### Example 227

### Methyl 2-(benzhydryloxy)-5-[({[4-(acetylamino)phenyl]amino}carbonyl)amino]benzoate

1.6 mg, LC-MS: purity 86%, Rt = 3.23 mjn, m/z: 510 [M+H]⁺

### Example 228

### Methyl 5-(anilinocarbonylamino)-2-benzyloxy benzoate

### (1) Methyl 2-benzyloxy-5-nitrobenzoate

To a solution (60 mL) of methyl 2-hydroxy-5-nitrobenzoate (3.94 g, 20.0 mmol) in DMF was added sodium hydride (60%, 0.96 g, 24.0 mmol) under ice-cooling, and the mixture was stirred at room temperature for 30 minutes. To the reaction solution was added benzyl bromide (2.85 mL, 24.0 mmol), the mixture was stirred at 70°C for 3 hours, poured into an ice-cooled aqueous solution of saturated ammonium chloride, and was extracted with ethyl acetate. The extracted solution was washed with water and saturated brine, and was dried with anhydrous magnesium sulfate, and the solvent was distilled off under reduced pressure. The residue was purified by silicagel column chromatography (hexane:ethyl acetate = 3:1) to obtain crystals, which were filtrated and were washed with ethyl acetate and hexane. 4.41 g (77%)
¹H-NMR (CDCl₃) δ; 3.95 (3H, s), 5.31 (2H, s), 7.10 (1H, d, J = 9.2 Hz), 7.26 to 7.50 (5H, m), 8.32 (1H, dd, J = 9.2, 3.0 Hz), 8.74 (1H, d, J = 3.0 Hz)

### (2) Methyl 5-amino-2-benzyloxy benzoate

A mixed solution of methyl 2-benzyloxy-5-nitrobenzoate (2.88 g, 10 mmol), 5% iridium carbon (0.71 g) and ethyl acetate (30 mL) was stirred under hydrogen atmosphere for 5 hours. The catalyst was removed, and the residue was purified by silicagel column chromatography (hexane:ethyl acetate = 3:1), to obtain the titled compound as oil. 1.74 g (67%)
¹H-NMR (CDCl₃) δ; 3.52 (2H, bs), 3.88 (3H, s), 5.08 (2H, s), 6.77 (1H, dd, J = 8.8, 2.8 Hz), 6.86 (1H, d, J = 8.8 Hz), 7.17 (1H, d, J = 2.8 Hz), 7.28 to 7.50 (5H, m)

### (3) Methyl 5-(anilinocarbonylamino)-2-benzyloxy benzoate

A mixed solution of methyl 5-amino-2-benzyloxy benzoate (2.13 g, 8.3 mmol), phenyl isocyanate (0.99 mL, 9.1 mmol) and THF (50 mL) was stirred at room temperature for 2 hours. The solvent was distilled off under reduced pressure, and the resulting crude crystals were recrystallized from ethyl acetate and methanol. 2.56 g (82%)
¹H-NMR (CDCl₃) δ; 3.85 (3H, s), 5.05 (2H, s), 6.89 (1H, d, J = 8.8 Hz), 7.02 to 7.10 (3H, m), 7.16 to 7.52 (10H, m), 7.64 (1H, d, J = 2.6 Hz)
Anal.Calcd for C₂₂H₂₀N₂O₄: C, 70.20; H, 5.36; N, 7.44
Found: C, 70.40; H, 5.29; N, 7.60

### Example 229

### Methyl 5-[(anilinocarbonyl)amino]-2-phenoxybenzoate

### (1) Methyl 5-nitro-2-phenyloxybenzoate

A mixed solution of methyl 2-chloro-5-nitrobenzoate (1.08 g, 5.0 mmol), phenol (0.47 g, 5.0 mmol), potassium carbonate (0.69 g, 5.0 mmol) and DMF (15 mL) was heated with stirring at 120°C for 1 hour. The reaction solution was poured into ice-water, and was extracted with ethyl acetate. The extracted solution was washed with water and saturated brine, and was dried with anhydrous magnesium sulfate, and the solvent was distilled off under reduced pressure. The resulting crystal was collected by filtration, and was washed with hexane. 1.12 g (82%)
¹H-NMR (CDCl₃) δ; 3.94 (3H, s), 6.92 (1H, d, J = 9.0 Hz), 7.07 to 7.12 (2H, m), 7.22 to 7.30 (1H, m), 7.39 to 7.49 (2H, m), 8.24 (1H, dd, J = 9.0, 2.8 Hz), 8.80 (1H, d, J = 2.8 Hz)

### (2) Methyl 5-amino-2-phenyloxybenzoate

A mixed solution of methyl 5-nitro-2-phenyloxybenzoate (0.89 g, 3.3 mmol), 10% palladium carbon (0.24 g) and ethyl acetate (20 mL) was stirred under hydrogen atmosphere for 2 hours. The catalyst was removed, and the solvent was distilled off under reduced pressure, and the obtained crude crystals were recrystallized from ethyl acetate and hexane. 0.74 g (94%)
¹H-NMR (CDCl₃) δ; 3.70 (2H, bd) , 3.73 (3H, s), 6.79 to 7.03 (5H, m), 7.22 to 7.31 (3H, m)

### (3) Methyl 5-[(anilinocarbonyl)amino]-2-phenoxybenzoate

A mixed solution of methyl 5-amino-2-phenyloxybenzoate (0.61 g, 2.5 mmol), phenyl isocyanate (0.30 mL, 2.8 mmol) and THF (15 mL) was stirred at room temperature for 14 hours. The solvent was distilled off under reduced pressure, and the obtained crude crystals were recrystallized from ethyl acetate and hexane. 0.86 g (95%)
¹H-NMR (CDCl₃) δ; 3.74 (3H, s), 6.84 to 6.92 (3H, m), 7.00 to 7.12 (3H, m), 7.19 to 7.30 (7H, m), 7.55 (1H, dd, J = 8.8, 2.6 Hz), 7.76 (1H, d, J = 2.6 Hz)

The compound of Example 230 was obtained in the same manner as Example 229.

### Example 230

### Methyl 5-[(anilinocarbonyl)amino]-2-(pyridin-3-yloxy)benzoate

### (1) Methyl 5-nitro-2-(pyridin-3-yloxy)benzoate

¹H-NMR (CDCl₃) δ; 3.94 (3H, s), 6.98 (1H, d, J = 9.2 Hz), 7.38 to 7.42 (2H, m), 8.32 (1H, dd, J = 9.2, 2.6 Hz), 8.46 to 8.54 (2H, m), 8.84 (1H, d, J = 3.0 Hz)

### (2) Methyl 5-amino-2-(pyridin-3-yloxy)benzoate

1.66 g (97%)
¹H-NMR (CDCl₃) δ; 3.73 (3H, s), 3.78 (2H, bs), 6.85 (1H, dd, J = 8.8, 2.8 Hz), 6.92 (1H, d, J = 8.8 Hz), 7.08 to 7.26 (3H, m), 8.26 (1H, dd, J = 4.4, 1.6 Hz), 8.30 (1H, d, J = 2.8 Hz)

### (3) Methyl 5-[(anilinocarbonyl)amino]-2-(pyridin-3-yloxy)benzoate

¹H-NMR (CDCl₃) δ; 3.72 (3H, s), 6.95 (1H, d, J = 8.8 Hz), 7.06 to 7.35 (7H, m), 7.43 (1H, s), 7.57 (1H, dd, J = 8.8, 3.0 Hz), 7.61 (1H,s), 7.84 (1H, d, J = 3.0 Hz), 8.24 (1H, d, J = 2.6 Hz), 8.32 (1H, dd, J = 4.4, 1.8 Hz)

### Example 231

### Methyl 2-[(4-isopropylbenzyl)oxy]-5-({[(4-methoxyphenyl)amino]carbonyl}amino)benzoate

### (1) Methyl 2-(4-isopropylbenzyl)oxy-5-nitrobenzoate

To a solution (150 mL) of methyl 2-hydroxy-5-nitrobenzoate (9.86 g, 50.0 mmol) in DMF was added sodium hydride (60%, 2.40 g, 60.0 mmol) under ice-cooling, the mixture was stirred for 1 hour at room temperature, (4-isopropyl)benzyl bromide (11.9 g, 60.0 mmol) was added to the mixture, and the mixture was stirred at 80°C for 16 hours. The mixture was poured into an ice-cooled aqueous solution of saturated ammonium chloride, and was extracted with ethyl acetate. The extracted solution was washed with water and saturated brine, was dried with anhydrous magnesium sulfate, and the solvent was distilled off under reduced pressure. The residue was purified by silicagel column chromatography (toluene) to obtain the titled compound as oil. 5.65 g (34%)
¹H-NMR (CDCl₃) δ; 1.25 (6H, d, J = 6.8 Hz), 2.85 - 2.99 (1H, m), 3.95 (3H, s), 5.28 (2H, s), 7.11 (1H, d, J = 9.2 Hz), 7.27 (2H, d, J = 8.6 Hz), 7.40 (2H, d, J = 8.6 Hz), 8.32 (1H, dd, J = 9.2, 3.0 Hz), 8.73 (1H, d, J = 3.0 Hz)

### (2) Methyl 5-amino-2-(4-isopropylbenzyl)oxy benzoate

A mixed solution of methyl 2-(4-isopropylbenzyl)oxy-5-nitrobenzoate (3.05 g, 9.3 mmol), 5% iridium carbon (0.61 g) and ethyl acetate (40 mL)-methanol (40 mL) was stirred under hydrogen atmosphere for 20 hours. The catalyst was removed, and the filtrate was concentrated and the resulting residue was filtrated. 2.19 g (79%)
¹H-NMR (CDCl₃) δ; 1.25 (6H,d, J = 7.0 Hz), 2.84 - 2.98 (1H, m), 3.51 (2H, bs), 3.88 (3H, s), 5.04 (2H, s), 6.77 (1H, dd, J = 8.8, 3.0 Hz), 6.88 (1H, d, J = 8.8 Hz), 7.16 (1H, d, J = 3.0 Hz), 7.23 (2H, d, J = 8.2 Hz), 7.39 (2H, d, J = 8.2 Hz)

### (3) Methyl 2-[(4-isopropylbenzyl)oxy]-5-({[(4-methoxyphenyl)amino]carbonyl}amino)benzoate

A mixed solution of methyl 5-amino-2-(4-isopropylbenzyl)oxy benzoate (299 mg, 1.0 mmol), 4-methoxyphenyl isocyanate (143 µl, 1.1 mmol) and THF (10 mL) was stirred at room temperature for 5 hours. The solvent was distilled off under reduced pressure, and the obtained crude crystals were recrystallized from ethyl acetate and hexane. 382 mg (85%)
¹H-NMR (CDCl₃) δ; 1.23 (6H,d, J = 7.0 Hz), 2.82 - 2.97 (1H, m), 3.75 (3H, s), 3.84 (3H, s), 5.03 (2H, s), 6.78 to 6.93 (5H, m), 7.15 to 7.23 (4H, m), 7.36 (2H, d, J = 8.0 Hz), 7.51 (1H, dd, J = 9.0, 2.8 Hz), 7.61 (1H, d, J = 2.8 Hz)

The following compounds were obtained in the same manner as Example 231.

### Example 232

### Methyl 2-[(4-isopropylbenzyl)oxy]-5-({[(3-methoxyphenyl)amino]carbonyl}amino)benzoate

¹H-NMR (CDCl₃) δ; 1.23 (6H, d, J = 7.0 Hz), 2.81 - 2.95 (1H, m), 3.71 (3H, s), 3.84 (3H, s), 5.01 (2H, s), 6.56 to 6.61 (1H, m), 6.75 to 6.80 (1H, m), 6.90 (1H, d, J = 8.8 Hz), 7.02 (1H, t, J = 2.2 Hz), 7.09 to 7.22 (5H, m), 7.34 (2H, d, J = 8.2 Hz), 7.49 (1H, dd, J = 8.8, 3.0 Hz), 7.62 (1H, d, J = 3.0 Hz)

### Example 233

### Methyl 2-[(4-isopropylbenzyl)oxy]-5-({[(3,4-dimethoxyphenyl)amino]carbonyl}amino)benzoate

¹H-NMR (CDCl₃) δ; 1.23 (6H, d, J = 6.6 Hz), 2.82 - 2.96 (1H, m), 3.82 (3H, s), 3.83 (3H, s), 3.85 (3H, s), 5.05 (2H, s), 6.70 (1H, dd, J = 8.6, 2.0 Hz), 6.77 (1H, d, J = 8.6 Hz), 6.87 (1H, s), 6.93 (1H, d, J = 8.6 Hz), 6.95 (1H, s), 7.03 (1H, d, J = 2.0 Hz), 7.21 (2H, d, J = 8.0 Hz), 7.36 (2H, d, J = 8.0 Hz), 7.54 (1H, dd, J = 8.8, 3.0 Hz), 7.62 (1H, d, J = 3.0 Hz)

### Example 234

### Methyl 2-[(4-tert-butylbenzyl)oxy]-5-({[(3,4-dimethoxyphenyl)amino]carbonyl}amino)benzoate

### (1) Methyl 2-(4-tert-butylbenzyl)oxy-5-nitrobenzoate

¹H-NMR (CDCl₃) δ; 1.33 (9H, s), 3.95 (3H, s), 5.28, (2H, s), 7.11 (1H, d, J = 9.2 Hz), 7.42 to 7.46 (4H, m), 8.33 (1H, dd, J = 9.2, 3.0 Hz), 8.73 (1H, d, J = 3.0 Hz)

### (2) Methyl 5-amino-2-(4-tert-butylbenzyl)oxy benzoate

¹H-NMR (CDCl₃) δ; 1.32 (9H, s), 3.51 (2H, bs), 3.88 (3H, s), 5.05, (2H, s), 6.78 (1H, dd, J = 8.8, 2.8 Hz), 6.88 (1H, d, J = 8.8 Hz), 7.17 (1H, d, J = 2.8 Hz), 7.40 (4H, s)

### (3) Methyl 2-[(4-tert-butylbenzyl)oxy]-5-({[(3,4-dimethoxyphenyl)amino]carbonyl}amino)benzoate

A mixed solution of methyl 5-amino-2-(4-tert-butylbenzyl)oxy benzoate (313 mg, 1.0 mmol), 3,4-dimethoxyphenyl isocyanate (0.16 mL, 1.1 mmol) and THF (10 mL) was stirred at room temperature for 16 hours. The solvent was distilled off under reduced pressure, and the obtained crude crystals were recrystallized from ethyl acetate and hexane. 365 mg (74%)
¹H-NMR (CDCl₃) δ; 1.31 (9H, s), 3.88 (3H, s), 3.90 (3H, s) , 3.91 (3H, s), 5.12 (2H, s), 6.82 to 6.89 (4H, m), 6.96 (1H, d, J = 8.8 Hz), 7.08 (1H, s), 7.39 (4H, s), 7.61 (1H, d, J = 2.8 Hz), 7.75 (1H, dd, J = 8.8, 2.8 Hz)

### Example 235

### Methyl 2-(benzhydryloxy)-5-{[(2,3-dihydro-1-benzofuran-5-ylamino)carbonyl]amino}benzoate

A mixed solution of methyl 5-amino-2-(benzhydryloxy)benzoate (333 mg, 1.0 mmol), 5-isocyanato-2,3-dihydro-1-benzofuran (161 mg, 1.0 mmol) and THF (10 mL) was stirred at room temperature for 20 hours. The solvent was distilled off under reduced pressure, and the obtained crude crystals were recrystallized from ethyl acetate and hexane. 454 mg (92%)
¹H-NMR (CDCl₃) δ; 3.12 (2H, t, J = 8.6 Hz), 3.86 (3H, s), 4.53 (2H, t, J = 8.6 Hz), 6.20 (1H, s), 6.53 (1H, s), 6.65 (1H, s), 6.69 (1H, d, J = 8.4 Hz), 6.82 (1H, d, J = 8.8 Hz), 6.89 (1H, dd, J = 8.4, 2.2 Hz), 7.14 to 7.51 (12H, m), 7.59 (1H, d, J = 2.8 Hz)

### Example 236

### Methyl 5-({[(3,4-dimethoxyphenyl)amino]carbonyl}amino)-2-[(2-chlorophenyl)(4-fluorophenyl)methoxy]benzoate

### (1) (2-Chlorophenyl)(4-fluorophenyl)methanol

To a solution (200 mL) of 2-chlorobromobenzene (10.2 g, 53.3 mmol) in THF was added dropwise 1.6N solution of butyl lithium in hexane (40 mL, 65 mmol) at -78°C, the mixture was stirred at -78°C for 10 minutes, a solution (20 mL) of 4-fluorobenzaldehyde (8.0 g, 65.0 mmol) in THF was added dropwise to the mixture, and the mixture was stirred at -78 to -65°C for 1 hour. The reaction solution was poured into an aqueous solution of saturated ammonium chloride, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 4:1), to obtain the titled compound as oil. 8.1 g (64.2%)
¹H-NMR (CDCl₃) δ: 2.32 (1H, d, J = 3.8 Hz), 6.22 (1H, d, J = 3.8 Hz), 6.8 to 7.6 (8H, m)
IR (neat) cm⁻¹: 3385, 1723, 1604, 1509, 1225, 1158, 1025, 843, 813, 753, 569

### (2) Methyl 2-[(2-chlorophenyl)(4-fluorophenyl)methoxy]-5-nitrobenzoate

A mixture of methyl 2-hydroxy-5-nitrobenzoate (2.1 g, 10.6 mmol), (2-chlorophenyl)(4-fluorophenyl)methanol (3.00 g, 12.7 mmol), 40% solution of diethyl azodicarbonate in touluen (7.4 g, 17.0 mmol) and a solution (5 mL) of triphenylphosphine (3.3 g, 12.7 mmol) in DMF was stirred at room temperature for 12 hours, the reaction solution was poured into ice-water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 20:1), to obtain the titled compound as oil. 1.95 g (44.2%)
¹H-NMR (CDCl₃) δ: 3.99(3H, s), 6.88 (1H, m), 6.99 (1H, d, J = 9.2 Hz), 7.0 (2H, m), 7.2 to 7.3 (5H, m), 7.4 (1H, m), 7.6 (2H, m)
IR (KBr) cm⁻¹: 1727, 1615, 1586, 1520, 1488, 1438, 1344, 1276, 1246, 1129, 1073, 1000, 820, 748

### (3) Methyl 5-amino-2-[(2-chlorophenyl)(4-fluorophenyl)methoxy]benzoate

A mixture of methyl 2-[(2-chlorophenyl)(4-fluorophenyl)methoxy]-5-nitrobenzoate (1.7 g, 4.1 mmol), iron (1.1 g, 20.4 mmol), calcium chloride (222 mg, 2 mmol), ethanol (25 mL) and water (5 mL) was stirred at reflux temperature for 2 hours. The insolubles were filtered off, and the filtrate was concentrated. The residue was purified by silicagel column chromatography (hexane:ethyl acetate = 3:1), to obtain the titled compound as oil. 1.6 g (100%)
¹H-NMR (CDCl₃) δ: 3.48 (2H, br), 3.84 (3Hs), 6.59(1H, s), 6.7 (1H, m), 6.9 to 7.4 (7H, m), 7.4 to 7.6 (2H, m), 7.79 (1H, dd, J = 1.7 and 7.8 Hz)
IR (KBr) cm⁻¹: 3452, 1723, 1499, 1445, 1318, 1221, 1078, 755, 732

### (4) Methyl 5-({[(3,4-dimethoxyphenyl)amino]carbonyl}amino)-2-[(2-chlorophenyl)(4-fluorophenyl)methoxy]benzoate

To a solution of methyl 5-amino-2-[(2-chlorophenyl)(4-fluorophenyl)methoxy]benzoate (1.4 g, 3.6 mmol) in THF (28 mL) was added 3,4-dimethoxyphenyl isocyanate (0.78 g, 4.4 mmol) under ice-cooling, the mixture was stirred at 0°C for 1 hour and at room temperature for 12 hours, and the solvent was distilled off under reduced pressure. The residue was purified by silicagel column chromatography (hexane:ethyl acetate = 1:1), to obtain the titled compound as a solid. 2.1 g (100%)
¹H-NMR (CDCl₃) δ: 3.83 (3H, s), 3.84 (3Hs), 3.86 (3Hs), 6.68 to 6.79 (6H, m), 7.0 (3H, m), 7.15 to 7.24 (2H, m), 7.34 (1H, dd, J = 1.2 and 7.8 Hz), 7.4 (1H, m), 7.5 (2H, m), 7.65 (1H, d, J = 2.7 Hz), 7.73 (1H, dd, J = 1.7 and 7.8 Hz)
IR (KBr) cm⁻¹: 3343, 1731, 1606, 1556, 1511, 1412, 1220, 1159, 1079, 1026, 809, 754

### Example 237

### N-(tert-butyl)-5-({[(3,4-dimethoxyphenyl)amino]carbonyl}amino)-2-[(2-chlorophenyl)(4-fluorophenyl)methoxy]benzamide

### (1) 5-({[(3,4-Dimethoxyphenyl)amino]carbonyl}amino)-2-[(2-chlorophenyl)(4-fluorophenyl)methoxy]benzoic acid

To a solution (30 mL) of methyl 5-({[(3,4-dimethoxyphenyl)amino]carbonyl}amino)-2-[(2-chlorophenyl)(4-fluorophenyl)methoxy]benzoate (1.0 g, 1.77 mmol) in methanol was added 1N aqueous solution of sodium hydroxide (5 mL) at reflux temperature, and the mixture was stirred for 3 hours. The reaction solution was poured into water, and neutralized with 1N-hydrochloric acid. The precipitated crystals were collected by filtration, washed with water, and dried under reduced pressure to obtain the titled compound as a solid. 910 mg (93.3%)
¹H-NMR (CDCl₃) δ: 3.84 (3H, s), 3.87 (3H, s), 6.38 (1H, s), 6.82 (1H, d, J = 8.5 Hz), 6.9(2H, m), 7.1 (3H, m), 7.2 to 7.4 (5H, m), 7.44 (1H, br), 7.59 (1H, br), 7.72 (1H, d, J = 2.7 Hz), 8.26 (1H, dd, J = 2.7 and 9.1 Hz), 11.1 (1H, br)
IR (KBr) cm⁻¹: 3348, 1702, 1606, 1552, 1511, 1418, 1221, 1160, 1025, 812, 755

### (2) N-(tert-Butyl)-5-({[(3,4-dimethoxyphenyl)amino]carbonyl}amino)-2-[(2-chlorophenyl)(4-fluorophenyl)methoxy]benzamide

A mixed solution of 5-({[(3,4-dimethoxyphenyl)amino]carbonyl}amino)-2-[(2-chlorophenyl)(4-fluorophenyl)methoxy]benzoic acid (550 mg, 1.0 mmol), 1-hydroxy-1H-benzotriazole (230 mg, 1.5 mmol), tert-butyl amine (146 mg, 2 mmol), 1-(3-dimethylaminopropyl)-3-ethylcarbodiimide hydrochloride (240 mg, 1.25 mmol) and DMF (6 mL) was stirred under ice-cooling for 1 hour and at room temperature for 12 hours, was poured into water, and the precipitates were collected by filtration. This was purified by silicagel column chromatography (hexane:ethyl acetate = 1:1), to obtain the titled compound as a solid. 0.93 g (94.4%)
¹H-NMR (CDCl₃) δ: 1.18 (9H, s), 3.81 (3H, s), 3.83 (3H, s), 6.60 (1H, s), 6.71 to 6.76 (3H, m), 6.92 to 7.81 (14H, m)
IR (KBr) cm⁻¹: 3361, 2964, 1661, 1607, 1511, 1490, 1203, 1028, 755

According to the same method as Example 236, the compound of Example 238 was synthesized.

### Example 238

### Methyl 5-({[(3,4-dimethoxyphenyl)amino]carbonyl}amino)-2-{(3-chlorophenyl)(4-fluorophenyl)methoxy}benzoate

### (1) Synthesis of (4-fluorophenyl)(3-chlorophenyl)methanol

¹H-NMR (CDCl₃) δ: 2.24 (1H, d, J = 3.4 Hz), 5.80 (1H, d, J = 3.4 Hz), 7.0 (2H, m), 7.2 to 7.4 (6H, m)
IR (neat) cm⁻¹: 3364, 1709, 1604, 1509, 1428, 1225, 1158, 1040, 836, 797, 768, 565

### (2) Methyl 2-{(3-chlorophenyl)(4-fluorophenyl)methoxy}-5-nitrobenzoate

¹H-NMR (CDCl₃) δ: 4.00 (3H, s), 6.36 (1H, m), 6.98 (1H, d, J = 9.3 Hz), 7.0 (2H, m), 7.26 to 7.39 (3H, m), 7.5 (3H, m), 8.21 (1H, dd, J = 3.0 and 9.3 Hz), 8.74 (1H, d, J = 3.0 Hz)
IR (neat) cm⁻¹: 1727, 1615, 1586, 1520, 1488, 1438, 1344, 1275, 1246, 1129, 1074, 1000, 907, 843, 820, 748

### (3) Methyl 5-amino-2-{(3-chlorophenyl)(4-fluorophenyl)methoxy}benzoate

¹H-NMR (CDCl₃) δ: 3.45 (2H, br), 3.85 (3H, s), 6.06 (1H, m), 6.62 (2H, m), 7.0 (2H, m), 7.13 (1H, dd, J = 1.3 and 2.1 Hz), 7.22 to 7.29 (3H, m), 7.4 (2H, m), 7.5 (1H, m)
IR (neat) cm⁻¹: 1720, 1499, 1221, 1078, 834

### (4) Methyl 5-({[(3,4-dimethoxyphenyl)amino]carbonyl}amino)-2-{(3-chlorophenyl)(4-fluorophenyl)methoxy}benzoate

¹H-NMR(CDCl₃) δ: 3.85 (3H, s), 3.86 (3H, s), 3.90 (3H, s), 6.16 (1H, s), 6.48 (1H, s), 6.60 (1H, s), 6.73 (1H, dd, J = 2.3 and 8.5 Hz), 6.8 (3H, m), 7.0 (3H, m), 7.2 to 7.6 (6H, m), 7.61 (1H, d, J = 2.9 Hz)
IR (KBr) cm⁻¹: 3335, 1729, 1606, 1558, 1510, 1413, 1220, 1158, 1079, 1026, 799

According to the same method as Example 237, the compound of Example 239 was synthesized.

### Example 239

### N-(tert-Butyl)-5-({[(3,4-dimethoxyphenyl)amino]carbonyl}amino)-2-[(3-chlorophenyl)(4-fluorophenyl)methoxy]benzamide

### (1) 5-({[(3,4-Dimethoxyphenyl)amino]carbonyl}amino)-2-{(3-chlorophenyl)(4-fluorophenyl)methoxy}benzoic acid

¹H-NMR(CDCl₃) δ: 3.82 (3H, s), 3.85 (3H, s), 6.36 (1H, s), 6.79(1H, d, J = 8.5 Hz), 6.9 (2H, m), 7.1 (2H, m), 7.19 (1H, d, J = 2.4 Hz), 7.25 to 7.37 (6H, m), 7.67 (1H, br), 7.70 (1H, d, J = 2.9 Hz), 7.78 (1H, br), 8.28 (1H, dd, J = 2.9 and 9.1 Hz), 11.2 (1H, br)
IR (KBr) cm⁻¹: 3375, 1693, 1606, 1552, 1511, 1416, 1224, 1161, 1027

### (2) N-(tert-Butyl)-5-({[(3,4-dimethoxyphenyl)amino]carbonyl}amino)-2-[(3-chlorophenyl)(4-fluorophenyl)methoxy]benzamide

¹H-NMR(CDCl₃) δ: 1.20 (9H, s), 3.81 (3H, s), 3.83 (3H, s), 6.20 (1H, s), 6.7 to 6.8 (3H, m), 7.0 (2H, m), 7.10 (1H, d, J = 1.9 Hz), 7.2 (1H, m), 7.27 to 7.33 (6H, m), 7.59 (1H, br), 7.7 (2H, m), 7.72 (1H, dd, J = 2.8 and 8.9 Hz)
IR (KBr) cm⁻¹: 3358, 2965, 1637, 1607, 1510, 1490, 1203, 1027, 798, 687

According to the same method as Example 237, the compound of Example 240 was synthesized.

### Example 240

### N-(tert-Butyl)-5-({[(3,4-dimethoxyphenyl)amino]carbonyl}amino)-2-{(4-fluorophenyl)[4-(trifluoromethyl)phenyl]methoxy}benzamide

### (1) 5-({[(3,4-Dimethoxyphenyl)amino]carbonyl}amino)-2-{(4-fluorophenyl)[4-(trifluoromethyl)phenyl]methoxy}benzoic acid

¹H-NMR (CDCl₃) δ: 3.82 (3H, s), 3.85 (3H, s), 6.45 (1H, s), 6.80 (1H, d, J = 8.5 Hz), 6.86 to 6.90 (2H, m), 7.0 (2H, m), 7.18 (1H, d, J = 2.2 Hz), 7.3 (2H, m), 7.5 (3H, m), 7.7 (4H, m), 8.26 (1H, dd, J = 2.8 and 9.1 Hz), 11.1 (1H, br)
IR (KBr) cm⁻¹: 3375, 1730, 1606, 1552, 1512, 1416, 1224, 1026

### (2) N-(tert-Butyl)-5-({[(3,4-dimethoxyphenyl)amino]carbonyl}amino)-2-{(4-fluorophenyl)[4-(trifluoromethyl)phenyl]methoxy}benzamide

¹H-NMR(CDCl₃) δ: 1.18 (9H, s), 3.82 (3H, s), 3.84 (3H, s), 6.28 (1H, s), 6.7 to 6.8 (3H, m), 7.0 (4H, m), 7.2 to 7.3 (2H, m), 7.4 (3H, m), 7.6 (4H, m), 7.74 (1H, dd, J = 2.7 and 9.0 Hz)
IR (KBr) cm⁻¹: 3365, 1660, 1608, 1512, 1490, 1414, 1327, 1205, 1165, 1128, 1067, 1017, 822

### Example 241

### Methyl 2-[(3,4-dichlorophenyl)(phenyl)methoxy]-5-({[(3,4-dimethoxyphenyl)amino]carbonyl}amino)benzoate

### (1) Methyl 5-amino-2-[(3,4-dichlorophenyl)(phenyl)methoxy]benzoate

To a solution (25 mL) of methyl 2-hydroxy-5-nitrobenzoate (1.08 g, 5.5 mmol), (3,4-dichlorophenyl)(phenyl)methanol (1.40 g, 5.5 mmol) and triphenylphosphine (1.44 g, 5.5 mmol) in acetonitrile was added diethyl azodicarbonate (0.87 mL, 5.5 mmol), and the mixture was stirred at room temperature for 20 hours. The solvent was distilled off under reduced pressure, and the precipitated crystals were filtered off and were washed with toluene. The filtrate was concentrated, the residue was purified by silicagel column chromatography (hexane:ethyl acetate = 5:1). The resulting oil was dissolved in 80% aqueous ethanol solution (30 mL), iron (0.95 g, 17.0 mmol) and calcium chloride (189 mg, 1.7 mmol) were added thereto, and the mixture was heated to reflux for 2 hours. The insolubles were filtered off with Celite in heat, and the filtrate was concentrated, and diluted with water. The mixture was extracted with ethyl acetate, the extracted solution was washed with water and saturated brine, and was dried with anhydrous magnesium sulfate, and the solvent was distilled off under reduced pressure. The residue was purified by silicagel column chromatography (hexane:ethyl acetate = 2:1), to obtain the titled compound as oil. 0.55 g (2 steps, 25%)
¹H-NMR (CDCl₃) δ; 3.50 (2H, bs), 3.87 (3H, s), 6.06 (1H, s), 6.63 (2H, d, J = 2.0 Hz), 7.13 to 7.16 (1H, m), 7.28 to 7.46 (7H, m), 7.65 (1H, d, J = 1.8Hz)

### (2) Methyl 2-[(3,4-dichlorophenyl)(phenyl)methoxy]-5-({[(3,4-dimethoxyphenyl)amino]carbonyl}amino)benzoate

A mixed solution of methyl 5-amino-2-[(3,4-dichlorophenyl)(phenyl)methoxy]benzoate (0.55 g, 1.4 mmol), 3,4-dimethoxyphenyl isocyanate (0.21 mL, 1.4 mmol) and THF (10 mL) was stirred at room temperature for 4 hours. The solvent was distilled off under reduced pressure, and the resulting residue was purified by silicagel column chromatography (hexane:ethyl acetate = 1:1), to obtain the titled compound as amorphous. 0.58 g (73%)
¹H-NMR (CDCl₃) δ; 3.82 (3H, s), 3.83 (3H, s), 3.90 (3H, s), 6.12 (1H, s), 6.66 to 6.80 (5H, m), 6.99 (1H, d, J = 2.2 Hz), 7.25 to 7.48 (8H, m), 7.61 (1H, d, J = 2.6 Hz), 7.66 (1H, d, J = 1.8Hz)

### Example 242

### Methyl 2-[(3,4-difluorophenyl)(phenyl)methoxy]-5-({[(3,4-dimethoxyphenyl)amino]carbonyl}amino)benzoate

### (1) Methyl 5-amino-2-[(3,4-difluorophenyl)(phenyl)methoxy]benzoate

To a solution (30 mL) of 2-hydroxy-5-nitrobenzoate methyl (1.13 g, 5.7 mmol), (3,4-difluorophenyl)(phenyl)methanol (1.26 g, 5.7 mmol) and triphenylphosphine (1.50 g, 5.7 mmol) in acetonitrile was added diethyl azodicarbonate (0.90 mL, 5.7 mmol), and the mixture was stirred at room temperature for 20 hours. The solvent was distilled off under reduced pressure, and the precipitated crystals were filtered off and were washed with toluene. The filtrate was concentrated, and the residue was purified by silicagel column chromatography (hexane:ethyl acetate = 5:1). The resulting oil was dissolved in 80% aqueous ethanol solution (20 mL), iron (0.67 g, 12.0 mmol) and calcium chloride (133 mg, 1.2 mmol) were added thereto, and the mixture was heated to reflux for 2 hours. The insolubles were filtered off with Celite in heat, and the filtrate was concentrated and diluted with water. The mixture was extracted with ethyl acetate, the extracted solution was washed with water and saturated brine, and was dried with anhydrous magnesium sulfate, and the solvent was distilled off under reduced pressure. The residue was purified by silicagel column chromatography (hexane:ethyl acetate = 1:2), to obtain the titled compound as oil. 0.47 g (2 step, 22%)
¹H-NMR (CDCl₃) δ; 3.50 (2H, bs), 3.86 (3H, s), 6.07 (1H, s), 6.62 to 6.64 (2H, m), 7.02 to 7.46 (9H, m)

### (2) Methyl 2-[(3,4-difluorophenyl)(phenyl)methoxy]-5-({[(3,4-dimethoxyphenyl)amino]carbonyl}amino)benzoate

A mixed solution of methyl 5-amino-2-[(3,4-difluorophenyl)(phenyl)methoxy]benzoate (455 mg, 1.23 mmol), 3,4-dimethoxyphenyl isocyanate (183 µl, 1.23 mmol) and THF (10 mL) was stirred at room temperature for 16. The solvent was distilled off under reduced pressure, and the resulting residue was purified by silicagel column chromatography (hexane:ethyl acetate = 1:2), to obtain the titled compound as amorphous. 32 g (47%)
H-NMR (CDCl₃) δ; 3.83 (3H, s), 3.84 (3H, s), 3.88 (3H, s), 6.14 (1H, s), 6.65 to 6.81 (5H, m), 6.98 to 7.48 (10H, m), 7.61 (1H, d, J = 2.6 Hz)

According to the same method as Example 242, the following compounds were obtained.

### Example 243

### Ethyl 2-{bis[3-(trifluoromethyl)phenyl]methoxy}-5-({[(3,4-dimethoxyphenyl)amino]carbonyl}amino)benzoate

### 1) Methyl 2-{bis[3-(trifluoromethyl)phenyl]methoxy}-5-nitrobenzoate

H-NMR (CDCl₃) δ; 4.02 (3H,s),6.52 (1H,s),6.98 (1H,d,J = 9.6 Hz),7.48 to 7.73 (6H, m), 7.84 (2H, s), 8.24 (1H, dd, J = 9.6, 3.0 Hz), 8.77 (1H, d, J = 3.0 Hz)

### (2) Methyl 5-amino-2-{bis[3-(trifluoromethyl)phenyl]methoxy}benzoate

¹H-NMR (CDCl₃) δ; 3.52 (2H, bd), 3.85 (3H,s), 6.22 (1H,s), 6.61 to 6.64 (2H, m), 7.15 to 7.17 (1H, m), 7.42 to 7.66 (6H, m), 7.79 (2H, d, J = 0.8 Hz)

### (3) Methyl 2-{bis[3-(trifluoromethyl)phenyl]methoxy}-5-({[(3,4-dimethoxyphenyl)amino]carbonyl}amino)benzoate

¹H-NMR (CDCl₃) δ; 3.84 (3H, s), 3.86 (3H, s), 3.90 (3H, s), 6.31 (1H, s), 6.54 (1H, s), 6.67 to 6.78 (4H, m), 6.98 (1H, d, J = 2.2 Hz), 7.42 to 7.67 (8H, m), 7.81 (2H, s)

### Example 244

### Ethyl 2-[bis(4-fluorophenyl)methoxy]-5-({[(3,4-dimethoxyphenyl)amino]carbonyl}amino)benzoate

### (1) Ethyl 2-[bis(4-fluorophenyl)methoxy]-5-nitrobenzoate

¹H-NMR (CDCl₃) δ; 1.39 (3H, t, J = 7.2 Hz), 4.43 (2H, q, J = 7.2 Hz), 6.40 (1H,s), 6.95 to 7.11 (5H, m), 7.43 to 7.50 (4H, m), 8.20 (1H, dd, J = 9.2, 3.0 Hz), 8.71 (1H, d, J = 3.0 Hz)

### (2) Ethyl 5-amino-2-[bis(4-fluorophenyl)methoxy]benzoate

¹H-NMR (CDCl₃) δ; 1.29 (3H, t, J = 7.2 Hz), 3.32 (2H, bd), 4.31 (2H, q, J = 7.2 Hz), 6.11 (1H, s), 6.61 to 6.63 (2H, m), 6.94 to 7.13 (5H, m), 7.36 to 7.47 (4H, m)

### (3) Ethyl 2-[bis(4-fluorophenyl)methoxy]-5-({[(3,4-dimethoxyphenyl)amino]carbonyl}amino)benzoate

¹H-NMR (CDCl₃) δ; 1.30 (3H, t, J = 7.2 Hz), 3.83 (3H, s), 3.84 (3H, s), 4.33 (2H, q, J = 7.2 Hz), 6.18 (1H, s), 6.66-6.68 (5H, m), 6.95 to 7.05 (5H, m), 7.36 to 7.49 (5H, m), 7.58 (1H, d, J = 2.6 Hz)

### Example 245

### tert-Butyl 2-[bis(4-fluorophenyl)methoxy]-5-({[(3,4-dimethoxyphenyl)amino]carbonyl)amino)benzoate

### (1) tert-Butyl 2-[bis(4-fluorophenyl)methoxy]-5-nitrobenzoate

To a solution (10 mL) of tert-butyl 2-hydroxy-5-nitrobenzoate (0.55 g, 2.3 mmol), bis(4-fluorophenyl)methanol (0.51 g, 2.3 mmol) and triphenylphosphine (0.60 g, 2.3 mmol) in acetonitrile was added diethyl azodicarbonate (0.36 mL, 2.3 mmol), and the mixture was stirred at room temperature for 20 hours. The solvent was distilled off under reduced pressure, and the precipitated crystals were filtered off and were washed with toluene. The filtrate was concentrated, and the residue was purified with VARIAN MEGA BOND ELUT NH₂ (60 CC, 20 GRM, hexane:ethyl acetate = 10:1), to obtain the titled compound as oil. 0.61 g (60%)
¹H-NMR (CDCl₃) δ; 1.55 (9H, s), 6.37 (1H,s), 6.92 (1H, d, J = 9.6 Hz), 7.00 to 7.12 (4H, m), 7.39 to 7.47 (4H, m), 8.15 (1H, dd, J = 9.6, 3.0 Hz), 8.52 (1H, d, J = 3.0 Hz)

### (2) tert-Butyl 5-amino-2-[bis(4-fluorophenyl)methoxy]benzoate

A mixed solution of tert-butyl 2-[bis(4-fluorophenyl)methoxy]-5-nitrobenzoate (353 mg, 0.8 mmol), iron (223 mg, 4.0 mmol), calcium chloride (44 mg, 0.4 mmol) and 80% aqueous ethanol solution (10 mL) was heated to reflux for 3 hours. The insolubles were filtered off with Celite in heat, and the filtrate was concentrated, and diluted with water. The mixture was extracted with ethyl acetate, and the extracted solution was washed with water and saturated brine and was dried with anhydrous magnesium sulfate. The solvent was distilled off under reduced pressure, and the resulting residue was purified with VARIAN MEGA BOND ELUT SI (60 CC, 10 GRM, hexane:ethyl acetate = 3:1), to obtain the titled compound as oil. 269 mg (82%)
¹H-NMR (CDCl₃) δ; 1.50 (9H, s), 3.32 (2H, bd), 6.10 (1H, s), 6.55 to 6.57 (2H, m), 6.96 to 7.06 (5H, m), 7.35 to 7.43 (4H, m)

### (3) tert-Butyl 2-[bis(4-fluorophenyl)methoxy]-5-({[(3,4-dimethoxyphenyl)amino]carbonyl}amino)benzoate

A mixed solution of tert-butyl 5-amino-2-[bis(4-fluorophenyl)methoxy]benzoate (260 mg, 0.63 mmol), 3,4-dimethoxyphenyl isocyanate (0.10 mL, 0.70 mmol) and THF (5 mL) was stirred at room temperature for 16 hours. The solvent was distilled off under reduced pressure, and the obtained crude crystals were recrystallized from ethyl acetate and hexane. 246 mg (66%)
¹H-NMR (CDCl₃) δ; 1.50 (9H, s), 3.84 (3H, s), 3.86 (3H, s), 6. 12 (1H, s), 6.48 (1H, s), 6.56 (1H, s), 6.68 to 6.82 (3H, m), 6.96 to 7.07 (2H, m), 7.34 to 7.42 (6H, m)

### Example 246

### Isopropyl 2-[bis(4-fluorophenyl)methoxy]-5-({[(3,4-dimethoxyphenyl)amino]carbonyl}amino)benzoate

### (1) Isopropyl 2-hydroxy-5-nitrobenzoate

A mixture of 2-hydroxy-5-nitrobenzoate (10.0 g, 54.0 mmol), 2-propanol (4.13 mL, 54.0 mmol), 1,3-dicyclohexylcarbodiimide (12.2 g, 60.4 mmol) and 4-dimethylaminopyridine (658 mg, 5.40 mmol) in tetrahydrofuran (100 mL) and diethylether (200 mL) was stirred at 0°C for 2 hours and at room temperature for 12 hours. The insolubles were filtered off, and the filtrate was concentrated. The residue was purified by silicagel column chromatography (hexane:ethyl acetate = 9:1), to obtain the titled compound as a solid. 5.70 g (46.7%)
¹H-NMR (CDCl₃) δ; 1.45 (6H, d, J = 6.4 Hz), 5.30 to 5.42 (1H, m), 7.07 (1H, d, J = 9.2 Hz), 8.33 (1H, dd, J = 9.2, 3.0 Hz), 8.77 (1H, d, J = 3.0 Hz), 11.67 (1H, s)

### (2) Isopropyl 2-[bis(4-fluorophenyl)methoxy]-5-nitrobenzoate

To a solution (20 mL) of iso-propyl 2-hydroxy-5-nitrobenzoate (1.13 g, 5.0 mmol), bis(4-fluorophenyl)methanol (1.10 g, 5.0 mmol) and triphenylphosphine (1.31 g, 5.0 mmol) in acetonitrile was added diethyl azodicarbonate (0.79 mL, 5.0 mmol), and the mixture was stirred at room temperature for 16 hours. The solvent was distilled off under reduced pressure, and the precipitated crystals were filtered off and were washed with toluene. The filtrate was concentrated, and the residue was purified with basic silicagel column chromatography (hexane:ethyl acetate = 5:1), to obtain the titled compound as oil. 983 mg (46%)
¹H-NMR (CDCl₃) δ; 1.35 (6H, d, J = 6.2 Hz), 5.24 to 5.37 (1H, m), 6.39 (1H, s), 6.66 (1H, d, J = 9.2 Hz), 7.00 to 7.12 (4H, m), 7.42 to 7.49 (4H, m), 8.19 (1H, dd, J = 9.2, 2.8 Hz), 8.65 (1H, d, J = 2.8 Hz)

### (3) Isopropyl 5-amino-2-[bis(4-fluorophenyl)methoxy]benzoate

A mixed solution of isopropyl 2-[bis(4-fluorophenyl)methoxy]-5-nitrobenzoate (975 mg, 2.3 mmol), iron (642 mg, 11.5 mmol), calcium chloride (133 mg, 1.2 mmol) and 80% aqueous ethanol solution (20 mL) was heated to reflux for 2.5 hours. The insolubles were filtered off with Celite in heat, and the filtrate was concentrated, and diluted with water. The mixture was extracted with ethyl acetate, the extracted solution was washed with water and saturated brine, was dried with anhydrous magnesium sulfate. The solvent was distilled off under reduced pressure and the resulting residue was purified by silicagel column chromatography (hexane:ethyl acetate = 2:1), to obtain the titled compound as oil. 799 mg (88%)
¹H-NMR (CDCl₃) δ; 1.24 (6H, d, J = 6.2 Hz), 3.44 (2H, bd), 5.12 to 5.25 (1H, m), 6.09 (1H, s), 6.56 to 6.58 (2H, m), 6.91 to 7.06 (5H, m), 7.32 to 7.43 (4H, m)

### (4) Isopropyl 2-[bis(4-fluorophenyl)methoxy]-5-({[(3,4-dimethoxyphenyl)amino]carbonyl}amino)benzoate

A mixed solution of isopropyl 5-amino-2-[bis(4-fluorophenyl)methoxy]benzoate (793 mg, 2.0 mmol), 3,4-dimethoxyphenyl isocyanate (0.39 mL, 2.2 mmol) and THF (10 mL) was stirred at room temperature for 16 hours. The solvent was distilled off under reduced pressure, and the obtained crude crystals were recrystallized from ethyl acetate and hexane. 1.05 g (91%)
¹H-NMR (CDCl₃) δ; 1.28 (6H, d, J = 6.2 Hz), 3.84 (3H, s), 3.85 (3H, s), 5.16 to 5.30 (1H, m), 6.20 (1H, s), 6.56 (1H, s), 6.65 (1H, s), 6.67 to 6.82 (3H, m), 6.96 to 7.05 (5H, m), 7.37 to 7.44 (5H, m), 7.53 (1H, d, J = 3.0 Hz)

The compounds of Example 247 to 279 were synthesized as described below.

A solution (1.0 mL) of methyl 5-[(anilinocarbonyl)amino]-2-hydroxybenzoate (0.060 mmol), halogenated alkyl (0.070 mmol) and potassium carbonate (0.090 mmol) in DMF was stirred at 60°C for 3 hours. Ethyl acetate (2 mL), water (2.0 mL) and saturated brine (2 mL) were added thereto with shaking. The ethyl acetate layer was taken from a syringe, and was concentrated with Dry Thermo-unit PTU-1C. The residue was purified with preparative HPLC made by Gilson Inc. (PLRP-S column 5 µm 100 A, 50 x 25 mm, 40% to 100% aqueous solution of acetonitrile). The resulting compound was analyzed by LC MASS made by Gilson Inc. (Shiseido capsule pack C18 2 x 5 cm, λ = 220 nm, temperature 40°C, A liquid 0.05% trifluoroacetic acid solution: B liquid acetonitrile: 10 to 95% B liquid (for 4 minutes) 95% B solution (for 1.5 minutes), electrospray ionization mass spectrum).

### Example 247

### Methyl 5-[(anilinocarbonyl)amino]-2-(propa-2-ynyloxy)benzoate

8.1 mg
LC-MS: purity 99%, Rt = 1.82 min, m/z: 325 [M+H]⁺

### Example 248

### Methyl 5-[(anilinocarbonyl)amino]-2-[(3-methylbutan-2-enyl)oxy]benzoate

7.5 mg
LC-MS: purity 92%, Rt = 2.05 min, m/z: 377 [M+H]⁺+Na⁺

### Example 249

### Methyl 5-[(anilinocarbonyl)amino]-2-(cyclopentyloxy)benzoate

4.4 mg
LC-MS: purity 92%, Rt = 2.07 min, m/z: 355 [M+H]⁺

### Example 250

### Methyl 5-[(anilinocarbonyl)amino]-2-{[(2E)-3-phenylpropa-2-enyl]oxy}benzoate

8.5 mg
LC-MS: purity 97%, Rt = 2.14 min, m/z: 403 [M+H]⁺

### Example 251

### Methyl 5-[(anilinocarbonyl)amino]-2-(2-methoxy-2-oxoethoxy)benzoate

7.3 mg
LC-MS: purity 99%, Rt = 1.75 min, m/z: 359 [M+H]⁺

### Example 252

### Methyl 5-[(anilinocarbonyl)amino]-2-[(2,5-dimethylbenzyl)oxy]benzoate

8.4 mg
LC-MS: purity 99%, Rt = 2.19 min, m/z: 405 [M+H]⁺

### Example 253

### Methyl 5-[(anilinocarbonyl)amino]-2-[(4-ethylbenzyl)oxy]benzoate

7.3 mg
LC-MS: purity 99%, Rt = 2.21 min, m/z: 405 [M+H]⁺

### Example 254

### Methyl 5-[(anilinocarbonyl)amino]-2-[(3,4-dimethylbenzyl)oxy]benzoate

8.4 mg
LC-MS: purity 97%, Rt = 2.19 min, m/z: 405 [M+H]⁺

### Example 255

### Methyl 5-[(anilinocarbonyl)amino]-2-[(2,4-dimethylbenzyl)oxy]benzoate

8.6 mg
LC-MS: purity 99%, Rt = 2.19 min, m/z: 405 [M+H]⁺

### Example 256

### Methyl 5-[(anilinocarbonyl)amino]-2-[(4-chlorobenzyl)oxy]benzoate

8.5 mg
LC-MS: purity 99%, Rt = 2.16 min, m/z: 411 [M+H]⁺

### Example 257

### Methyl 5-[(anilinocarbonyl)amino]-2-[(2,6-difluorobenzyl)oxy]benzoate

2.4 mg
LC-MS: purity 99%, Rt = 2.05 min, m/z: 413 [M+H]⁺

### Example 258

### Methyl 5-[(anilinocarbonyl)amino]-2-(pyridin-3-ylmethoxy)benzoate

8.2 mg
LC-MS: purity 98%, Rt = 1.46 min, m/z: 378 [M+H]⁺

### Example 259

### Methyl 5-[(anilinocarbonyl)amino]-2-(pyridin-4-ylmethoxy)benzoate

5.4 mg
LC-MS: purity 99%, Rt = 1.45 min, m/z: 378 [M+H]⁺

### Example 260

### Methyl 2-[2-(acetyloxy)ethoxy]-5-[(anilinocarbonyl)amino]benzoate

6.4 mg
LC-MS: purity 98%, Rt = 1.79 min, m/z: 373 [M+H]⁺

### Example 261

### Methyl 5-[(anilinocarbonyl)amino]-2-(1-phenylethoxy)benzoate

10.2 mg
LC-MS: purity 98%, Rt = 2.10 min, m/z: 413 [M+H]⁺ + Na⁺

### Example 262

### Methyl 5-[(anilinocarbonyl)amino]-2-[(4-methylbenzyl)oxy]benzoate

6.2 mg,
LC-MS: purity 97%, Rt = 2.13 min, m/z: 391 [M+H]⁺

### Example 263

### Methyl 5-[(anilinocarbonyl)amino]-2-(2-phenylethoxy)benzoate

4.3 mg
LC-MS: purity 92%, Rt = 2.10 min, m/z: 391 [M+H]⁺

### Example 264

### Methyl 5-[(anilinocarbonyl)amino]-2-[(3-methylbenzyl)oxy]benzoate

8.8 mg
LC-MS: purity 99%, Rt = 2.13 min, m/z: 391 [M+H]⁺

### Example 265

### Methyl 5-[(anilinocarbonyl)amino]-2-[(2-methylbenzyl)oxy]benzoate

11.0 mg
LC-MS: purity 99%, Rt = 2.12 min, m/z: 391 [M+H]⁺

### Example 266

### Methyl 5-[(anilinocarbonyl)amino]-2-[(2-fluorobenzyl)oxy]benzoate

10.5 mg
LC-MS: purity 99%, Rt = 2.08 min, m/z: 395 [M+H]⁺

### Example 267

### Methyl 5-[(anilinocarbonyl)amino]-2-[(3-fluorobenzyl)oxy]benzoate

10.5 mg
LC-MS: purity 99%, Rt = 2.08 min, m/z: 395 [M+H]⁺

### Example 268

### Methyl 5-[(anilinocarbonyl)amino]-2-[(4-fluorobenzyl)oxy]benzoate

10.0 mg
LC-MS: purity 96%, Rt = 2.08 min, m/z: 395 [M+H]⁺

### Example 269

### Methyl 5-[(anilinocarbonyl)amino]-2-[(3,4-dichlorobenzyl)oxy]benzoate

9.9 mg
LC-MS: purity 90%, Rt = 2.25 min, m/z: 445 [M+H]⁺

### Example 270

### Methyl 5-[(anilinocarbonyl)amino]-2-[(2-cyanobenzyl)oxy]benzoate

1.0 mg
LC-MS: purity 96%, Rt = 1.99 min, m/z: 402 [M+H]⁺

### Example 271

### Methyl 5-[(anilinocarbonyl)amino]-2-[(3-cyanobenzyl)oxy]benzoate

11.8 mg
LC-MS: purity 96%, Rt = 2.00 min, m/z: 402 [M+H]⁺

### Example 272

### Methyl 5-[(anilinocarbonyl)amino]-2-[(4-cyanobenzyl)oxy)benzoate

10.3 mg
LC-MS: purity 99%, Rt = 2.00 min, m/z: 402 [M+H]⁺

### Example 273

### Methyl 5-[(anilinocarbonyl)amino]-2-(3-phenylpropoxy)benzoate

9.5 mg
LC-MS: purity 98%, Rt = 2.18 min, m/z: 405 [M+H]⁺

### Example 274

### Methyl 5-[(anilinocarbonyl)amino]-2-[(3-chlorobenzyl)oxy]benzoate

11.3 mg
LC-MS: purity 98%, Rt = 2.16 min, m/z: 411 [M+H]⁺

### Example 275

### Methyl 5-[(anilinocarbonyl)amino]-2-[(2-chlorobenzyl)oxy]benzoate

8.3 mg
LC-MS: purity 97%, Rt = 2.15 min, m/z: 411 [M+H]⁺

### Example 276

### Methyl 5-[(anilinocarbonyl)amino]-2-[(2,4-difluorobenzyl)oxy]benzoate

6.1 mg
LC-MS: purity 96%, Rt = 2.10 min, m/z: 413 [M+H]⁺

### Example 277

### Methyl 5-[(anilinocarbonyl)amino]-2-[(4-nitrobenzyl)oxy]benzoate

7.9 mg
LC-MS: purity 98%, Rt = 2.07 min, m/z: 422 [M+H]⁺

### Example 278

### Methyl 5-[(anilinocarbonyl)amino]-2-{[4-(methoxycarbonyl)benzyl]oxy}benzoate

8.7 mg
LC-MS: purity 99%, Rt = 2.04 min, m/z: 435 [M+H]⁺

### Example 279

### Methyl 5-[(anilinocarbonyl)amino]-2-{[3-(trifluoromethyl)benzyl]oxy}benzoate

11.8 mg
LC-MS: purity 95%, Rt = 2.19 min, m/z: 445 [M+H]⁺

### Example 280

### Methyl 2-{(2-chlorophenyl)[4-(trifluoromethyl)phenyl]methoxy}-5-({[(3,4-dimethoxyphenyl)amino]carbonyl}amino)benzoate

### (1) (2-Chlorophenyl)[4-(trifluoromethyl)phenyl]methanol

To a solution THF (200 mL) of 2-chlorobromobenzene (10.2 g, 53.3 mmol) in was added dropwise 1.6N solution of butyl lithium in hexane (40 mL, 65 mmol) at -78°C, the mixture was stirred at -78°C for 10 minutes, a solution of 4-trifluoromethyl benzaldehyde (11.3 g, 65.0 mmol) in THF (20 mL) was added dropwise thereto, and the mixture was stirred at -78 to -65°C for 1 hour. The reaction solution was poured into an aqueous solution of saturated ammonium chloride, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 4:1), to obtain the titled compound as oil. 6.5 g (42.7%)
¹H-NMR (CDCl₃) δ: 2.42 (1H, d, J = 3.9 Hz), 6.31 (1H, d, J = 3.9 Hz), 7.2 to 7.4 (3H, m), 7.5 to 7.6 (5H, m)
IR (neat) cm⁻¹: 3385, 1723, 1604, 1509, 1225, 1158, 1025, 843, 813, 753, 569

### (2) Methyl 2-{(2-chlorophenyl)[4-(trifluoromethyl)phenyl]methoxy}-5-nitrobenzoate

A mixture of methyl 2-hydroxy-5-nitrobenzoate (2.1 g, 10.6 mmol), (2-chlorophenyl)[4-(trifluoromethyl)phenyl]methanol (3.60 g, 12.7 mmol), 40% solution of diethyl azodicarbonate in toluene (7.4 g, 17.0 mmol) and a solution (10 mL) of triphenylphosphine (3.3 g, 12.7 mmol) in acetonitrile was stirred at room temperature for 12 hours, the reaction solution was poured into ice-water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 5:1), to obtain the titled compound as oil. 2.4 g (48.6%)
¹H-NMR (CDCl₃) δ: 3.99 (3H, s), 6.88 (1H, m), 6.96 (1H, s), 6.99 (1H, d, J = 9.4 Hz), 7.2 to 7.3 (2H, m), 7.4 (1H, m), 7.6 (2H, m), 7.80 (2H, d, J = 8.3 Hz), 8.25 (1H, dd, J = 2.9 and 9.4 Hz), 8.75 (1H, d, J = 2.9 Hz)
IR (KBr) cm⁻¹: 1735, 1616, 1524, 1487, 1439, 1347, 1325, 1276, 1127, 1068, 1018, 752

### (3) Methyl 5-amino-2-{(2-chlorophenyl)[4-(trifluoromethyl)phenyl]methoxy}benzoate

A mixture of methyl 2-{(2-chlorophenyl)[4-(trifluoromethyl)phenyl)methoxy}-5-nitrobenzoate (2.3 g, 4.9 mmol), iron (1.4 g, 24.7 mmol), calcium chloride (274 mg, 2.5 mmol), ethanol (30 mL) and water (6 mL) was stirred at reflux temperature for 2 hours. The insolubles were filtered off, and the filtrate was concentrated. The residue was dried under reduced pressure to obtain the titled compound as oil. 1.9 g (88.2%)
¹H-NMR (CDCl₃) δ: 3.49 (2H, br), 3.86 (3Hs), 6.69 (3H, m), 7.0 to 7.6 (5H, m), 7.66 (2H, d, J = 7.8 Hz), 7.7 (3H, m)
IR (KBr) cm⁻¹: 3370, 1720, 1499, 1325, 1221, 1126, 1068, 1018, 753

### (4) Methyl 2-{(2-chlorophenyl)[4-(trifluoromethyl)phenyl]methoxy}-5-({[(3,4-dimethoxyphenyl)amino]carbonyl}amino)benzoate

To a solution (20 mL) of methyl 5-amino-2-{(2-chlorophenyl)[4-(trifluoromethyl)phenyl]methoxy}benzoate (1.7 g, 3.6 mmol) in THF was added 3,4-dimethoxyphenyl isocyanate (0.84 g, 4.7 mmol) under ice-cooling, the mixture was stirred at 0°C for 1 hour and at room temperature for 12 hours, and the solvent was distilled off under reduced pressure. The residue was purified by silicagel column chromatography (hexane:ethyl acetate = 1:1), to obtain the titled compound as a solid. 1.6 g (66.8%)
¹H-NMR (CDCl₃) δ: 3.84 (3H, s), 3.85 (3Hs), 3.88 (3Hs), 6.52 (1H, brs), 6.63 (1H, brs), 6.72 (1H, dd, J = 2.4 Hz), 6.78 (1H, d, J = 4.4 Hz), 6.81 (2H, 8.5 Hz), 6.99 (1H, d, J = 2.4 Hz), 7.18 to 7.25 (2H, m), 7.36 (1H, dd, J = 1.5 Hz), 7.47 (1H, dd, J = 2.8 and 8.9 Hz), 7.59 (2H, d, J = 8.4 Hz), 7.66 (1H, d, J = 2.8 Hz), 7.70 (1H, dd, J = 2.0 and 7.6 Hz), 7.75 (2H, d, J = 8.4 Hz)
IR (KBr) cm⁻¹: 3346, 1732, 1610, 1550, 1498, 1414, 1326, 1221, 1165, 1125, 1068, 1017, 810, 755

### Example 281

### N-(tert-Butyl)-2-{(2-chlorophenyl)[4-(trifluoromethyl)phenyl]methoxy}-5-({[(3,4-dimethoxyphenyl)amino]carbonyl}amino)benzamide

### (1) 2-{(2-Chlorophenyl)[4-(trifluoromethyl)phenyl]methoxy}-5-({[(3,4-dimethoxyphenyl)amino]carbonyl}amino) benzoate

To a solution (30 mL) of methyl 2-{(2-chlorophenyl)[4-(trifluoromethyl)phenyl]methoxy}-5-({[(3,4-dimethoxyphenyl)amino]carbonyl}amino)benzoate (0.9 g, 1.46 mmol) in methanol was added 1N aqueous solution of sodium hydroxide (5 mL) at reflux temperature, and the mixture was stirred for 3 hours. The reaction solution was poured into water, and neutralized with 1N-hydrochloric acid. The precipitated crystals were collected, washed with water, and dried under reduced pressure to obtain the titled compound as a solid. 800 mg (91.0%)
¹H-NMR (CDCl₃) δ: 3.81 (3H, s), 3.84 (3H, s), 6.74 to 6.89 (4H, m), 7.19 to 7.76 (13H, m), 8.23 (1H, dd, J = 2.8 and 9.2 Hz), 11.0 (1H, br)
IR (KBr) cm⁻¹: 3336, 1697, 1609, 1556, 1514, 1419, 1325, 1221, 1166, 1129, 1068, 1018, 910, 812, 755, 733

### (2) N-(tert-Butyl)-2-{(2-chlorophenyl)[4-(trifluoromethyl)phenyl]methoxy}-5-({[(3,4-dimethoxyphenyl)amino]carbonyl}amino)benzamide

A mixed solution of 2-{(2-chlorophenyl)[4-(trifluoromethyl)phenyl]methoxy}-5-({[(3,4-dimethoxyphenyl)amino]carbonyl}amino)benzoate (300 mg, 0.5 mmol), 1-hydroxy-1H-benzotriazole (115 mg, 0.75 mmol), tert-butyl amine (105 mg, 1 mmol), 1-(3-dimethylaminopropyl)-3-ethylcarbodiimide hydrochloride (120 mg, 0.63 mmol) and DMF (3 mL) was stirred under ice-cooling for 1 hour and at room temperature for 12 hours, was poured into water, and the precipitates were collected. This was purified by silicagel column chromatography (hexane:ethyl acetate = 1:1), to obtain the titled compound as a solid. 0.24 g (74.7%)
1H-NMR (CDCl₃) δ: 1.17 (9H, s), 3.84 (3H, s), 3.85 (3H, s), 6.6 to 6.8 (4H, m), 6.99 (1H, brs), 7.08 (1H, d, J = 3.0 Hz,), 7.3 (4H, m), 7.4 (1H, m), 7.50 (2H, d, J = 8.0 Hz), 7.57 (1H, s), 7.6 (3H, m), 7.74 (1H, dd, J = 2.8 and 8.9 Hz)
IR (KBr) cm⁻¹: 3361, 2954, 1727, 1652, 1609, 1556, 1514, 1498, 1466, 1439, 14.15, 1326, 1222, 1165, 1127, 1068, 1018, 909, 810, 733

According to the same method as Example 280, the compound of Example 282 was synthesized.

### Example 282

### Methyl 2-{(2-fluorophenyl)[4-(trifluoromethyl)phenyl]methoxy}-5-({[(3,4-dimethoxyphenyl)amino]carbonyl}amino)benzoate

### (1) (2-Fluorophenyl)[4-(trifluoromethyl)phenyl]methanol

¹H-NMR (CDCl₃) δ: 2.37 (1H, d, J = 3.8 Hz), 6.31 (1H, d, J = 4.1 Hz), 7.0 to 7.1 (1H, m), 7.16 (1H, td, 1.2 and 7.4 Hz), 7.27 to 7.31 (1H, m), 7.46 (1H, td, J = 1.7 and 7.4 Hz), 7.54 (2H, d, J = 8.4 Hz), 7.60 (2H, d, J = 8.4 Hz)
IR (neat) cm⁻¹: 3375, 1710, 1620, 1588, 1488, 1458, 1418, 1326, 1230, 1165, 1126, 1068, 1017, 820, 759

### (2) Methyl 2-{(2-fluorophenyl)[4-(trifluoromethyl)phenyl]methoxy}-5-nitrobenzoate

¹H-NMR (CDCl₃) δ: 4.01 (3H, s), 6.85 (1H, s), 7.03 (1H, d, J = 9.3 Hz), 7.13 (1H, ddd, J = 1.22, 8.3, and 10.3 Hz), 7.2 to 7.3 (1H, m), 7.6 (3H, m), 7.75 (2H, d, J = 8.1 Hz), 8.24 (1H, dd, J = 2.9 and 9.3 Hz), 8.76 (1H, d, J = 2.9 Hz)
IR (KBr) cm⁻¹: 1736, 1615, 1586, 1524, 1488, 1348, 1326, 1277, 1128, 1068, 1017, 826, 760

### (3) Methyl 5-amino-2-{(2-fluorophenyl)[4-(trifluoromethyl)phenyl]methoxy}benzoate

¹H-NMR (CDCl₃) δ: 3.55 (2H, br), 3.86 (3H, s), 6.57 (1H, m), 6.7 (2H, m), 7.0 to 7.5 (4H, m), 7.58 (2H, d, J = 7.8 Hz), 7.7 (3H, m)
IR (KBr) cm⁻¹: 3375, 1723, 1621, 1499, 1326, 1222, 1166, 1125, 1068, 1018, 760

### (4) Methyl 2-{(2-fluorophenyl)[4-(trifluoromethyl)phenyl]methoxy}-5-({[(3,4-dimethoxyphenyl)amino]carbonyl}amino)benzoate

¹H-NMR (CDCl₃) δ: 3.85 (3H, s), 3.86 (3H, s), 3.89 (3H, s), 6.47 (1H, s), 6.59 (1H, s), 6.66 (1H, s), 6.73 (1H, dd, J = 2.4 and 8.5 Hz), 6.80 (1H, d, J = 8.5 Hz), 6.85 (1H, d, J = 9.0 Hz), 6.98 (1H, d, J = 2.4 Hz), 7.0 (1H, m), 7.1 (1H, m), 7.2 (1H, m), 7.47 (1H, dd, J = 2.8 and 9.0 Hz), 7.59 (2H, d, J = 8.4 Hz), 7.71 (2H, d, J = 8.4 Hz)
IR (KBr) cm⁻¹: 3345, 1727, 1606, 1554, 1477, 1326, 1221, 1165, 1124, 1068, 1018, 762

According to the same method as Example 281, the compound of Example 283 was synthesized.

### Example 283

### N-(tert-Butyl)-2-{(2-fluorophenyl)[4-(trifluoromethyl)phenyl]methoxy}-5-({[(3,4-dimethoxyphenyl)amino]carbonyl}amino)benzamide

### (1) 2-{(2-Fluorophenyl)[4-(trifluoromethyl)phenyl]methoxy}-5-({[(3,4-dimethoxyphenyl)amino]carbonyl}amino)benzoate

¹H-NMR (CDCl₃) δ: 3.83 (3H, s), 3.85 (3H, s), 6.78 (1H, m), 6.81 (1H, s), 6.90 (1H, dd, 2.4 and 8.5 Hz), 6.93 (1H, d, J = 9.3 Hz), 7.14 to 7.21 (3H, m), 7.3 to 7.4 (2H, m), 7.57 (2H, d, J = 8.1 Hz), 7.57 (1H, brs), 7.67 (2H, d, J = 8.1 Hz), 7.70 (1H, brs), 7.73 (1H, d, J = 2.6 Hz), 8.25 (1H, dd, J = 2.6 and 9.3), 11.0 (1H, br)
IR (KBr) cm⁻¹: 3294, 1676, 1540, 1497, 1454, 1327, 1300, 1259, 1221, 1167, 1135, 1067, 1020, 815, 764

### (2) N-(tert-Butyl)-2-{(2-fluorophenyl)[4-(trifluoromethyl)phenyl]methoxy}-5-({[(3,4-dimethoxyphenyl)amino]carbonyl}amino)benzamide

¹H-NMR (CDC13) δ: 1.22 (9H, s), 3.81 (3H, s), 3.83 (3H, s), 6.59 (1H, s), 6.7 (3H, m), 7.1 (3H, m), 7.3 (4H, m), 7.51 (2H, d, J = 8.3 Hz), 7.56 (1H, s), 7.6 (3H, m), 7.71 (1H, dd, J = 2.8 and 8.9 Hz)
IR (KBr) cm⁻¹: 3347, 2968, 1637, 1610, 1543, 1515, 1491, 1456, 1326, 1207, 1167, 1130, 1068, 1028, 911, 760, 734

According to the same method as Example 280, the compound of Example 284 was synthesized.

### Example 284

### Methyl 2-{(3-chlorophenyl)[4-(trifluoromethyl)phenyl]methoxy}-5-({[(3,4-dimethoxyphenyl)amino]carbonyl}amino)benzoate

### (1) (3-Chlorophenyl)[4-(trifluoromethyl)phenyl]methanol

¹H-NMR (CDCl₃) δ: 2.37 (1H, d, J = 2.9 Hz), 5.86 (1H, brs), 7.22 to 7.31 (3H, m), 7.38 (1H, s), 7.50 (2H, d, J = 8.2 Hz), 7.61 (2H, d, J = 8.2 Hz)
IR (neat) cm⁻¹. 3406, 1710, 1620, 1597, 1476, 1417, 1376, 1327, 1260, 1166, 1126, 1068, 1045, 1017, 846, 787, 762

### (2) Methyl 2-{(3-chlorophenyl)[4-(trifluoromethyl)phenyl]methoxy}-5-nitrobenzoate

¹H-NMR (CDCl₃) δ: 4.01 (3H, s), 6.85 (1H, s), 7.03 (1H, d, J = 9.3 Hz), 7.13 (1H, ddd, J = 1.22, 8.3, and 10.3 Hz), 7.2 to 7.3 (2H, m), 7.6 (3H, m), 7.75 (2H, d, J = 8.1 Hz), 8.24 (1H, dd, J = 2.9 and 9.3 Hz), 8.76 (1H, d, J = 2.9 Hz)
IR (KBr) cm⁻¹: 1736, 1615, 1589, 1524, 1486, 1347, 1326, 1277, 1128, 1068, 1017, 825, 748

### (3) Methyl 5-amino-2-{(3-chlorophenyl)[4-(trifluoromethyl)phenyl]methoxy}benzoate

¹H-NMR (CDCl₃) δ: 3.51 (2H, br), 3.86 (3H, s), 6.12 (1H, s), 6.6 (2H, m), 7.15 (1H, d, J = 2.4 Hz), 7.22 to 7.34 (3H, m), 7.50 (1H, s), 7.6 (4H, m)
IR (KBr) cm⁻¹: 1722, 1499, 1325, 1222, 1124, 1067, 1018, 787

### (4) Methyl 2-{(3-chlorophenyl)[4-(trifluoromethyl)phenyl]methoxy}-5-({[(3,4-dimethoxyphenyl)amino]carbonyl}amino)benzoate

¹H-NMR (CDCl₃) δ: 3.81 (3H, s), 3.82 (3H, s), 3.88 (3H, s), 6.17 (1H, s), 6.63 (1H, s), 6.6 to 6.8 (3H, m), 6.81 (1H, brs), 6.91 (1H, brs), 6.99(1H, d, J = 2.2 Hz), 7.22 to 7.33 (2H, m), 7.45 (1H, dd, J = 3.9 and 9.0 Hz), 7.51 (1H, brs), 7.60 (5H, m)
IR (KBr) cm⁻¹: 3334, 1725, 1654, 1608, 1556, 1523, 1498, 1326, 1220, 1165, 1127, 1067, 1018, 767

According to the same method as Example 281, the compound of Example 285 was synthesized.

### Example 285

### N-(tert-Butyl)-2-{(3-chlorophenyl)[4-(trifluoromethyl)phenyl]methoxy}-5-({[(3,4-dimethoxyphenyl)amino]carbonyl}amino)benzamide

### (1) 2-{(3-Chlorophenyl)[4-(trifluoromethyl)phenyl]methoxy}-5-({[(3,4-dimethoxyphenyl)amino]carbonyl}amino)benzoate

¹H-NMR (CDCl₃) δ: 3.82 (3H, s), 3.85 (3H, s), 6.40 (1H, s), 6.80 (1H, d, J = 8.8 Hz), 6.9(2H, m), 7.15 (1H, d, J = 2.4 Hz), 7.2 to 7.4 (4H, m), 7.5 (3H, m), 7.64 (1H, brs), 7.67 (2H, d, J = 8.3 Hz), 7.71 (1H, d, J = 2.8 Hz) 8.22 (1H, dd, J = 2.8 and 9.2 Hz), 11.0 (1H, br)
IR (KBr) cm⁻¹: 3308, 1703, 1555, 1495, 1325, 1222, 1128, 1068, 1018

### (2) N-(tert-Butyl)-2-{(3-chlorophenyl)[4-(trifluoromethyl)phenyl]methoxy}-5-({[(3,4-dimethoxyphenyl)amino]carbonyl}amino)benzamide

¹H-NMR (CDCl₃) δ: 1.21 (9H, s), 3.81 (3H, s), 3.82 (3H, s), 6.24 (1H, s), 6.68 to 6.76 (3H, m), 7.10 (1H, s) 7.2 (1H, m), 7.30 to 7.34 (4H, m), 7.45 (2H, d, J = 8.1 Hz), 7.6 (5H, m), 7.71 (1H, dd, J = 2.6 and 8.9 Hz)
IR (KBr) cm⁻¹: 3370, 1640, 1514, 1493, 1326, 1206, 1167, 1130, 1068, 1018, 734

According to the same method as Example 280, the compound of Example 286 was synthesized.

### Example 286

### Methyl 2-{(3-fluorophenyl)[4-(trifluoromethyl)phenyl]methoxy}-5-({[(3,4-dimethoxyphenyl)amino]carbonyl}amino)benzoate

### (1) (3-Fluorophenyl)[4-(trifluoromethyl)phenyl]methanol

¹H-NMR (CDCl₃) δ: 2.38 (1H, d, J = 3.1 Hz), 5.87 (1H, d, J = 3.1 Hz), 7.0 (1H, m), 7.07 to 7.14 (2H, m), 7.28 to 7.34 (1H, m), 7.50 (2H, d, J = 8.2 Hz), 7.60 (2H, d, 8.3 Hz)
IR (neat) cm⁻¹: 3405, 1710, 1614, 1592, 1487, 1450, 1415, 1377, 1327, 1251, 1167, 1127, 1068, 1017, 851, 762

### (2) Methyl 2-{(3-fluorophenyl)[4-(trifluoromethyl)phenyl]methoxy}-5-nitrobenzoate

¹H-NMR (CDCl₃) δ: 4.01 (3H, s), 6.44 (1H, s), 7.0 (2H, m), 7.2 to 7.3 (3H, m), 7.64 (2H, d, J = 8.4 Hz), 7.68 (2H, d, J = 8.4 Hz), 8.22 (1H, dd, J = 2.8 and 8.1 Hz), 8.76 (1H, d, J = 2.8 Hz)
IR (KBr) cm⁻¹: 1734, 1615, 1591, 1523, 1488, 1348, 1326, 1277, 1167, 1128, 1068, 1016, 823, 777, 748

### (3) Methyl 5-amino-2-{(3-fluorophenyl)[4-(trifluoromethyl)phenyl]methoxy}benzoate

¹H-NMR (CDCl₃) δ: 3.51 (2H, brs), 3.85 (3H, s), 6.15 (1H, m), 6.6 (2H, m), 7.0 (1H, m), 7.1 (1H, m), 7.2 to 7.3 (3H, m), 7.59 (2H, d, J = 8.7 Hz), 7.62 (2H, d, J = 8.7 Hz)
IR (KBr) cm⁻¹: 3374, 1721, 1615, 1592, 1500, 1326, 1222, 1125, 1067, 1018, 789

### (4) Methyl 2-{(3-fluorophenyl)[4-(trifluoromethyl)phenyl]methoxy}-5-({[(3,4-dimethoxyphenyl)amino]carbonyl}amino)benzoate

¹H-NMR (CDCl₃) δ: 3.85 (3H, s), 3.86 (3H, s), 3.89 (3H, s), 6.21 (1H, s), 6.68 to 6.81 (5H, m), 6.92 to 6.97 (1H, m), 6.99 (1H, d, J = 2.2 Hz), 7.20 to 7.30 (3H, m), 7.46 (1H, dd, J = 2.9 and 9.0 Hz), 7.6 (5H, m)
IR (KBr) cm⁻¹: 3344, 1710, 1654, 1613, 1558, 1497, 1416, 1326, 1222, 1166, 1129, 1068, 1018, 731

According to the same method as Example 281, the compound of Example 287 was synthesized.

### Example 287

### N-(tert-Butyl)-2-{(3-fluorophenyl)[4-(trifluoromethyl)phenyl]methoxy}-5-({[(3,4-dimethoxyphenyl)amino]carbonyl}amino)benzamide

### (1) 2-{(3-Fluorophenyl)[4-(trifluoromethyl)phenyl]methoxy}-5-({[(3,4-dimethoxyphenyl)amino]carbonyl}amino)benzoate

¹H-NMR (CDCl₃) δ: 3.83 (3H, s), 3.86 (3H, s), 6.43 (1H, s), 6.80 (1H, d, J = 8.7 Hz), 6.9(2H, m), 7.0 to 7.2 (4H, m), 7.3 to 7.5 (2H, m), 7.52 (2H, d, J = 8.3 Hz), 7.61 (1H, brs), 7.68 (2H, d, J = 8.3 Hz), 7.71 (1H, d, J = 3.0 Hz), 8.23 (1H, dd, J = 2.6 and 9.2 Hz), 11.0 (1H, br)
R (KBr) cm⁻¹: 3308, 1704, 1613, 1556, 1515, 1495, 1325, 1225, 1130, 1068, 1018, 763

### (2) N-(tert-Butyl)-2-{(3-fluorophenyl)[4-(trifluoromethyl)phenyl]methoxy}-5-({[(3,4-dimethoxyphenyl)amino]carbonyl}amino)benzamide

¹H-NMR(CDCl₃) δ: 1.20 (9H, s), 3.82 (3H, s), 3.84 (3H, s), 6.27 (1H, s), 6.7 to 6.8 (3H, m), 7.0 to 7.2 (5H, m), 7.19 (1H, s), 7.3 (1H, m), 7.46 (2H, d, J = 7.8 Hz), 7.56 (1H, s), 7.6 (3H, m), 7.71 (1H, dd, J = 2.8 and 8.9 Hz)
IR (KBr) cm⁻¹: 3346, 1636, 1515, 1490, 1326, 1206, 1167, 1131, 1068

### Example 288

### 5-({[(3,4-Dimethoxyphenyl)amino]carbonyl}amino)-2-{(4-fluorophenyl)[4-(trifluoromethyl)phenyl]methoxy}-N-isopropyl benzamide

A mixture of 2-{(4-fluorophenyl)[4-(trifluoromethyl)phenyl]methoxy}-5-({[(3,4-dimethoxyphenyl)amino]carbonyl}amino)benzoate (200 mg, 0.34 mmol), 1-hydroxy-1H-benzotriazole (78 mg, 0.51 mmol), iso-propyl amine (0.58 mL, 0.68 mmol), 1-(3-dimethylaminopropyl)-3-ethylcarbodiimide hydrochloride (82 mg, 0.43 mmol) and DMF (2 mL) was stirred under ice-cooling for 1 hour and at room temperature for 12 hours, and was poured into water, and the precipitates were collected. This was purified by silicagel column chromatography (hexane:ethyl acetate = 1:1), to obtain the titled compound as a solid. 210 mg (98.7%)
¹H-NMR(400 MHz) δ: 0.94 (6H, s), 3.82 (6H, s), 4.10 (1H, t, J = 6.6 Hz), 6.31 (1H, s), 6.68 to 6.77 (3H, m), 7.0 (2H, s) 7.12 (1H, s), 7.2 (2H, m), 7.46 (2H, d, J = 7.8 Hz), 7.64 (2H, d, J = 7.8 Hz), 7.77 (1H, s), 7.9 to 8.11 (3H, m), 8.23 (1H, s)
IR (KBr) cm⁻¹: 3348, 1635, 1607, 1511, 1491, 1326, 1205, 1166, 1129, 1068, 1017, 824, 734

The compounds of Example 289 to 377 were synthesized as described below.

To a solution (1 mL) of methyl 5-amino-2-benzhydryloxy benzoate (0.900 mmol) and diisopropylethyl amine (0.108 mmol) in acetonitrile was added N,N-disuccinimidyl carbamate (0.108 mmol) at 0°C, and the mixture was stirred at 0°C for 40 minutes. This solution was added to a solution of diisopropylethyl amine (0.108 mmol) and amine (0.09 mmol), and the mixture was stirred for 12 hours at room temperature. The solvent was evaporated with Dry Thermo-unit, to the residue were added water and dichloromethane, the layer of dichloromethane was separated with PTFE filter (1 µm pore size, Whatman Inc.). Dichloromethane was evaporated with Dry Thermo-unit, and the residue was purified with preparative HPLC (PLRP-S column 5 µm 100 A, 50 x 25 mm, 40% to 100% aqueous solution of acetonitrile). The resulting cbmpound was analyzed by LC MASS made by Gilson Inc. (Shiseido capsule pack C18 2 x 5 cm, λ = 220 nm, temperature 40°C, A liquid 0.05% trifluoroacetic acid solution: B liquid acetonitrile: 10 to 95% B liquid (for 4 minutes) 95% B liquid (for 1.5 minutes), electrospray ionization mass spectrum).

### Example 289

### Methyl 2-(benzhydryloxy)-5-{[(butylamino)carbonyl]amino}benzoate

20.0 mg, LC-MS: purity 95%, Rt = 3.56 mjn, m/z: 433 [M+H]⁺

### Example 290

### Methyl 2-(benzhydryloxy)-5-({[(cyclohexylmethyl)amino]carbonyl}amino)benzoate

22.3 mg, LC-MS: purity 95%, Rt = 3.86 mjn, m/z: 473 [M+H]⁺

### Example 291

### Methyl 2-(benzhydryloxy)-5-{[(cyclopropylamino)carbonyl]amino}benzoate

21.2 mg, LC-MS: purity 92%, Rt = 3.25 mjn, m/z: 417 [M+H]⁺

### Example 292

### Methyl 2-(benzhydryloxy)-5-{[(benzylamino)carbonyl]amino}benzoate

21.5 mg, LC-MS: purity 99%, Rt = 3.58 mjn, m/z: 467 [M+H]⁺

### Example 293

### Methyl 2-(benzhydryloxy)-5-({[(1,3-benzodioxol-5-ylmethyl)amino]carbonyl}amino)benzoate

27.5 mg, LC-MS: purity 97%, Rt = 3.53 mjn, m/z: 511 [M+H]⁺

### Example 294

### Methyl 2-(benzhydryloxy)-5-({[(2-phenylethyl)amino]carbonyl}amino)benzoate

21.5 mg, LC-MS: purity 99%, Rt = 3.67 mjn, m/z: 481 [M+H]⁺

### Example 295

### Methyl 2-(benzhydryloxy)-5-({[(3-phenylpropyl)amino]carbonyl}amino)benzoate

24.5 mg, LC-MS: purity 99%, Rt = 3.77 mjn, m/z: 495 [M+H]⁺

### Example 296

### Methyl 5-{[(benzhydrylamino)carbonyl]amino}-2-(benzhydryloxy)benzoate

23.3 mg, LC-MS: purity 89%, Rt = 3.93 mjn, m/z: 543 [M+H]⁺

### Example 297

### Methyl 2-(benzhydryloxy)-5-({[(2-methoxyethyl)amino]carbonyl}amino)benzoate

21.8 mg, LC-MS: purity 97%, Rt = 3.18 mjn, m/z: 435 [M+H]⁺

### Example 298

### Methyl 2-(benzhydryloxy)-5-[({[3-(methylthio)propyl]amino}carbonyl)amino]benzoate

24.0 mg, LC-MS: purity 98%, Rt = 3.45 mjn, m/z: 465 [M+H]⁺

### Example 299

### Methyl 2-(benzhydryloxy)-5-({[(tetrahydrofuran -2-ylmethyl)amino]carbonyl}amino)benzoate

21.0 mg, LC-MS: purity 100%, Rt = 3.27 mjn, m/z: 461 [M+H]⁺

### Example 300

### Methyl 2-(benzhydryloxy)-5-[({[2-(1H-indol-3-yl)ethyl]amino}carbonyl)amino]benzoate

26.3 mg, LC-MS: purity 99%, Rt = 3.59 mjn, m/z: 520 [M+H]⁺

### Example 301

### Methyl 2-(benzhydryloxy)-5-({[(1-ethylpropyl)amino]carbonyl}amino)benzoate

19.0 mg, LC-MS: purity 99%, Rt = 3.64 mjn, m/z: 447 [M+H]⁺

### Example 302

### Methyl 2-(benzhydryloxy)-5-{[(tert-butylamino)carbonyl]amino}benzoate

18.0 mg, LC-MS: purity 96%, Rt = 3.63 mjn, m/z: 455 [M+H]⁺

### Example 303

### Methyl 2-(benzhydryloxy)-5-{[(cyclohexylamino)carbonyl]amino}benzoate

22.4 mg, LC-MS: purity 97%, Rt = 3.71 mjn, m/z: 459 [M+H]⁺

### Example 304

### Methyl 2-(benzhydryloxy)-5-{[(propa-2-ynylamino)carbonyl]amino}benzoate

17.4 mg, LC-MS: purity 97%, Rt = 3.27 mjn, m/z: 437 [M+H]⁺

### Example 305

### Methyl 2-(benzhydryloxy)-5-[({[4-(trifluoromethyl)benzyl]amino}carbonyl)amino]benzoate

22.4 mg, LC-MS: purity 99%, Rt = 3.83 mjn, m/z: 535 [M+H]⁺

### Example 306

### Methyl 2-(benzhydryloxy)-5-[({[2-(3,4-dimethoxyphenyl)ethyl]amino}carbonyl)amino]benzoate

26.5 mg, LC-MS: purity 90%, Rt = 3.49 mjn, m/z: 541 [M+H]⁺

### Example 307

### Methyl 2-(benzhydryloxy)-5-({[(3,3-diphenylpropyl)amino]carbonyl}amino)benzoate

32.4 mg, LC-MS: purity 92%, Rt = 4.02 mjn, m/z: 571 [M+H]⁺

### Example 308

### Methyl 2-(benzhydryloxy)-5-{[(2,3-dihydro-1H-indene-2-ylamino)carbonyl]amino}benzoate

23.6 mg, LC-MS: purity 99%, Rt = 3.74 mjn, m/z: 493 [M+H]⁺

### Example 309

### Methyl 2-(benzhydryloxy)-5-({[(3-isopropoxypropyl)amino]carbonyl}amino)benzoate

22.9 mg, LC-MS: purity 99%, Rt = 3.48 mjn, m/z: 477 [M+H]⁺

### Example 310

### Methyl 2-(benzhydryloxy)-5-({[(2-oxoazepan-3-yl)amino]carbonyl}amino)benzoate

23.0 mg, LC-MS: purity 97%, Rt = 3.19 mjn, m/z: 488 [M+H]⁺

### Example 311

### Methyl 2-(benzhydryloxy)-5-({[(2-furylmethyl)amino]carbonyl}amino)benzoate

23.4 mg, LC-MS: purity 97%, Rt = 3.44 mjn, m/z: 457 [M+H]⁺

### Example 312

### Methyl 2-(benzhydryloxy)-5-[({[3-(2-oxypyrrolidin-1-yl)propyl)amino}carbonyl)amino]benzoate

24.1 mg, LC-MS: purity 96%, Rt = 3.08 mjn, m/z: 502 [M+H]⁺

### Example 313

### Methyl 2-(benzhydryloxy)-5-{[(dipropylamino)carbonyl]amino}benzoate

19.8 mg, LC-MS: purity 100%, Rt = 3.85 mjn, m/z: 461 [M+H]⁺

### Example 314

### Methyl 2-(benzhydryloxy)-5-({[methyl(1-naphthylmethyl)amino]carbonyl}amino)benzoate

29.4 mg, LC-MS: purity 92%, Rt = 3.97 mjn, m/z: 531 [M+H]⁺

### Example 315

### Methyl 2-(benzhydryloxy)-5-({[[2-(3,4-dimethoxyphenyl)ethyl](methyl)amino]carbonyl}amino)benzoate

24.4 mg, LC-MS: purity 98%, Rt = 3.58 mjn, m/z: 555 [M+H]⁺

### Example 316

### Methyl 2-(benzhydryloxy)-5-({[bis(2-methoxyethyl)amino]carbonyl}amino)benzoate

22.6 mg, LC-MS: purity 98%, Rt = 3.54 mjn, m/z: 493 [M+H]⁺

### Example 317

### Methyl 2-(benzhydryloxy)-5-[(piperidin-1-ylcarbonyl)amino]benzoate

21.3 mg, LC-MS: purity 99%, Rt = 3.57 mjn, m/z: 445 [M+H]⁺

### Example 318

### Methyl 2-(benzhydryloxy)-5-{[(2,6-dimethylmorpholin-4-yl)carbonyl]amino}benzoate

22.5 mg, LC-MS: purity 99%, Rt = 3.47 mjn, m/z: 475 [M+H]⁺

### Example 319

### Methyl 2-(benzhydryloxy)-5-[(3,4-dihydroisoquinolin-2 (1H)-ylcarbonyl)amino]benzoate

22.5 mg, LC-MS: purity 99%, Rt = 3.76 mjn, m/z: 493 [M+H]⁺

### Example 320

### Methyl 5-({[4-(aminocarbonyl)piperidin-1-yl]carbonyl}amino)-2-(benzhydryloxy)benzoate

21.3 mg, LC-MS: purity 97%, Rt = 2.95 mjn, m/z: 488 [M+H]⁺

### Example 321

### Methyl 2-(benzhydryloxy)-5-({[4-(2-hydroxyethyl)piperidin-1-yl]carbonyl}amino)benzoate

24.6 mg, LC-MS: purity 97%, Rt = 3.19 mjn, m/z: 489 [M+H]⁺

### Example 322

### Methyl 2-(benzhydryloxy)-5-[(thiomorpholin-4-ylcarbonyl)amino]benzoate

24.9 mg, LC-MS: purity 98%, Rt = 3.46 mjn, m/z: 463 [M+H]⁺

### Example 323

### Methyl 2-(benzhydryloxy)-5-{[(4-benzylpiperidin-1-yl)carbonyl]amino}benzoate

26.7 mg, LC-MS: purity 91%, Rt = 4.07 mjn, m/z: 535 [M+H]⁺

### Example 324

### Methyl 5-({[3-(acetylamino)pyrrolidin-1-yl]carbonyl}amino)-2-(benzhydryloxy)benzoate

24.7 mg, LC-MS: purity 98%, Rt = 2.95 mjn, m/z: 488 [M+H]⁺

### Example 325

### Methyl 2-(benzhydryloxy)-5-({[cyclohexyl(methyl)amino]carbonyl}amino)benzoate

21.3 mg, LC-MS: purity 99%, Rt = 3.86 mjn, m/z: 473 [M+H]⁺

### Example 326

### Methyl 2-(benzhydryloxy)-5-({[benzyl(methyl)amino]carbonyl}amino)benzoate

21.1 mg, LC-MS: purity 98%, Rt = 3.72 mjn, m/z: 481 [M+H]⁺

### Example 327

### N-({[4-(benzhydryloxy)-3-(methoxycarbonyl)phenyl]amino}carbonyl)-N-benzyl-beta-alanine ethyl ester

22.9 mg, LC-MS: purity 98%, Rt = 3.84 mjn, m/z: 567 [M+H]⁺

### Example 328

### Methyl 2-(benzhydryloxy)-5-({[ethyl(2-methoxyethyl)amino]carbonyl}amino)benzoate

23.2 mg, LC-MS: purity 96%, Rt = 3.53 mjn, m/z: 463 [M+H]⁺

### Example 329

### Methyl 2-(benzhydryloxy)-5-{[(3,5-dimethylpiperidin-1-yl)carbonyl]amino}benzoate

20.6 mg, LC-MS: purity 99%, Rt = 3.92 mjn, m/z: 473 [M+H]⁺

### Example 330

### Methyl 2-(benzhydryloxy)-5-[(octahydroisoquinolin-2 (1H)-ylcarbonyl)amino]benzoate

26.2 mg, LC-MS: purity 99%, Rt = 4.03 mjn, m/z: 499 [M+H]⁺

### Example 331

### Ethyl 1-({[4-(benzhydryloxy)-3-(methoxycarbonyl)phenyl]amino}carbonyl)piperidin-4carbonate

25.5 mg, LC-MS: purity 99%, Rt = 3.57 mjn, m/z: 517 [M+H]⁺

### Example 332

### Methyl 2-(benzhydryloxy)-5-{[(4-hydroxypiperidin-1-yl)carbonyl]amino}benzoate

23.8 mg, LC-MS: purity 99%, Rt = 3.03 mjn, m/z: 461 [M+H]⁺

### Example 333

### Methyl 2-(benzhydryloxy)-5-{[(3-{[(2,6-dimethylphenyl)amino]methyl}pyrrolidin-1-yl)carbonyl]amino}benzoate

31.4 mg, LC-MS: purity 90%, Rt = 3.06 mjn, m/z: 564 [M+H]⁺

### Example 334

### Methyl 2-(benzhydryloxy)-5-({[4-(4-chlorophenyl)-4-hydroxypiperidin-1-yl]carbonyl}amino)benzoate

30.6 mg, LC-MS: purity 98%, Rt = 3.69 mjn, m/z: 572 [M+H]⁺

### Example 335

### Methyl 5-[({3-[acetyl(ethyl)amino]pyrrolidin-1-yl}carbonyl)amino]-2-(benzhydryloxy)benzoate

29.6 mg, LC-MS: purity 98%, Rt = 3.16 mjn, m/z: 516 [M+H]⁺

### Example 336

### Methyl 2-(benzhydryloxy)-5-{[(1,3-benzothiazol-2-ylamino)carbonyl]amino}benzoate

22.6 mg, LC-MS: purity 92%, Rt = 3.82 mjn, m/z: 510 [M+H]⁺

### Example 337

### Methyl 2-(benzhydryloxy)-5-[({[4-(methoxycarbonyl)phenyl]amino}carbonyl)amino]benzoate

14.9 mg, LC-MS: purity 99%, Rt = 3.61 mjn, m/z: 533 [M+H]⁺

### Example 338

### Methyl 2-(benzhydryloxy)-5-({[(2-chloro-4-cyanophenyl)amino]carbonyl}amino)benzoate

1.8 mg, LC-MS: purity 84%, Rt = 3.86 mjn, m/z: 512 [M+H]⁺

### Example 339

### Methyl 2-(benzhydryloxy)-5-({[(3-chloro-4-cyanophenyl)amino]carbonyl}amino)benzoate

6.5 mg, LC-MS: purity 92%, Rt = 3.85 mjn, m/z: 512 [M+H]⁺

### Example 340

### Methyl 2-(benzhydryloxy)-5-({[(3,5-dimethoxyphenyl)amino]carbonyl}amino)benzoate

18.9 mg, LC-MS: purity 98%, Rt = 3.67 mjn, m/z: 513 [M+H]⁺

### Example 341

### Methyl 2-(benzhydryloxy)-5-[({[2-(1H-pyrrol-1-yl)phenyl]amino}carbonyl)amino]benzoate

18.0 mg, LC-MS: purity 92%, Rt = 3.93 mjn, m/z: 518 [M+H]⁺

### Example 342

### Methyl 2-(benzhydryloxy)-5-[({[3-(trifluoromethyl)phenyl]amino}carbonyl)amino]benzoate

21.3 mg, LC-MS: purity 98%, Rt = 3.97 mjn, m/z: 543 [M+Na]⁺

### Example 343

### Methyl 2-(benzhydryloxy)-5-({[(3,4-dichlorophenyl)amino]carbonyl}amino)benzoate

21.2 mg, LC-MS: purity 95%, Rt = 4.09 mjn, m/z: 543 [M+Na]⁺

### Example 344

### Methyl 2-(benzhydryloxy)-5-({[(4-pentylphenyl)amino]carbonyl}amino)benzoate

27.2 mg, LC-MS: purity 100%, Rt = 4.35 mjn, m/z: 523 [M+H]⁺

### Example 345

### Methyl 2-(benzhydryloxy)-5-({[(4-cyano-1-naphthyl)amino]carbonyl}amino)benzoate

6.0 mg, LC-MS: purity 94%, Rt = 3.88 mjn, m/z: 550 [M+Na]⁺

### Example 346

### Methyl 2-(benzhydryloxy)-5-{[(1,1'-biphenyla-3-ylamino)carbonyl]amino}benzoate

23.4 mg, LC-MS: purity 96%, Rt = 4.06 mjn, m/z: 529 [M+H]⁺

### Example 347

### Methyl 2-(benzhydryloxy)-5-{[(1,1'-biphenyla-2-ylamino)carbonyl]amino}benzoate

28.5 mg, LC-MS: purity 87%, Rt = 4.00 mjn, m/z: 529 [M+H]⁺

### Example 348

### Methyl 2-(benzhydryloxy)-5-({[(3-bromophenyl)amino]carbonyl}amino)benzoate

30.7 mg, LC-MS: purity 87%, Rt = 3.94 mjn, m/z: 553 [M+Na]⁺

### Example 349

### Methyl 2-(benzhydryloxy)-5-({[(2-bromophenyl)amino]carbonyl}amino)benzoate

19.1 mg, LC-MS: purity 82%, Rt = 3.90 mjn, m/z: 553 [M+Na]⁺

### Example 350

### Methyl 2-(benzhydryloxy)-5-({[(4-bromophenyl)amino]carbonyl}amino)benzoate

25.5 mg, LC-MS: purity 98%, Rt = 3.93 mjn, m/z: 553 [M+Na]⁺

### Example 351

### Methyl 5-[({[4-(aminosulfonyl)phenyl]amino}carbonyl)amino]-2-(benzhydryloxy)benzoate

4.3 mg, LC-MS: purity 83%, Rt = 3.26 mjn, m/z: 554 [M+Na]⁺

### Example 352

### Methyl 2-(benzhydryloxy)-5-[({[4-(trifluoromethoxy)phenyl]amino]carbonyl)amino]benzoate

24.7 mg, LC-MS: purity 99%, Rt = 4.00 mjn, m/z: 559 [M+Na]⁺

### Example 353

### Methyl 2-(benzhydryloxy)-5-[({[4-fluoro-3-(trifluoromethyl)phenyl]amino}carbonyl)amino]benzoate

20.6 mg, LC-MS: purity 99%, Rt = 4.00 mjn, m/z: 561 [M+Na]⁺

### Example 354

### Methyl 2-(benzhydryloxy)-5-[({[4-(2-ethoxy-2-oxoethyl)phenyl]amino}carbonyl)amino]benzoate

27.0 mg, LC-MS: purity 99%, Rt = 3.74 mjn, m/z: 539 [M+H]⁺

### Example 355

### Methyl 2-(benzhydryloxy)-5-[({[4-(pentyloxy)phenyl]amino}carbonyl)amino]benzoate

30.5 mg, LC-MS: purity 99%, Rt = 4.21 mjn, m/z: 539 [M+H]⁺

### Example 356

### Methyl 2-(benzhydryloxy)-5-({[(6-methoxy-1,3-benzothiazol-2-yl)amino]carbonyl}amino)benzoate

23.8 mg, LC-MS: purity 91%, Rt = 3.78 mjn, m/z: 540 [M+H]⁺

### Example 357

### Methyl 2-(benzhydryloxy)-5-[({[2-methoxy-4-(methoxycarbonyl)phenyl]amino}carbonyl)amino]benzoate

23.6 mg, LC-MS: purity 99%, Rt = 3.74 mjn, m/z: 541 [M+H]⁺

### Example 358

### Methyl 2-(benzhydryloxy)-5-({[(4-benzylphenyl)amino]carbonyl}amino)benzoate

34.6 mg, LC-MS: purity 90%, Rt = 4.10 mjn, m/z: 543 [M+H]⁺

### Example 359

### Methyl 5-({[(2-anilin ophenyl)amino]carbonyl}amino)-2-(benzhydryloxy)benzoate

28.8 mg, LC-MS: purity 99%, Rt = 4.00 mjn, m/z: 544 [M+H]⁺

### Example 360

### Methyl 2-(benzhydryloxy)-5-({[(3-phenoxyphenyl)amino]carbonyl}amino)benzoate

33.1 mg, LC-MS: purity 83%, Rt = 4.07 mjn, m/z: 545 [M+H]⁺

### Example 361

### Methyl 2-(benzhydryloxy)-5-({[(4-phenoxyphenyl)amino]carbonyl}amino)benzoate

34.0 mg, LC-MS: purity 88%, Rt = 4.04 mjn, m/z: 545 [M+H]⁺

### Example 362

### Methyl 2-(benzhydryloxy)-5-({[(2-phenoxyphenyl)amino]carbonyl}amino)benzoate

25.3 mg, LC-MS: purity 99%, Rt = 4.14 mjn, m/z: 545 [M+H]⁺

### Example 363

### Methyl 2-(benzhydryloxy)-5-({[(3,4,5-trimethoxyphenyl)amino]carbonyl}amino)benzoate

31.0 mg, LC-MS: purity 98%, Rt = 3.53 mjn, m/z: 543 [M+H]⁺

### Example 364

### Methyl 2-(benzhydryloxy)-5-[({[4-(4-methylpiperazin-1-yl)phenyl]amino}carbonyl)amino]benzoate

32.9 mg, LC-MS: purity 98%, Rt = 2.77 mjn, m/z: 551 [M+H]⁺

### Example 365

### Methyl 2-(benzhydryloxy)-5-({[(9-oxo-9H-fluoren-2-yl)amino]carbonyl)amino)benzoate

26.8 mg, LC-MS: purity 89%, Rt = 3.95 mjn, m/z: 555 [M+H]⁺

### Example 366

### Methyl 2-(benzhydryloxy)-5-{[({4-[(E)-2-phenylethenyl]phenyl}amino)carbonyl]amino}benzoate

22.5 mg, LC-MS: purity 99%, Rt = 4.20 mjn, m/z: 555 [M+H]⁺

### Example 367

### Methyl 2-(benzhydryloxy)-5-({[(4-benzoylphenyl)amino]carbonyl}amino)benzoate

14.3 mg, LC-MS: purity 98%, Rt = 3.91 mjn, m/z: 579 [M+Na]⁺

### Example 368

### Methyl 2-(benzhydryloxy)-5-({[(4-methoxy-1,1'-biphenyla-3-yl)amino]carbonyl}amino)benzoate

24.3 mg, LC-MS: purity 100%, Rt = 4.18 mjn, m/z: 559 [M+H]⁺

### Example 369

### Methyl 2-(benzhydryloxy)-5-[({[3-(benzyloxy)phenyl]amino}carbonyl)amino]benzoate

29.2 mg, LC-MS: purity 81%, Rt = 4.05 mjn, m/z: 559 [M+H]⁺

### Example 370

### Methyl 2-(benzhydryloxy)-5-[({[4-(heptyloxy)phenyl]amino}carbonyl)amino]benzoate

22.8 mg, LC-MS: purity 97%, Rt = 4.49 mjn, m/z: 567 [M+H]⁺

### Example 371

### Dimethyl 5-[({[4-(benzhydryloxy)-3-(methoxycarbonyl)phenyl]amino}carbonyl)amino]isophthalate

26.1 mg, LC-MS: purity 98%, Rt = 3.76 mjn, m/z: 569 [M+H]⁺

### Example 372

### Methyl 2-(benzhydryloxy)-5-({[(4'-nitro-1,1'-biphenyla-4-yl)amino]carbonyl}amino)benzoate

24.2 mg, LC-MS: purity 87%, Rt = 4.04 mjn, m/z: 596 [M+Na]⁺

### Example 373

### Methyl 2-(benzhydryloxy)-5-({[(1-benzyl-1H-benzoimidazol-2-yl)amino]carbonyl}amino)benzoate

25.4 mg, LC-MS: purity 96%, Rt = 3.57 mjn, m/z: 583 [M+H]⁺

### Example 374

### Methyl 2-(benzhydryloxy)-5-{[({4-[(E)-2-(4-methoxyphenyl)ethenyl]phenyl}amino)carbonyl]amino}benzoate

23.0 mg, LC-MS: purity 99%, Rt = 4.14 mjn, m/z: 585 [M+H]⁺

### Example 375

### Methyl 2-(benzhydryloxy)-5-[({[3,5-bis(trifluoromethyl)phenyl]amino}carbonyl)amino]benzoate

17.3 mg, LC-MS: purity 93%, Rt = 4.27 mjn, m/z: 611 [M+Na]⁺

### Example 376

### Methyl 2-(benzhydryloxy)-5-[({[4-(benzyloxy)phenyl]amino}carbonyl)amino]benzoate

29.9 mg, LC-MS: purity 97%, Rt = 3.99 mjn, m/z: 559 [M+H]⁺

### Example 377

### Methyl 2-(benzhydryloxy)-5-{[({4-[(phenylsulfonyl)amino]phenyl}amino)carbonyl]amino}benzoate 34.5 mg, LC-MS: purity 99%, Rt = 3.71 mjn, m/z: 622 [M+H]⁺

### Synthetic method of amide derivatives by combinatorial synthesis

The compounds of Examples 378 to 425 were synthesized as described below.

To a mixed solution of 5-(anilinocarbonylamino)-2-benzhydryloxy benzoate (0.0684 mmol), 1-hydroxy-7-azabenzotriazole (0.0821 mmol) and 1-ethyl-3-(3-dimethylaminopropyl)carbodiimide (0.1026 mmol) in DMF (0.3 mL) and dichloromethane (0.7 mL) was added amine (0.0821 mmol), the mixture was stirred at room temperature for 2 hours, and water and dichloromethane were added to the mixture. The layer of dichloromethane was separated with PTFE filter (1µm, Whatman Inc.), and concentrated with Dry Thermo-unit PTU-1C. The residue was purified with preparative HPLC made by Gilson Inc. (PLRP-S column 5 µm 100 A, 50 x 25 mm, 40% to 100% aqueous solution of acetonitrile). The resulting compound was analyzed by LC MASS made by Gilson Inc. (Shiseido capsule pack C18 2 x 5 cm, λ = 220 nm, temperature 40°C, A liquid 0.05% trifluoroacetic acid solution: B liquid acetonitrile: 10 to 95% B liquid (for 4 minutes) 95% B liquid (for 1.5 minutes), electrospray ionization mass spectrum).

### Example 378

### 5-[(Anilinocarbonyl)amino]-2-(benzhydryloxy)-N,N-dipropyl benzamide

23.5 mg, LC-MS: purity 97%, Rt = 3.88 mjn, m/z: 522 [M+H]⁺

### Example 379

### 5-[(Anilinocarbonyl)amino]-2-(benzhydryloxy)-N-methyl-N-(1-naphthylmethyl)benzamide

30.4 mg, LC-MS: purity 97%, Rt = 4.01 mjn, m/z: 592 [M+H]⁺

### Example 380

### 5-[(Anilinocarbonyl)amino]-2-(benzhydryloxy)-N-[2-(3,4-dimethoxyphenyl)ethyl]-N-methyl benzamide

31.0 mg, LC-MS: purity 97%, Rt = 3.61 mjn, m/z: 616 [M+H]⁺

### Example 381

### 5-[(Anilinocarbonyl)amino]-2-(benzhydryloxy)-N,N-bis(2-methoxyethyl)benzamide

27.5 mg, LC-MS: purity 96%, Rt = 3.46 mjn, m/z: 554 [M+H]⁺

### Example 382

### N-[4-(Benzhydryloxy)-3-(piperidin-1-ylcarbonyl)phenyl]-N'-phenylurea

26.5 mg, LC-MS: purity 97%, Rt = 3.65 mjn, m/z: 506 [M+H]⁺

### Example 383

### N-{4-(Benzhydryloxy)-3-[(2,6-dimethylmorpholin-4-yl)carbonyl]phenyl}-N'-phenylurea

27.4 mg, LC-MS: purity 98%, Rt = 3.54 mjn, m/z: 536 [M+H]⁺

### Example 384

### N-[4-(Benzhydryloxy)-3-(3,4-dihydroisoquinolin-2 (1H)-ylcarbonyl)phenyl]-N'-phenylurea

25.0 mg, LC-MS: purity 99%, Rt = 3.79 mjn, m/z: 554 [M+H]⁺

### Example 385

### 1-[5-[(Anilinocarbonyl)amino]-2-(benzhydryloxy)benzoyl]piperidin-4-carboxamide

26.7 mg, LC-MS: purity 98%, Rt = 2.94 mjn, m/z: 549 [M+H]⁺

### Example 386

### N-(4-(Benzhydryloxy)-3-{[4-(2-hydroxyethyl)piperidin-1-yl]carbonyl}phenyl)-N'-phenylurea

24.4 mg, LC-MS: purity 99%, Rt = 3.15 mjn, m/z: 550 [M+H]⁺

### Example 387

### N-[4-(Benzhydryloxy)-3-(thiomorpholin-4-ylcarbonyl)phenyl]-N'-phenylurea

26.8 mg, LC-MS: purity 99%, Rt = 3.57 mjn, m/z: 524 [M+H]⁺

### Example 388

### N-{4-(Benzhydryloxy)-3-[(4-benzylpiperidin-1-yl)carbonyl]phenyl}-N'-phenylurea

27.1 mg, LC-MS: purity 99%, Rt = 4.09 mjn, m/z: 596 [M+H]⁺

### Example 389

### N-{1-[5-[(Anilinocarbonyl)amino]-2-(benzhydryloxy)benzoyl]pyrrolidin-3-yl}acetamide

23.0 mg, LC-MS: purity 99%, Rt = 2.97 mjn, m/z: 549 [M+H]⁺

### Example 390

### 5-[(Anilinocarbonyl)amino]-2-(benzhydryloxy)-N-cyclohexyl-N-methyl benzamide

27.1 mg, LC-MS: purity 98%, Rt = 3.92 mjn, m/z: 534 [M+H]⁺

### Example 391

### 5-[(Anilinocarbonyl)amino]-2-(benzhydryloxy)-N-benzyl-N-methyl benzamide

28.4 mg, LC-MS: purity 100%, Rt = 3.79 mjn, m/z: 542 [M+H]⁺

### Example 392

### Ethyl N-[5-[(Anilinocarbonyl)amino]-2-(benzhydryloxy)benzoyl]-N-benzyl-beta-alanate

25.7 mg, LC-MS: purity 97%, Rt = 3.89 mjn, m/z: 628 [M+H]⁺

### Example 393

### 5-[(Anilinocarbonyl)amino]-2-(benzhydryloxy)-N-ethyl-N-(2-methoxyethyl)benzamide

28.1 mg, LC-MS: purity 98%, Rt = 3.51 mjn, m/z: 524 [M+H]⁺

### Example 394

### N-[3-(Azepan-1-ylcarbonyl)-4-(benzhydryloxy)phenyl]-N'-phenylurea

27.8 mg, LC-MS: purity 98%, Rt = 3.75 mjn, m/z: 520 [M+H]⁺

### Example 395

### N-{4-(Benzhydryloxy)-3-[(3,5-dimethylpiperidin-1-yl)carbonyl]phenyl}-N'-phenylurea

26.8 mg, LC-MS: purity 99%, Rt = 3.95 mjn, m/z: 534 [M+H]⁺

### Example 396

### N-[4-(Benzhydryloxy)-3-(octahydroisoquinolin-2 (1H)-ylcarbonyl)phenyl]-N'-phenylurea

27.5 mg, LC-MS: purity 99%, Rt = 4.11 mjn, m/z: 560 [M+H]⁺

### Example 397

### Ethyl 1-[5-[(anilinocarbonyl)amino]-2-(benzhydryloxy)benzoyl]piperidin-4 carbonate

22.5 mg, LC-MS: purity 98%, Rt = 3.60 mjn, m/z: 578 [M+H]⁺

### Example 398

### N-{4-(Benzhydryloxy)-3-[(4-hydroxypiperidin-1-yl)carbonyl]phenyl}-N'-phenylurea

25.1 mg, LC-MS: purity 99%, Rt = 3.05 mjn, m/z: 522 [M+H]⁺

### Example 399

### N-{4-(Benzhydryloxy)-3-[(2-{[(2,6-dimethylphenyl)amino]methyl}pyrrolidin-1-yl)carbonyl]phenyl}-N'-phenylurea

29.7 mg, LC-MS: purity 100%, Rt = 3.13 mjn, m/z: 625 [M+H]⁺

### Example 400

### N-(4-(Benzhydryloxy)-3-{[4-(4-chlorophenyl)-4-hydroxypiperidin-1-yl]carbonyl}phenyl)-N'-phenylurea

24.4 mg, LC-MS: purity 99%, Rt = 3.71 mjn, m/z: 632 [M+H]⁺

### Example 401

### N-{1-[5-[(Anilinocarbonyl)amino]-2-(benzhydryloxy)benzoyl]pyrrolidin-3-yl}-N-ethyl acetamide

26.7 mg, LC-MS: purity 99%, Rt = 3.18 mjn, m/z: 577 [M+H]⁺

### Example 402

### 5-[(Anilinocarbonyl)amino]-2-(benzhydryloxy)-N-[2-(dimethylamino)ethyl]-N-methyl benzamide

28.6 mg, LC-MS: purity 99%, Rt = 2.57 mjn, m/z: 523 [M+H]⁺

### Example 403

### 5-[(Aanilinocarbonyl)amino]-2-(benzhydryloxy)-N-(1-benzylpyrrolidin-3-yl)-N-methyl benzamide

31.4 mg, LC-MS: purity 99%, Rt = 2.86 mjn, m/z: 611 [M+H]⁺

### Example 404

### 5-[(Anilinocarbonyl)amino]-2-(benzhydryloxy)-N-ethyl-N-(pyridin-4-ylmethyl)benzamide

29.6 mg, LC-MS: purity 99%, Rt = 2.66 mjn, m/z: 557 [M+H]⁺

### Example 405

### 5-[(Anilinocarbonyl)amino]-2-(benzhydryloxy)-N,N-bis(pyridin-3-ylmethyl)benzamide

29.3 mg, LC-MS: purity 98%, Rt = 2.33 mjn, m/z: 620 [M+H]⁺

### Example 406

### N-{4-(Benzhydryloxy)-3-[(4-ethylpiperazin-1-yl)carbonyl]phenyl}-N'-phenylurea

24.4 mg, LC-MS: purity 98%, Rt = 2.55 mjn, m/z: 535 [M+H]⁺

### Example 407

### {4-[5-[(Anilinocarbonyl)amino]-2-(benzhydryloxy)benzoyl]piperazin-1-yl}ethyl acetate

22.9 mg, LC-MS: purity 99%, Rt = 2.71 mjn, m/z: 593 [M+H]⁺

### Example 408

### N-{4-(Benzhydryloxy)-3-[(4-benzylpiperazin-1-yl)carbonyl]phenyl}-N'-phenylurea

29.0 mg, LC-MS: purity 99%, Rt = 2.78 mjn, m/z: 597 [M+H]⁺

### Example 409

### N-{4-(Benzhydryloxy)-3-[(4-pyridin-2-ylpiperazin-1-yl)carbonyl]phenyl}-N'-phenylurea

26.0 mg, LC-MS: purity 98%, Rt = 2.61 mjn, m/z: 584 [M+H]⁺

### Example 410

### N-{4-(Benzhydryloxy)-3-[(4-benzhydrylpiperazin-1-yl)carbonyl]phenyl}-N'-phenylurea

37.4 mg, LC-MS: purity 98%, Rt = 3.15 mjn, m/z: 673 [M+H]⁺

### Example 411

### N-{4-(Benzhydryloxy)-3-[(4-phenylpiperazin-1-yl)carbonyl]phenyl}-N'-phenylurea

23.5 mg, LC-MS: purity 98%, Rt = 3.66 mjn, m/z: 583 [M+H]⁺

### Example 412

### N-(4-(Benzhydryloxy)-3-{[4-(2-methoxyphenyl)piperazin-1-yl]carbonyl}phenyl)-N'-phenylurea

30.4 mg, LC-MS: purity 96%, Rt = 3.37 mjn, m/z: 613 [M+H]⁺

### Example 413

### N-[4-(Benzhydryloxy)-3-(1,4'-bipiperidin-1'-ylcarbonyl)phenyl]-N'-phenylurea

28.1 mg, LC-MS: purity 89%, Rt = 2.62 mjn, m/z: 589 [M+H]⁺

### Example 414

### 5-[(Anilinocarbonyl)amino]-2-(benzhydryloxy)-N-methyl-N-(1-methylpyrrolidin-3-yl)benzamide

28.3 mg, LC-MS: purity 95%, Rt = 2.61 mjn, m/z: 535 [M+H]⁺

### Example 415

### 5-[(Anilinocarbonyl)amino]-2-(benzhydryloxy)-N-benzyl-N-(1-benzylpyrrolidin-3-yl)benzamide

35.0 mg, LC-MS: purity 98%, Rt = 3.14 mjn, m/z: 687 [M+H]⁺

### Example 416

### 5-[(Anilinocarbonyl)amino]-2-(benzhydryloxy)-N,N-bis(pyridin-2-ylmethyl)benzamide

33.6 mg, LC-MS: purity 96%, Rt = 2.73 mjn, m/z: 620 [M+H]⁺

### Example 417

### N-{4-(Benzhydryloxy)-3-[(4-methyl-1,4-diazepan-1-yl)carbonyl]phenyl}-N'-phenylurea

27.2 mg, LC-MS: purity 97%, Rt = 2.53 mjn, m/z: 535 [M+H]⁺

### Example 418

### N-(4-(Benzhydryloxy)-3-{[4-(2-hydroxyethyl)piperazin-1-yl]carbonyl}phenyl)-N'-phenylurea

28.0 mg, LC-MS: purity 99%, Rt = 2.47 mjn, m/z: 551 [M+H]⁺

### Example 419

### N-(4-(Benzhydryloxy)-3-{[4-(1,3-benzodioxol-5-ylmethyl)piperazin-1-yl]carbonyl}phenyl)-N'-phenylurea

30.6 mg, LC-MS: purity 100%, Rt = 2.78 mjn, m/z: 641 [M+H]⁺

### Example 420

### N-{4-(Benzhydryloxy)-3-[(4-pyrimidin-2-ylpiperazin-1-yl)carbonyl]phenyl}-N'-phenylurea

30.9 mg, LC-MS: purity 97%, Rt = 3.34 mjn, m/z: 585 [M+H]⁺

### Example 421

### N-[4-(Benzhydryloxy)-3-({4-[(2E)-3-phenylpropa-2-enyl]piperazin-1-yl}carbonyl)phenyl]-N'-phenylurea

32.9 mg, LC-MS: purity 97%, Rt = 2.93 mjn, m/z: 623 [M+H]⁺

### Example 422

### 5-[(Anilinocarbonyl)amino]-2-(benzhydryloxy)-N-benzyl-N-[2-(dimethylamino)ethyl]benzamide

25.7 mg, LC-MS: purity 97%, Rt = 2.89 mjn, m/z: 599 [M+H]⁺

### Example 423

### 5-[(Anilinocarbonyl)amino]-2-(benzhydryloxy)-N-methyl-N-(1-methylpiperidin-4-yl)benzamide

28.0 mg, LC-MS: purity 98%, Rt = 2.55 mjn, m/z: 549 [M+H]⁺

### Example 424

### N-(4-(Benzhydryloxy)-3-{[2-(pyrrolidin-1-ylmethyl)pyrrolidin-1-yl]carbonyl}phenyl)-N'-phenylurea

26.2 mg, LC-MS: purity 95%, Rt = 2.79 mjn, m/z: 575 [M+H]⁺

### Example 425

### N-{4-(Benzhydryloxy)-3-[(4-pyrrolidin-1-ylpiperidin-1-yl)carbonyl]phenyl}-N'-phenylurea

7.0 mg, LC-MS: purity 89%, Rt = 2.62 mjn, m/z: 575 [M+H]⁺

According to the same method as Example 236, the following compounds were synthesized.

### Example 426

### Methyl 5-({[(3,4-dimethoxyphenyl)amino]carbonyl}amino)-2-(dipyridin-2-ylmethoxy)benzoate

### (1) Methyl 2-(dipyridin-2-ylmethoxy)-5-nitrobenzoate

¹H-NMR (CDCl₃) δ; 4.02 (3H, s), 6.68 (1H, s), 6.98 to 7.81 (9H, m), 8.22 (1H, d, J = 2.8, 9.0 Hz), 8.77 (2H, d, J = 2.8 Hz)

### (2) Methyl 5-({[(3,4-dimethoxyphenyl)amino]carbonyl}amino)-2-(dipyridin-2-ylmethoxy)benzoate

¹H-NMR (CDCl₃) δ; 3.79 (3H, s), 3.81 (3H, s), 3.82 (3H, s), 6.50 (1H, s), 6.68 to 7.85 (13H, m), 7.97 (1H, s), 8.51 (2H, d, J = 4.8 Hz)

### Example 427

### Methyl 2-[bis(4-methylphenyl)methoxy]-5-({[(3,4-dimethoxyphenyl)amino]carbonyl}amino)benzoate

### (1) Methyl 2-[bis(4-methylphenyl)methoxy]-5-nitrobenzoate

¹H-NMR (CDCl₃) δ; 2.32 (6H, s), 3.97 (3H, s), 6.35 (1H, s), 7. 02 (1H, d = 9. 0 Hz), 7.15 (4H, d, J = 8. 0 Hz), 7.35 (4H, d, J = 8.0 Hz), 8.16 (1H, dd, J = 3.0, 9.0 Hz), 8.70 (1H, d, J = 3.0 Hz)

### (2) Methyl 2-[bis(4-methylphenyl)methoxy]-5-({[(3,4-dimethoxyphenyl)amino]carbonyl}amino)benzoate

¹H-NMR (CDCl₃) δ; 2.28 (6H, s), 3.81 (3H, s), 3.82 (3H, s), 3.86 (3H, s), 6.15 (1H, s), 6.68 to 7.42 (15H, m), 7.58 (1H, d, J = 2.8 Hz)

### Example 428

### Methyl 2-[bis(4-chlorophenyl)methoxy]-5-({[(3,4-dimethoxyphenyl)amino]carbonyl}amino)benzoate

### (1) Methyl 2-[bis(4-chlorophenyl)methoxy]-5-nitrobenzoate

¹H-NMR (CDCl₃) δ; 4.04 (3H, s), 6.36 (1H, s), 6.97 (1H, d, J = 7.6 Hz), 7.34 (4H, d, J = 10.8 Hz), 7.43 (4H, d, J = 10.8 Hz), 8.20 (1H, dd, J = 2.4, 7.6 Hz), 8.73 (1H, d, J = 2.4 Hz)

### (2) Methyl 2-[bis(4-chlorophenyl)methoxy]-5-({[(3,4-dimethoxyphenyl)amino]carbonyl}amino)benzoate

¹H-NMR (DMSO-d6) δ; 3.70 (3H, s), 3.72 (3H, s), 3.89 (3H, s), 6.69 (1H, s), 6.85 to 7.59 (13H, m), 7.89 (1H, d, J = 2.4 Hz), 8.45 (1H, s), 8.53 (1H, s)

### Example 429

### Methyl 2-[bis(4-chlorophenyl)methoxy]-5-({[(3-methoxyphenyl)amino]carbonyl}amino)benzoate

¹H-NMR (DMSO-d6) δ; 3.72 (3H, s), 3.89 (3H, s), 6.53 to 7.59 (15H, m), 7.89 (1H, d, J = 2.8 Hz), 8.60 (1H, s), 8.62 (1H, s)

### Example 430

### Methyl 2-[bis(4-chlorophenyl)methoxy]-5-({[(4-methoxyphenyl)amino]carbonyl}amino)benzoate

¹H-NMR (DMSO-d6) δ; 3.71 (3H, s), 3.89 (3H, s), 6.68 (1H, s), 6.84 to 7.59 (14H, m), 7.89 (1H, d, J = 2.4 Hz), 8.41 (1H, s), 8.54 (1H, s)

### Example 431

### Methyl 2-[bis(4-methylphenyl)methoxy]-5-({[(3-methoxyphenyl)amino]carbonyl}amino)benzoate

¹H-NMR (CDCl₃) δ; 2.26 (6H, s), 3.62 (3H, s), 3.81 (3H, s), 6.07 (1H, s), 6.69 to 7.57 (17H, m)

### Example 432

### Methyl 2-[bis(4-methylphenyl)methoxy]-5-({[(4-methoxyphenyl)amino]carbonyl}amino)benzoate

¹H-NMR (CDCl₃) δ; 2.28 (6H, s), 3.76 (3H, s), 3.86 (3H, s), 6.15 (1H, s), 6.56 (1H, s), 6.63 (1H, s), 6.81 to 7.41 (14H, m), 7.58 (1H, d, J = 2.8 Hz)

### Example 433

### Methyl 5-[(anilinocarbonyl)amino]-2-[bis(4-methylphenyl)methoxy]benzoate

¹H-NMR (CDCl₃) δ; 2.28 (6H, s), 3.85 (3H, s), 6.13 (1H, s), 6.80 to 7.36 (17H, m), 7.59 (1H, d, J = 2.4 Hz)

### Example 434

### Methyl 2-[bis(4-fluorophenyl)methoxy]-5-({[(3-methoxyphenyl)amino]carbonyl}amino)benzoate

### (1) Methyl 2-[bis(4-fluorophenyl)methoxy]-5-nitrobenzoate

¹H-NMR (CDCl₃) δ; 3.98 (3H, s), 6.39 (1H, s), 6.99 (1H, d, J = 9.2 Hz), 7.04 to 7.49 (8H, m), 8.20 (1H, dd, J = 2.8, 9.2 Hz), 8.73 (1H, d, J = 2.8 Hz)

### (2) Methyl 2-[bis(4-fluorophenyl)methoxy]-5-({[(3-methoxyphenyl)amino]carbonyl}amino)benzoate

¹H-NMR (CDCl₃) δ; 3.71 (3H, s), 3.82 (3H, s), 6.13 (1H, s), 6.58 to 7.42 (16H, m), 7.60 (1H, d, J = 2.8 Hz)

### Example 435

### Methyl 2-[bis(4-fluorophenyl)methoxy]-5-({[(4-methoxyphenyl)amino]carbonyl}amino)benzoate

¹H-NMR (CDCl₃) δ; 3.76 (3H, s), 3.84 (3H, s), 6.16 (1H, s), 6.57 to 7.43 (16H, m), 7.60 (1H, d, J = 2.8 Hz)

### Example 436

### Methyl 5-[(anilinocarbonyl)amino]-2-[bis(4-fluorophenyl)methoxy]benzoate

¹H-NMR (CDCl₃) δ; 3.83 (3H, s), 6.14 (1H, s), 6.74 (1H, d, J = 9.2 Hz), 6.90 to 7.42 (16H, m), 7.62 (1H, d, J = 2.8 Hz)

### Example 437

### Methyl 2-[bis(4-fluorophenyl)methoxy]-5-({[(3,4-dimethoxyphenyl)amino]carbonyl}amino)benzoate

¹H-NMR (CDCl₃) δ; 3.70 (3H, s), 3.72 (3H, s), 3.88 (3H, s), 6.67 (1H, s), 6.84 (2H, s), 7.06 to 7.61 (11H, m), 7.87 (1H, d, J = 2.8 Hz), 8.44 (1H, s), 8.52 (1H, s).

### Example 438

### Methyl 2-[bis(4-fluorophenyl)methoxy]-5-{[({4-[(2,2-dimethylpropanoyl)oxy]-3-methoxyphenyl}amino)carbonyl]amino}benzoate

A solution (10 mL) of 4-[(2,2-dimethylpropanoyl)oxy]-3-methoxy benzoate (253 mg, 1.00 mmol) and triethyl amine (0.294 mL, 2.10 mmol), diphenylphosphoryl azide (0.230 mL, 1.05 mmol) in toluene was stirred at room temperature for 30 minutes at 93°C for 1.5 hours, and the reaction solution was cooled to room temperature. Methyl 5-amino-2-[bis(4-fluorophenyl)methoxy]benzoate (259 mg, 0.700 mmol) was added thereto and the mixture was stirred at room temperature for 15 hours. The reaction solution was poured into water, extracted with ethyl acetate, and the extracted solution was washed with water. The extracted solution was dried with anhydrous magnesium sulfate, and the solvent was distilled off under reduced pressure. The residue was purified by silicagel column chromatography (hexane:ethyl acetate = 3:2), to obtain the titled compound as powder. 433 mg (100%)
¹H-NMR (DMSO-d6) δ; 1.29 (9H, s), 3.72 (3H, s), 3.89 (3H, s), 6.68 (1H, s), 6.91 to 7.61 (13H, m), 7.89 (1H, d, J = 3.2 Hz), 8.61 (1H, s), 8.68 (1H, s)

### Example 439

### Methyl 2-[bis(4-fluorophenyl)methoxy]-5-[({[4-(methylthio)phenyl]amino}carbonyl)amino]benzoate

To a solution (15 mL) of methyl 5-amino-2-[bis(4-fluorophenyl)methoxy]benzoate (999 mg, 2.50 mmol) in THF was added 4-methylthiophenyl isocyanate (0.342 mL, 2.50 mmol), and the mixture was stirred at room temperature for 15 hours. The reaction solution was poured into water, extracted with ethyl acetate, and the extracted solution was washed with water. The extracted solution was dried with anhydrous magnesium sulfate, and the solvent was distilled off under reduced pressure. The residue was purified by silicagel column chromatography (hexane:ethyl acetate = 3:2), to obtain the titled compound as a solid. 1.35 g (98%)
¹H-NMR (DMSO-d6) δ; 2.43 (3H, s), 3.89 (3H, s), 6.68 (1H, s), 7.07 to 7.61 (14H, m), 7.87 (1H, d, J = 2.4 Hz), 8.60 (1H, s), 8.63 (1H, s)

### Example 440

### Methyl 2-[bis(4-fluorophenyl)methoxy]-5-[({(4-(methylsulfinyl)phenyl]amino}carbonyl)amino]benzoate

To a solution (10 mL) of methyl 2-[bis(4-fluorophenyl)methoxy]-5-[({[4-(methylthio)phenyl]amino}carbonyl)amino]benzoate (268 mg, 0.500 mmol) in methylene chloride was added m-chloroperbenzoate (70%, 123 mg, 0.500 mmol) under ice-cooling, and the mixture was stirred at room temperature for 15 hours. The solvent was distilled off under reduced pressure, and the residue was added to an aqueous solution of sodium bicarbonate, and was extracted with ethyl acetate-THF (2:1). The extracted solution was dried with anhydrous magnesium sulfate, and the solvent was distilled off under reduced pressure. The residue was purified by silicagel column chromatography (methanol:ethyl acetate = 1:20), to obtain the titled compound as a solid. 159 mg (57%)
¹H-NMR (DMSO-d6) δ; 2.70 (3H, s), 3.89 (3H, s), 6.70 (1H, s), 7.09 to 7.64 (14H, m), 7.90 (1H, d, J = 2.0 Hz), 8.73 (1H, s), 8.96 (1H, s)

### Example 441

### Methyl 2-[bis(4-fluorophenyl)methoxy]-5-[({[4-(methylsulfonyl)phenyl]amino}carbonyl)amino]benzoate

To a solution (10 mL) of methyl 2-[bis(4-fluorophenyl)methoxy]-5-[({[4-(methylthio)phenyl]amino}carbonyl)amino]benzoate (268 mg, 0.500 mmol) in methylene chloride was added m-chloroperbenzoate (70%, 247 mg, 1.00 mmol) under ice-cooling, and the mixture was stirred at room temperature for 1 hour. The solvent was distilled off under reduced pressure, and the residue was added to an aqueous solution of sodium bicarbonate, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 3:1), to obtain the titled compound as a solid. 261 mg (84%)
¹H-NMR (DMSO-d6) δ; 3.15 (3H, s), 3.89 (3H, s), 6.70 (1H, s), 7.10 to 7.81 (14H, m), 7.91 (1H, d, J = 2.8 Hz), 8.80 (1H, s), 9.18 (1H, s)

### Example 442

### Methyl 2-[bis(4-fluorophenyl)methoxy]-5-({[(4-ethoxy-3-methoxyphenyl)amino]carbonyl}amino)benzoate

From 4-ethoxy-3-methoxy benzoate and methyl 5-amino-2-[bis(4-fluorophenyl)methoxy]benzoate was synthesized the titled compound in the same manner as Example 438.
¹H-NMR (DMSO-d6) δ; 1.29 (3H, t, J = 7.2 Hz), 3.73 (3H, s), 3.89 (3H, s), 3.94 (2H, q, J = 7.2 Hz), 6.68 (1H, s), 6.82 to 7.61 (13H, m), 7.87 (1H, d, J = 2.8 Hz), 8.44 (1H, s), 8.52 (1H, s)

### Example 443

### Methyl 2-[bis(4-fluorophenyl)methoxy]-5-[({[3-methoxy-4-(methoxymethoxy)phenyl]amino}carbonyl)amino]benzoate

From 3-methoxy-4-(methoxymethoxy)benzoate and methyl 5-amino-2-[bis(4-fluorophenyl)methoxy]benzoate was synthesized the titled compound in the same manner as Example 438.
¹H-NMR (DMSO-d6) δ; 3.39 (3H, s), 3.75 (3H, s), 3.89 (3H, s), 5.04 (2H, s), 6.68 (1H, s), 6.80 to 7.61 (13H, m), 7.87 (1H, d, J = 3.2 Hz), 8.52 (1H, s), 8.54 (1H, s)

### Example 444

### Methyl 2-[bis(4-fluorophenyl)methoxy]-5-{[({3-methoxy-4-[(methylsulfonyl)oxy]phenyl}amino)carbonyl]amino}benzoate

From 3-methoxy-4-[(methylsulfonyl)oxy]benzoate and methyl 5-amino-2-[bis(4-fluorophenyl)methoxy]benzoate was synthesized the titled compound in the same manner as Example 438.
¹H-NMR (DMSO-d6) δ; 3.29 (3H, s), 3.81 (3H, s), 3.89 (3H, s), 6.69 (1H, s), 6.90 to 7.61 (13H, m), 7.88 (1H, d, J = 2.4 Hz), 8.66 (1H, s), 8.82 (1H, s)

### Example 445

### Methyl 2-[bis(4-fluorophenyl)methoxy]-5-[({[4-(2-ethoxy-2-oxoethoxy)-3-methoxyphenyl]amino}carbonyl)amino]benzoate

A solution (30 mL) of 4-hydroxy-3-methoxybenzaldehyde (4.57 g, 30.0 mmol), potassium carbonate (4.98 g, 36.0 mmol) and bromoethyl acetate (4.00 mL, 36.0 mmol) in DMF was stirred at room temperature for 3 hours, and the reaction solution was poured into water and was extracted with ethyl acetate. The extracted solution was washed with water, was dried with anhydrous magnesium sulfate, and the solvent was distilled off under reduced pressure. To the residue (2.39 g) were added acetic acid (12 mL) and sulfamic acid (1.31 g, 13.5 mmol) and to this solution was added a solution of sodium chlorite (1.47 g, 13.0 mmol) in water (3 mL). The mixture was stirred at room temperature for 30 minutes, water (50 mL) was added thereto, and the solid were collected. A solution of this solid (255 mg), triethyl amine (0.150 mL, 1.05 mmol) and diphenylphosphoryl azide (0.230 mL, 1.05 mmol) in toluene (10 mL) was stirred at room temperature for 15 minutes and at 93°C for 1.5 hours, and the reaction solution was cooled to room temperature. Methyl 5-amino-2-[bis(4-fluorophenyl)methoxy]benzoate (259 mg, 0.700 mmol) was added thereto, and the mixture was stirred at room temperature for 15 hours. The reaction solution was poured into water, extracted with ethyl acetate, and the extracted solution was washed with water. The extracted solution was dried with anhydrous magnesium sulfate, and the solvent was distilled off under reduced pressure. The residue was purified by silicagel column chromatography (hexane:ethyl acetate = 1:2), to obtain the titled compound as powder. 436 mg (70.3%)
¹H-NMR (DMSO-d6) δ; 1.21 (3H, t, J = 7.2 Hz), 3.75 (3H, s), 3.89 (3H, s), 4.15 (2H, q, J = 7.2 Hz), 4.66 (2H, s), 6.68 (1H, s), 6.80 to 7.60 (13H, m), 7.87 (1H, d, J = 2.4 Hz), 8.49 (1H, s), 8.54 (1H, s)

### Example 446

### Methyl 2-[bis(4-fluorophenyl)methoxy]-5-{[({4-[(ethoxycarbonyl)oxy]-3-methoxyphenyl}amino)carbonyl]amino}benzoate

From 4-[(ethoxycarbonyl)oxy]-3-methoxy benzoate and methyl 5-amino-2-[bis(4-fluorophenyl)methoxy]benzoate was synthesized the titled compound in the same manner as Example 438.
¹H-NMR (DMSO-d6) δ; 1.27 (3H, t, J = 7.2 Hz), 3.76 (3H, s), 3.89 (3H, s), 4.21 (2H, q, J = 7.2 Hz), 6.69 (1H, s), 6.87 to 7.61 (13H, m), 7.89 (1H, d, J = 2.4 Hz), 8.63 (1H, s), 8.73 (1H, s)

### Example 447

### Methyl 2-[bis(4-fluorophenyl)methoxy]-5-({[(4-hydroxy-3-methoxyphenyl)amino]carbonyl}amino)benzoate

A solution (2 mL) of methyl 2-[bis(4-fluorophenyl)methoxy]-5-([({4-[(ethoxycarbonyl)oxy]-3-methoxyphenyl}amino)carbonyl]amino}benzoate (122 mg, 0.200 mmol) and potassium carbonate (28 mg, 0.200 mmol) in methanol was stirred under ice-cooling for 15 minutes and at room temperature for 1.5 hours, the reaction solution was poured into water and was extracted with ethyl acetate, and the extracted solution was washed with water. The extracted solution was dried with anhydrous magnesium sulfate, and the solvent was distilled off under reduced pressure. The residue was purified by silicagel column chromatography (hexane:ethyl acetate = 1:2), to obtain the titled compound as oil. 88.0 mg (82.2%)
¹H-NMR (DMSO-d6) δ; 3.73 (3H, s), 3.88 (3H, s), 6.65 to 7.61 (14H, m), 7.87 (1H, d, J = 2.4 Hz), 8.32 (1H, s), 8.48 (1H, s), 8.57 (1H, s)

### Example 448

### Methyl 2-[bis(4-fluorophenyl)methoxy]-5-({[(4-isopropoxy-3-methoxyphenyl)amino]carbonyl}amino)benzoate

From methyl 4-isopropoxy-3-methoxy benzoate and 5-amino-2-[bis(4-fluorophenyl)methoxy]benzoate was synthesized the titled compound in the same manner as Example 438.
¹H-NMR (DMSO-d6) δ; 1.21 (6H, d, J = 6.0 Hz), 3.72 (3H, s), 3.89 (3H, s), 4.36 to 4.39 (1H, m), 6.68 (1H, s), 6.81 to 7.61 (13H, m), 7.87 (1H, d, J = 2.0 Hz), 8.46 (1H, s), 8.53 (1H, s)

### Example 449

### Methyl 5-({[(3,4-dimethoxyphenyl)amino]carbonyl}amino)-2-(dithien-2-ylmethoxy)benzoate

According to the same method as Example 236 was synthesized the titled compound.

### (1) Methyl 2-(dithien-2-ylmethoxy)-5-nitrobenzoate

¹H-NMR (CDCl₃) δ; 4.02 (3H, s), 6.36 (1H, s), 6.85 to 7.26 (6H, m), 8.26 (1H, d, J = 2.4 Hz), 8.73 (1H, d, J = 2.4 Hz), 11.93 (1H, s)

### (2) Methyl 5-({[(3,4-dimethoxyphenyl)amino]carbonyl}amino)-2-(dithien-2-ylmethoxy)benzoate

¹H-NMR (DMSO-d6) δ; 3.70 (3H, s), 3.73 (3H, s), 3.92 (3H, s), 6.29 (1H, s), 6.84 to 7.45 (10H, m), 8.11 (1H, d, J = 2.0 Hz), 8.26 (1H, s), 8.61 (1H, s), 10.74 (1H, s)

### Example 450

### Methyl 5-[({[4-(2-aminoethoxy)-3-methoxyphenyl]amino}carbonyl)amino]-2-[bis(4-fluorophenyl)methoxy]benzoate

### (1) 4-[2-(1,3-Dioxo-1,3-dihydro-2H-isoindol-2-yl)ethoxy]-3-methoxy benzoate

A mixture of 4-hydroxy-3-methoxybenzaldehyde (4.57 g, 30.0 mmol), 1,2-dibromoethane (20 mL), 10% tetrabutylammonium hydroxide solution (12 mL), potassium hydroxide (12 g) and water (18 mL) was stirred at 53°C for 2 hours. After cooling, the organic layer was collected, and the aqueous layer was extracted with methylene chloride. The organic layer was combined with the extracted solution, washed with water, was dried with anhydrous magnesium sulfate, and the solvent was distilled off under reduced pressure. The residue was recrystallized from hexane and ether. These crystals (2.59 g) and potassium phthalimide (2.04 g, 11.0 mmol) were suspended in DMF (10 mL), the mixture was stirred at 57°C for 2 hours, and the reaction solution was poured into ice-water to obtain crystals. To these crystals (2.93 g) were added acetic acid (12 mL) and sulfamic acid (1.18 g, 12.2 mmol) and to this solution was added a solution of sodium chlorite (1.32 g, 11.7 mmol) in water (3 mL). The mixture was stirred for 1 hour at room temperature, and water (50 mL) was added thereto, to obtain the titled compound as a solid. 2.86 g (93%)
¹H-NMR (DMSO-d6) δ; 3.67 (3H, s), 3.98 (2H, t, J = 6.0 Hz), 4.31 (2H, t, J = 6.0 Hz), 7.07 to 7.91 (7H, m)

### (2) 3-Methoxy-4-{2-[(trifluoroacetyl)amino]ethoxy}benzoate

A suspension of 4-[2-(1,3-dioxo-1,3-dihydro-2H-isoindol-2-yl)ethoxy]-3-methoxy benzoate (6.32 g, 18.5 mmol), methylhydrazine (3.92 mL, 74.0 mmol) and ethanol (120 mL) was heated to reflux for 4 hours. After cooling, the precipitated crystals were collected, washed with small amount of ethanol, and dried. These crystals (1.33 g) were suspended in methylene chloride (10 mL), trifluoroacetic anhydride (3 mL, 21.7 mmol) was added to the suspension under ice-cooling, and the mixture was stirred at room temperature for 1.5 hours. The solvent was distilled off under reduced pressure, and the residue was poured into ice-water, and was extracted with ethyl acetate. The ethyl acetate layer was washed with water and was extracted with saturated aqueous solution of sodium bicarbonate. This sodium bicarbonate solution was acidified with 1 N hydrochloric acid, and further extracted with ethyl acetate. The extracted solution was washed with water. The extracted solution was dried with anhydrous magnesium sulfate, and the solvent was distilled off under reduced pressure, to obtain the titled compound as a solid. 910 mg (47%)
¹H-NMR (DMSO-d6) δ; 3.57 to 3.62 (2H, m), 3.80 (3H, s), 4.18 (2H, t, J = 5.6 Hz), 7.08 (1H, d, J = 8.4 Hz), 7.46 (1H, s), 7.54 (1H, d, J = 8.4 Hz), 9.95 (1H, s), 12.69 (1H, s)

### (3) Methyl 2-[bis(4-fluorophenyl)methoxy]-5-({[(3-methoxy-4-{2-[(trifluoroacetyl)amino]ethoxy}phenyl)amino]carbonyl}amino) benzoate

A solution (6 mL) of 3-methoxy-4-{2-[(trifluoroacetyl)amino]ethoxy}benzoate (185 mg, 0.600 mmol), triethyl amine (0.190 mL, 1.32 mmol) and diphenylphosphoryl azide (0.130 mL, 0.66 mmol) in toluene was stirred at room temperature for 1 hour and at 93°C for 1.5 hours, and the reaction solution was cooled to room temperature. Methyl 5-amino-2-[bis(4-fluorophenyl)methoxy]benzoate (148 mg, 0.400 mmol) was added thereto and the mixture was stirred at room temperature for 15 hours. The reaction solution was poured into water, extracted with ethyl acetate, and the extracted solution was washed with water. The extracted solution was dried with anhydrous magnesium sulfate, and the solvent was distilled off under reduced pressure. The residue was purified by silicagel column chromatography (hexane:ethyl acetate = 2:3), to obtain the titled compound as powder. 255 mg (94%)
¹H-NMR (DMSO-d6) δ; 3.50 to 3.54 (2H, m), 3.73 (3H, s), 3.89 (3H, s), 4.03 (2H, t, J = 5.2 Hz), 6.68 (1H, s), 6.81 to 7.61 (13H, m), 7.87 (1H, d, J = 2.4 Hz), 8.48 (1H, s), 8.53 (1H, s), 9.60 (1H, s)

### (4) Methyl 5-[({[4-(2-aminoethoxy)-3-methoxyphenyl]amino}carbonyl)amino]-2-[bis(4-fluorophenyl)methoxy]benzoate

A suspension of methyl 2-[bis(4-fluorophenyl)methoxy]-5-({[(3-methoxy-4-{2-[(trifluoroacetyl)amino]ethoxy}phenyl)amino]carbonyl}amino) benzoate (81.5 mg, 0.120 mmol), potassium carbonate (20 mg, 0.144 mmol) in methanol (2 mL) was stirred at room temperature for 15 hours and at 63°C for 4 hours, and the solvent was distilled off under reduced pressure. The residue was poured into water, extracted with ethyl acetate, and the extracted solution was washed with water. The extracted solution was dried with anhydrous magnesium sulfate, and the solvent was distilled off under reduced pressure. The residue was purified by silicagel column chromatography (chloroform:methanol = 3:1), to obtain the titled compound as powder. 49 mg (71%)
¹H-NMR (DMSO-d6) δ; 2.90 (2H, t, J = 5.6 Hz), 3.74 (3H, s), 3.88 (3H, s), 3.93 (2H, t, J = 5.6 Hz), 6.67 (1H, s), 6.82 to 7.60 (13H, m), 7.86 (1H, d, J = 2.4 Hz), 8.63 (1H, s), 8.70 (1H, s)

### Example 451

### Methyl 2-[(4-cyanophenyl)(phenyl)methoxy]-5-({[(3,4-dimethoxyphenyl)amino]carbonyl}amino)benzoate

According to the same method as Example 236, the titled compound was synthesized.

### (1) Methyl 2-[(4-cyanophenyl)(phenyl)methoxy]-5-nitrobenzoate

¹H-NMR (CDCl₃) δ; 4.00 (3H, s), 6.45 (1H, s), 7.00 (2H, d, J = 9.2 Hz), 7.26 to 7.73 (9H, m), 8.22 (1H, dd, J = 3.2, 9.2 Hz), 8.75 (2H, d, J = 3.2 Hz)

### (2) Methyl 2-[(4-cyanophenyl)(phenyl)methoxy]-5-({[(3,4-dimethoxyphenyl)amino]carbonyl}amino)benzoate

¹H-NMR (DMSO-d6) δ; 3.70 (3H, s), 3.72 (3H, s), 3.90 (3H, s), 6.77 (1H, s), 6.85 to 7.90 (15H, m), 8.45 (1H, s), 8.53 (1H, s)

### Example 452

### Methyl 2-[bis(4-fluorophenyl)methoxy]-5-[({[4-(ethoxymethoxy)-3-methoxyphenyl]amino}carbonyl)amino]benzoate

### (1) 4-(ethoxymethoxy)-3-methoxy benzoate

A solution (10 mL) of 4-hydroxy-3-methoxybenzaldehyde (1.53 g, 10.0 mmol), potassium carbonate (1.66 g, 12.0 mmol) and chloromethylethyl ether (1.12 mL, 12.0 mmol) in DMF was stirred at room temperature for 2 hours, the reaction solution was poured into water, and was extracted with ethyl acetate. The extracted solution was washed with water, was dried with anhydrous magnesium sulfate, and the solvent was distilled off under reduced pressure. To the residue were added acetic acid (12 mL) and sulfamic acid (1.17 g, 12.0 mmol), and to this solution was added a solution of sodium chlorite (1.25 g, 11.0 mmol) in water (3 mL). The mixture was stirred at room temperature for 1 hour, water (50 mL) was added thereto, to obtain the titled compound as a solid. 1.64 g (63%)
¹H-NMR (DMSO-d6) δ; 1.13 (3H, t, J = 6.8 Hz), 3.67 (2H, q, J = 6.8 Hz), 3.82 (3H, s), 5.29 (2H, s), 7.15 (1H, d, J = 8.8 Hz), 7.48 (1H, d, J = 2.0 Hz), 7.53 (1H, dd, J = 2.0, 8.8 Hz)

### (2) Methyl 2-[bis(4-fluorophenyl)methoxy]-5-[({[4-(ethoxymethoxy)-3-methoxyphenyl]amino}carbonyl)amino]benzoate

A solution (10 mL) of 4-(ethoxymethoxy)-3-methoxy benzoate (227 mg, 1.00 mmol), triethyl amine (0.170 mL, 1.10 mmol) and diphenylphosphoryl azide (0.240 mL, 1.10 mmol) in toluene was stirred at room temperature for 15 minutes and at 93°C for 1.5 hours, and the reaction solution was cooled to room temperature. Methyl 5-amino-2-[bis(4-fluorophenyl)methoxy]benzoate (259 mg, 0.700 mmol) was added thereto, and the mixture was stirred at room temperature for 15 hours. The reaction solution was poured into water, extracted with ethyl acetate, and the extracted solution was washed with water. The extracted solution was dried with anhydrous magnesium sulfate, and the solvent was distilled off under reduced pressure. The residue was purified by silicagel column chromatography (hexane:ethyl acetate = 1:1), to obtain the titled compound as powder. 357 mg (86%)
¹H-NMR (DMSO-d6) δ; 1.13 (3H, t, J = 7.2 Hz), 3.67 (2H, q, J = 7.2 Hz), 3.74 (3H, s), 3.89 (3H, s), 5.08 (2H, s), 6.68 (1H, s), 6.79 to 7.61 (13H, m), 7.88 (1H, d, J = 2.8 Hz), 8.51 (1H, s), 8.55 (1H, s)

### Example 453

### Methyl 2-[bis(4-fluorophenyl)methoxy]-5-({[(4-{[(2,2-dimethylpropanoyl)oxy]methoxy}-3-methoxyphenyl)amino]carbonyl}amino)benzoate

### (1) (4-Formyl-2-methoxyphenoxy)methyl pivalic acid

A solution (15 mL) of 4-hydroxy-3-methoxybenzaldehyde (1.53 g, 10.0 mmol), potassium carbonate (1.80 g, 13.0 mmol) and chloromethyl pivalic acid (1.89 mL, 13.0 mmol) in DMF was stirred at room temperature for 15 hours, the reaction solution was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 7:1), to obtain the titled compound as oil. 1.75 g (65%)
¹H-NMR (CDCl₃) δ; 1.21 (9H, s), 3.75 (3H, s), 5.88 (2H, s), 7.17 to 7.46 (3H, m), 9.90 (1H, s)

### (2) 4-{[(2,2-Dimethylpropanoyl)oxy]methoxy}-3-methoxy benzoate

To a solution of (4-formyl-2-methoxyphenoxy)methyl pivalic acid (1.75 g, 6.50 mmol) were added acetic acid (8 mL) and sulfamic acid (0.850 g, 8.77 mmol), and to this solution was added a solution of sodium chlorite (0.765 g, 8.450 mmol) in water (2 mL). The mixture was stirred at room temperature for 1 hour and water (20 mL) was added thereto, to obtain the titled compound as a solid. 1.53 g (83%)
¹H-NMR (DMSO-d6) δ; 1.12 (9H, s), 3.85 (3H, s), 5.85 (2H, s), 7.17 to 7.57 (3H, m), 12.81 (1H, bs)

### (3) Methyl 2-[bis(4-fluorophenyl)methoxy]-5-({[(4-{[(2,2-dimethylpropanoyl)oxy]methoxy}-3-methoxyphenyl)amino]carbonyl}amino)benzoate

A solution (10 mL) of 4-{[(2,2-dimethylpropanoyl)oxy]methoxy}-3-methoxy benzoate (283 mg, 1.00 mmol), triethyl amine (0.308 mL, 2.20 mmol) and diphenylphosphoryl azide (0.240 mL, 1.10 mmol) in toluene was stirred at room temperature for 15 minutes and at 93°C for 1.5 hours, and the reaction solution was cooled to room temperature. Methyl 5-amino-2-[bis(4-fluorophenyl)methoxy]benzoate (259 mg, 0.700 mmol) was added thereto, and the mixture was stirred at room temperature for 15 hours. The reaction solution was poured into water, extracted with ethyl acetate, and the extracted solution was washed with water. The extracted solution was dried with anhydrous magnesium sulfate, and the solvent was distilled off under reduced pressure. The residue was purified by silicagel column chromatography (hexane:ethyl acetate = 1:1), to obtain the titled compound as powder. 453 mg (100%)
¹H-NMR (DMSO-d6) δ; 1.12 (9H, s), 3.75 (3H, s), 3.89 (3H, s), 5.62 (2H, s), 6.68 (1H, s), 6.83 to 7.61 (13H, m), 7.88 (1H, d, J = 2.8 Hz), 8.57 (2H, s)

### Example 454

### Methyl 2-[bis(4-fluorophenyl)methoxy]-5-[({[3-methoxy-4-(methoxymethoxy)benzyl]amino}carbonyl)amino]benzoate

### (1) Methyl [3-methoxy-4-(methoxymethoxy)phenyl]acetate

A solution (20 mL) of methyl (4-hydroxy-3-methoxyphenyl)acetate (1.11 g, 5.65 mmol), diisopropylethyl amine (1.19 mL, 6.78 mmol) and chloromethylm ethyl ether (0.515 mL, 6.78 mmol) in methylene chloride was stirred under ice-cooling for 1 hour and at room temperature for 3 hours, and the solvent was distilled off under reduced pressure. The residue was poured into water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 7:3), to obtain the titled compound as oil. 930 mg (68%)
¹H-NMR (CDCl₃) δ; 3.51 (3H, s), 3.57 (2H, s), 3.70 (3H, s), 3.88 (3H, s), 5.21 (2H, s), 6.78 to 7.11 (3H, m)

### (2) [3-Methoxy-4-(methoxymethoxy)phenyl]acetic acid

To a solution of methyl [3-methoxy-4-(methoxymethoxy)phenyl]acetate (930 mg, 3.87 mmol) in methanol (5 mL) was added 1N aqueous solution of sodium hydroxide (4 mL), and the mixture was stirred at room temperature for 1 hour. The reaction solution was poured into water, washed with ether, neutralized with 1 N hydrochloric acid, and was extracted with ethyl acetate.

The extracted solution was washed with water, was dried with anhydrous magnesium sulfate, and the solvent was distilled off under reduced pressure, to obtain the titled compound as a solid. 875 mg (100%)
¹H-NMR (CDCl₃) δ; 3.51 (3H, s), 3.60 (2H, s), 3.87 (3H, s), 5.21 (2H, s), 6.79 to 7.12 (3H, m)

### (3) Methyl 2-[bis(4-tluorophenyl)methoxy]-5-[({[3-methoxy-4-(methoxymethoxy)benzyl]amino}carbonyl)amino]benzoate

From [3-methoxy-4-(methoxymethoxy)phenyl]acetic acid and methyl 5-amino-2-[bis(4-fluorophenyl)methoxy]benzoate was synthesized the titled compound in the same manner as Example 453.
¹H-NMR (DMSO-d6) δ; 3.37 (3H, s), 3.75 (3H, s), 3.88 (3H, s), 4.20 (2H, d, J = 5.6 Hz), 5.09 (2H, s), 6.51 (1H, t, J = 5.6 Hz), 6.65 (1H, s), 6.80 to 7.60 (13H, m), 7.81 (1H, d, J = 2.8 Hz), 8.50 (1H, s)

### Example 455

### Methyl 2-[bis(4-fluorophenyl)methoxy]-5-({[(3,4-dimethoxybenzyl)amino]carbonyl}amino)benzoate

From (3,4-dimethoxyphenyl)acetic acid and methyl 5-amino-2-[bis(4-fluorophenyl)methoxy]benzoate was synthesized the titled compound in the same manner as Example 453.
¹H-NMR (DMSO-d6) δ; 3.72 (3H, s), 3.78 (3H, s), 4.19 (2H, d, J = 5.6 Hz), 6.48 (1H, t, J = 5.6 Hz), 6.65 (1H, s), 6.79 to 7.60 (13H, m), 7.82 (1H, d, J = 1.6 Hz), 8.49 (1H, s)

### Example 456

### Methyl 2-[(4-chlorophenyl)(2-fluorophenyl)methoxy]-5-({[(3,4-dimethoxyphenyl)amino]carbonyl}amino)benzoate

### (1) (4-Chlorophenyl)(2-fluorophenyl)methanol

To a solution of 2-fluorobromobenzene (9.2 g, 52.6 mmol) in THF (200 mL) was added dropwise 1.6N solution of butyl lithium in hexane (40 mL, 65 mmol) at -78°C, and the mixture was stirred at -78°C for 10 minutes. A solution (30 mL) of 4-chlorobenzaldehyde (8.9 g, 63.2 mmol) in THF was added dropwise thereto, and the mixture was stirred at - 78 to -65°C for 1 hour. The reaction solution was poured into an aqueous solution of saturated ammonium chloride, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 4:1), to obtain the titled compound as oil. 6.6 g (42.7%)
¹H-NMR (CDCl₃) δ: 2.31 (1H, d, J = 4.1 Hz), 6.12 (1H, d, J = 4.1 Hz), 7.02 (1H, ddd, J = 1.2, 8.3 and 10.5 Hz), 7.15 (1H, td, J = 1.2 and 7.5 Hz), 7.25 to 7.36 (5H, m), 7.47 (1H, td, J = 1.7 and 7.5 Hz)

### (2) Methyl 2-[(4-chlorophenyl)(2-fluorophenyl)methoxy]-5-nitrobenzoate

A mixture of methyl 2-hydroxy-5-nitrobenzoate (2.1 g, 10.6 mmol), (4-chlorophenyl)(2-fluorophenyl)methanol (3.0 g, 12.7 mmol), 40% solution of diethyl azodicarbonate in toluene (7.4 g, 17.0 mmol) and a solution (10 mL) of triphenylphosphine (3.3 g, 12.7 mmol) in acetonitrile was stirred at room temperature for 12 hours, the reaction solution was poured into ice-water, and was extracted with ethyl acetate. The extracted solution was washed with water, was dried with anhydrous magnesium sulfate, and the solvent was distilled off under reduced pressure. The residue was purified by silicagel column chromatography (hexane:ethyl acetate = 5:1), to obtain the titled compound as oil. 1.7 g (38.6%)
¹H-NMR (CDCl₃) δ: 4.00 (3H, s), 6.77 (1H, m), 7.02 to 7.18 (4H, m), 7.26 to 7.36 (2H, m), 7.54 (2H, m, J = 8.6 Hz), 7.64 (1H, td, J = 1.7 and 7.7 Hz), 8.23 (1H, dd, J = 2.9 and 9.3 Hz), 8.75 (1H, d, J = 2.9 Hz)
IR (KBr) cm⁻¹: 1734, 1620, 1580, 1525, 1489, 1346, 1277

### (3) Methyl 5-amino-2-[(4-chlorophenyl)(2-fluorophenyl)methoxy]benzoate

A mixture of methyl 2-[(4-chlorophenyl)(2-fluorophenyl)methoxy]-5-nitrobenzoate (1.6 g, 3.9 mmol), iron (1.1 g, 19.2 mmol), calcium chloride (211 mg, 1.9 mmol), ethanol (25 mL) and water (5 mL) was stirred at reflux temperature for 2 hours. The insolubles were filtered off, and the filtrate was concentrated. The residue was dried under reduced pressure to obtain the titled compound as oil. 1.4 g (94.3%)
¹H-NMR (CDCl₃) δ: 3.47 (2H, br), 3.85 (3H, s), 6.49 (1H, s), 6.64 (1H, dd, J = 2.9 and 8.3 Hz), 6.70 (1H, d, J = 8.5 Hz), 6.98 to 7.36 (6H, m), 7.49 (2H, d, J = 7.8 Hz), 7.69 (1H, td, J = 1.7 and 7.5 Hz)
IR (KBr) cm⁻¹: 1721, 1491, 1448, 1250, 1220, 100, 788

### (4) Methyl 2-[(4-chlorophenyl)(2-fluorophenyl)methoxy]-5-({[(3,4-dimethoxyphenyl)amino]carbonyl}amino)benzoate

To a solution (20 mL) of methyl 5-amino-2-[(4-chlorophenyl)(2-fluorophenyl)methoxy]benzoate (1.35 g, 3.5 mmol) in THF was added 3,4-dimethoxyphenyl isocyanate (0.75 g, 4.2 mmol) under ice-cooling, the mixture was stirred at 0°C for 1 hour and at room temperature for 12 hours, and the solvent was distilled off under reduced pressure. The residue was purified by silicagel column chromatography (hexane:ethyl acetate = 1:1), to obtain the titled compound as a solid. 1.36 g (65.7%)
¹H-NMR (CDCl₃) δ: 3.84 (3H, s), 3.85 (3H, s), 3.88 (3H, s), 6.46 (1H, s), 6.58 (2H, m), 6.73 (1H, dd, J = 8.5 and 2.4 Hz), 6.79 to 6.86 (2H, m), 6.97 to 7.30 (6H, m), 7.46 (1H, dd, J = 8.5 and 2.4 Hz), 7.49 (1H, m), 7.51 (1H,m), 7.63 to 7.68 (2H, m)
IR (KBr) cm⁻¹: 1725, 1650, 1590, 1556, 149, 1448, 1222, 1082, 1014, 786
LC/MS (APCI+) m/z: 587.1 (Na⁺)

### Example 457

### N-(tert-butyl)-2-[(4-chlorophenyl)(2-fluorophenyl)methoxy]-5-({[(3,4-dimethoxyphenyl)amino]carbonyl}amino)benzamide

### (1) 2-[(4-Chlorophenyl)(2-fluorophenyl)methoxy]-5-({[(3,4-dimethoxyphenyl)amino]carbonyl}amino)benzoate

To a solution (25 mL) of methyl 2-{(4-chlorophenyl)(2-fluorophenyl)methoxy}-5-({[(3,4-dimethoxyphenyl)amino]carbonyl}amino)benzoate (0.8 g, 1.42 mmol) in methanol was added 1N aqueous solution of sodium hydroxide (4 mL) at reflux temperature, and the mixture was stirred for 3 hours. The reaction solution was poured into water, and neutralized with 1N-hydrochloric acid. The precipitated crystals were collected, washed with water, and dried under reduced pressure to obtain the titled compound as a solid. 790 mg (100%)
¹H-NMR (CDCl₃) δ: 3.85 (3H, s), 3.87 (3H, s), 6.71 (1H, s), 6.83 (1H, d, J = 8.5 Hz), 6.89(1H, dd, J = 2.4 and 8.5 Hz), 6.94 (1H, d, J = 9.3 Hz), 7.11 to 7.43 (11H, m), 7.73 (1H, d, J = 2.9 Hz), 8.21 (1H, dd, J = 2.8 and 9.3 Hz), 11.0 (1H, br)
IR (KBr) cm⁻¹: 3330, 2919, 1700, 1610, 1520, 1493, 1222, 761

### (2) N-(tert-Butyl)-2-[(4-chlorophenyl)(2-fluorophenyl)methoxy]-5-({[(3,4-dimethoxyphenyl)amino]carbonyl}amino)benzamide

A mixed solution of 2-[(4-chlorophenyl)(2-fluorophenyl)methoxy]-5-({[(3,4-dimethoxyphenyl)amino]carbonyl}amino)benzoate (600 mg, 1.09 mmol), 1-hydroxy-1H-benzotriazole (250 mg, 1.64 mmol), tert-butyl amine (160 mg, 2.18 mmol), 1-(3-dimethylaminopropyl)-3-ethylcarbodiimide hydrochloride (261 mg, 1.36 mmol) and DMF (6 mL) was stirred under ice-cooling for 1 hour and at room temperature for 12 hours, was poured into water, and the precipitates were collected. This was purified by silicagel column chromatography (hexane:ethyl acetate = 1:1), to obtain the titled compound as a solid. 620 mg (93.9%)
¹H-NMR (CDCl₃) δ: 1.22 (9H, s), 3.82 (3H, s), 3.83 (3H, s), 6.52 (1H, s), 6.71 to 6.77 (3H, m), 7.06 to 7.15 (3H, m), 7.27 to 7.36 (7H, m), 7.53 (1H, s), 7.64 to 7.67 (2H, m), 7.71 (1H, dd, J = 2.8 and 8.9 Hz)
IR (KBr) cm⁻¹: 3360, 1663, 1610, 1546, 1515, 1491, 1206, 1093, 1028, 910, 812, 761, 733
LC/MS (APCl +) m/z: 628.1 (Na⁺), 606.1 (M⁺)

### Example 458

### Methyl 2-[(4-chlorophenyl)(3-fluorophenyl)methoxy]-5-({[(3,4-dimethoxyphenyl)amino]carbonyl}amino)benzoate

The titled compound was synthesized in the same manner as Example 456.

### (1) (4-Chlorophenyl)(3-fluorophenyl)methanol

¹H-NMR (CDCl₃) δ: 2.31 (1H, d, J = 4.1 Hz), 5.80 (1H, d, J = 3.5 Hz), 6.93 to 6.99(1H, m), 7.07 to 7.13 (2H, m), 7.27 to 7.34 (5H, m)

### (2) Methyl 2-{(4-chlorophenyl)(3-fluorophenyl)methoxy}-5-nitrobenzoate

¹H-NMR (CDCl₃) δ: 4.00 (3H, s), 6.37 (1H, s), 6.96 to 7.13 (3H, m), 7.25 to 7.36 (4H, m), 7.45 to 7.48 (2H, m), 8.21 (1H, dd, J = 2.9 and 9.3 Hz), 8.75 (1H, d, J = 2.9 Hz)
IR (KBr) cm⁻¹: 3464, 1734, 1614, 1591, 1522, 1489, 1442, 1346, 1279, 1130, 1076, 1014, 824, 788, 771

### (3) Methyl 5-amino-2-[(4-chlorophenyl)(3-fluorophenyl)methoxy]benzoate

¹H-NMR (CDCl₃) δ: 3.49(2H, brs), 3.85 (3H, s), 6.07 (1H, m), 6.25 (2H, d, J = 1.7 Hz), 6.9 to 7.5 (9H, m)
IR (KBr) cm⁻¹: 3367, 1723, 1591, 1492, 1222, 1014, 786

### (4) Methyl 2-[(4-chlorophenyl)(3-fluorophenyl)methoxy]-5-({[(3,4-dimethoxyphenyl)amino]carbonyl}amino)benzoate

¹H-NMR (CDCl₃) δ: 3.79(3H, s), 3.80 (3H, s), 3.85 (3H, s), 6.12 (1H, s), 6.66 (1H, dd, J = 2.4 and 8.5 Hz), 6.71 to 6.75 (2H, m), 6.88 (1H, s), 6.90 to 7.00 (3H, m), 7.17 to 7.29(5H, m), 7.37 to 7.43 (3H, m), 7.61 (1H, d, J = 2.7 Hz)
IR (KBr) cm⁻¹: 3341, 1725, 1654, 1612, 1557, 1497, 1451, 1413, 1221, 1166, 1136, 1084, 1027, 910, 788, 772, 733
LC/MS (APCI +) m/z: 587.0 (Na⁺), 565.0 (M⁺)

### Example 459

### N-(tert-Butyl)-2-[(4-chlorophenyl)(3-fluorophenyl)methoxy]-5-({[(3,4-dimethoxyphenyl)amino]carbonyl}amino)benzamide

The titled compound was synthesized in the same manner as Example 457.

### (1) 2-[(4-Chlorophenyl)(3-fluorophenyl)methoxy}-5-({[(3,4-dimethoxyphenyl)amino]carbonyl}amino)benzoate

¹H-NMR (CDCl₃) δ: 3.84 (3H, s), 3.86 (3H, s), 6.37 (1H, m), 6.82 (1H, d, J = 8.6 Hz), 6.88 to 6.92 (2H, m), 7.05 to 7.08 (2H, m), 7.15 (2H, m), 7.30 (2H, d, J = 8.3 Hz), 7.36 to 7.43 (4H, m), 7.58 (1H, brs), 7.71 (1H, d, J = 2.7 Hz), 8.23 (1H, dd, J = 2.7 and 9.1 Hz), 10.8 (1H, br)
IR (KBr) cm⁻¹: 3380, 1702, 1552, 1516, 1493, 1260, 1220, 1196

### (2) N-(tert-Butyl)-2-[(4-chlorophenyl)(3-fluorophenyl)methoxy]-5-({[(3,4-dimethoxyphenyl)amino]carbonyl}amino)benzamide

¹H-NMR (CDCl₃) δ: 1.21 (9H, s), 3.80 (3H, s), 3.82 (3H, s), 6.19(1H, s), 6.70 to 6.76 (3H, m), 7.00 to 7.05 (2H, m), 7.07 to 7.12 (2H, m), 7.23 to 7.26 (2H, m), 7.30 to 7.36 (3H, m), 7.43 (1H, s), 7.65 to 7.72 (4H, m)
IR (KBr) cm⁻¹: 3368, 1637, 1610, 1514, 1491, 1412, 1205, 1027, 733
LC/MS (APCl +) m/z: 628.1 (Na⁺), 606.1 (M⁺)

### Example 460

### Methyl 2-[(4-chlorophenyl)(4-fluorophenyl)methoxy]-5-({[(3,4-dimethoxyphenyl)amino]carbonyl}amino)benzoate

The titled compound was synthesized in the same manner as Example 456.

### (1) (4-Chlorophenyl)(4-fluorophenyl)methanol

¹H-NMR (CDCl₃) δ: 2.24 (1H, d, J = 3.3 Hz), 5.80 (1H, d, J = 3.3 Hz), 6.99 to 7.05 (2H, m), 7.26 to 7.35 (6H, m)
IR (KBr) cm⁻¹: 3340, 1605, 1508, 1225, 1090, 1013, 830, 551

### (2) Methyl 2-[(4-chlorophenyl)(4-fluorophenyl)methoxy]-5-nitrobenzoate

¹H-NMR (CDCl₃) δ: 3.98 (3H, s), 6.38 (1H, s), 6.96 to 7.08 (3H, m), 7.30 to 7.36 (3H, m), 7.43 to 7.50 (3H, m), 8.20 (1H, dd, J = 2.7 and 9.2 Hz), 8.73 (1H, d, J = 2.7 Hz)
IR (KBr) cm⁻¹: 1734, 1612, 1588, 1510, 1489, 1346, 1346, 1278, 1130, 1076, 1013, 822

### (3) Methyl 5-amino-2-[(4-chlorophenyl)(4-fluorophenyl)methoxy]benzoate

¹H-NMR (CDCl₃) δ: 3.49(2H, br), 3.83 (3H, s), 6.07 (1H, s), 6.99(2H, d, J = 4.4 Hz), 6.98 to 7.05 (3H, m), 7.12 (1H, t, J = 1.6 Hz), 7.27 to 7.43 (5H, m)
IR (KBr) cm⁻¹: 1721, 1605, 1499, 1448, 1319, 1222, 1158, 1089, 1014, 816, 551

### (4) Methyl 2-[(4-chlorophenyl)(4-fluorophenyl)methoxy]-5-({[(3,4-dimethoxyphenyl)amino]carbonyl}amino)benzoate

¹H-NMR (CDCl₃) δ: 3.83 (3H, s), 3.85 (3H, s), 3.86 (3H, s), 6.16 (1H, s), 6.59(1H, s), 6.70 to 6.80 (4H, m), 6.98 to 7.02 (3H, m), 7.27 to 7.31 (2H, m), 7.37 to 7.46 (5H, m), 7.62 (1H, d, J = 3.0 Hz)
IR (KBr) cm⁻¹: 3346, 1729, 1606, 1554, 1499, 1413, 1221, 1082, 1013, 815
LC/MS (APCI +) m/z: 587.0 (Na⁺)

### Example 461

### N-(tert-Butyl)-2-[(4-chlorophenyl)(4-fluorophenyl)methoxy)-5-({[(3,4-dimethoxyphenyl)amino]carbonyl}amino)benzamide

The titled compound was synthesized in the same manner as Example 457.

### (1) 2-[(4-Chlorophenyl)(4-fluorophenyl)methoxy]-5-({[(3,4-dimethoxyphenyl)amino]carbonyl}amino)benzoate

¹H-NMR (CDCl₃) δ: 3.85 (3H, s), 3.87 (3H, s), 6.40 (1H, s), 6.79 to 6.92 (4H, m), 7.03 to 7.12 (3H, m), 7.21 to 7.39(7H, m), 7.70 (1H, d, J = 2.9 Hz), 8.19(1H, d, J = 8.3 Hz), 10.2 (1H, br)
IR (KBr) cm⁻¹: 3381, 1699, 1607, 1542, 1509, 1491, 1222, 1026, 820

### (2) N-(tert-Butyl)-2-[(4-chlorophenyl)(4-fluorophenyl)methoxy]-5-({[(3,4-dimethoxyphenyl)amino]carbonyl}amino)benzamide

¹H-NMR (CDCl₃) δ: 1.19 (9H, s), 3.82 (3H, s), 3.84 (3H, s), 6.22 (1H, s), 6.71 to 6.78 (3H, m), 7.03 to 7.09 (3H, m), 7.19 (1H, s), 7.23 to 7.29 (5H, m), 7.32 to 7.36 (1H, m), 7.46 (1H, s), 7.67 (1H, d, J = 2.7 Hz), 7.70 to 7.75 (2H, m)
IR (KBr) cm⁻¹: 3373, 1636, 1607, 1528, 1512, 1491, 1222, 1091, 1028, 816, 733

The structures of the compounds of Examples 1 to 461 are shown in the following Table 1.

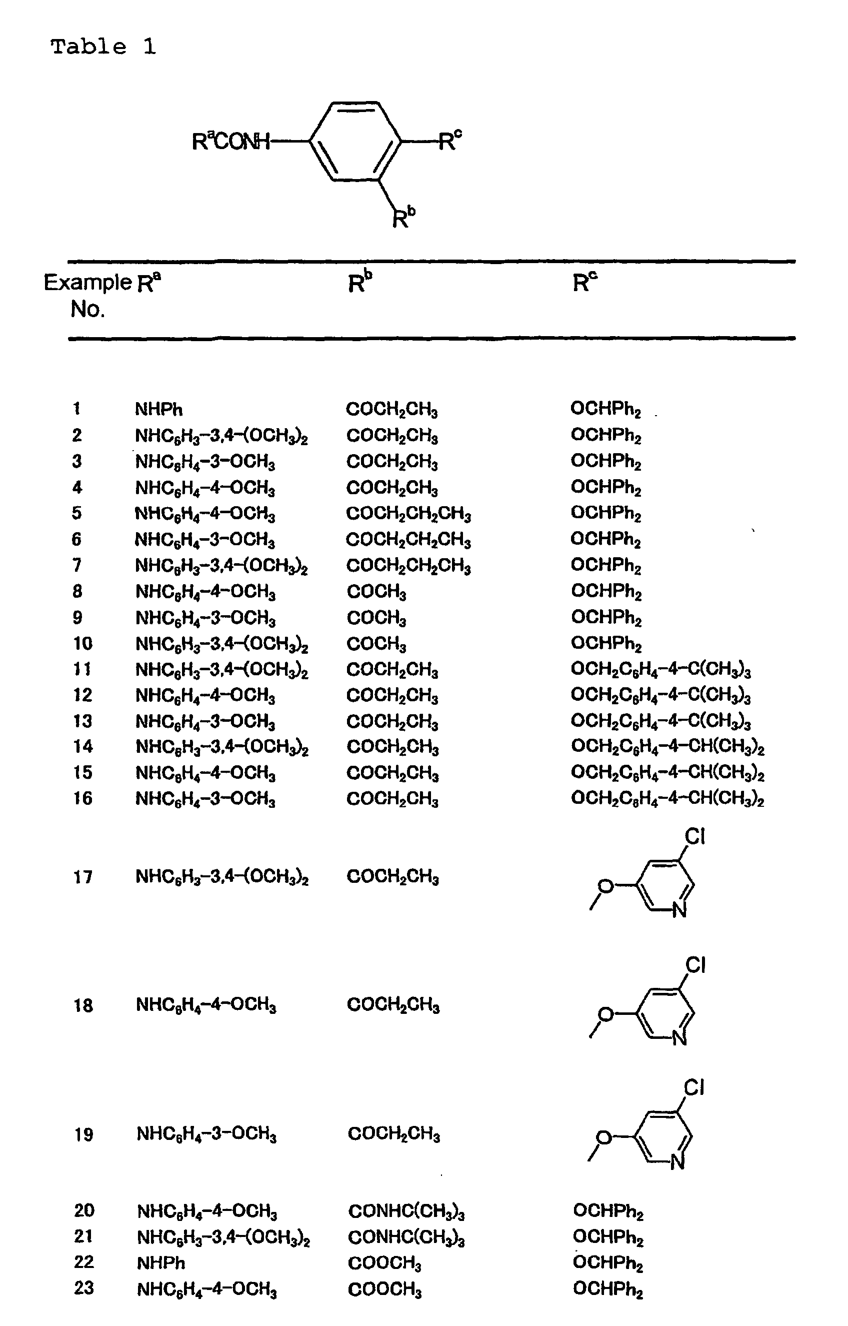

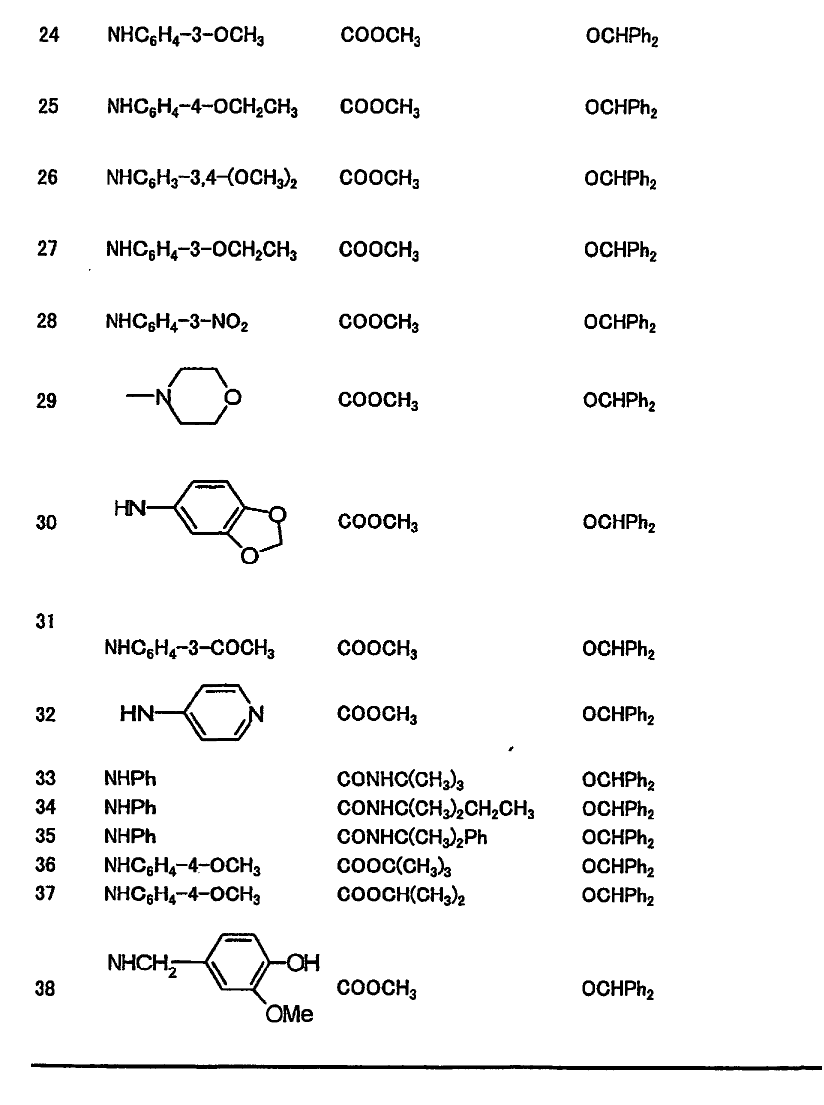

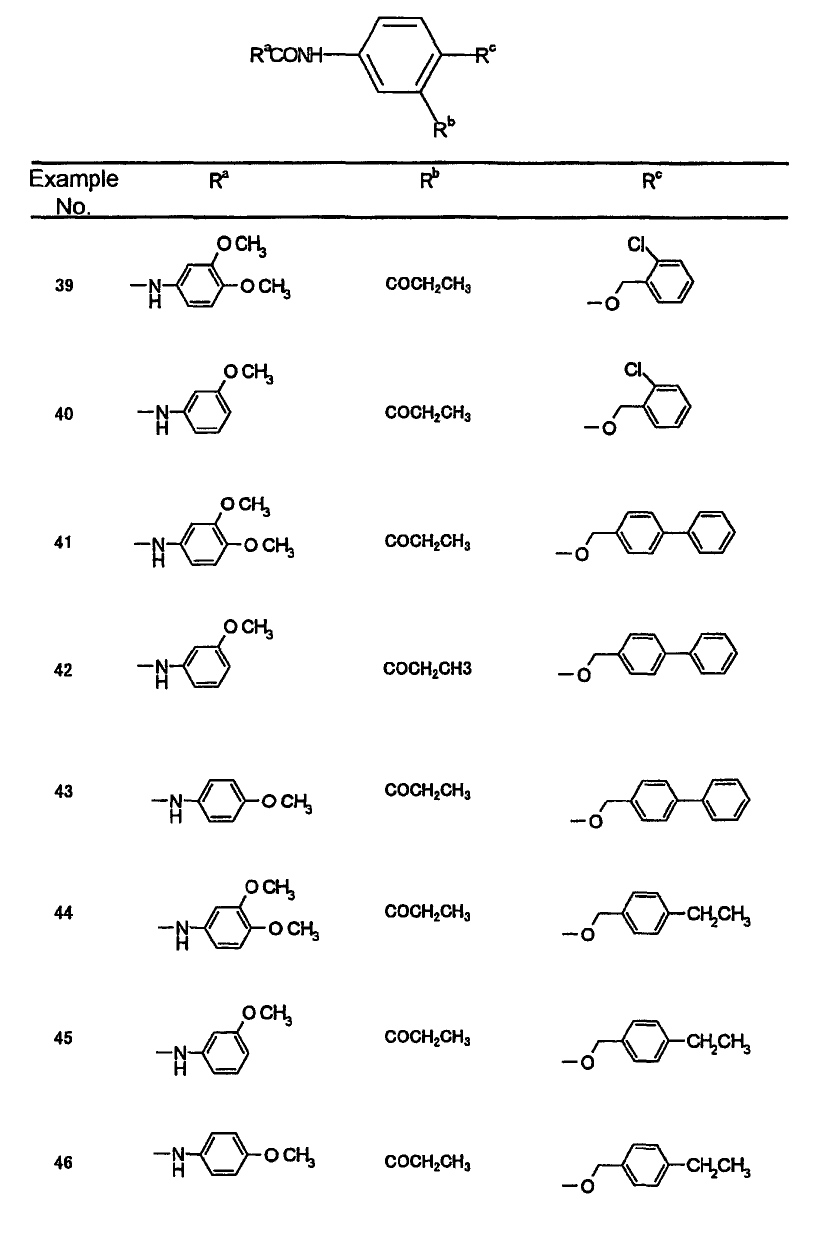

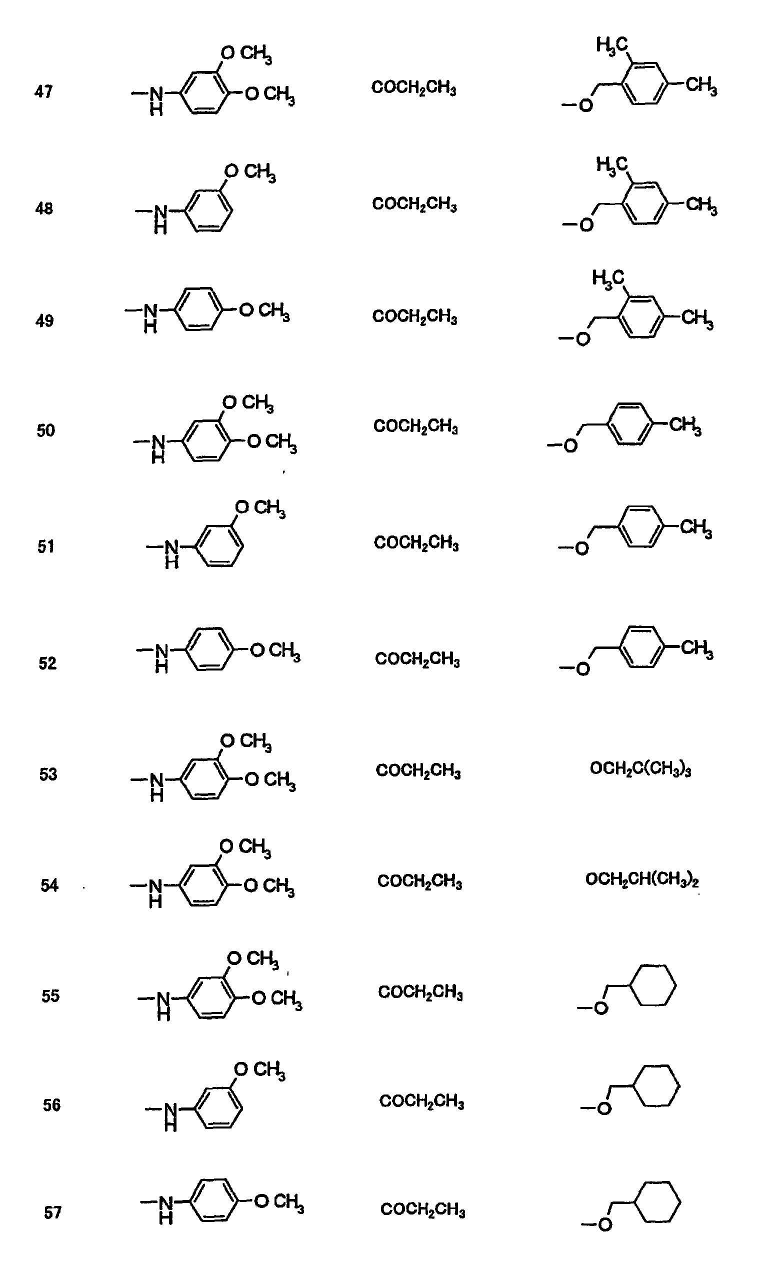

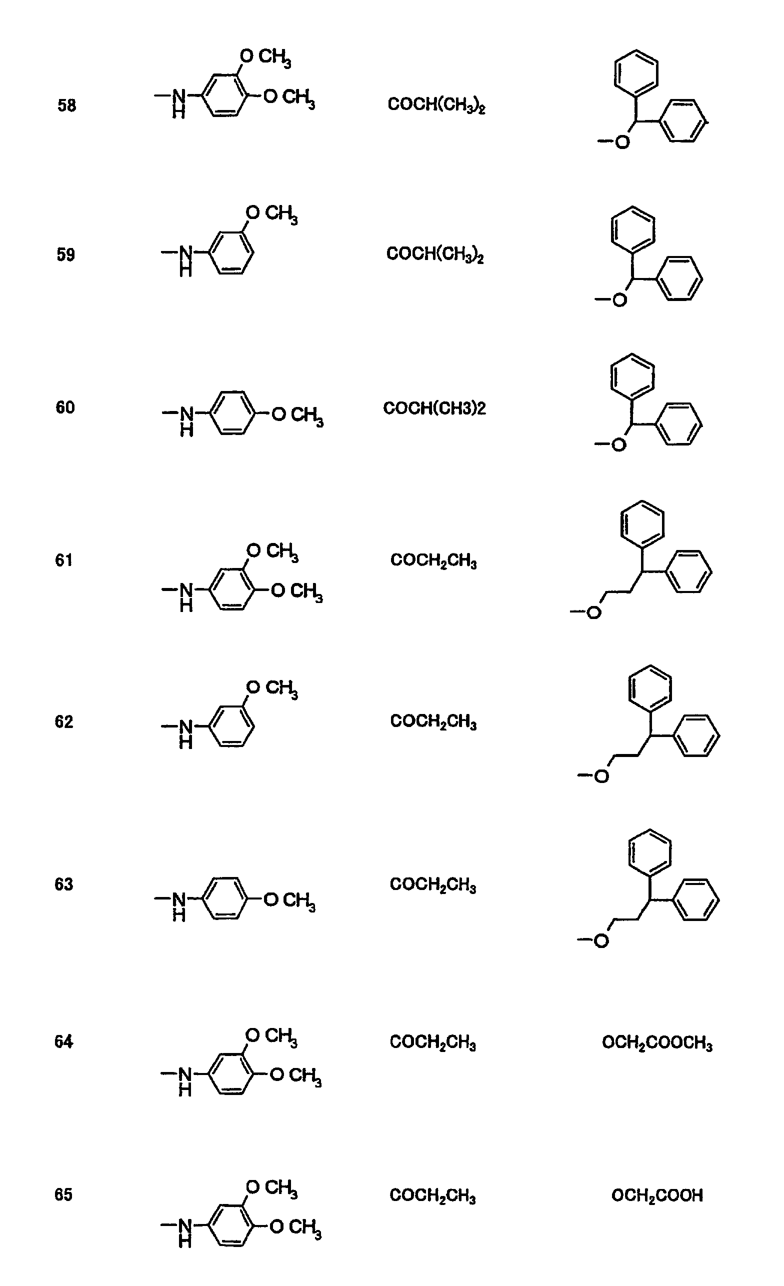

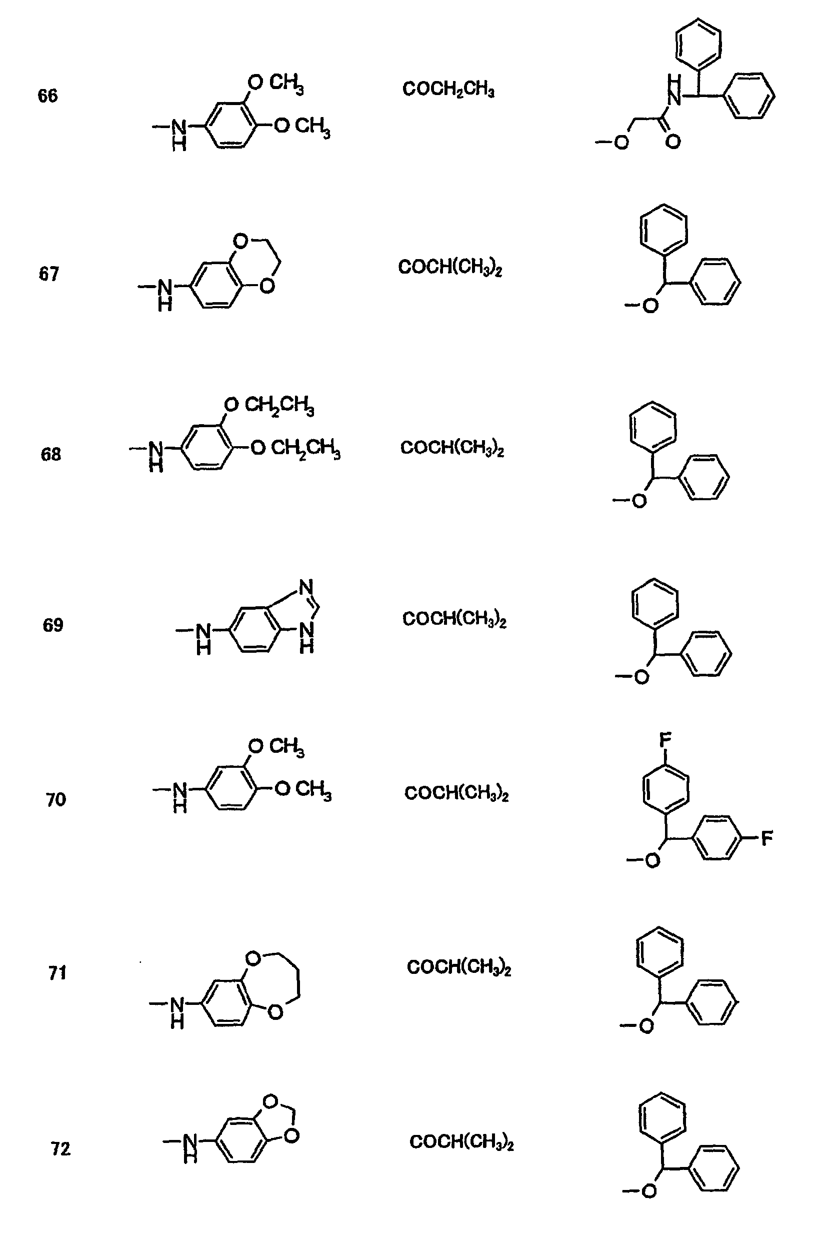

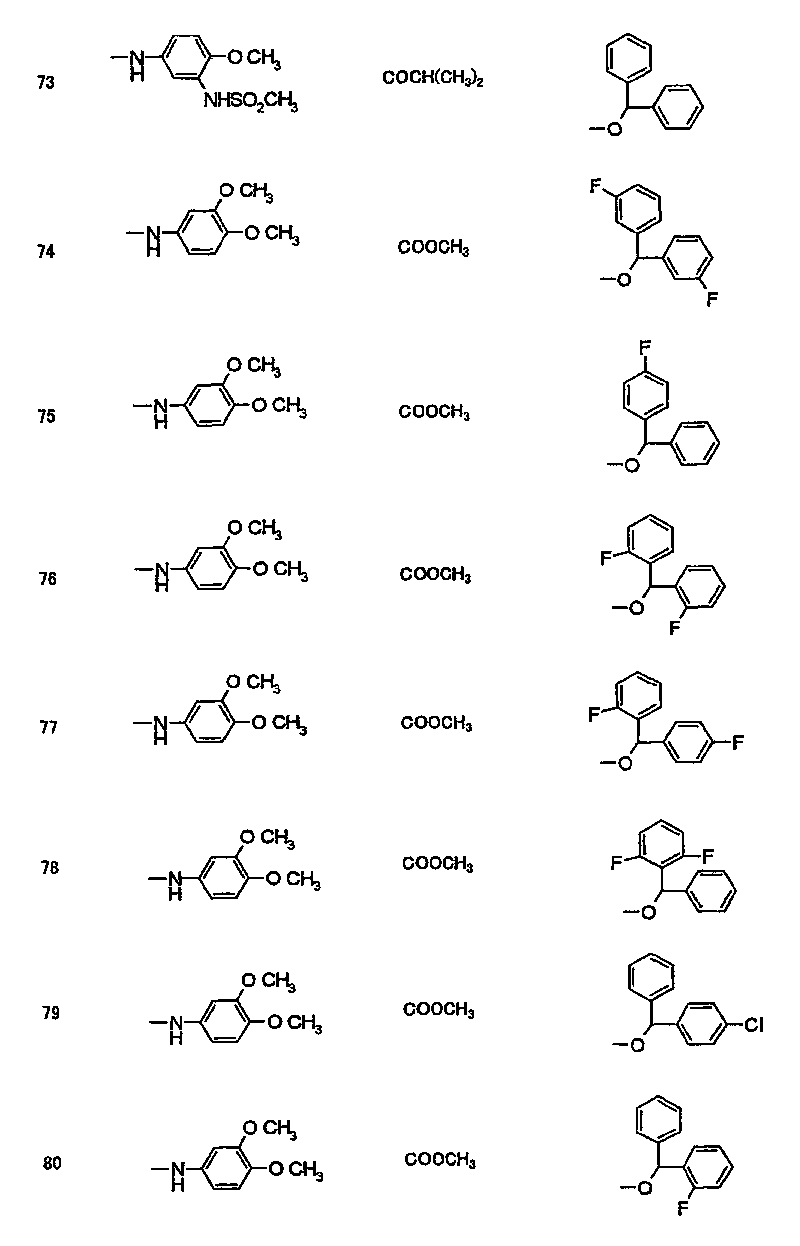

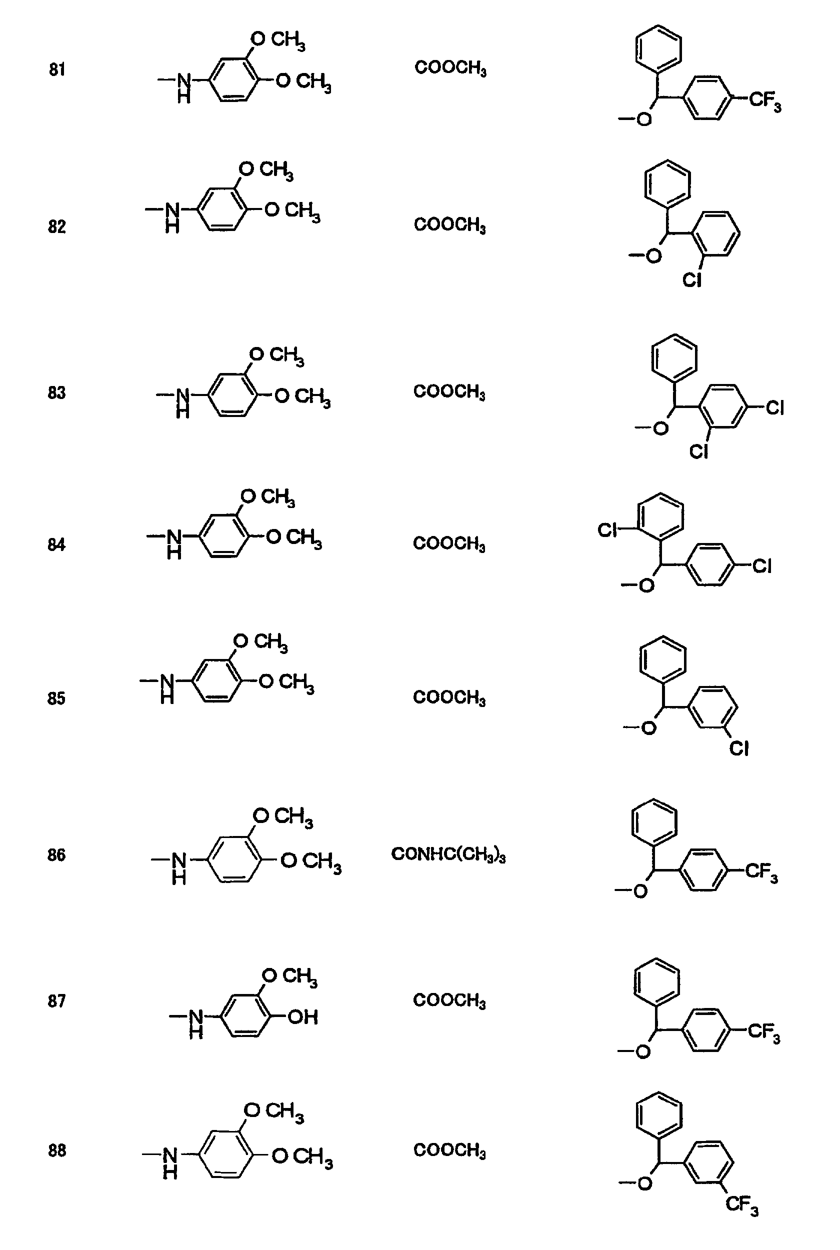

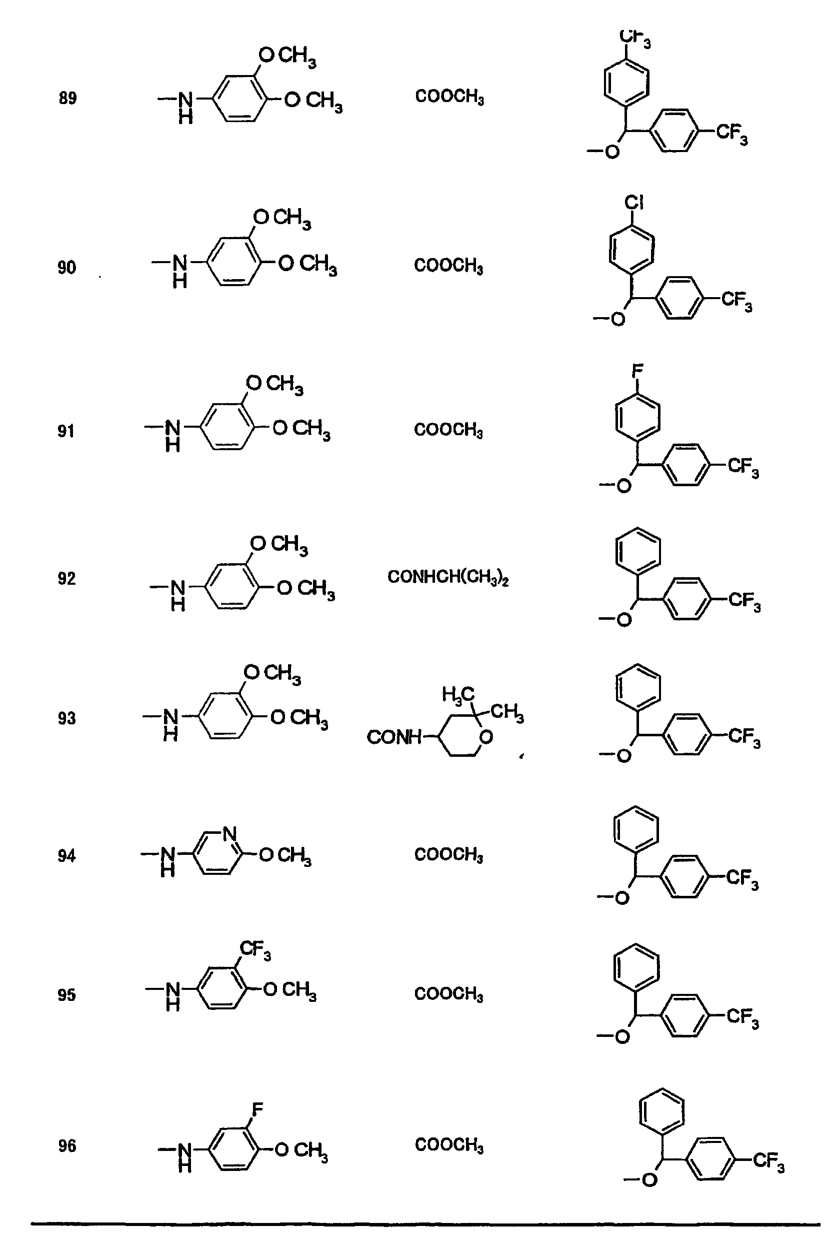

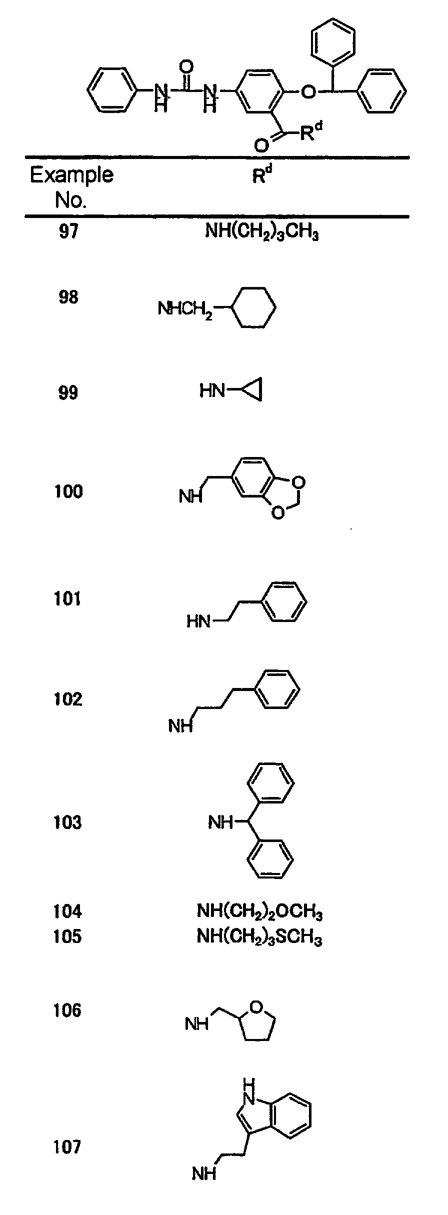

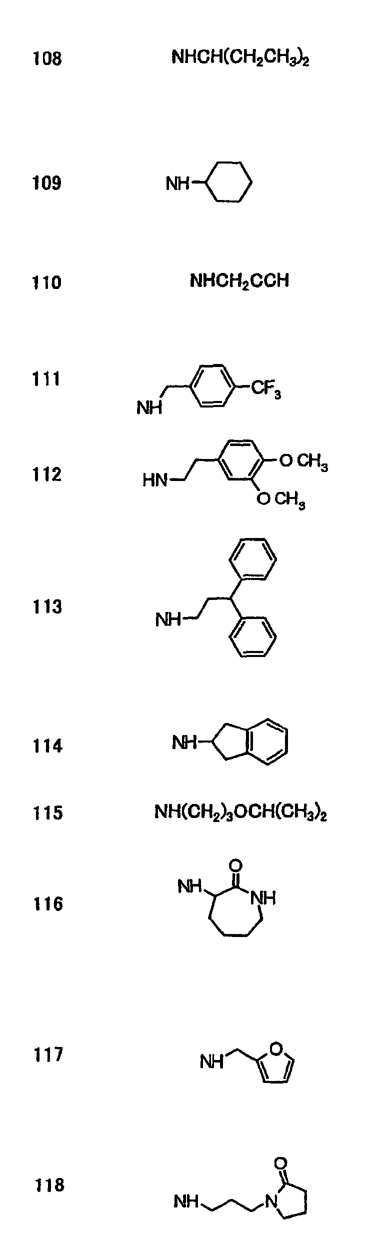

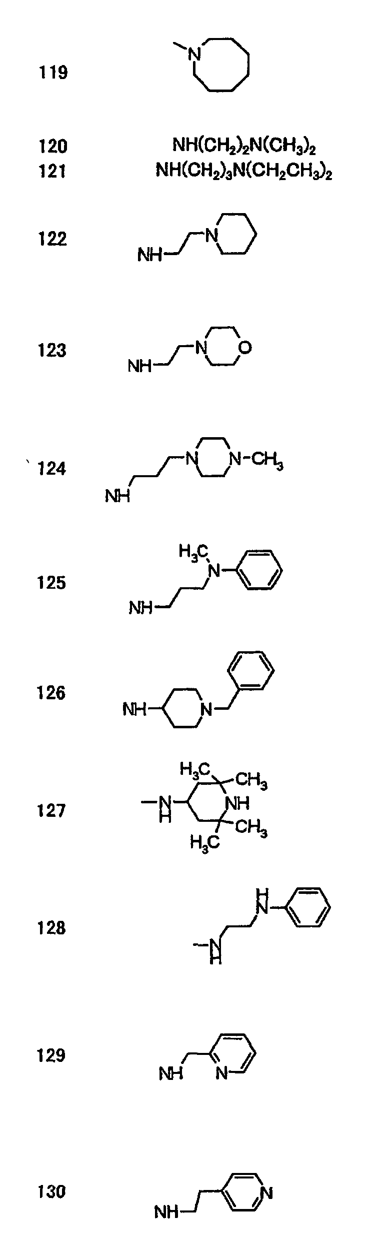

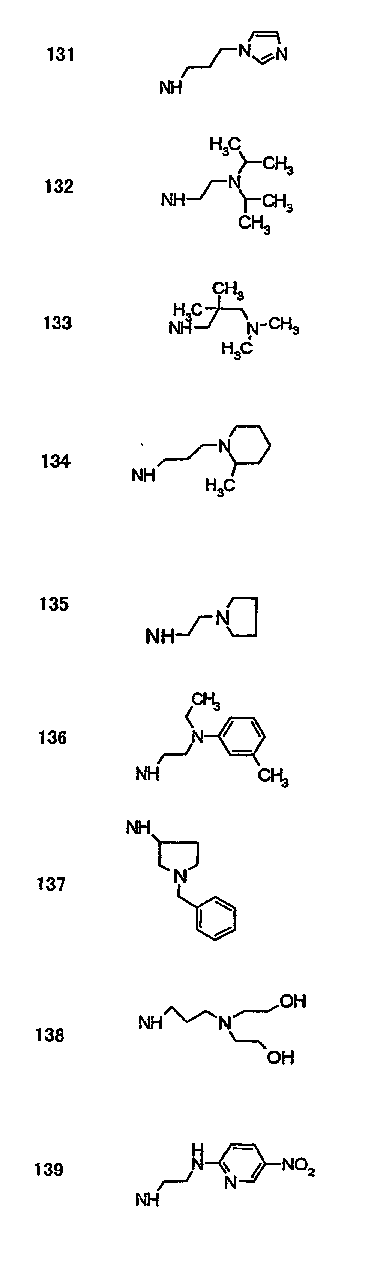

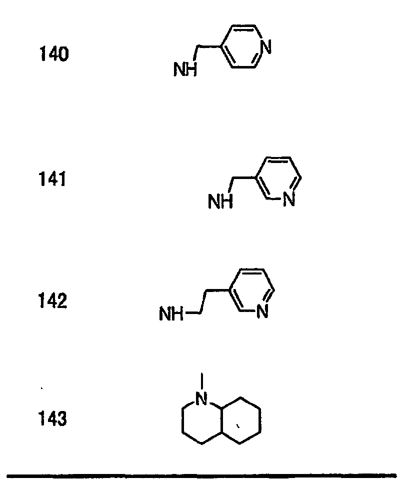

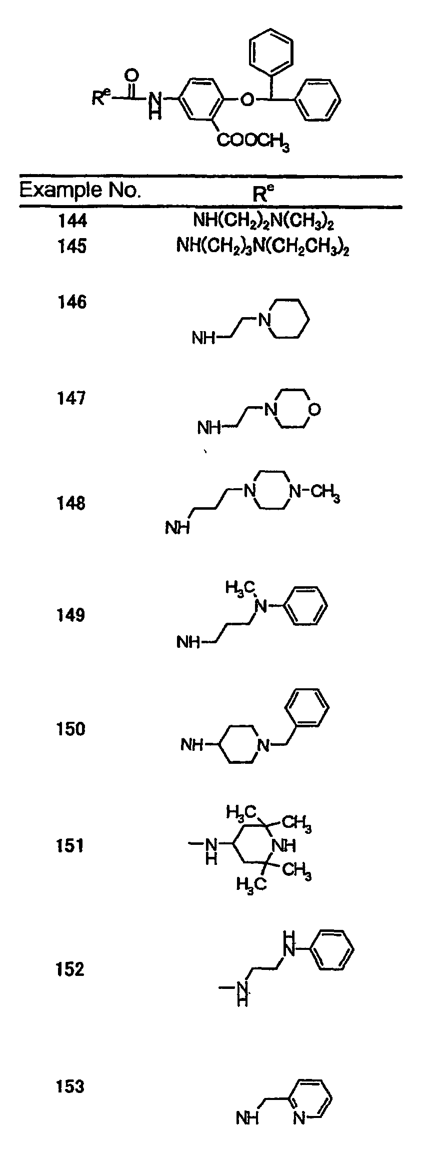

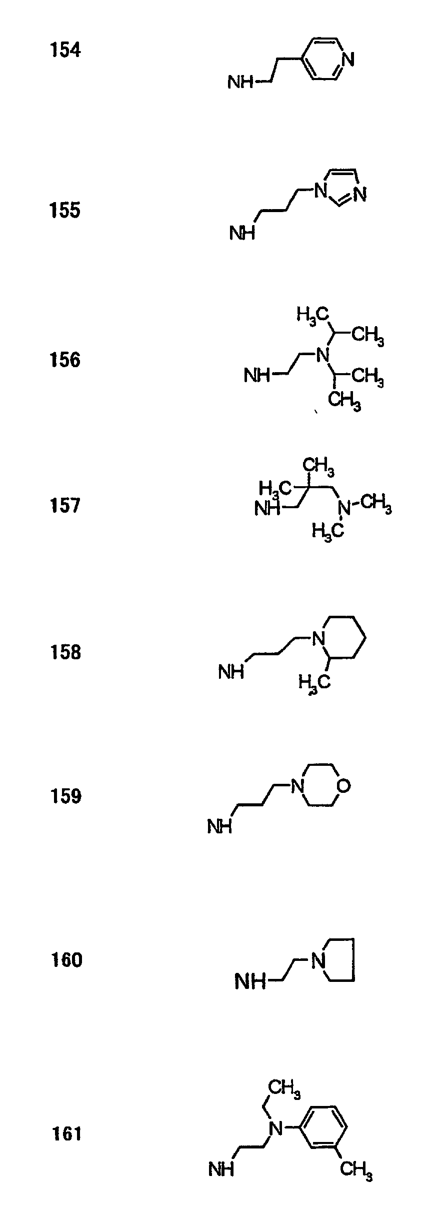

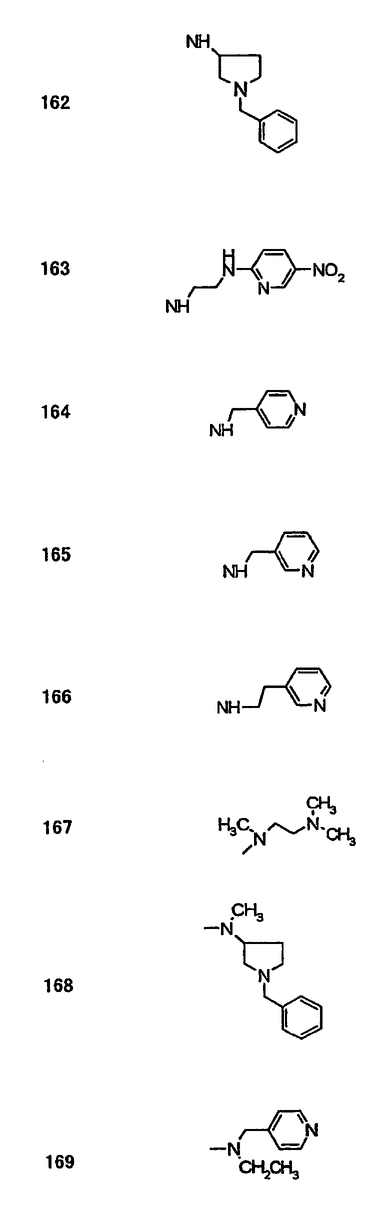

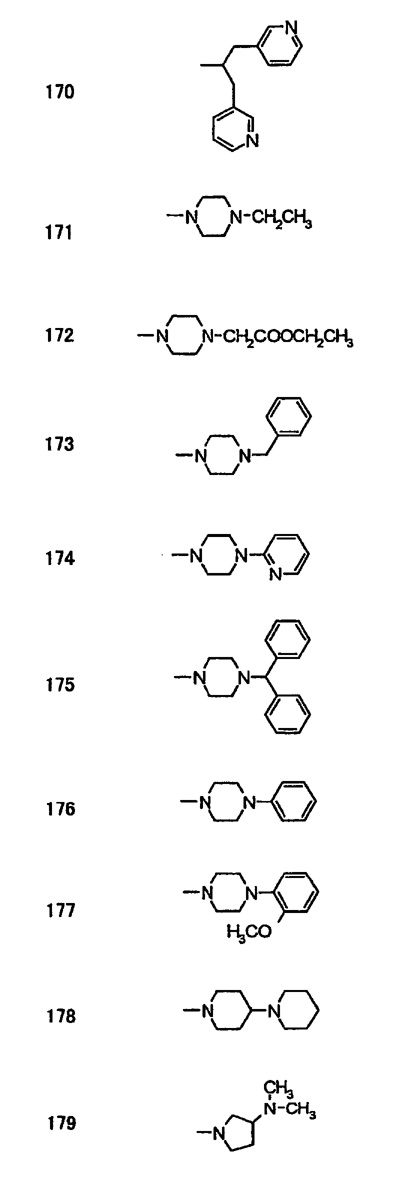

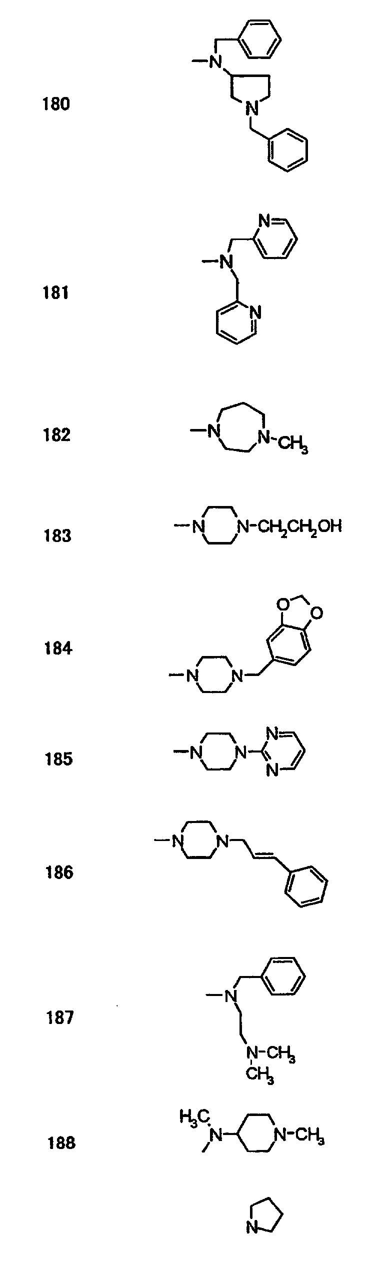

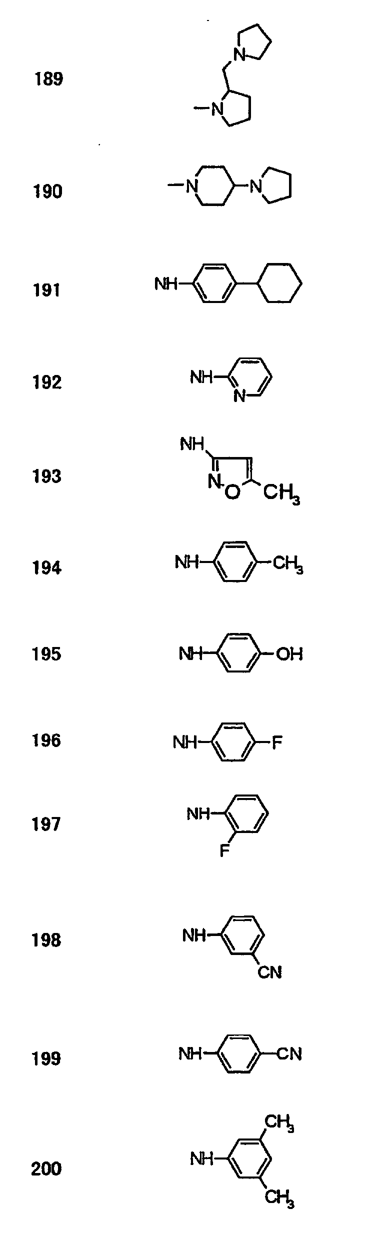

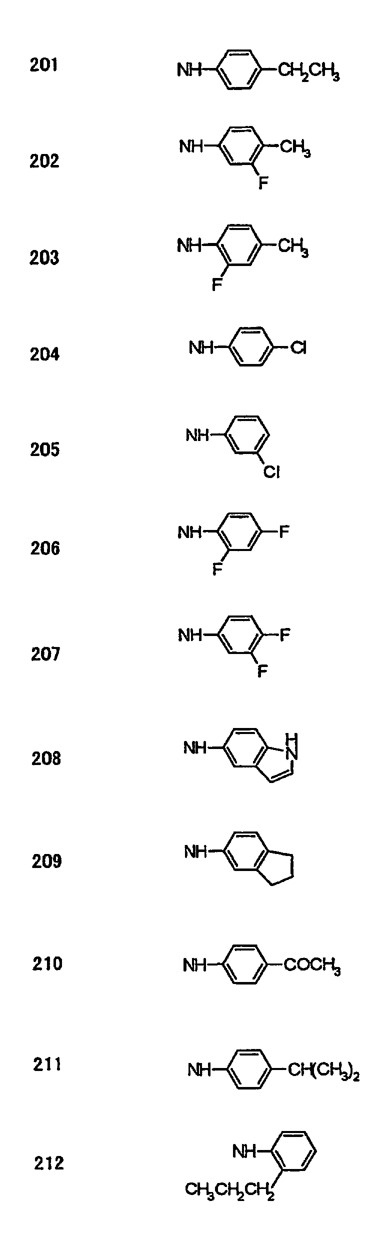

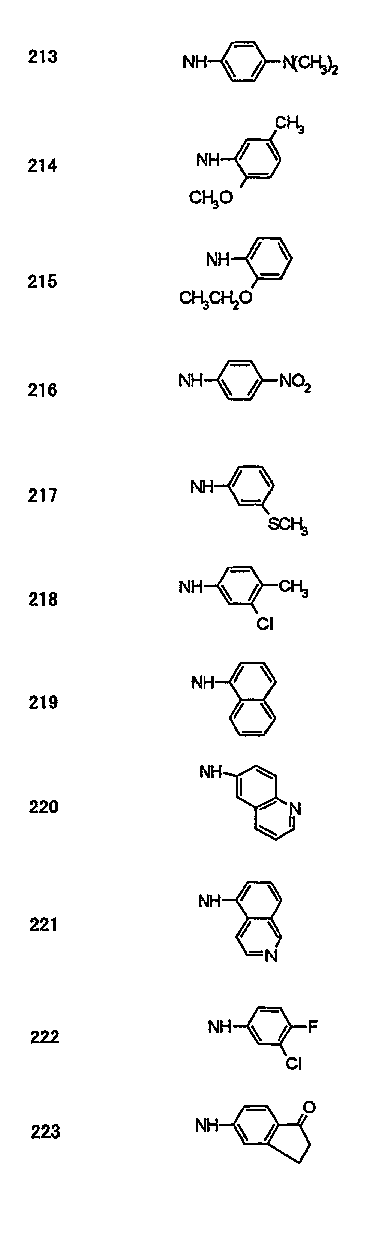

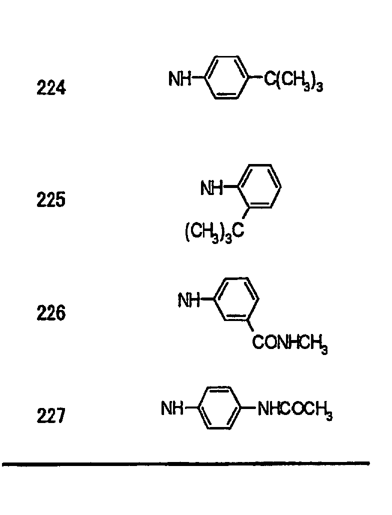

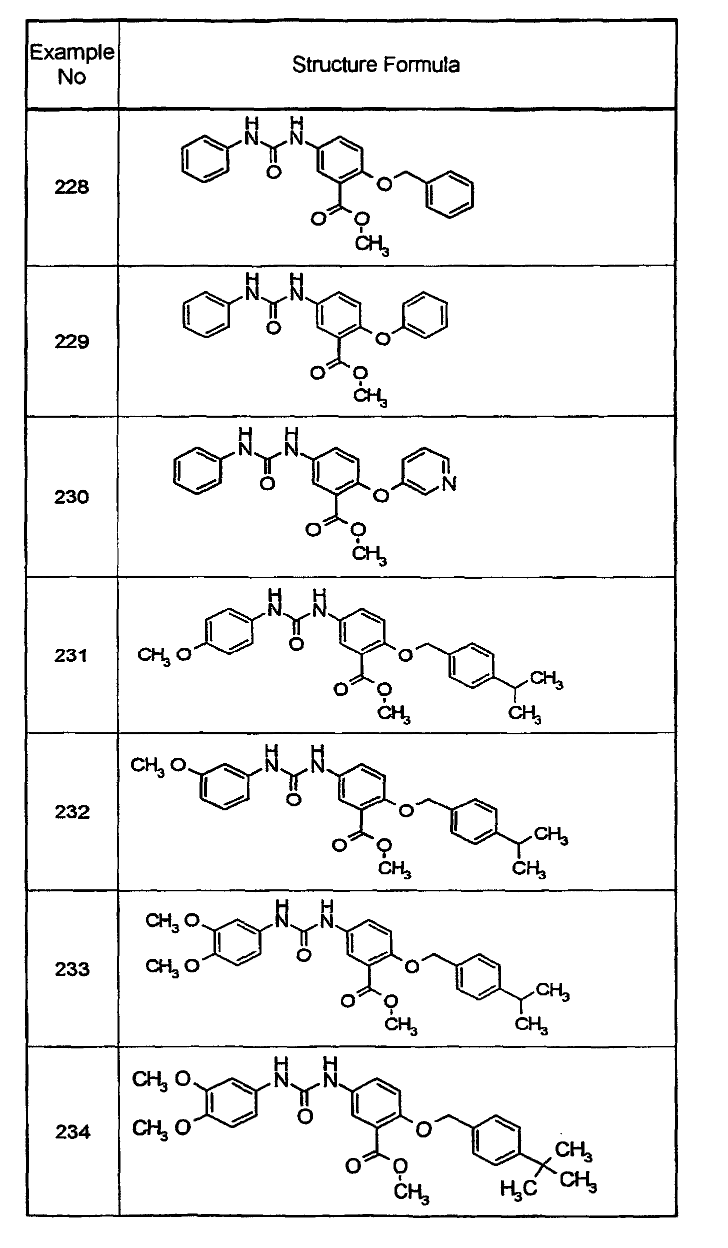

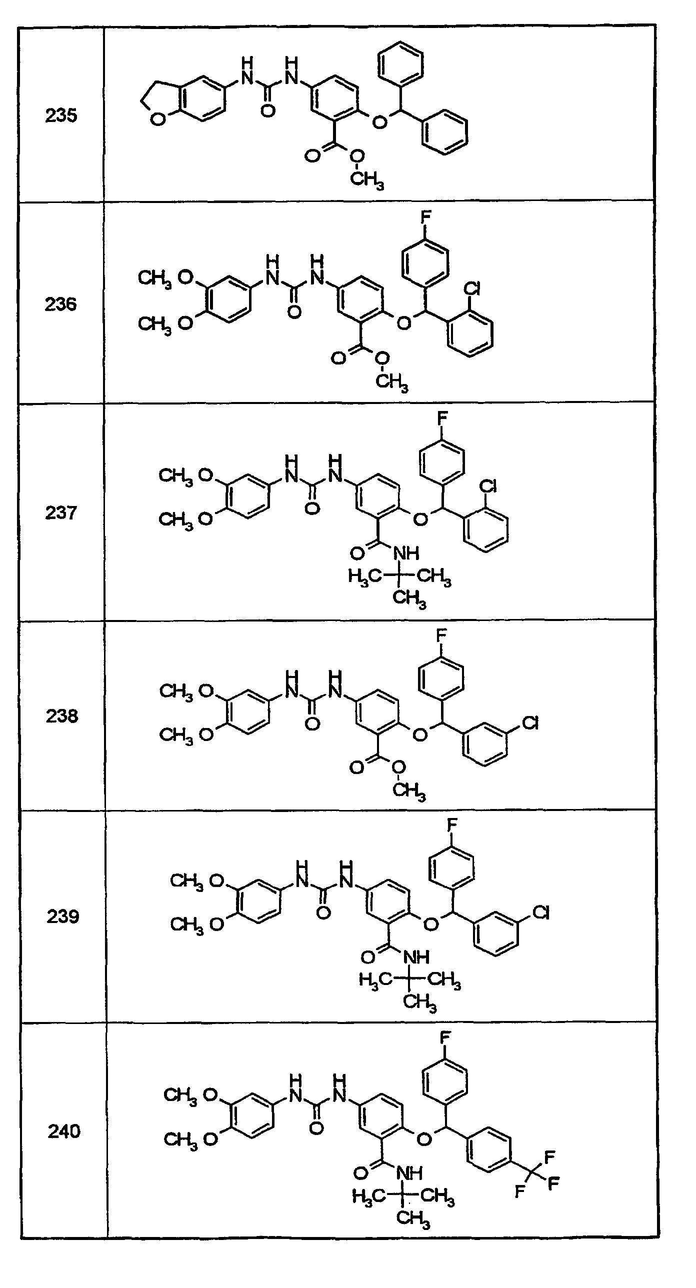

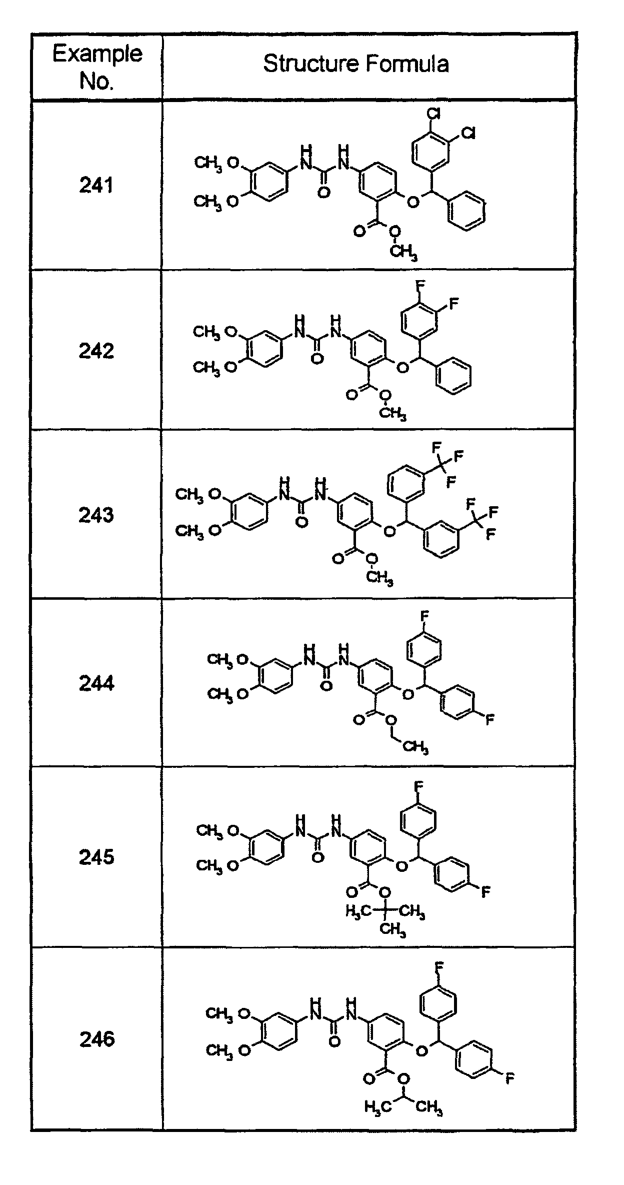

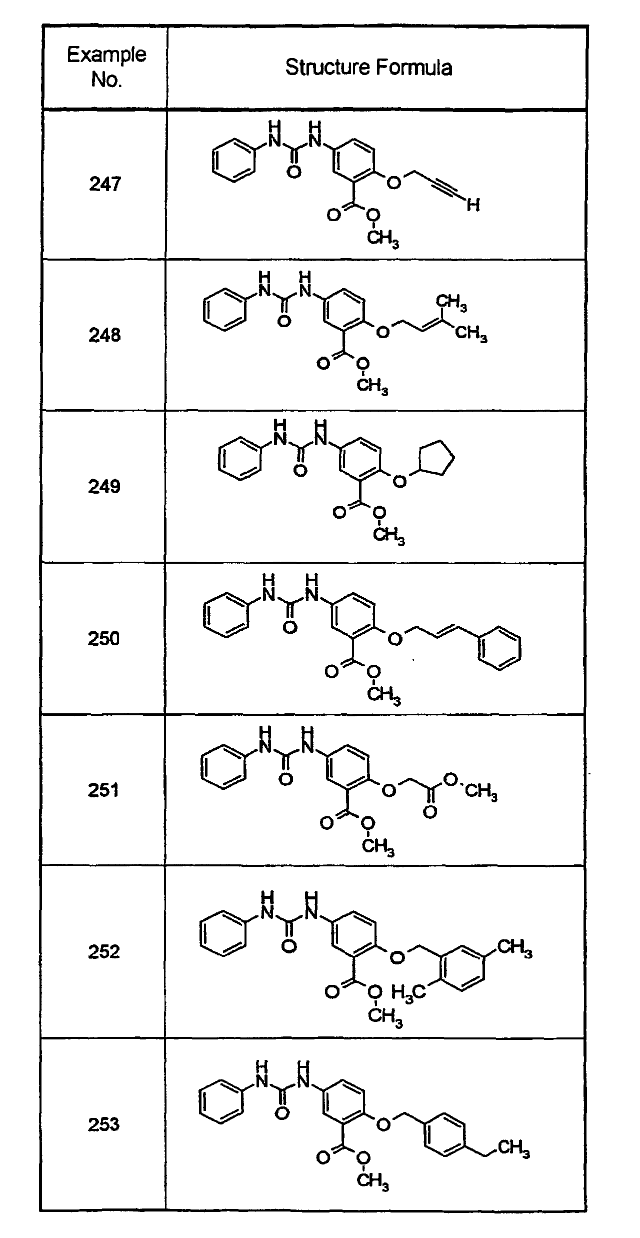

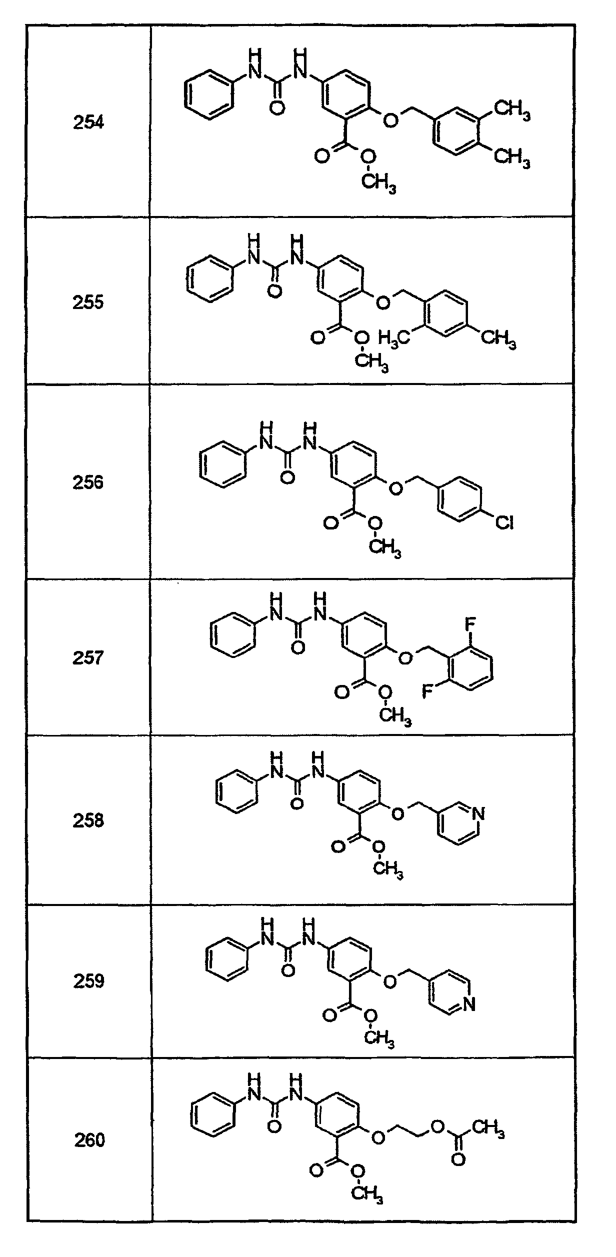

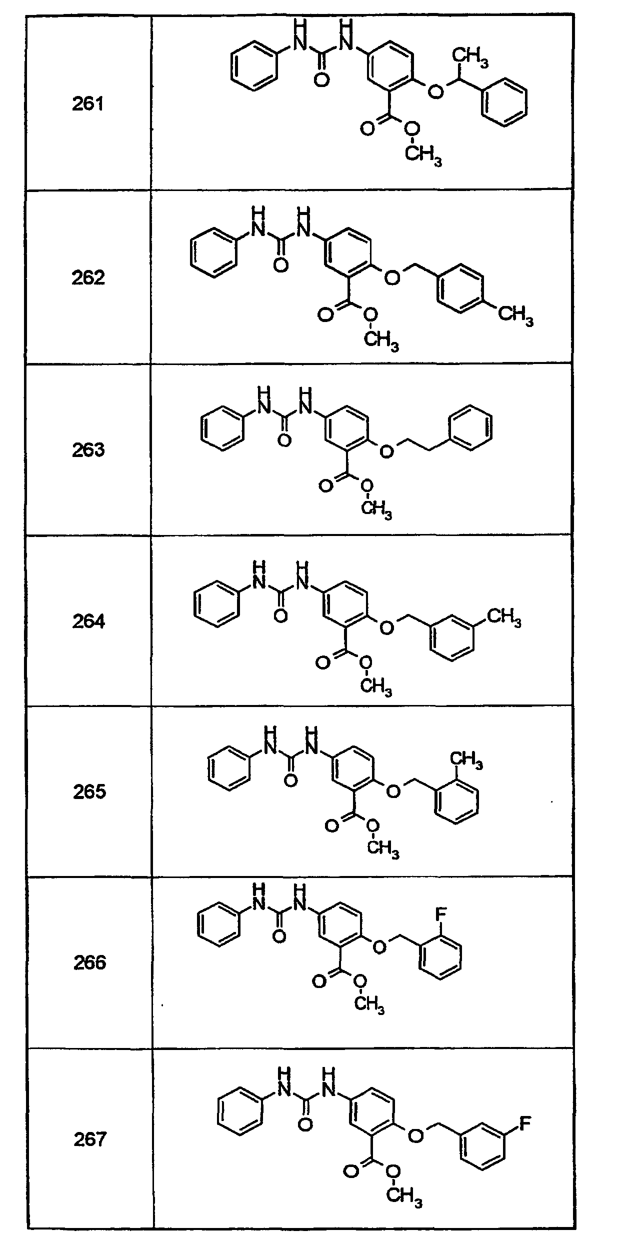

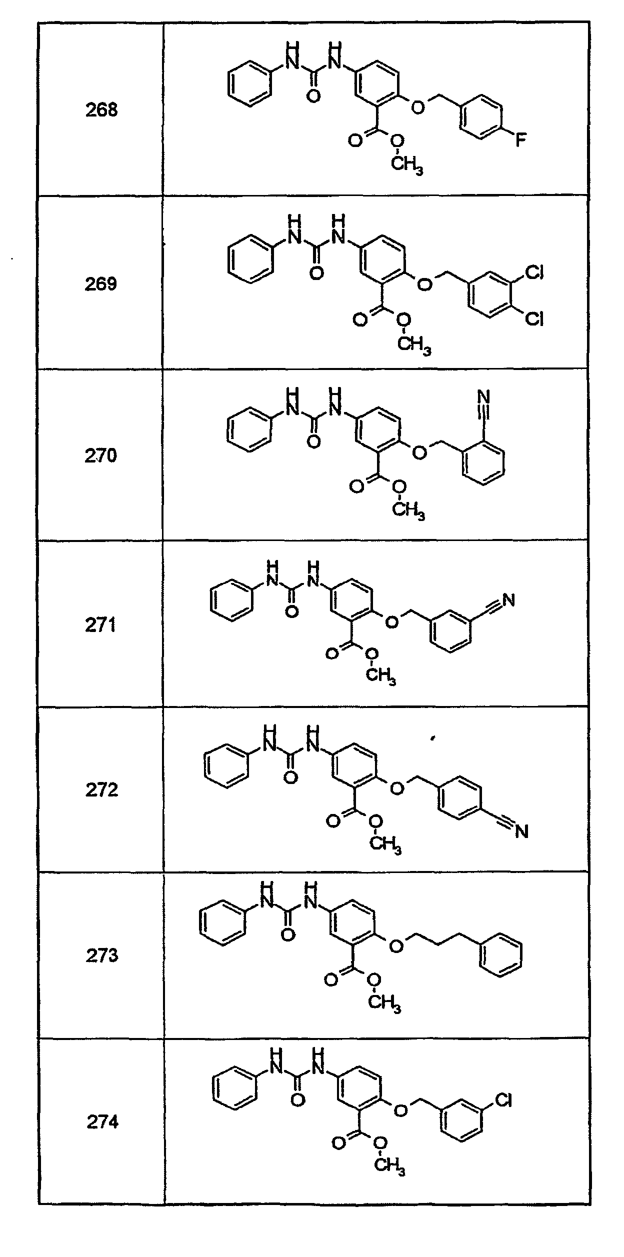

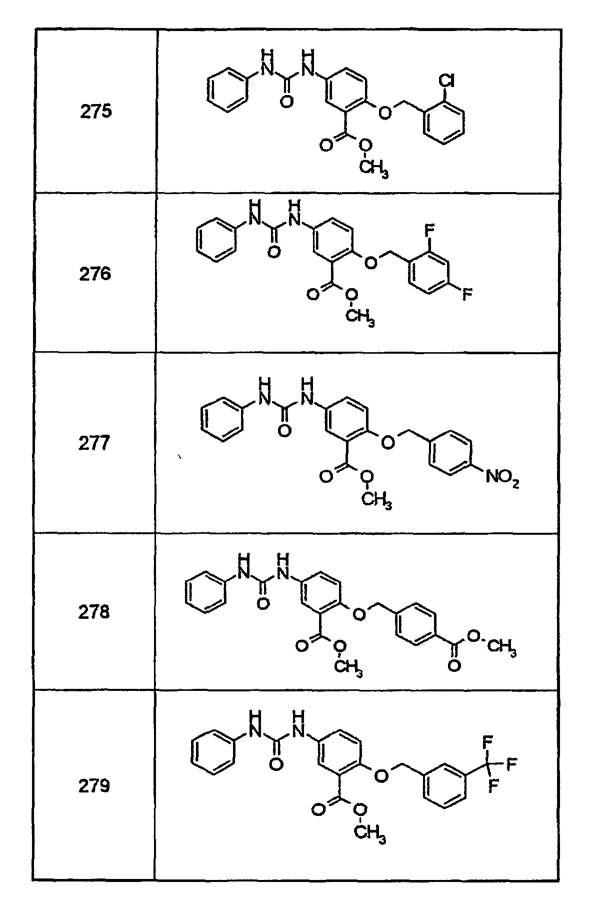

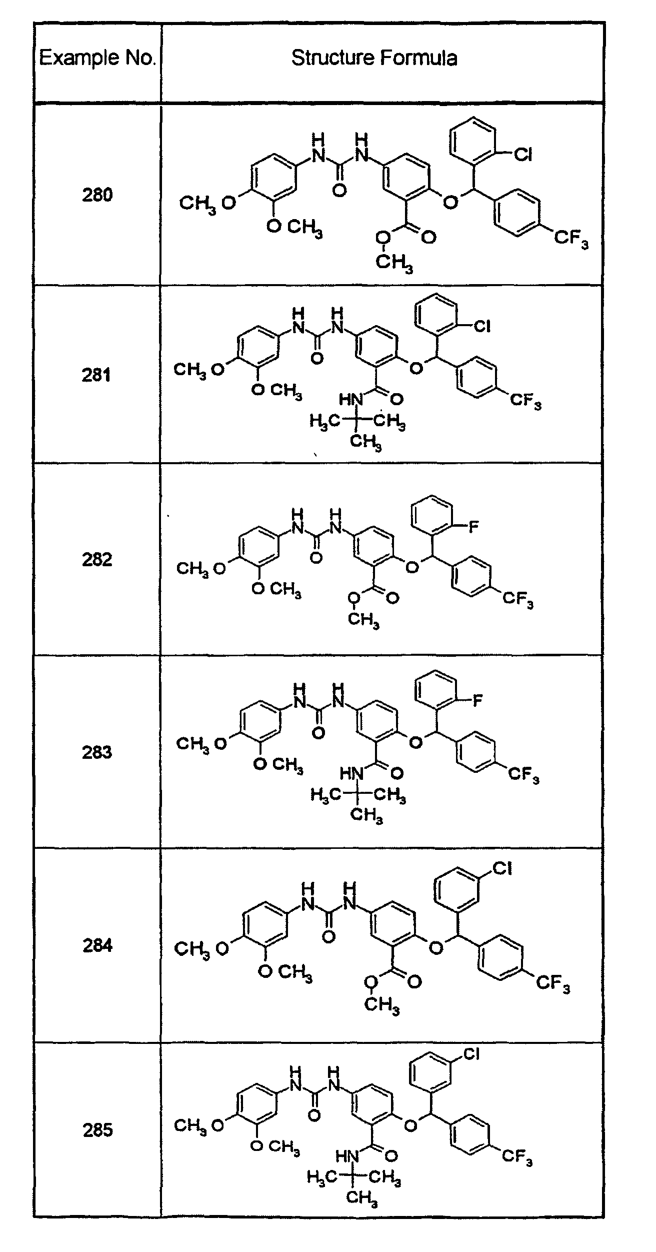

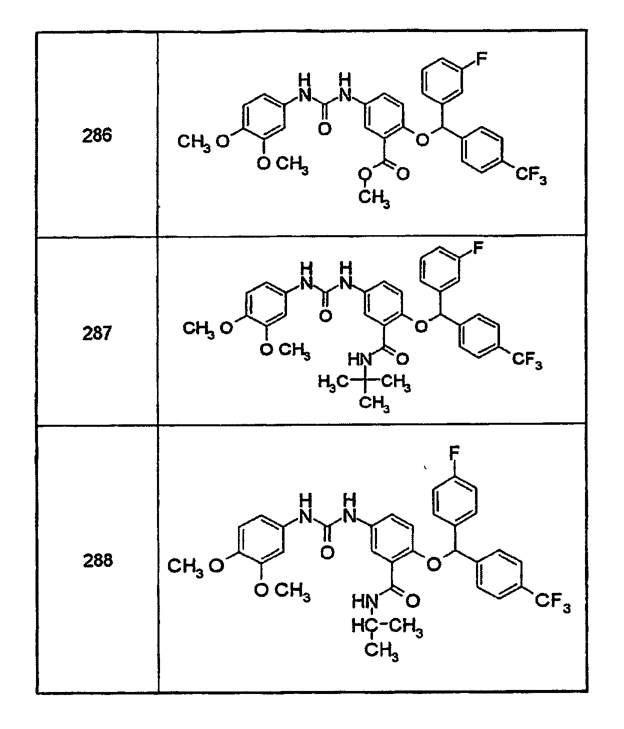

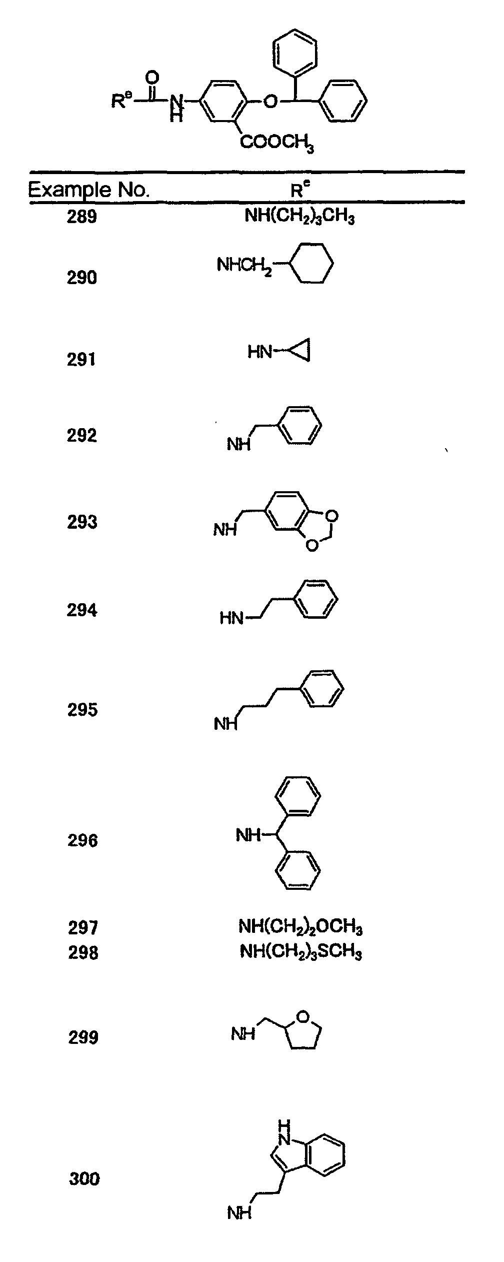

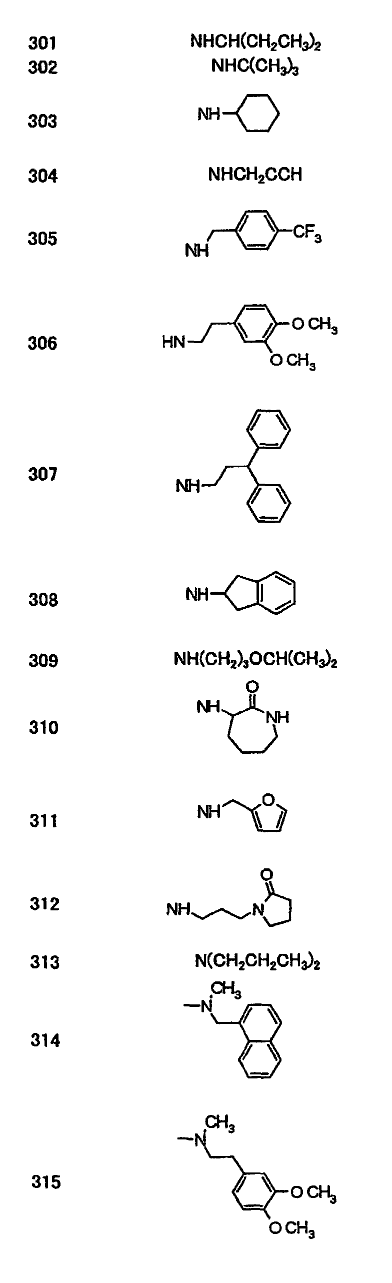

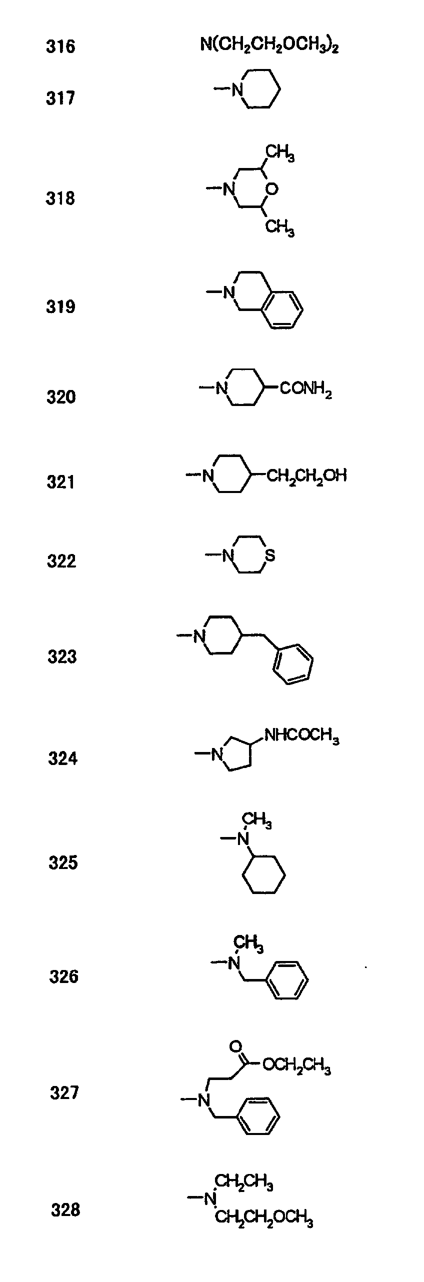

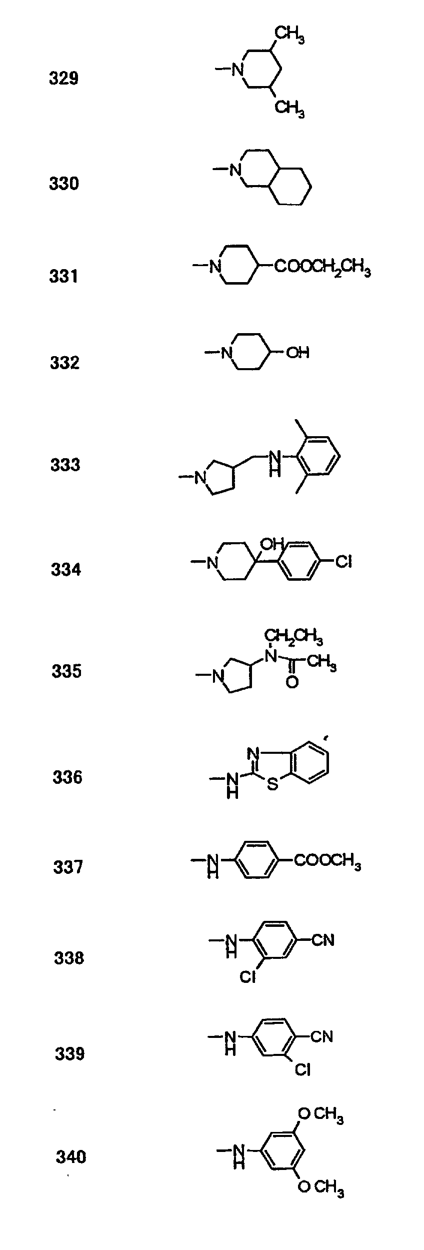

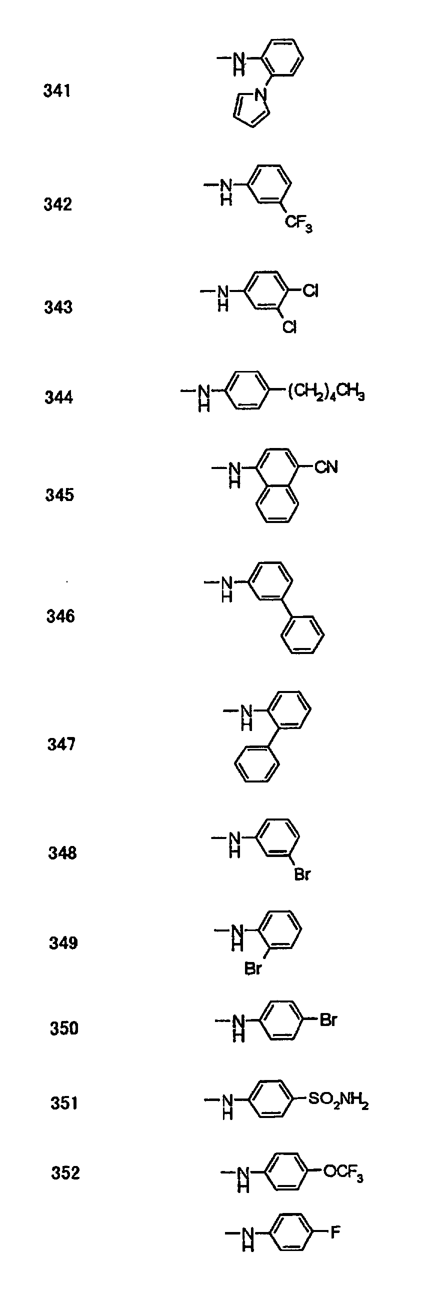

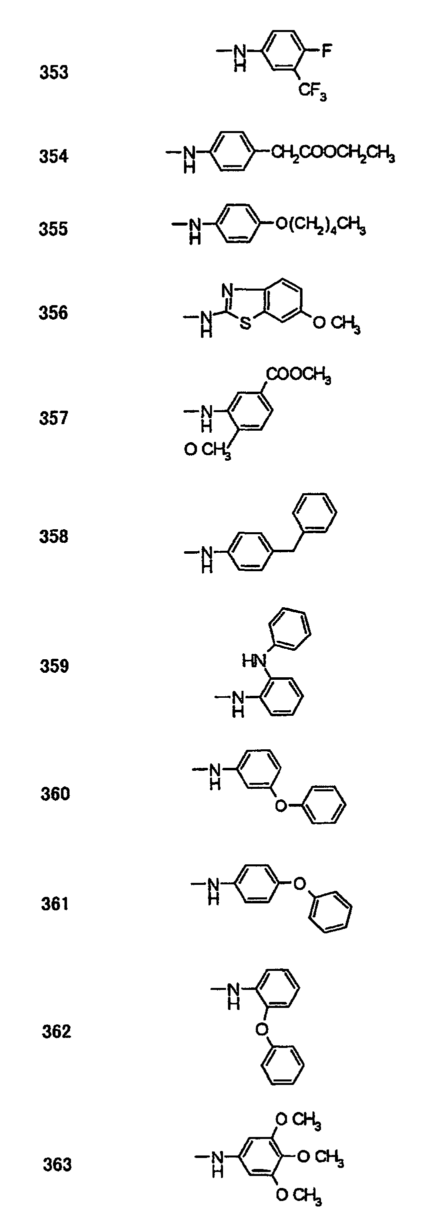

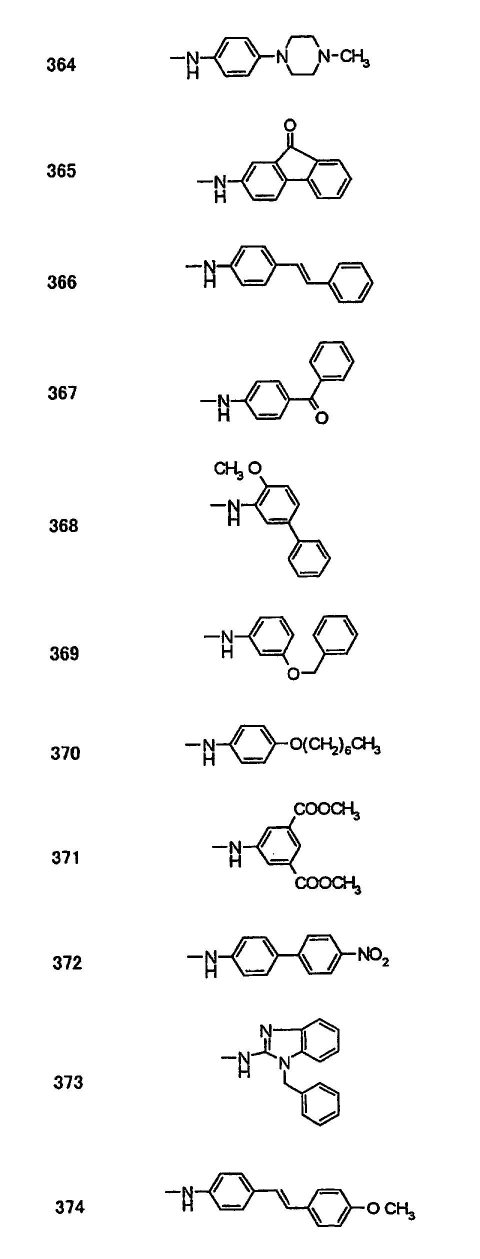

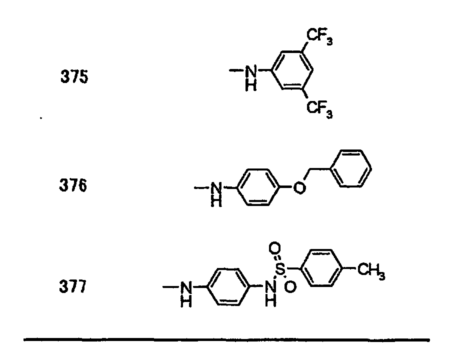

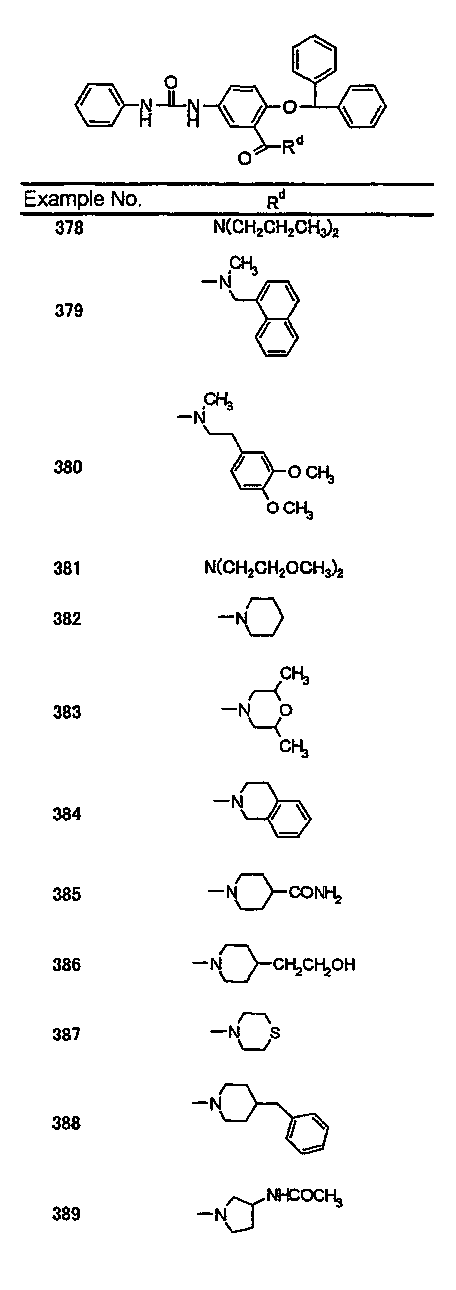

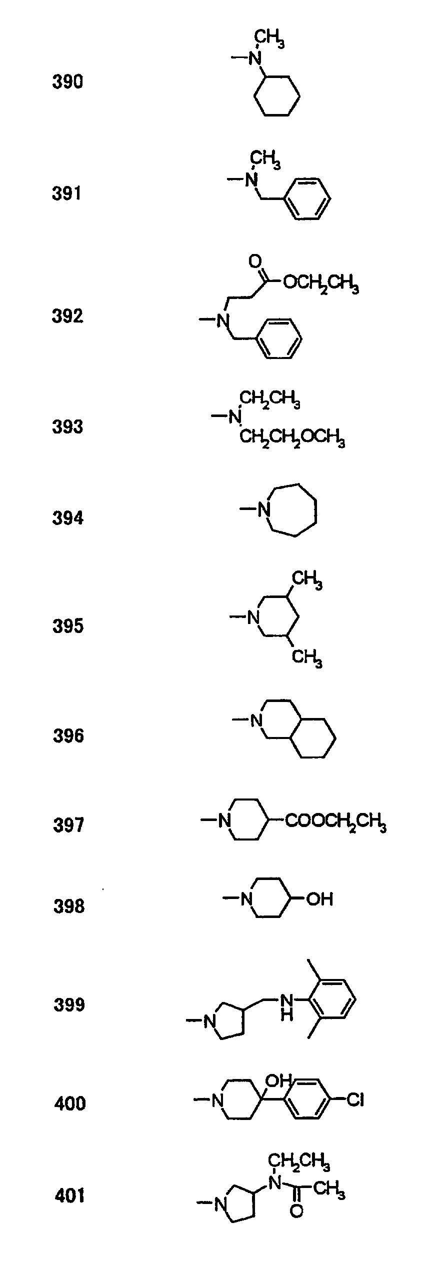

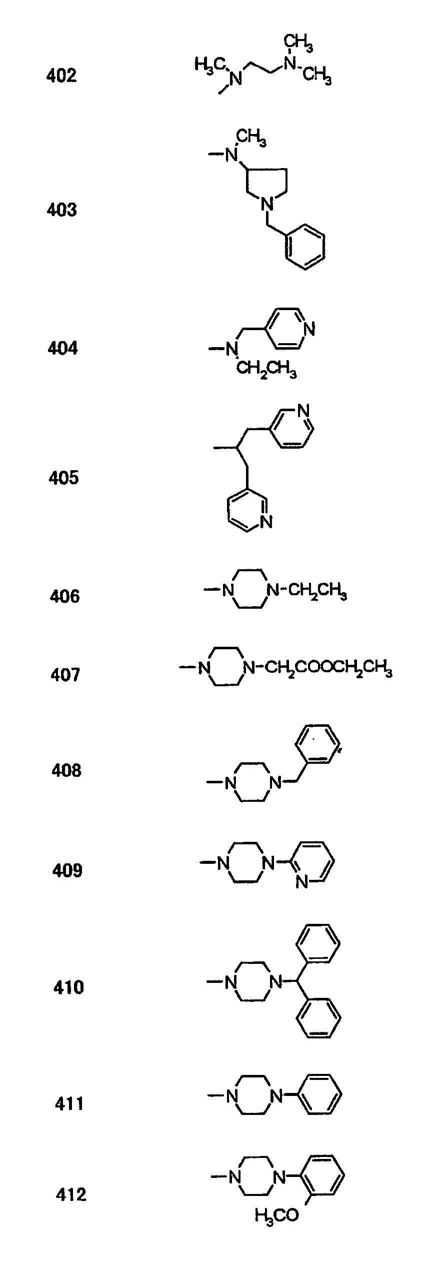

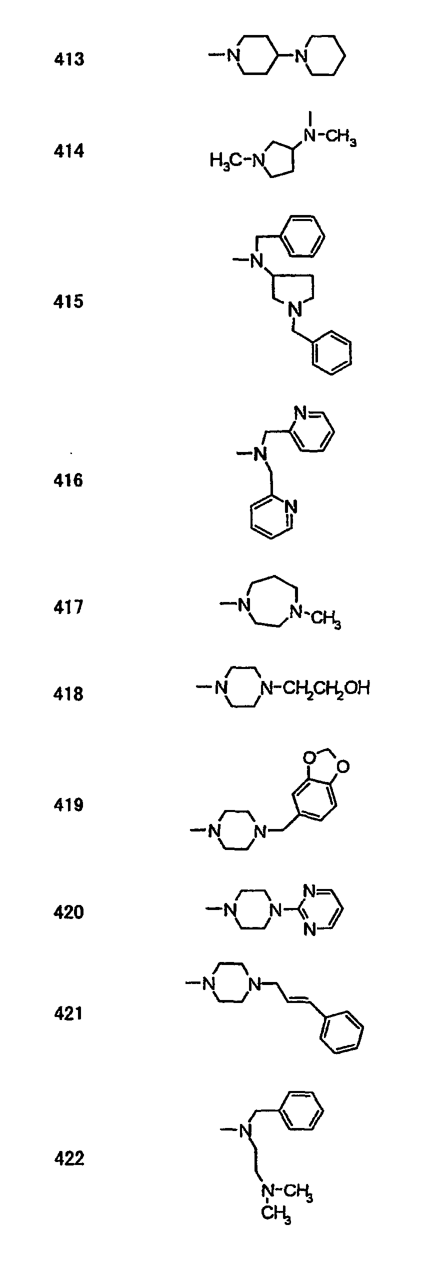

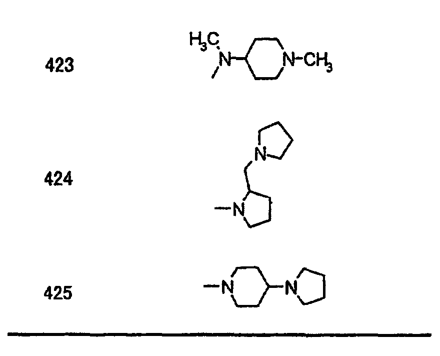

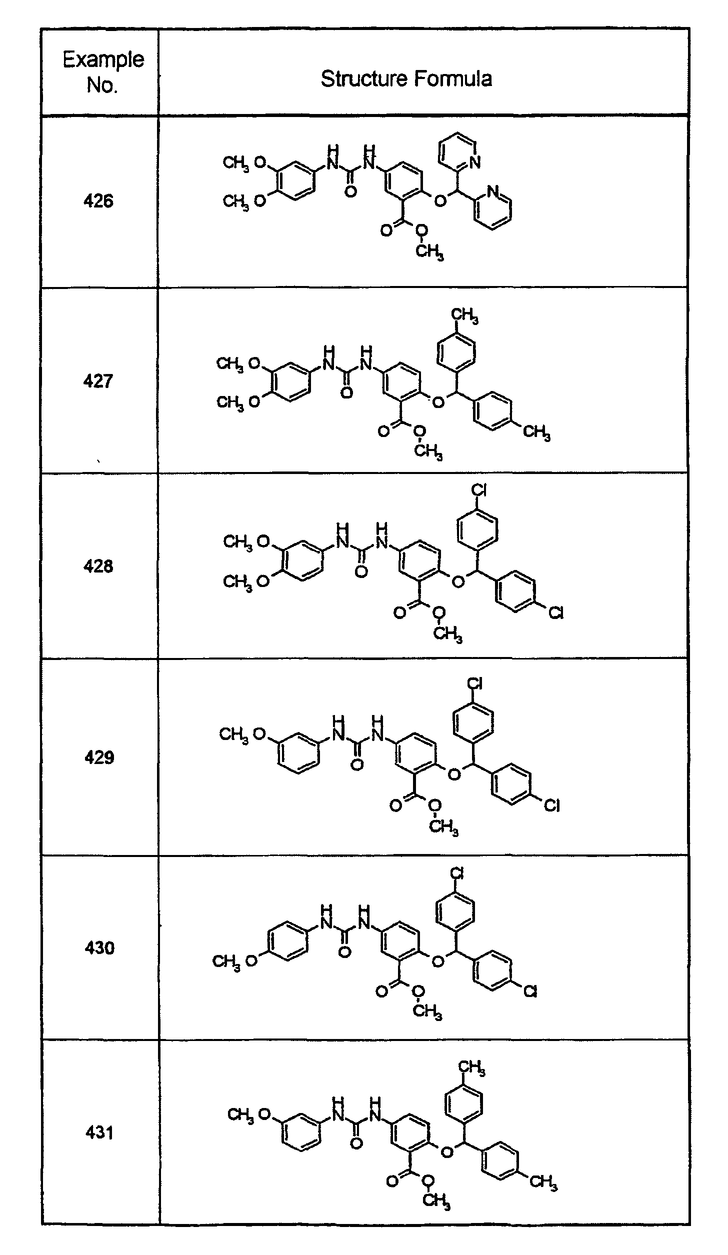

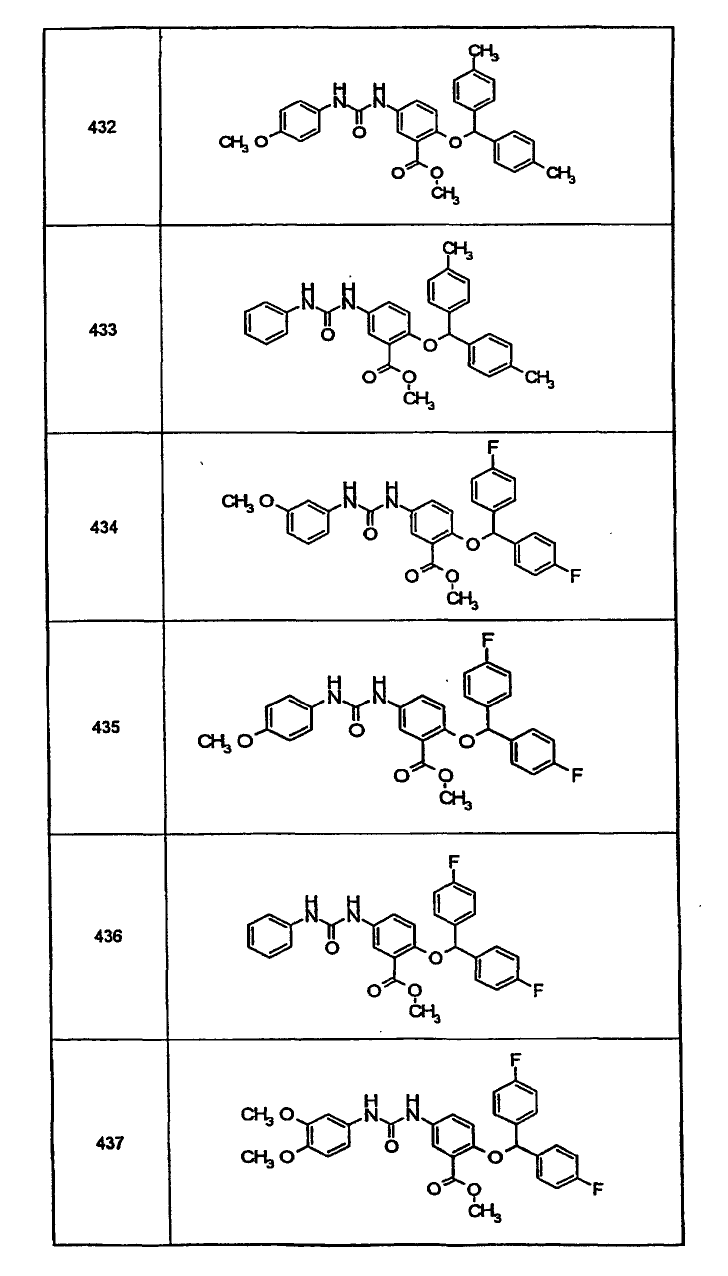

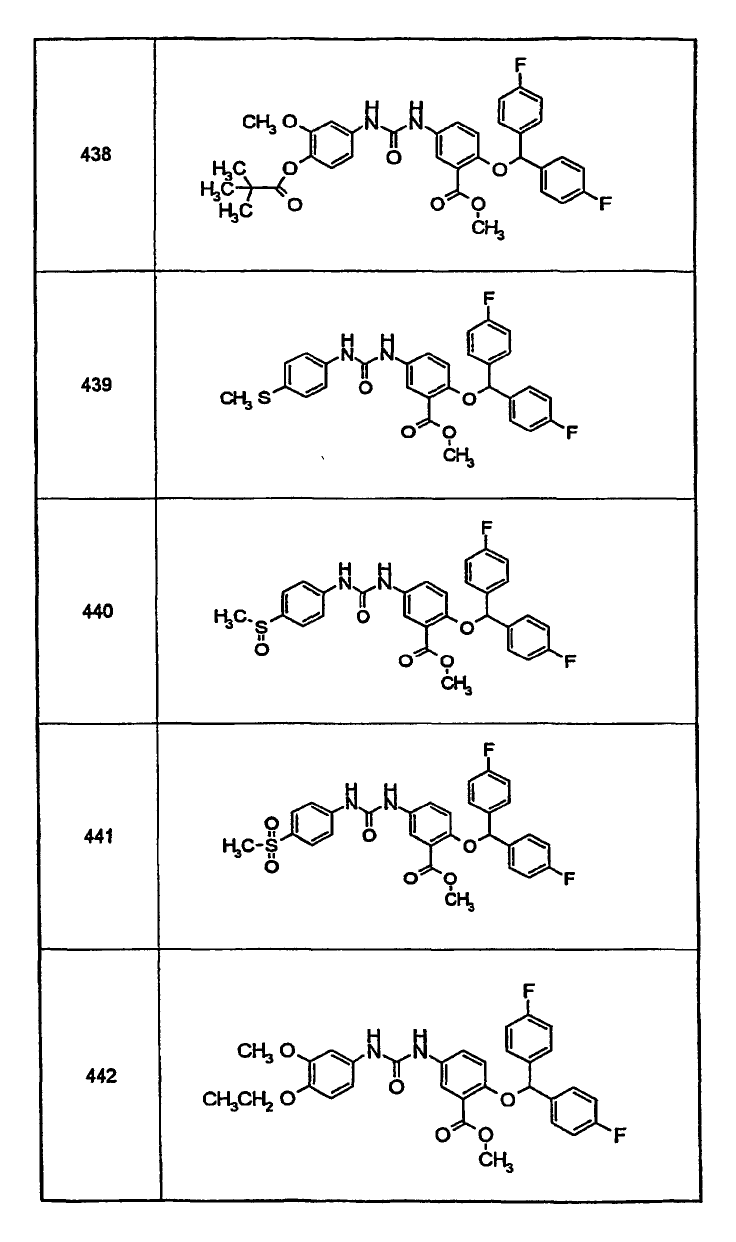

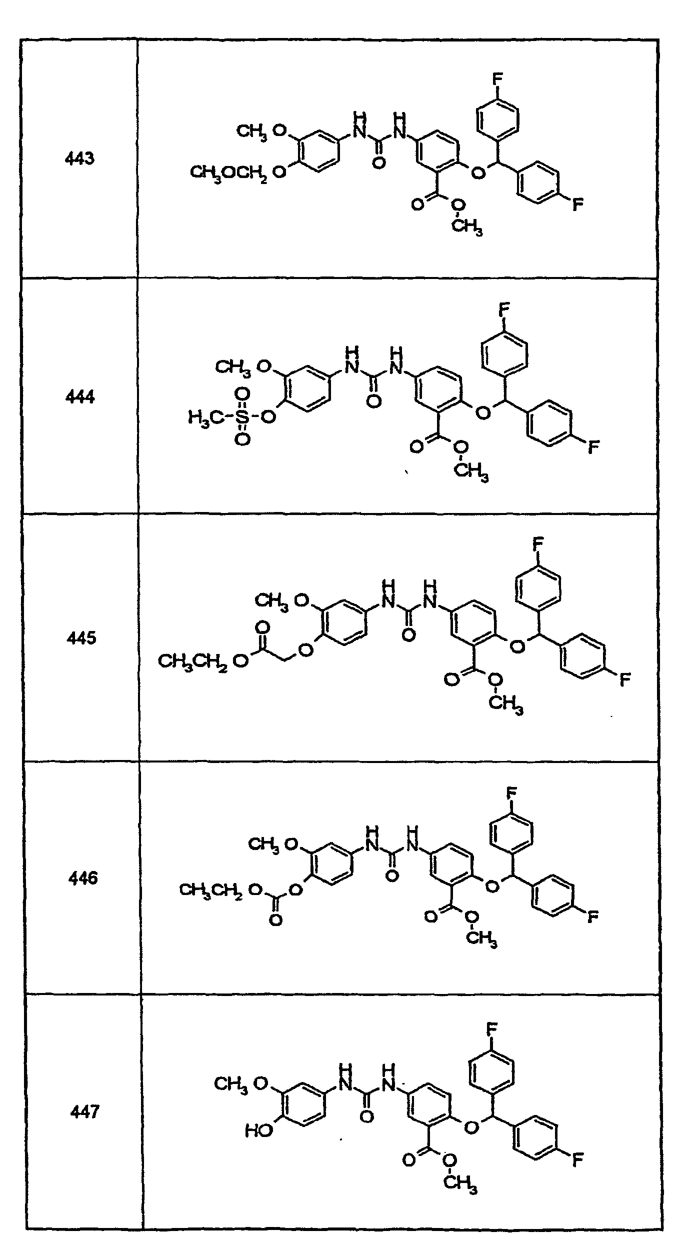

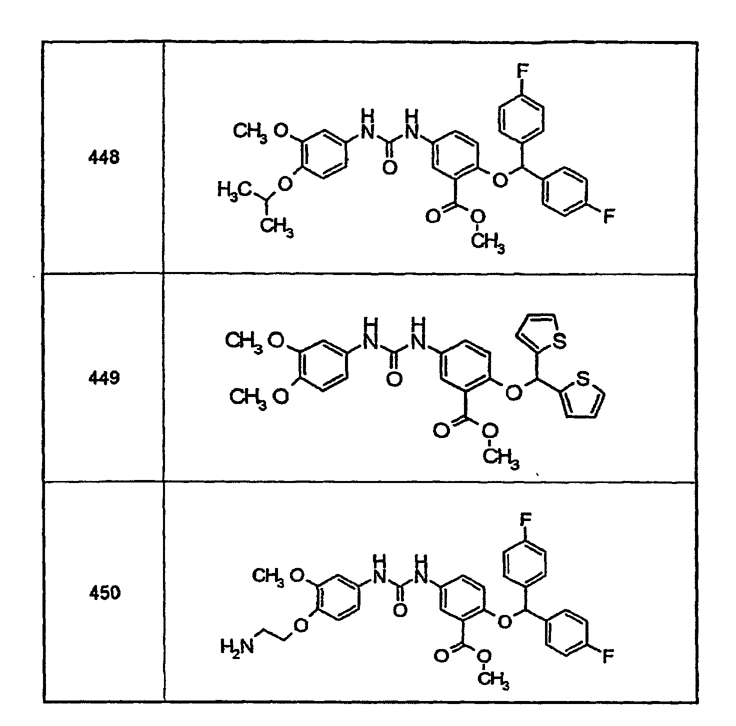

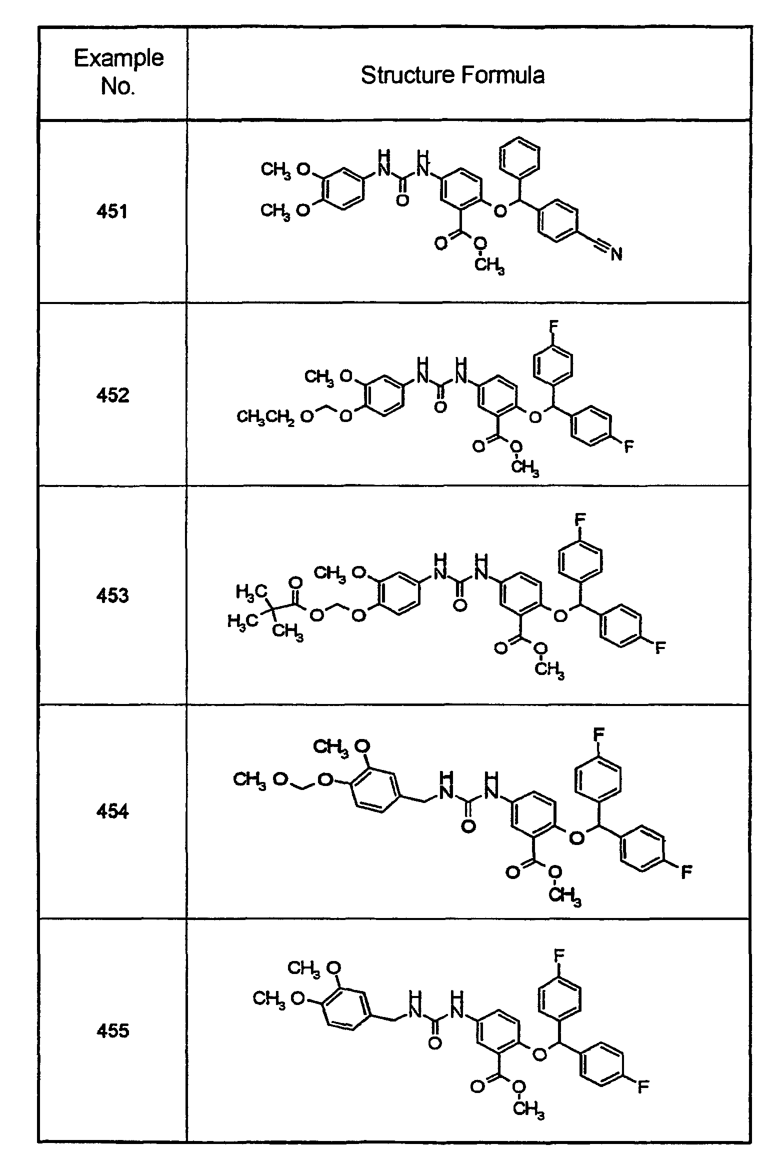

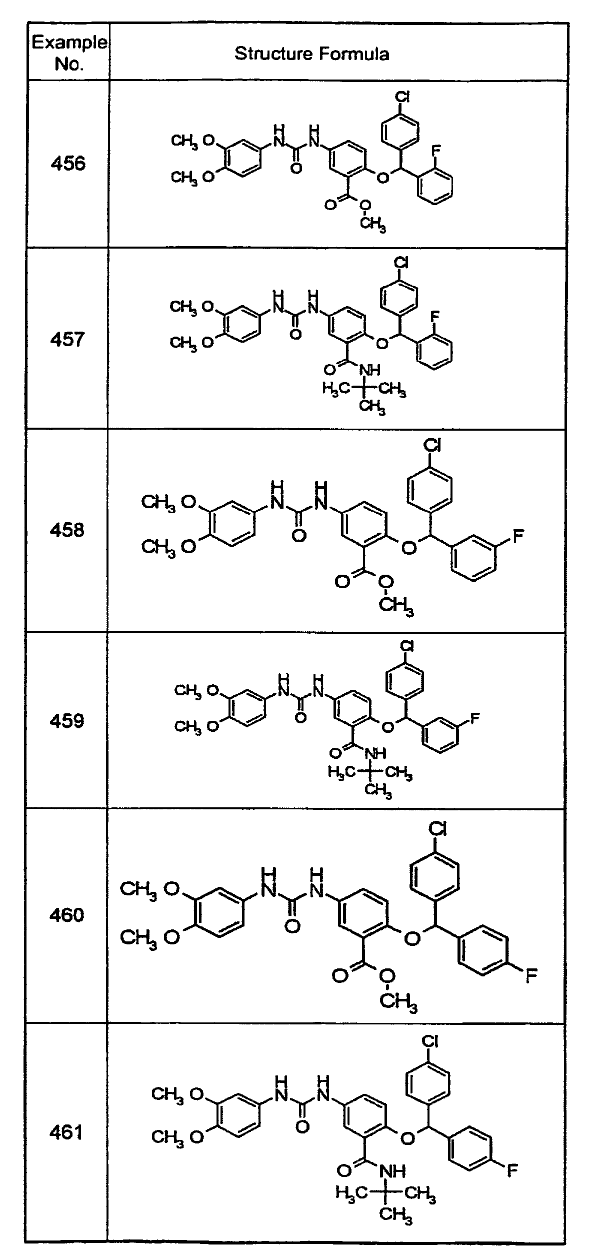

### Test Example 1

### (1) Cloning of Rat VR1

Cloning of VR1 gene was conducted by PCR method from rat brain cDNA. Using 0.5 ng of rat brain cDNA (Takara Shuzo Co., Ltd.) as a template, PCR reaction was carried out in Gene Amp PCR System 9700 (Applied Biosystems) using KON DNA Polymerase (Toyobo Co., Ltd.) (reaction conditions: 35 cycles of treatments at 95°C for 30 seconds, at 55°C for 30 seconds and at 72°C for 2 minutes) by adding 50 pmol of primers, 5'-GGGGAATTCGCCACCATGGAACAACGGGCTAGCTTA-3' and 5'-GGGGCGGCCGCTTATTTCTCCCCTGGGACCATGGAATCCTT-3', respectively, which were prepared by referring to the base sequence of the VR1 gene reported by Caterina, M.J. et al. (Nature 389 (6653):816-24 (1997)).

### (2) Preparation of Plasmid for Expression of Rat VR1

The PCR fragment obtained above was digested with restriction enzymes EcoRI (Takara Shuzo Co., Ltd.) and NotI (Takara Shuzo Co., Ltd.), and subjected to agarose gel electrophoresis to collect DNA fragment of 2.5kb. The DNA fragment and a plasmid pMSR α neo (WO 00/44756) for expression in animal cells, which was previously digested with EcoRI and NotI, were mixed and ligated by DNA Ligation Kit Ver. 2 (Takara Shuzo Co., Ltd.). Transformation of E. coli JM109 competent cells gave plasmid pRVR1.

### (3) Introduction of the Plasmid for Expression of Rat VR1 into CHO-K1 Cells and Expression thereof

CHO-K1 cells (ATCC No.: CCL-61) were grown in a 150 cm² of cell culture flask (Corning Coaster) using Ham's F12 medium (GIBCO BRL) containing 10% fetal calf serum (GIBCO BRL) and were collected from the flask by using 0.5 g/L trypsin-0.2 g/L EDTA (GIBCO BRL). The cells were washed with PBS (GIBCO BRL), centrifuged (1000 rpm, 5 minutes) and suspended in PBS. Next, the DNA was introduced into the cells using Gene Pulser (Bio-Rad Laboratories Inc.) under the following conditions. Namely, 1 × 10⁷ cells and 15 µg of plasmid pRVR1 for expression of rat VR1 were added into a cuvette having a 0.4 cm of gap, and electroporation was carried out under 0.25 kV of electric voltage and 960 µF of capacitance. Subsequently, the cells were transferred into Ham's F12 medium containing 10% fetal calf serum, and incubated for 24 hours. The cells were then collected, centrifuged and suspended in Ham's F12 medium containing 10% fetal calf serum and Geneticin (GIBCO BRL) at a concentration of 500 µg/ml. The suspension of cells was diluted to a concentration of 10⁴ cells/mL, and inoculated on 96-well plates (Corning Coaster) to obtain Geneticin-resistant strains.

Subsequently, the resulting Geneticin-resistant strains were cultured in the 96-well plates (Corning Coaster). Next, after the medium was removed by suction, 100 µl of an assay buffer (1 mM CaCl₂, HBSS W/O sodium bicarbonate (GIBCO BRL), 0.5% BSA and 20 mM HEPES (Dojindo Molecular Technologies, Inc.), pH 7.5) was added to each well, and the cells were washed twice. Subsequently, 100 µl of the assay buffer containing 2.5 µCi/ml of ⁴⁵Ca (Daiichi Pure Chemicals) and 1 µM of capsaicin (Wako Pure Chemical Industries, Ltd.) was added to each well and the reaction was carried out for 30 minutes. After the assay buffer was removed by suction, the well plate containing the cells were washed twice with 100 µl of ice-cooled PBS (GIBCO BRL), and to each well was added 150 µl of MicroScint-20 (Packard Industry Company, Inc.) with stirring. Subsequently, the radioactivity was measured with TopCount (Packard Industry Company, Inc.) to select RVR1/CHO strains of which calcium concentration is increased when capsaicin is added to.

### (4) Cloning of Human VR1

Cloning of VR1 gene was conducted by PCR method from human brain cDNA. Using 0.5ng of human brain cDNA (Clonetech Inc., Quick-Clone cDNA) as the template, PCR reaction was carried out in Gene Amp PCR System 9700 (Applied Biosystems) using KON DNA Polymerase (Toyobo Co., Ltd.) (reaction conditions: 35 cycles of treatments at 95°C for 30 seconds, at 55°C for 30 seconds and at 72°C for 2 minutes), by adding a 50 pmol of primers, 5'-GGGGAATTCGCCACCATGAAGAAATGGAGCAGCACAGACTT-3' and 5'-GGGGCGGCCGCTCACTTCTCCCCGGAAGCGGCAGGACTCTT-3', respectively, which were prepared referring to the base sequence (WO 99/37675) of the VR1 gene reported by Caterina, M.J. et al.

### (5) Preparation of Plasmid for Expression of Human VR1

The PCR fragment obtained above was digested with restriction enzymes EcoRI (Takara Shuzo Co., Ltd.) and NotI (Takara Shuzo Co., Ltd.), and subjected to agarose gel electrophoresis to collect DNA fragment of 2.5kb. The DNA fragment and a plasmid pMSR α neo (WO 00/44756) for expression in animal cells, which was previously digested with EcoRI and NotI, were mixed and ligated by DNA Ligation Kit Ver. 2 (Takara Shuzo Co., Ltd.). The resulting plasmid was subjected to transformation of E. coli JM109 competent cells to obtain plasmid pHVR1.

### (6) Introduction of the Plasmid for Expression of Human VR1 into CHO-K1 Cells and Expression thereof

CHO-K1 cells (ATCC No.: CCL-61) were grown in a 150 cm² of cell culture flask (Corning Coaster) using Ham's F12 medium (GIBCO BRL) containing 10% fetal calf serum (GIBCO BRL) and were collected by using 0.5 g/L trypsin-0.2 g/L EDTA (GIBCO BRL). The cells were washed with PBS (GIBCO BRL), centrifuged (1000 rpm, 5 minutes) and suspended in PBS. Next, DNA was introduced into the cells using Gene Pulser (Bio-Rad Laboratories Inc.) under the following conditions. Namely, 1 × 10⁷ cells and 15 µg of plasmid pHVR1 for expression of human VR1 were added into a cuvette of a 0.4 cm gap, and electroporation was carried out at 0.25 kv of electric voltage and 960 µF of capacitance. Subsequently, the cells were transferred into Ham's F12 medium containing 10% fetal calf serum, and incubated for 24 hours. The cells were then collected, centrifuged, and then suspended in Ham's F12 medium containing 10% fetal calf serum and Geneticin (GIBCO BRL) at a concentration of 500 µg/ml. The suspension of cells was diluted to a concentration of 10⁴ cells/mL, which was inoculated on 96-well plates (Corning Coaster) to obtain Geneticin-resistant strains.

Subsequently, the resulting Geneticin-resistant strains were cultured in the 96-well plates (Corning Coaster). Next, after the medium was removed by suction, 100 µl of an assay buffer (1 mM CaCl₂, HBSS W/O sodium bicarbonate (GIBCO BRL), 0.5% BSA and 20 mM HEPES (Dojindo Molecular Technologies, Inc.), pH 7.5) was added to each well, and the cells were washed twice. Subsequently, 100 µl of the assay buffer containing 2.5 µCi/ml of ⁴⁵Ca (Daiichi Pure Chemicals) and 1 µM of capsaicin (Wako Pure Chemical Industries, Ltd.) was added to each well and the reaction was carried out for 30 minutes. After the assay buffer was removed by suction, the cells were washed twice with 100 µl of ice-cooled PBS (GIBCO BRL), and to each well was added 150 µl of MicroScint-20 (Packard Industry Company, Inc.) with stirring. Subsequently, the radioactivity was determined with Top Count (Packard Industry Company, Inc.) to select HVR1/CHO strains of which calcium concentration is increased when capsaicin is added thereto.

### (7) Evaluation of Compounds Based on Cell Death

Caterina, M.J. et al. (Nature 389 (6653): 816-24 (1997)) have reported that cells expressing vanilloid receptor died in the presence of capsaicin which is VR1 agonist. The compounds were evaluated based on this. RVR1/CHO and HVR1/CHO strains were inoculated on 96-well microplates (Corning Coaster) at a concentration of 4 × 10⁴ cells/well, respectively and were cultured for 20 hours. After the culture was removed by suction, 180 µl of Ham's F12 medium (GIBCO BRL) containing 10% fetal calf serum (GIBCO BRL) was added to each well. Next, 20 µl of the assay buffer (Ham's F12 medium (GIBCO BRL), 0.5% BSA and 20 mM HEPES (Dojindo Molecular Technologies, Inc.), pH 7.5) containing a test compound at a final concentration of 1 µM was added to each well and the reaction was carried out in a carbon dioxide incubator for 3 hours. Subsequently, and Alama Blue (Wako Pure Chemical Industries, Ltd.) at a concentration of 25 µl was added to each well, and again cultured for 20 hours. Next, fluorescence was measured at excitation wavelength of 530nm and fluorescence wavelength of 590nm using multilabel counter (Wallac-Berthold, Japan), and survival rate of cell was determined.

According to the above-mentioned method, the agonist activity of test compounds was determined. As a result, the survival rate of the cells in the presence of 1 µM of the compound which was obtained in Example 2 was 0%.

### Test Example 2

### Mouse eye dropping test

Eight of 4-5 weeks old ICR male mice (Japan SLC) were used in a group. 0.01 mL of the drug was added dropwise to the eye, and the time when the mouse continued to close the eye was measured. The stimulation of the drug was evaluated as positive when the time is more than 10 seconds, and as negative when the time is less than 10 seconds. At 1 hour after the initial treatment, a solution of capsaicin (0.3 µg) was added dropwise to the eye to evaluate whether the stimulation is or not, and examined the desensitizing activity of the compound. The stimulating and desensitizing activity of the drug were calculated as a value of ED₅₀. The drug was dissolved in a mixture of 10% ethanol, 20% Tween and 80-70% physiological salt solution.

ED₅₀ of the compound obtained in Example 2 was 2.2 µg.

### Test Example 3

### Tail-flick test

Four to five weeks old ICR male mice (Japan SLC) were used in this test. The mouse was put into a retainer, the tail end was dipped into 55°C water bath, and the time until the tail end rose to the surface was measured. The time was previously measured before the administration of the drug, and the mouse having the time of less than 2 seconds were selected and used in the test. Cut-off time was set to 10 seconds. The drug was administered subcutaneously, and the time was measured after 1, 3 and 6 hours to evaluate the effects of the drug. The drug was dissolved in a mixture of 10% ethanol, 20% Tween and 80-70% physiological salt solution.

Minimum effective dose of the compound obtained in Example 2 was 1 mg/Kg.

### Test Example 4

### Measurement of mouse bladder volume

Five weeks old ICR male mice were used in this test. At 3 hours after the subcutaneous administration of the drug, the abdomen of mouse was cut open under urethane anesthesia (30 mg/mouse) to expose the bladder. Physiological salt solution was infused into the bladder at the rate of 0.05mL/minutes to check excretion reflection. All of the urine was drained off, and the physiological salt solution was infused again, and then, the bladder volume of mouse was calculated by measuring the time to urination. The results are shown in Table 2.

Resiniferatoxin (RTX) and the compound obtained in Example 22 increased significantly the bladder volume of the mouse.

**Table 2**

| The influence of RTX and the compound obtained in Example 22 on the bladder volume of a mouse anesthetized with urethane | | | |
|---|---|---|---|
| Compound | Dose (mg/kg, sc) | N | Bladder volume (mL) |
| Vehicle | - | 14 | 0.12 ± 0.01 |
| RTX | 0.03 | 8 | 0.24 ± 0.04 |
| | 0.1 | 8 | 0.39 ± 0.04* |
| Example 22 | 3 | 6 | 0.17 ± 0.03 |
| | 10 | 7 | 0.28 ± 0.05* |
| | 100 | 8 | 0.31 ± 0.06** |
| Mean ± S.E. * p ≤ 0.05, ** p ≤ 0.01 vs. vehicle (Dunnett's test). | | | |

### Industrial Applicability

The present invention provides novel benzene derivatives which have vanilloid receptor agonist activity and are useful as a drug such as an analgesic and an agent for preventing and/or treating urinary frequency and/or urinary incontinence.

## Claims

1. A compound represented by the formula (I): 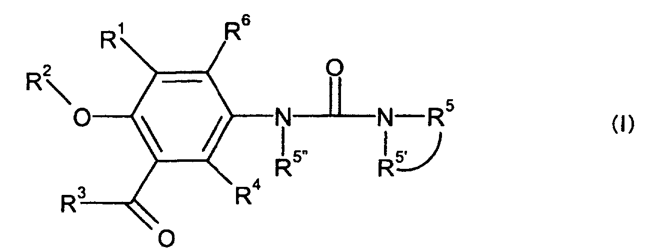 wherein R¹, R⁴ and R⁶ each independently represents a hydrogen atom, a halogen atom or an optionally substituted hydrocarbon group;
R² represents an optionally substituted hydrocarbon group or an optionally substituted heterocyclic group;
R³ represents an optionally substituted hydrocarbon group, NR^{7'}R⁷ or OR⁸ (wherein R^{7'} represents a hydrogen atom or an optionally substituted hydrocarbon group, and R⁷ represents an optionally substituted non-aromatic group, or R^{7'} and R⁷ may form an optionally substituted ring together with the adjacent nitrogen atom, and R⁸ represents an optionally substituted hydrocarbon group or an optionally substituted heterocyclic group);
R⁵ represents an optionally substituted hydrocarbon group (except for an optionally substituted benzoyl group) or an optionally substituted heterocyclic group (except for a quinolyl group); R⁵' represents a hydrogen atom, or an optionally substituted hydrocarbon group, or R⁵ and R⁵' may form an optionally substituted ring together with the adjacent nitrogen atom; and R^{5"} represents a hydrogen atom or an optionally substituted hydrocarbon group; or
a salt thereof.

2. The compound according to claim 1, wherein the optionally substituted hydrocarbon group represented by R¹, R², R³, R⁴, R⁵, R⁵', R⁵", R⁶, R⁷' and R⁸ each independently represents a C₁₋₆alkyl group, a C₂₋₆alkenyl group, a C₂₋₆alkynyl group, a C₃₋₁₀cycloalkyl group, a C₃₋₁₀cycloalkenyl group, a C₄₋₁₂cycloalkylalkyl group, a C₄₋₁₂cycloalkenylalkyl group, a C₆₋₁₄aryl group or a C₇₋₁₉aralkyl group, which may be independently substituted;
the optionally substituted non-aromatic group represented by R⁷ represents a C₁₋₆alkyl group, a C₂₋₆alkenyl group, a C₂₋₆alkynyl group, a C₃₋₁₀cycloalkyl group, a C₃₋₁₀cycloalkenyl group, a C₄₋₁₂cycloalkylalkyl group, a C₄₋₁₂cycloalkenylalkyl group, a C₇₋₁₉aralkyl group, or a 5- to 12-membered non-aromatic heterocyclic group containing 1 to 4 hetero atoms consisting of 1 to 3 kinds of hetero atoms selected from an oxygen atom, a sulfur atom and a nitrogen atom as ring-constituting atoms, which may be independently substituted;
the optionally substituted heterocyclic group represented by R², R⁵ and R⁸ each independently represents a 5- to 12-membered aromatic heterocyclic group, or a saturated or unsaturated non-aromatic heterocyclic group containing 1 to 4 hetero atoms consisting of 1 to 3 kinds of hetero atoms selected from an oxygen atom, a sulfur atom and a nitrogen atom as ring-constituting atoms, which may be substituted; and
the ring formed by R^{7'} and R⁷, and R⁵ and R^{5'} together with the adjacent nitrogen atom, represents an optionally substituted 3- to 12-membered nitrogen-containing heterocyclic ring which may contain 1 to 3 hetero atoms selected from an oxygen atom, a sulfur atom and a nitrogen atom in addition to carbon atoms and one nitrogen atom.

3. The compound according to claim 1 or 2, wherein the substituents are 1 to 4 groups selected from a halogen atom; a nitro group; a cyano group; a hydroxy group; a mercapto group; a sulfo group; a sulfino group; a phosphono group; an optionally halogenated C₁₋₆alkyl group; an oxo group; an amidino group; an imino group; a C₁₋₄alkylenedioxy group; an optionally halogenated C₁₋₆alkoxy group; an optionally halogenated C₁₋₆alkylthio group; a carboxyl group; a formyl group; an optionally halogenated C₁₋₆alkyl-carbonyl group; a formyloxy group; an optionally halogenated C₁₋₆alkyl-carbonyloxy group; an optionally halogenated C₁₋₆alkoxy-carbonyl; a C₇₋₁₁aralkyl group; a C₇₋₁₁aralkyloxy group; a C₇₋₁₁aralkyloxy-carbonyl group; a thiocarbamoyl group; an optionally halogenated C₁₋₆alkylsulfinyl group; an optionally halogenated C₁₋₆alkylsulfonyl group; a sulfamoyl group; an optionally halogenated mono-C₁₋₆alkylsulfamoyl group; an optionally halogenated di-C₁₋₆alkylsulfamoyl group; a C₆₋₁₀arylsulfamoyl group; a C₆₋₁₀aryl group; a C₆₋₁₀aryloxy group; a C₆₋₁₀arylthio group; a C₆₋₁₀arylsulfinyl group; a C₆₋₁₀arylsulfonyl group; a C₆₋₁₀aryl-carbonyl group; a C₆₋₁₀aryl-carbonyloxy group; a group represented by the formula -CONR⁹R¹⁰ (wherein R⁹ and R¹⁰ each represents (1) a hydrogen atom, (2) a C₁₋₆alkyl group which may have 1 to 4 substituents selected from a halogen atom and a hydroxy group, (3) a C₆₋₁₀aryl group which may have 1 to 4 substituents selected from a halogen atom, a hydroxy group, and an optionally halogenated C₁₋₆alkyl group or (4) a 5- to 12-membered heterocyclic group which may have 1 to 4 substituents selected from a halogen atom and an optionally halogenated C₁₋₆alkyl group, containing 1 to 4 hetero atoms consisting of 1 to 3 kinds of hetero atoms selected from an oxygen atom, a sulfur atom and a nitrogen atom as ring-constituting atoms, or R⁹ and R¹⁰ may form with the adjacent nitrogen atom a 3- to 8-membered nitrogen-containing heterocyclic ring which may contain 1 to 3 hetero atoms selected from an oxygen atom, a sulfur atom and a nitrogen atom in addition to carbon atom and one nitrogen atom); a group represented by the formula -NR⁹R¹⁰ (wherein R⁹ and R¹⁰ are as defined above); a group represented by the formula -NHCONR⁹R¹⁰ (wherein R⁹ and R¹⁰ are as defined above); a group represented by the formula -NR⁹COR¹⁰ (wherein R⁹ and R¹⁰ are as defined above); a group represented by the formula -NR⁹SO₂R¹⁰ (wherein R⁹ and R¹⁰ are as defined above); and a 5- to 12-membered heterocyclic group containing 1 to 4 hetero atoms consisting of 1 to 3 kinds of hetero atoms selected from an oxygen atom, a sulfur atom and a nitrogen atom as ring-constituting atoms.

4. The compound according to claim 1, wherein R¹ represents a hydrogen atom; R⁴ represents a hydrogen atom or an optionally halogenated C₁₋₄alkyl group; and R⁶ represents a hydrogen atom or an optionally halogenated C₁₋₄alkyl group.

5. The compound according to claim 1, wherein R² represents a C₇₋₁₉aralkyl group which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxy-carbonyl group, an optionally halogenated C₁₋₆alkylthio-carbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C₆₋₁₀arylsulfamoyl group, and a di-C₆₋₁₀arylsulfamoyl group.

6. The compound according to claim 1, wherein R² represents a C₇₋₁₉aralkyl group which may have 1 to 4 substituents selected from a halogen atom, an optionally halogenated C₁₋₄alkyl group, a nitro group, a cyano group, and a C₁₋₄alkoxy-carbonyl group.

7. The compound according to claim 6, wherein the C₇₋₁₉aralkyl group is a benzhydryl group.

8. The compound according to claim 1, wherein R³ represents a C₁₋₄alkyl group, a C₁₋₄alkylamino group or C₁₋₄alkoxy group.

9. The compound according to claim 1, wherein R⁵ represents a C₆₋₁₀aryl group, a pyridyl group or a C₇₋₁₁aralkyl group, each of which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxycarbonyl group, an optionally halogenated C₁₋₆alkylthiocarbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C₆₋₁₀arylsulfamoyl group, and a di-C₆₋₁₀arylsulfamoyl group.

10. The compound according to claim 1, wherein R⁵ represents a phenyl group which may have 1 or 2 C₁₋₆alkoxy groups.

11. The compound according to claim 1, wherein R¹ represents a hydrogen atom; R² represents a C₇₋₁₉aralkyl group which may have 1 to 4 substituents selected from a halogen atom, an optionally halogenated C₁₋₄alkyl group, a nitro group, a cyano group, and a C₁₋₄alkoxy-carbonyl group; R³ represents a C₁₋₄alkyl group, a C₁₋₄alkylamino group or C₁₋₄alkoxy group; R⁴ represents a hydrogen atom or an optionally halogenated C₁₋₄alkyl group; R⁵ represents a C₆₋₁₀aryl group, a pyridyl group or a C₇₋₁₁ aralkyl group, each of which may have 1 to 4 substituents selected from an optionally halogenated C₁₋₆alkyl group, a halogen atom, a nitro group, a cyano group, a carboxyl group, an amino group, an optionally halogenated C₁₋₆alkyl-carbonyl group, a C₆₋₁₀aryl-carbonyl group, an optionally halogenated C₁₋₆alkylthio group, an optionally halogenated C₁₋₆alkoxy group, a C₆₋₁₀aryl group, a C₆₋₁₀aryloxy group, a C₆₋₁₀arylthio group, an optionally halogenated C₁₋₆alkoxy-carbonyl group, an optionally halogenated C₁₋₆alkylthio-carbonyl group, an optionally halogenated C₁₋₆alkylsulfinyl group, an optionally halogenated C₁₋₆alkylsulfonyl group, an optionally halogenated mono-C₁₋₆alkylsulfamoyl group, an optionally halogenated di-C₁₋₆alkylsulfamoyl group, a C₆₋₁₀arylsulfinyl group, a C₆₋₁₀arylsulfonyl group, a mono-C_{6-10aryl}sulfamoyl group, and a di C₆₋₁₀arylsulfamoyl group; and R⁶ represents a hydrogen atom or an optionally halogenated C₁₋₄alkyl group.

12. The compound according to claim 1, wherein R⁵', R⁵", and R⁷' each represents a hydrogen atom.

13. The compound according to claim 1, which is N-(4-benzhydryloxy-3-isobutyrylphenyl)-N'-(3,4-dimethoxyphenyl)urea,
methyl 5-({[(3,4-dimethoxyphenyl)amino]carbonyl}amino)-2-[(4-fluorophenyl)(phenyl)methoxy]benzoate,
methyl 5-({[(3,4-dimethoxyphenyl)amino]carbonyl}amino)-2-[(4-trifluoromethylphenyl)(phenyl)methoxy]benzoate,
methyl 5-({[(3,4-dimethoxyphenyl)amino]carbonyl}amino)-2-[(2-chlorophenyl)(4'-chlorophenyl)methoxy]benzoate,
N-(tert-butyl)-5-({[(3,4-dimethoxyphenyl)amino]carbonyl}amino)-2-{phenyl[4-(trifluoromethyl)phenyl]methoxy}benzamide,
methyl 2-[(3,4-difluorophenyl)(phenyl)methoxy]-5-({[(3,4-dimethoxyphenyl)amino]carbonyl}amino)benzoate,
methyl 2-{(2-chlorophenyl)[4-(trifluoromethyl)phenyl]methoxy}-5-({[(3,4-dimethoxyphenyl)amino]carbonyl}amino)benzoate,
N-(tert-butyl)-2-{(2-chlorophenyl)[4-(trifluoromethyl)phenyl]methoxy}-5-({[(3,4-dimethoxyphenyl)amino]carbonyl}amino)benzamide,
N-(tert-butyl)-2-[(4-chlorophenyl)(2-fluorophenyl)methoxy]-5-({[(3,4-dimethoxyphenyl)amino]carbonyl}amino)benzamide,
N-(tert-butyl)-2-[(4-chlorophenyl)(3-fluorophenyl)methoxy]-5-({[(3,4-dimethoxyphenyl)amino]carbonyl}amino)benzamide, or
N-(tert-butyl)-2-[(4-chlorophenyl)(4-fluorophenyl)methoxy]-5-({[(3,4-dimethoxyphenyl)amino]carbonyl}amino)benzamide.

14. A prodrug of the compound according to claim 1 or a salt thereof.

15. A process for preparing the compound according to claim 1 or a salt thereof, which comprises subjecting a compound represented by the formula: 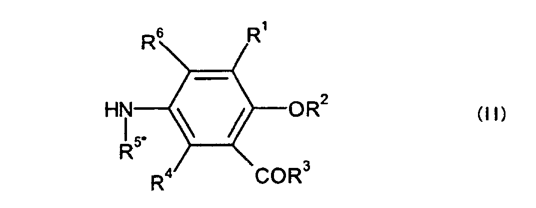 wherein each symbol is as defined in claim 1, or a salt thereof and a compound represented by the formula: 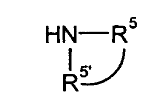 wherein each symbol is as defined in claim 1, or a salt thereof to urea synthesis reaction.

16. A pharmaceutical composition comprising the compound according to claim 1, a pharmaceutically acceptable salt thereof or a prodrug thereof.

17. A vanilloid receptor 'agonist comprising a compound represented by the formula: 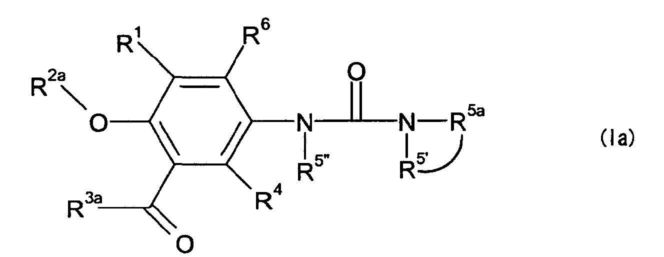 wherein R¹, R⁴ and R⁶ each independently represents a hydrogen atom, a halogen atom or an optionally substituted hydrocarbon group;
R^{2a} represents an optionally substituted hydrocarbon group or an optionally substituted heterocyclic group;
R^{3a} represents an optionally substituted hydrocarbon group, NR^{7'}R^{7a} or OR⁸ (wherein R^{7'} represents a hydrogen atom or an optionally substituted hydrocarbon group, R^{7a} and R⁸ each independently represents an optionally substituted hydrocarbon group or an optionally substituted heterocyclic group, or R^{7'} and R^{7a} may form an optionally substituted ring with the adjacent nitrogen atom);
R^{5a} represents an optionally substituted hydrocarbon group or an optionally substituted heterocyclic group;
R^{5'} represents a hydrogen atom, or an optionally substituted hydrocarbon group, or R^{5a} and R^{5'} may form an optionally substituted ring with the adjacent nitrogen atom;
R⁵" represents a hydrogen atom or an optionally substituted hydrocarbon group,
a pharmaceutically acceptable salt thereof or a prodrug thereof.

18. An agent for preventing and/or treating urinary frequency and/or urinary incontinence, which comprises the compound of the formula (Ia) according to claim 17, a pharmaceutically acceptable salt thereof or a prodrug thereof.

19. An analgesic which comprises the compound of the formula (Ia) according to claim 17, a pharmaceutically acceptable salt thereof or a prodrug thereof.

20. Use of the compound of the formula (Ia) according to claim 17, a pharmaceutically acceptable salt thereof or a prodrug thereof for manufacturing an agent for preventing and/or treating urinary frequency and/or urinary incontinence.

21. Use of the compound of the formula (Ia) according to claim 17, a pharmaceutically acceptable salt thereof or a prodrug thereof for manufacturing an analgesic.

22. Use of the compound of the formula (Ia) according to claim 17, a pharmaceutically acceptable salt thereof or a prodrug thereof for manufacturing a vanilloid receptor agonist.

23. A method for preventing and/or treating urinary frequency and/or urinary incontinence, which comprises administrating an effective amount of the compound of the formula (Ia) according to claim 17, a pharmaceutically acceptable salt thereof or a prodrug thereof to a mammal.

24. An analgesic method which comprises administrating an effective amount of the compound of the formula (Ia) according to claim 17, a pharmaceutically acceptable salt thereof or a prodrug thereof to a mammal.
